# EUROPEAN PATENT APPLICATION

(11) **EP 4 763 228 A1**
(43) Date of publication of application: **24.06.2026**
(21) Application number: 24855736.5
(22) Date of filing: 16.08.2024
(51) Int. Cl.: A61K 47/68, C07K 16/28, A61K 31/4745, A61K 31/513, A61P 35/00

(54) **PENTACYCLIC ACTIVE COMPOUND AND CONJUGATE THEREOF AND USE THEREOF**

(30) Priority: 18.08.2023 CN 202311046439
(71) Applicant: Keymed Biosciences (Chengdu) Co., Ltd., Chengdu, Sichuan 610219 (CN)
(72) Inventor: CHEN, Bo, Chengdu, Sichuan 610219 (CN); XU, Jianyan, Chengdu, Sichuan 610219 (CN); MIAO, Yu, Chengdu, Sichuan 610219 (CN); CHEN, Zhongyuan, Chengdu, Sichuan 610219 (CN)
(74) Representative: Gille Hrabal Partnerschaftsgesellschaft mbB Patentanwälte
(86) International application number: PCT/CN2024/112682
(87) International publication number: WO 2025/040012

(57) **Abstract**

The present invention relates to a pentacyclic active compound and a conjugate thereof and a use thereof. The compound and the conjugate have excellent tumor cell inhibition activity, stability and in vivo efficacy in animals.

## Description

This application claims the priority of the prior application filed with the State Intellectual Property Office of China on August 18, 2023 under the Patent Application Number 202311046439.3 with the title " Pentacyclic active compound and conjugate thereof and use thereof", the full text of which is incorporated in the present disclosure by reference.

### Technical field

The disclosure belongs to the technical field of medicine and relates to a pentacyclic active compound, conjugate and use thereof.

### Background

The concept of Antibody-Drug Conjugate (ADC) has been known for a long time. Early in the 20th century, Nobel Prize winner Professor Paul Ehrlich first proposed the concept of "magic bullet", that is, a complex that couples a cytotoxic small molecule drug to an antibody through a reasonably constructed linker, can selectively deliver potent cytotoxic drugs into tumors. Currently, ADC drugs that have been marketed are based on this theory. With the expansion of targets and indications, ADC is leading a new era of targeted therapy for cancer, and is expected to replace traditional chemotherapy drugs in the future.

In the ADC drug, the antibody is connected to the small molecule cytotoxic drug by a specific linker, and the main components include the antibody (mAb), the linker (linker) and the small molecule cytotoxic drug (payload). After the ADC drug enters blood, the antibody component can recognize the target and bind to tumor cells highly expressing cell surface antigens. After ADC (an antigen complex) enters tumor cells through endocytosis, the cytotoxic payload (drug) will be released under the degradation effect of lysosome, damaging DNA or preventing cell division of the tumor, thus killing tumor cells.

Use of an anti-murine leukocyte immunoglobulin conjugated with methotrexate in the treatment of leukemia had been attempted in 1958. In 2000, the U.S. Food and Drug Administration (FDA) first approved an ADC drug Mylotarg for treating adult acute myeloid leukemia, marking the beginning of the era of ADC-targeted therapy for cancer. Mylotarg is the first-generation ADC drug, but it was withdrawn from the market because of its severe and fatal liver injury without a significant survival benefit. The disadvantages of the first-generation ADC drugs are their antibodies were murine, their cytotoxicity was not potent enough, and their targets were low-expressed.

Trastuzumab Emtansine, the first approved breast cancer-targeted ADC drug, was the starting point for the development of the second-generation ADC drugs. Brentuximab Vedotin is the most successful ADC drug in hematologic oncology, targeting classical Hodgkin's lymphoma and anaplastic large cell lymphoma. The advantages of the second-generation ADC drugs are diversity in the development of target antigen and humanized antibodies, while the disadvantages are too low or too high drug loading ratio, narrow therapeutic window and low efficacy.

Enfortumab Vedotin is the typical representative of the third-generation ADC, is the second ADC drug targeting solid tumors. It is indicated for patients who have failed to respond to PD-1/PD-L1 antibody therapy but are insensitive to tubulin inhibitors.

At present, it is still necessary to develop drug molecules and conjugates thereof with reasonable molecular design, improved pharmacodynamic activity and controllable safety.

### SUMMARY

To overcome the above technical problem, the present disclosure provides a ligand-drug conjugate represented by the following formula (C), or a stereoisomer, racemate, tautomer, isotopologue, isotope-labeled compound, N-oxide, prodrug, solvate, or pharmaceutically acceptable salt thereof:

Tp-L-G (C)

wherein, Tp is the targeting moiety;
L is selected from a chemical bond or a linker;
G is a group represented by the following formula (G): 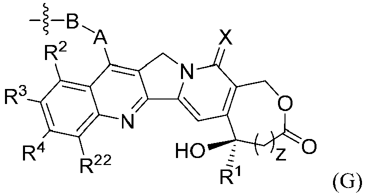
wherein, A is absent or selected from the groups consisting of alkylene, alkenylene, alkynylene, cycloalkyl, aryl, heteroaryl, heterocyclyl, and a combination of two or more of them, which are unsubstituted or optionally substituted with one, two or more R^{A}; wherein the alkylene, alkenylene, alkynylene, cycloalkyl, aryl, heteroaryl, heterocyclyl or a combination of two or more of them may be uninterrupted or optionally interrupted by one, two or more groups selected from-O-, -S-, -NR⁵-, -NR⁶C(=O)-, -C(=O)NR⁶-, -C(=O)-, -NR⁷C(=O)NR⁸-or -OC(=O)-;
R¹ is selected from the following groups which are unsubstituted or optionally substituted with one, two or more R^{B}: cycloalkyl, cycloalkylalkyl, cycloalkyloxy, heterocyclyl, heterocyclylalkyl, heterocyclyloxy;
X is selected from O or S;
Z is selected from 0 or 1;
R² is selected from the group consisting of hydrogen, deuterium, halogen, hydroxy, mercapto, amino, cyano, and the following groups which are unsubstituted or optionally substituted with one, two or more R^{C}: alkyl, alkyloxy, cycloalkyl, cycloalkylalkyl, or cycloalkyloxy;
R²² is selected from the group consisting of hydrogen, deuterium, halogen, hydroxy, mercapto, amino, cyano, and the following groups which are unsubstituted or optionally substituted with one, two or more R^{C}: alkyl, alkyloxy, cycloalkyl, cycloalkylalkyl, or cycloalkyloxy;
R³ is selected from the groups consisting of hydrogen, deuterium, halogen, hydroxy, mercapto, amino, cyano, and the following groups which are unsubstituted or optionally substituted with one, two or more R^{C}: alkyl, alkyloxy, cycloalkyl, cycloalkylalkyl, or cycloalkyloxy;
R⁴ is selected from the group consisting of hydrogen, deuterium, halogen, hydroxy, mercapto, amino, cyano, and the following groups which are unsubstituted or optionally substituted with one, two or more R^{C}: alkyl, alkyloxy, cycloalkyl, cycloalkylalkyl, or cycloalkyloxy;
alternatively, R² and R³, together with the atoms to which they are attached form a ring structure which is unsubstituted or optionally substituted with one, two or more R^{D};
alternatively, R³ and R⁴, together with the atoms to which they are attached form a ring structure which is unsubstituted or optionally substituted with one, two or more R^{D};
alternatively, R²² and R⁴, together with the atoms to which they are attached form a ring structure which is unsubstituted or optionally substituted with one, two or more R^{D};
B is absent or selected from-O-, -S-, -S(=O)-, -C(=O)-, -NR⁵-, -C=N-NR⁹-, -C=N-O- or -NR¹⁰-NR¹¹-;
R⁵, R⁶, R⁷, R⁸, R⁹, R¹⁰, and R¹¹ are identical or different and are independently selected from the group consisting of hydrogen, deuterium, and the following groups which are unsubstituted or optionally substituted with one, two or more R^{E}: alkyl, cycloalkyl, cycloalkylalkyl, aryl, arylalkyl, heteroaryl, heteroarylalkyl, heterocyclyl, heterocyclylalkyl, HC(=O)-, alkyl-C(=O)-, cycloalkyl-C(=O)NH-, heterocyclyl-C(=O)NH-, aryl-C(=O)NH-, or heteroaryl-C(=O)NH-;
each of R^{A}, R^{B}, R^{C}, R^{D}, and R^{E} is identical or different and is independently selected from the group consisting of deuterium, halogen, hydroxy, cyano, nitro, oxo (=O), and the following groups which are unsubstituted or optionally substituted with one, two or more R^{F}: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, NH₂, HC(=O)NH-, alkyl-C(=O)NH-, cycloalkyl-C(=O)NH-, heterocyclyl-C(=O)NH-, aryl-C(=O)NH-, or heteroaryl-C(=O)NH-;
each R^{F} is identical or different and is independently selected from the group consisting of deuterium, halogen, hydroxy, cyano, nitro, oxo (=O), alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, NH₂, HC(=O)NH-, alkyl-C(=O)NH-, cycloalkyl-C(=O)NH-, heterocyclyl-C(=O)NH-, aryl-C(=O)NH-, or heteroaryl-C(=O)NH-;
the wavy line represents the site linked to L.

According to an embodiment of the present disclosure, the groups in formula (G) may be independently selected from the following definitions:
wherein, A is absent or selected from the following groups which are unsubstituted or optionally substituted with one, two or more R^{A}: C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl, or a combination of two or more of them, wherei the C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl, or a combination of two or more of them may be uninterrupted or optionally interrupted by one, two or more groups selected from-O-, -S-, -NR⁵-, -NR⁶C(=O)-, -C(=O)NR⁶-, -C(=O)-, -NR⁷C(=O)NR⁸-, or -OC(=O)-; preferably, A is absent or selected from the following groups which are unsubstituted or optionally substituted with one, two or more R^{A}: C₁₋₆ alkylene, C₂₋₆ alkenylene, C₂₋₆ alkynylene, C₃₋₆ cycloalkyl, C₆₋₁₀ aryl, 5-10 membered heteroaryl, 5-10 membered heterocyclyl, or a combination of two or more of them, wherein the C₁₋₆ alkylene, C₂₋₆ alkenylene, C₂₋₆ alkynylene, C₃₋₆ cycloalkyl, C₆₋₁₀ aryl, 5-10-membered heteroaryl, 5-10-membered heterocyclyl, or a combination of two or more of them may be uninterrupted or optionally interrupted by one, two or more groups selected from -O-, -S-, -NR⁵-, -NR⁶C(=O)-, -C(=O)NR⁶-, -C(=O)-, -NR⁷C(=O)NR⁸-, or -OC(=O)-;
R¹ is selected from the following groups which are unsubstituted or optionally substituted with one, two or more R^{B}: C₃₋₁₀ cycloalkyl, C₃₋₁₀ cycloalkyl-C₁₋₁₀ alkyl, C₃₋₁₀ cycloalkyloxy, 3-10 membered heterocyclyl, 3-10 membered heterocyclyl-C₁₋₁₀ alkyl, 3-10 membered heterocyclyloxy; preferably, R¹ is selected from the following groups which are unsubstituted or optionally substituted with one, two or more R^{B}: C₃₋₆ cycloalkyl, C₃₋₆ cycloalkyl-C₁₋₆ alkyl, C₃₋₆ cycloalkyloxy, 3-6 membered heterocyclyl, 3-6 membered heterocyclyl-C₁₋₆ alkyl, or 3-6 membered heterocyclyloxy;
X is selected from O or S;
Z is selected from 0 or 1;
R² is selected from the group consisting of hydrogen, deuterium, halogen, hydroxy, mercapto, amino, cyano, and the following groups which are unsubstituted or optionally substituted with one, two or more R^{C}: C₃₋₁₀ alkyl, _{C3-10} alkyloxy, C₃₋₁₀ cycloalkyl, C₃₋₁₀ cycloalkyl-C₁₋₁₀ alkyl, or C₃₋₁₀ cycloalkyloxy; preferably, R² is selected from the group consisting of hydrogen, deuterium, halogen, hydroxy, mercapto, amino, cyano, or the following groups which are unsubstituted or optionally substituted with one, two or more R^{C}: C₁₋₆ alkyl, C₁₋₆ alkyloxy, C₃₋₆ cycloalkyl, C₃₋₆ cycloalkyl-C₁₋₆ alkyl, or C₃₋₆ cycloalkyloxy;
R²² is selected from the group consisting of hydrogen, deuterium, halogen, hydroxy, mercapto, amino, cyano, and the following groups which are unsubstituted or optionally substituted with one, two or more R^{C}: C₃₋₁₀ alkyl, C₃₋₁₀ alkyloxy, C₃₋₁₀ cycloalkyl, C₃₋₁₀ cycloalkyl-C₁₋₁₀ alkyl, or C₃₋₁₀ cycloalkyloxy; preferably, R²² is selected from the group consisting of hydrogen, deuterium, halogen, hydroxy, mercapto, amino, cyano, and the following groups which are unsubstituted or optionally substituted with one, two or more R^{C}: C₁₋₆ alkyl, C₁₋₆ alkyloxy, C₃₋₆ cycloalkyl, C₃₋₆ cycloalkyl-C₁₋₆ alkyl, or C₃₋₆ cycloalkyloxy;
R³ is selected from the group consisting of hydrogen, deuterium, halogen, hydroxy, mercapto, amino, cyano, and the following groups which are unsubstituted or optionally substituted with one, two or more R^{C}: C₁₋₁₀ alkyl, C₁₋₁₀ alkyloxy, C₃₋₁₀ cycloalkyl, C₃₋₁₀ cycloalkyl-C₁₋₁₀ alkyl, or C₃₋₁₀ cycloalkyloxy; preferably, R³ is selected from the group consisting of hydrogen, deuterium, halogen, hydroxy, mercapto, amino, cyano, and the following groups which are unsubstituted or optionally substituted with one, two or more R^{C}: C₁₋₆ alkyl, C₁₋₆ alkyloxy, C₃₋₆ cycloalkyl, C₃₋₆ cycloalkyl-C₁₋₆ alkyl, or C₃₋₆ cycloalkyloxy;
R⁴ is selected from the group consisting of hydrogen, deuterium, halogen, hydroxy, mercapto, amino, cyano, and the following groups which are unsubstituted or optionally substituted with one, two or more R^{C}: C₁₋₁₀ alkyl, C₁₋₁₀ alkyloxy, C₃₋₁₀ cycloalkyl, C₃₋₁₀ cycloalkyl-C₁₋₁₀ alkyl, or C₃₋₁₀ cycloalkyloxy; preferably, R⁴ is selected from the group consisting of hydrogen, deuterium, halogen, hydroxy, mercapto, amino, cyano, and the following groups which are unsubstituted or optionally substituted with one, two or more R^{C}: C₁₋₆ alkyl, C₁₋₆ alkyloxy, C₃₋₆ cycloalkyl, C₃₋₆ cycloalkyl-C₁₋₆ alkyl, or C₃₋₆ cycloalkyloxy;
alternatively, R² and R³, together with the atoms to which they are attached form a 4-10 membered ring structure which is unsubstituted or optionally substituted with one, two or more R^{D}; wherein the 4-10 membered ring structure may be selected from, for example, 4-, 5-, 6-, 7-, 8-, 9-, or 10-membered hetero-monocyclic hydrocarbon, hetero-bicyclic hydrocarbon, monocyclic hydrocarbon, or bicyclic hydrocarbon; wherein the heteromonocyclic hydrocarbon and heterobicyclic hydrocarbon contain one, two or more O, S, N or carbonyl or any combination thereof;
alternatively, R³ and R⁴, together with the atoms to which they are attached form a 4-10 membered ring structure which is unsubstituted or optionally substituted with one, two or more R^{D}; wherein the 4-10 membered ring structure may be selected from, for example, 4-, 5-, 6-, 7-, 8-, 9-, or 10-membered monocyclic hydrocarbon, bicyclic hydrocarbon, hetero-monocyclic hydrocarbon, or hetero-bicyclic hydrocarbon; wherein the hetero-monocyclic hydrocarbon and hetero-bicyclic hydrocarbon contain one, two or more O, S, N or carbonyl or any combination thereof;
alternatively, R²² and R⁴, together with the atoms to which they are attached form a 4-10 membered ring structure which is unsubstituted or optionally substituted with one, two or more R^{D}; wherein the 4-10 membered ring structure may be selected from, for example, 4-, 5-, 6-, 7-, 8-, 9-, or 10-membered monocyclic hydrocarbon, bicyclic hydrocarbon, hetero-monocyclic hydrocarbon, or hetero-bicyclic hydrocarbon; wherein the hetero-monocyclic hydrocarbon and hetero-bicyclic hydrocarbon contain one, two or more O, S, N or carbonyl or any combination thereof;
B is absent or selected from-O-, -S-, -S(=O)-, -C(=O)-, -NR⁵-, -C=N-NR⁹-, -C=N-O- or -NR¹⁰-NR¹¹-;
R³, R⁶, R⁷, R⁸, R⁹, R¹⁰, and R¹¹ are identical or different and are independently selected from the following group consisting of hydrogen, deuterium, or the following groups which are unsubstituted or optionally substituted with one, two or more R^{E}: C₁₋₁₀ alkyl, C₃₋₁₀ cycloalkyl, C₃₋₁₀ cycloalkyl-C₁₋₁₀ alkyl, C₆₋₁₂ aryl, C₆₋₁₂ aryl-C₁₋₁₀ alkyl, 5-12 membered heteroaryl, 5-12 membered heteroaryl-C₁₋₁₀ alkyl, 5-12 membered heterocyclyl, 5-12 membered heterocyclyl-C₁₋₁₀ alkyl, HC(=O)-, C₁₋₁₀ alkyl-C(=O)-, C₃₋₁₀ cycloalkyl-C(=O)NH-, 5-12 membered heterocyclyl-C(=O)NH-, or C₆₋₁₂ aryl-C(=O)NH-, 5-12 membered heteroaryl-C(=O)NH-; preferably, R⁵, R⁶, R⁷, R⁸, R⁹, R¹⁰, and R¹¹ are identical or different and are independently selected from the following group consisting of hydrogen, deuterium, and the following groups which are unsubstituted or optionally substituted with one, two or more R^{E}: C₁₋₆ alkyl, C₃₋₆ cycloalkyl, C₃₋₆ cycloalkyl-C₁₋₆ alkyl, C₆₋₁₀ aryl, C₆₋₁₀ aryl-C₁₋₆ alkyl, 5-6 membered heteroaryl, 5-6 membered heteroaryl-C₁₋₆ alkyl, 5-6 membered heterocyclyl, 5-6 membered heterocyclyl-C₁₋₆ alkyl, HC(=O)-, C₁₋₆ alkyl-C(=O)-, C₃₋₆ cycloalkyl-C(=O)NH-, 5-6 membered heterocyclyl-C(=O)NH-, C₆₋₁₀ aryl-C(=O)NH-, 5-6 membered heteroaryl-C(=O)NH-;
each of R^{A}, R^{B}, R^{C}, R^{D}, and R^{E} is identical or different and is independently selected from the group consisting of deuterium, halogen, hydroxy, cyano, nitro, and the following groups which are unsubstituted or optionally substituted with one, two or more R^{F}: C₁₋₁₀ alkyl, C₁₋₁₀ alkyloxy, C₃₋₁₀ cycloalkyl, C₃₋₁₀ cycloalkyl-C₁₋₁₀ alkyl, C₃₋₁₀ cycloalkyloxy, C₆₋₁₂ aryl, C₆₋₁₂ aryl-C₁₋₁₀ alkyl, C₆₋₁₂ aryloxy, 5-12-membered heteroaryl, 5-12-membered heteroaryl-C₁₋₁₀ alkyl, 5-12-membered heteroaryloxy, 5-12-membered heterocyclyl, 5-12-membered heterocyclyl-C₁₋₁₀ alkyl, 5-12-membered heterocyclyloxy, NH₂, HC(=O)NH-, C₁₋₁₀ alkyl-C(=O)NH-, C₃₋₁₀ cycloalkyl-C(=O)NH-, 5-12 membered heterocyclyl-C(=O)NH-, C₆₋₁₂ aryl-C(=O)NH-, 5-12 membered heteroaryl-C(=O)NH-; preferably, each of R^{A}, R^{B}, R^{C}, R^{D}, and R^{E} is identical or different and is independently selected from the group consisting of deuterium, halogen, hydroxy, cyano, nitro, and the following groups which are unsubstituted or optionally substituted with one, two or more R^{F}: C₁₋₆ alkyl, C₁₋₆ alkyloxy, C₃₋₆ cycloalkyl, C₃₋₆ cycloalkyl-C₁₋₆ alkyl, C₃₋₆ cycloalkyloxy, C₆₋₁₀ aryl, C₆₋₁₀ aryl-C₁₋₆ alkyl, C₆₋₁₀ aryloxy, 5-6-membered heteroaryl, 5-6-membered heteroaryl-C₁₋₆ alkyl, 5-6-membered heteroaryloxy, 5-6-membered heterocyclyl, 5-6-membered heterocyclyl-C₁₋₆ alkyl, 5-6-membered heterocyclyloxy, NH₂, HC(=O)NH-, C₁₋₆ alkyl-C(=O)NH-, C₃₋₆ cycloalkyl-C(=O)NH-, 5-6 membered heterocyclyl-C(=O)NH-, C₆₋₁₀ aryl-C(=O)NH-, or 5-6 membered heteroaryl-C(=O)NH-;
each R^{F} is identical or different and is independently selected from the group consisting of deuterium, halogen, hydroxy, cyano, nitro, alkyl, C₁₋₁₀ alkyloxy, C₃₋₁₀ cycloalkyl, C₃₋₁₀ cycloalkyl-C₁₋₁₀ alkyl, C₃₋₁₀ cycloalkyloxy, C₆₋₁₂ aryl, C₆₋₁₂ aryl-C₁₋₁₀ alkyl, C₆₋₁₂ aryloxy, 5-12 membered heteroaryl, 5-12 membered heteroaryl-C₁₋₁₀ alkyl, 5-12 membered heteroaryloxy, 5-12 membered heterocyclyl, 5-12 membered heterocyclyl-C₁₋₁₀ alkyl, 5-12 membered heterocyclyloxy, NH₂, HC(=O)NH-, C₁₋₁₀ alkyl-C(=O)NH-, C₃₋₁₀ cycloalkyl-C(=O)NH-, 5-12-membered heterocyclyl-C(=O)NH-, C₆₋₁₂ aryl-C(=O)NH-, and 5-12-membered heteroaryl-C(=O)NH-; preferably, each R^{F} is identical or different and is independently selected from the group consisting of deuterium, halogen, hydroxy, cyano, nitro, alkyl, C₁₋₆ alkyloxy, C₃₋₆ cycloalkyl, C₃₋₆ cycloalkyl-C₁₋₆ alkyl, C₃₋₆ cycloalkyloxy, C₆₋₁₀ aryl, C₆₋₁₀ aryl-C₁₋₆ alkyl, C₆₋₁₀ aryloxy, 5-6 membered heteroaryl, 5-6 membered heteroaryl-C₁₋₆ alkyl, 5-6 membered heteroaryloxy, 5-6 membered heterocyclyl, 5-6 membered heterocyclyl-C₁₋₆ alkyl, 5-6 membered heterocyclyloxy, NH2, HC(=O)NH-, C₁₋₆ alkyl-C(=O)NH-, C₃₋₆ cycloalkyl-C(=O)NH-, 5-6-membered heterocyclyl-C(=O)NH-, C₆₋₁₀ aryl-C(=O)NH-, and 5-6-membered heteroaryl-C(=O)NH-.

According to an embodiment of the present disclosure, the groups in formula (G) may be independently selected from the following definitions:
A is absent or selected from one of the following substructures, wherein the substructure may be unsubstituted or optionally substituted with one, two or more R^{A}:
-(CH₂)ₙ₁-, -O(CH₂)ₙ₂-, -S(CH₂)ₙ₃-, -NR⁵(CH₂)ₙ₄-, -NR⁶C(=O)(CH₂)ₙ₅-, -C(=O)(CH₂)ₙ₆-, - NR⁷C(=O)NR⁸(CH₂)ₙ₇-, -OC(=O)(CH₂)ₙ₈-, -C=C(CH₂)ₙ₉-, -C≡C(CH₂)ₙ₁₀-, 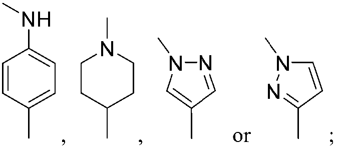
n1, n2, n3, n4, n5, n6, n7, n8, n9, and n10 are each independently selected from any integer between 0 and 6;
R1 is selected from the following groups which are unsubstituted or optionally substituted with one, two or more R^{B}: C₃₋₆ cycloalkyl, or C₃₋₆ cycloalkyl-C₁₋₆ alkyl; for example, R¹ is selected from the following groups which are unsubstituted or optionally substituted with one, two or more R^{B}: 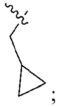
R² is selected from hydrogen;
R²² is selected from hydrogen;
R³ is selected from C₁₋₆ alkyl which is unsubstituted or optionally substituted with one, two or more R^{C}; for example, R³ is selected from methyl, ethyl, or propyl;
R⁴ is selected from halogen;
B is absent or selected from-O-, -S-, or NR⁵; R⁵ is selected from the group consisting of H, deuterium, C₁₋₆ alkyl, and C₃₋₆ cycloalkyl.

According to an embodiment of the present disclosure, R³ and R⁴, together with the atoms to which they are attached form a ring structure as follows, which is unsubstituted or substituted by one, two or more R^{D}:

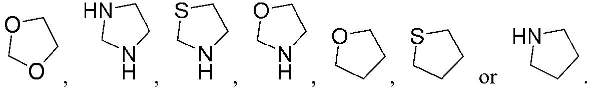

According to an embodiment of the present disclosure, R³ and R⁴, together with the phenyl ring to which they are attached form one of the following substructures, wherein the substructure may be unsubstituted or optionally substituted with one, two or more R^{D}: 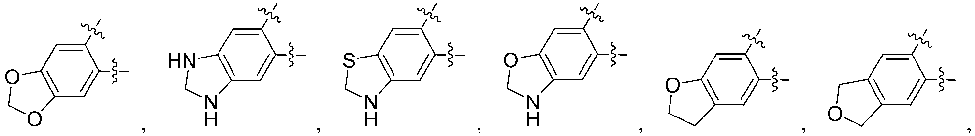 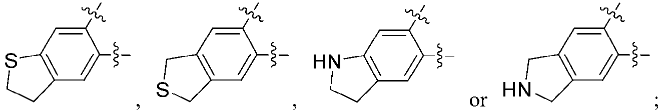 wherein R^{D} has the definition as described above.

According to an embodiment of the present disclosure, the definitions of the groups in formula (G) may be independently selected from:
A is absent or selected from one of the following substructures, wherein the substructure may be unsubstituted or optionally substituted with one, two or more R^{A}: 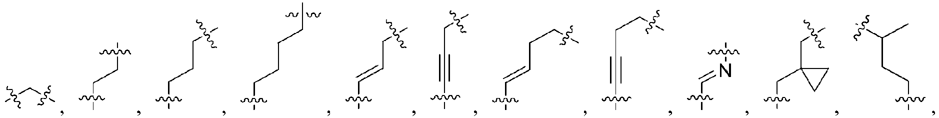 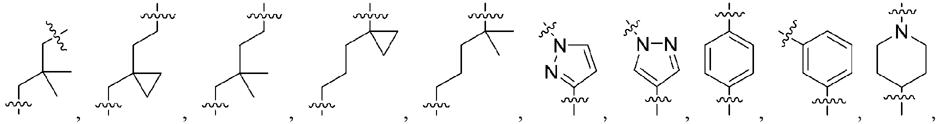 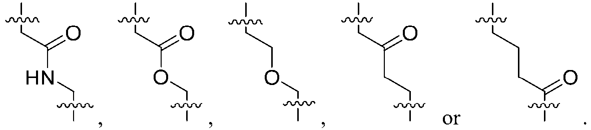
B is absent or selected from-O-, -S-, or NR⁵; R5 is selected from the group consisting of H, deuterium, C₁₋₆ alkyl, and C₃₋₆ cycloalkyl.

In some embodiments, A is selected from the following structures which are unsubstituted or optionally substituted with one, two or more R^{A}:

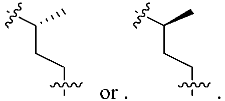

According to an embodiment of the present disclosure, in the G, 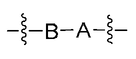 may be selected from the following groups:

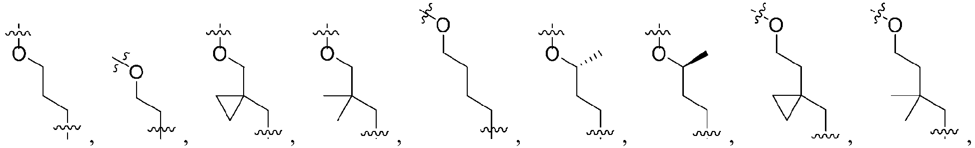

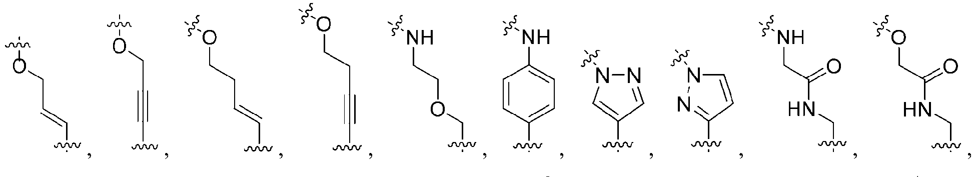

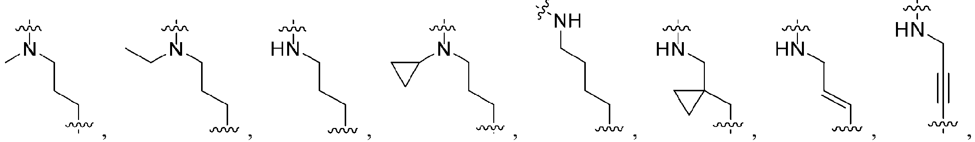

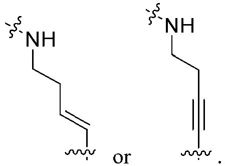

According to an embodiment of the present disclosure, G is selected from the group represented by the following formula (G-1) or (G-2): 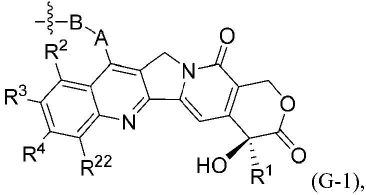 or 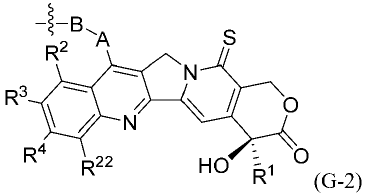 wherein, A, B, R¹, R², R²², R³, and R⁴ independently have the definitions as described above.

According to an embodiment of the present disclosure, G is selected from the group represented by formula (G-3), (G-4), (G-5) or (G-6): 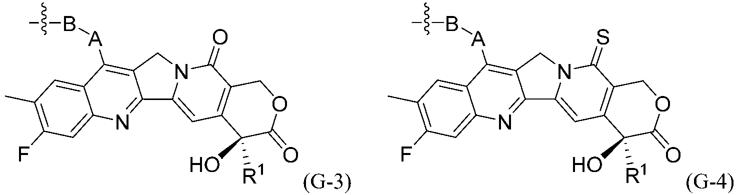 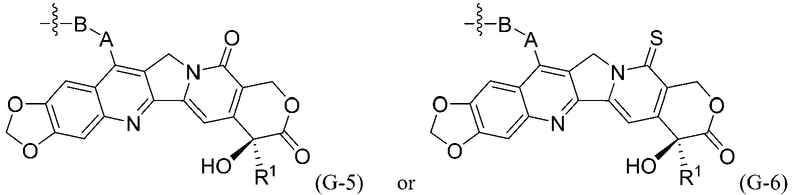 wherein A, B, and R¹ independently have the definitions as described above.

According to an embodiment of the present disclosure, G is selected from the following group: 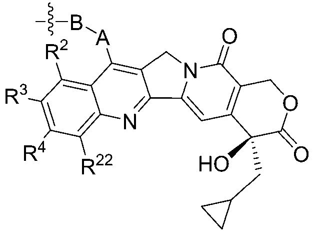 wherein A, B, R², R²², R³, and R⁴ independently have the definitions as described above.

According to an embodiment of the present disclosure, G is selected from the following group: 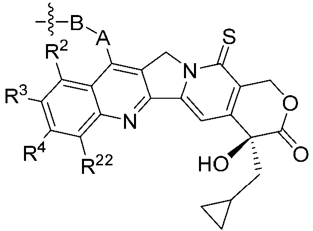 wherein A, B, R², R²², R³, and R⁴ independently have the definitions as described above.

According to an exemplary embodiment of the present disclosure, G is selected from the following groups:

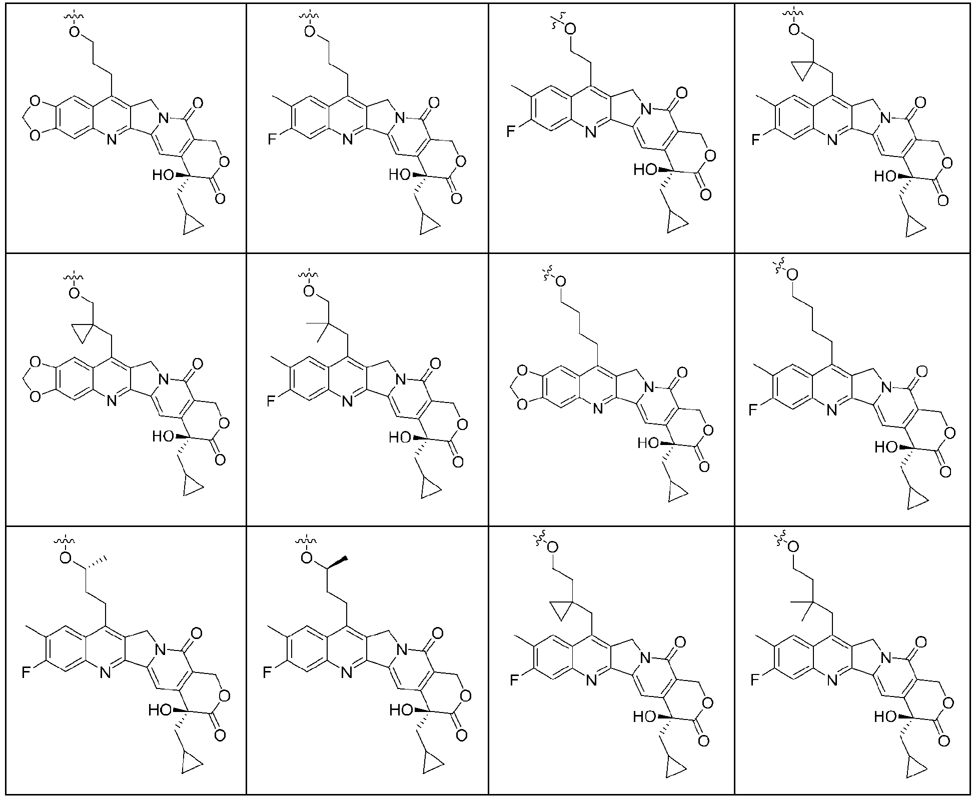

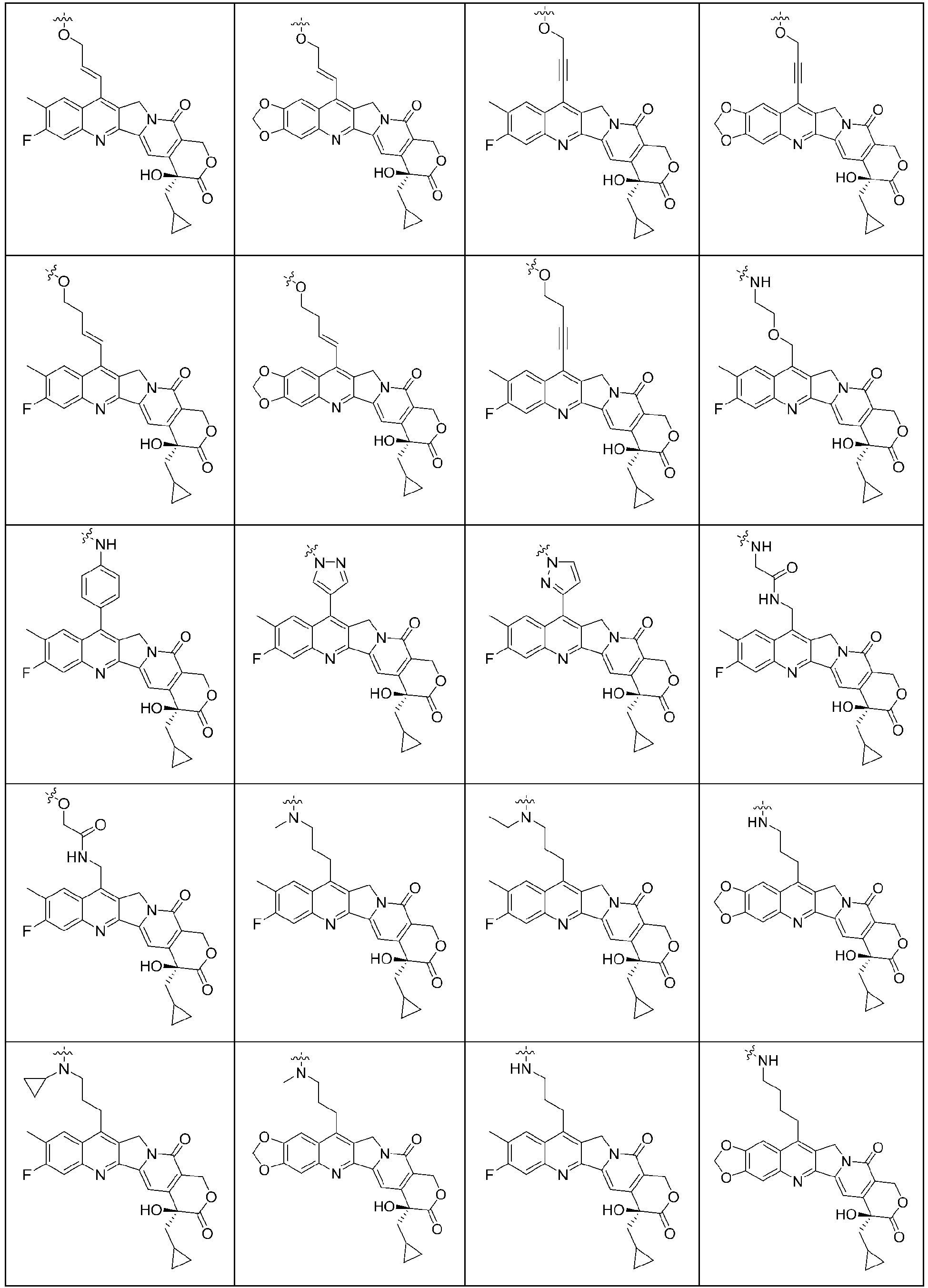

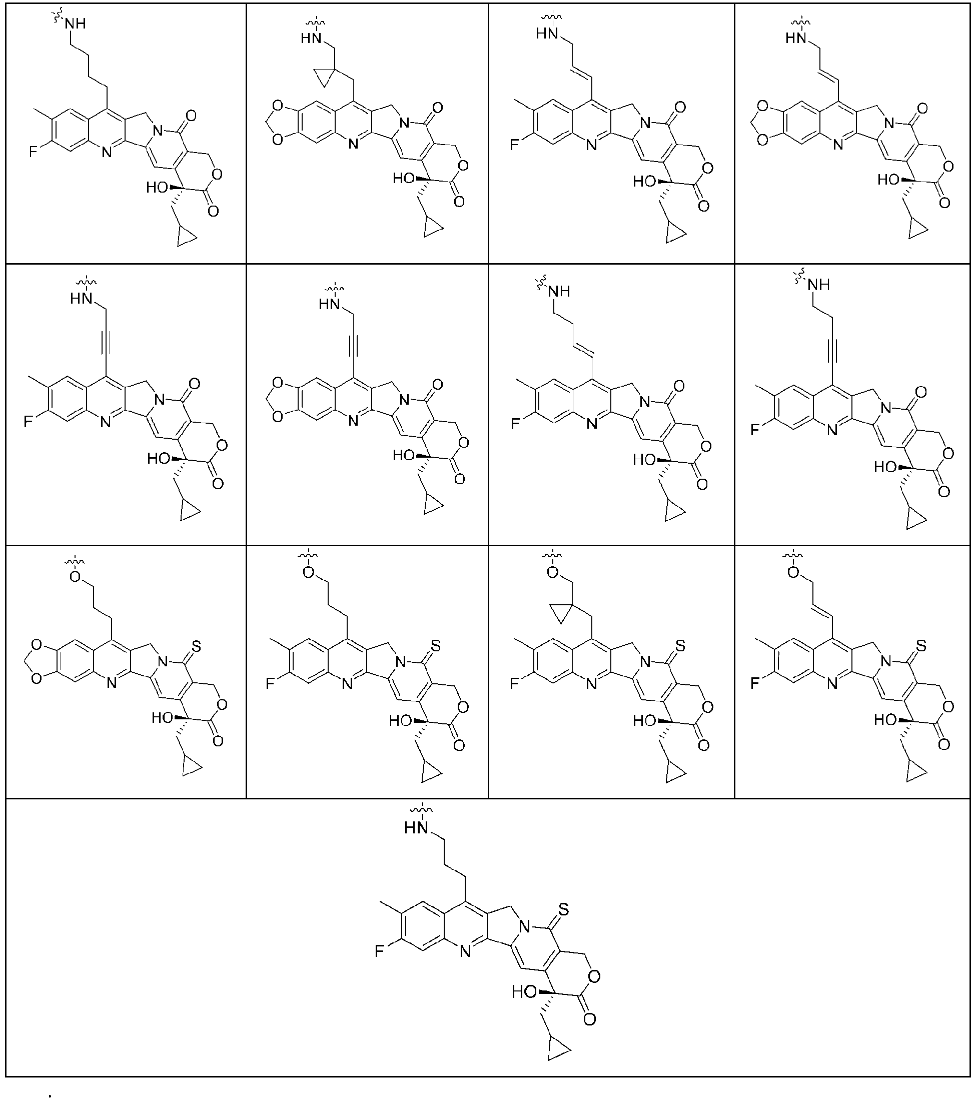

In the above aspects of the present disclosure, wherein L is selected from a chemical bond or a linker represented by formula (L) as defined below:

#L¹-L²-L³-L⁴ * (L)

wherein L¹ is a linking moiety to the targeting moiety Tp, which is formed by reacting a reactive group L^{1'} with the targeting moiety Tp, and # represents a linking site to the targeting moiety Tp;
for example, L^{1'} is a pyrimidine group or a maleimide group, then L¹ has the following structure: 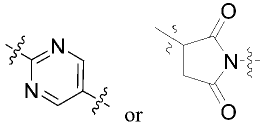 or its open-loop form: 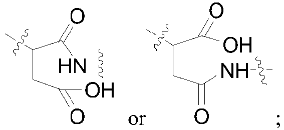
L² is absent or is a spacer between L¹ and L³;
L³ is a peptide moiety;
L⁴ is absent, or is a linking moiety between the peptide moiety and a biologically active molecule G, formed by reacting a reactive group L^{4'} with a biologically active molecule G or an intermediate thereof, and * represents a linking site attached to the biologically active molecule G;
optionally:
   a following hydrophilic moiety is contained between the aforementioned moieties of L, preferably located between L¹ and L², or between L² and L³, or in a form of replacement of L², or in a form of insertion into the L² unit;
   wherein the hydrophilic moiety is a divalent unit substituted with one, two or more hydrophilic groups, such as phenylene substituted with one, two or more hydrophilic groups or an amino acid residue substituted with one, two or more hydrophilic groups, preferably the hydrophilic moiety is: 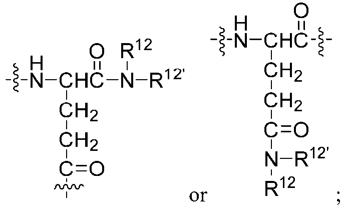 the hydrophilic moiety may also be a divalent unit formed by hydrophilic group itself, such as 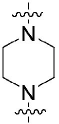 or -(CH₂CH₂O)ₚ-;
   R¹² and R^{12'} are identical or different, at least one is selected from a hydrophilic group and the other is selected from the groups consisting of: hydrogen, halogen, cyano, amino, nitro, and the following substituents which are unsubstituted or optionally substituted with one, two or more Rzg: C₁₋₁₀ alkyl, C₁₋₁₀ alkyloxy, C₃₋₁₀ cycloalkyl, C₃₋₁₀ cycloalkyl-C₁₋₁₀ alkyl, or C₃₋₁₀ cycloalkyloxy;.

The hydrophilic group is selected from a polyethylene glycol group or a polyethylene glycol divalent unit, a C₁₋₁₀ alkyl substituted with 1 to 10 hydroxyl, a group containing a sugar ring or a heterocyclic divalent unit containing a nitrogen atom such as a piperidinyl or piperazinyl, preferably a polyethylene glycol group, further preferably -(CH₂CH₂O)ₚ-C₁₋₁₀ alkyl, -C(=O)-NH-(CH₂CH₂O)ₚ-C₁₋₁₀ alkyl or -NH-(CH₂CH₂O)ₚ-C₁₋₁₀ alkyl, or preferably a C₁₋₁₀ alkyl substituted with 1 to 10 hydroxyl, further preferably 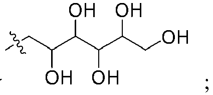
each p is identical or different and is independently selected from an integer from 0 to 10, preferably 1, 2, 3, 4, 5, 6, 7 or 8;
each R^{zg} is identical or different and is independently selected from the following groups: halogen, hydroxy, amino, cyano, nitro, C₁₋₁₀ alkyl, C₁₋₁₀ alkyloxy, C₃₋₁₀ cycloalkyl, C₃₋₁₀ cycloalkyl-C₁₋₁₀ alkyl, C₃₋₁₀ cycloalkyloxy;
preferably, each R^{zg} is identical or different and is independently selected from the following groups: halogen, hydroxy, amino, cyano, nitro, C₁₋₆ alkyl, C₁₋₆ alkyloxy, C₃₋₆ cycloalkyl, C₃₋₆ cycloalkyl-C₁₋₆ alkyl, C₃₋₆ cycloalkyloxy.

In certain embodiments of the present disclosure, the hydrophilic moiety in L as defined above is absent.

In certain embodiments of the present disclosure, L containing a hydrophilic moiety is, for example:

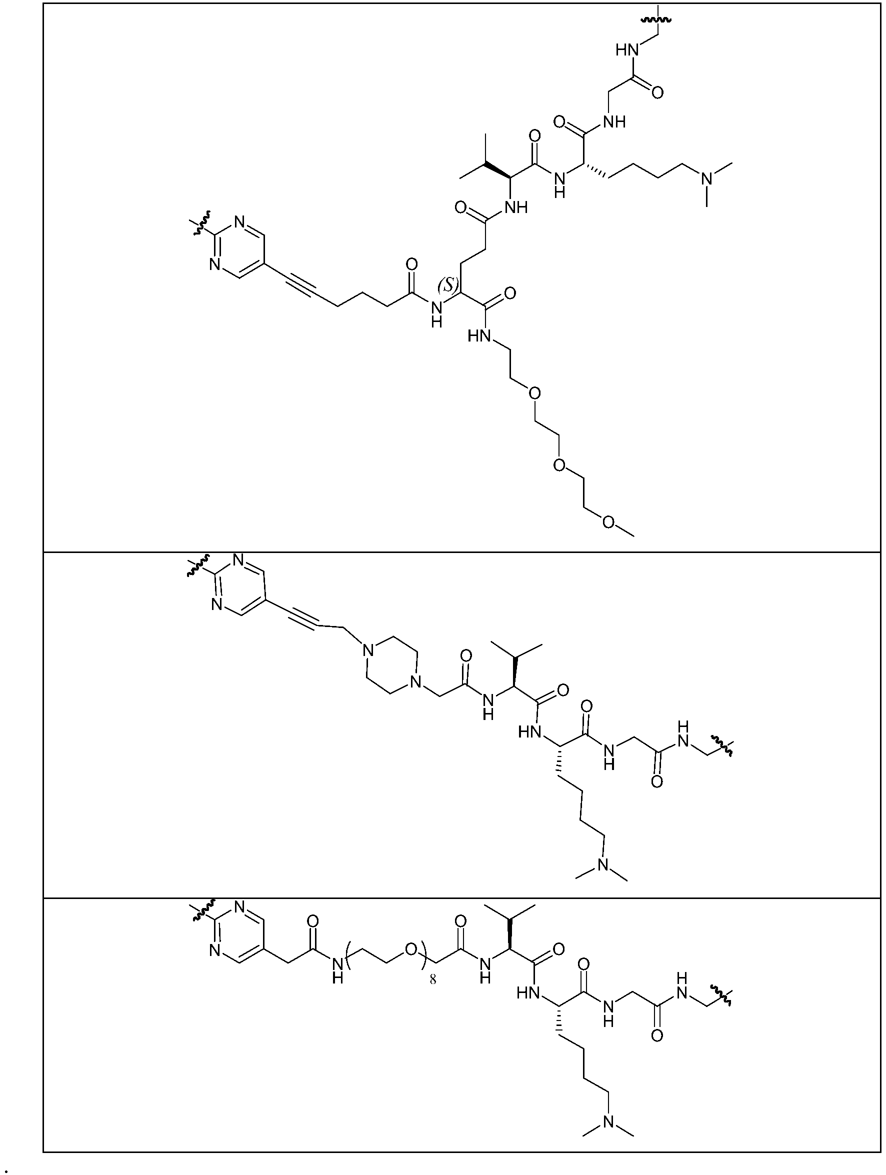

In a further preferred embodiment of the present disclosure, L¹ is formed from any group L^{1'} which reacts with the targeting moiety Tp, wherein L^{1'} is preferably a thiol-reactive group, an amino-reactive group, a carboxyl-reactive group, a dithiol bridging group, or the like; for antibodies directed to introduction of an unnatural amino acid, it may also be selected from a click chemical group such as ketone, hydrazine or hydrazide, azide, alkyne, cyclopropene or diene. When the Tp moiety is an antibody, the linking site comprises any applicable amino acid residue or N297 sugar chain coupling of the CH₂ domain, such as fucose, galactose, N-acetylgalactosamine (GalNAc), N-acetylglucosamine (GlcNAc) and sialic acid (SA) introduced by sugar engineering. The linking reaction comprises a chemical reaction or an enzymatic reaction, such as the transfer of an amine-containing linker-drug or reactive spacer into a deglycosylated antibody utilizing glutamine transaminase (MTGase).

L^{1'} is preferably a thiol-reactive group;
preferably, L^{1'} is a thiol-reactive group having the following structure:
Hal-Het-
Hal is selected from the group consisting of halogen, OMs, OTs, OTf, nitro, and the following groups which are optionally substituted with one, two or more R^{z8}: C₁₋₁₀ alkyl thioether group, C₆₋₁₂ aryl thioether group, 5-12 membered heteroaryl thioether group, C₁₋₁₀ alkyl sulfoxide group, C₆₋₁₂ aryl sulfoxide group, 5-12 membered heteroaryl sulfoxide group, C₁₋₁₀ alkylsulfonyl, C₆₋₁₂ arylsulfonyl, or 5-12 membered heteroarylsulfonyl; wherein, R^{z8} is independently selected from the group consisting of H (hydrogen), D (deuterium), halogen, CN, nitro, C₁₋₆ alkyl, halo-C₁₋₆ alkyl, C₁₋₆ alkoxy, C₆₋₁₂ aryl, and 5-12 membered heteroaryl;
Het is selected from a 5-12 membered heteroaryl optionally substituted with one, two or more R^{z9}; wherein, R^{z9} is independently selected from the group consisting of H (hydrogen), D (deuterium), halogen, CN, nitro, C₁₋₁₀ alkyl, and halo-C₁₋₁₀ alkyl. Preferably, Het is selected from a 5-10 membered heteroaryl optionally substituted with one, two or more R^{z9}; wherein, R^{z9} is independently selected from the group consisting of H (hydrogen), D (deuterium), halogen, CN, nitro, C₁₋₄ alkyl, and halo-C₁₋₄ alkyl.

In a preferred embodiment, Hal is methanesulfonyl and Het is pyrimidine;
in the preferred embodiment, Hal-Het-is: 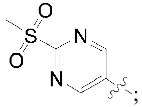
the corresponding L¹ structure is: 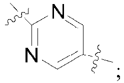
and L^{1'}-L² is preferably the following structure: 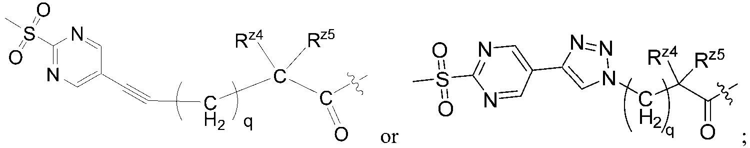
q is an integer selected from 0 to 10, preferably 1, 2, 3, 4, 5, 6, 7 or 8;
R^{z4} and R^{z5} are identical or different and independently of one another are selected from H, C₁₋₄ alkyl, C₃₋₆ cycloalkyl, C₃₋₆ cycloalkyl-C₁₋₄ alkyl, or R^{z4} and R^{z5} together form C₃₋₆ cycloalkyl.

In a further preferred embodiment of the present disclosure, L¹'-L² is selected from the following structure: 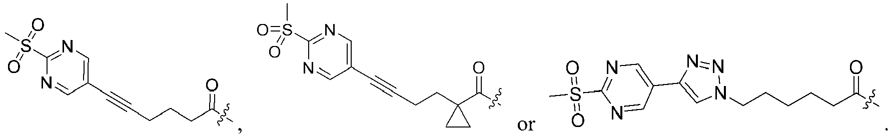 or L^{1'} is further preferably a maleimide group or a substituted maleimide group, and L¹-L² is preferably of the structure:
a fragment prepared from (N-maleimidomethyl)-carboxylic acid-N-hydroxysuccinimide ester, which has the following structure: 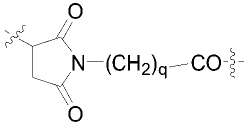 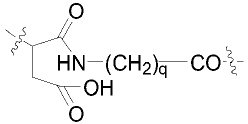 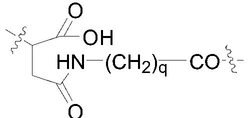
(q is an integer from 0 to 10, preferably 1, 2, 3, 4, 5, 6, 7 or 8);
a fragment prepared from m-maleimidobenzoyl-N-hydroxysuccinimide ester (MBS), which has the following structure: 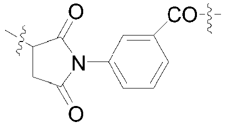 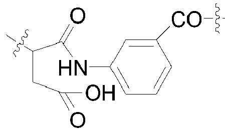 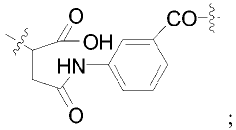
or a fragment prepared from 4-(N-maleimidomethyl)-cyclohexane-1-carboxylic acid succinimidyl ester (SMCC), which has the following structure:

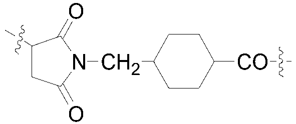

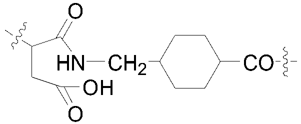

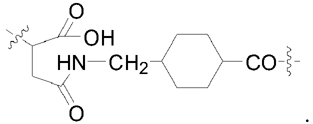

In a further embodiment of the present disclosure,
L² is absent or selected from the group consisting of C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl, anda combination of two or more of them; wherein the C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl or a combination of two or more of them may be uninterrupted or optionally interrupted by carbonyl, O, S, or N; the C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl or a combination of two or more of them may optionally be substituted with C₁₋₆ alkyl, C₃₋₆ cycloalkyl, halogen, halo-C₁₋₆ alkyl; Optionally, the C₁₋₆ alkyl or halo-C₁₋₆ alkyl may form a C₃₋₆ cycloalkyl with the C atom to which it is attached, and L² is attached to the L¹ or L³ fragment via a functional group or a covalent bond;
preferably, L² is selected from-(CH₂)_{q}-, -C(=O)-NH-(CH₂)_{q}-C(=O)-, -(CH₂)_{q}-C(=O)-, -(CH₂)_{q}-NH-C(=O)-, - (CH₂)q-NCH₃-C(=O)-, -(C≡C)-(CH₂)_{q}-C(R^{z4}R^{z5})-C(=O)- or -Cy-(CH₂)_{q}-C(R^{z4}R^{z5})-C(=O)-, wherein q is an integer selected from 0 to 10, preferably 1, 2, 3, 4, 5, 6, 7 or 8;
wherein Cy is a 5-12 membered heteroaryl or heterocyclyl optionally containing S, O or N and is optionally substituted with H (hydrogen), D (deuterium), halogen, CN, nitro, C₁₋₄ alkyl and halo-C₁₋₄ alkyl, preferably at least three atoms in Cy are N, further preferably three consecutive atoms in Cy are N, further preferably Cy is 1, 2, 3-triazolyl.

Preferably, R^{z4} and R^{z5} are identical or different and independently of one another are selected from H, C₁₋₄ alkyl, C₃₋₆ cycloalkyl, C₃₋₆ cycloalkyl-C¹⁻⁴ alkyl, or R^{z4} and R^{z5} together form C₃₋₆ cycloalkyl.

Preferably, L² is linked to the N-terminus of peptide fragment L³ via-NHC(=O)-, -NCH₃C(=O)- or -C(=O)-.

In a further embodiment of the present disclosure,
L³ is selected from a bivalent peptide radical comprising 2 to 8 amino acid residues, which are natural or non-natural, of L- or D-configuration, and are optionally substituted; each of the amino acid residues is identical or different and is independently selected from the residues of the following amino acid: alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), selenocysteine (Sec), pyrrolysine (Pyl), homoserine, homocysteine, demethylpyrrolysine, an analog of the above amino acid or an amino acid residue represented by AA¹ or a stereoisomer thereof: 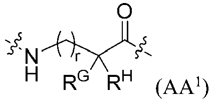
wherein R^{G} and R^{H} are not simultaneously H and are each independently selected from H,

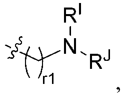

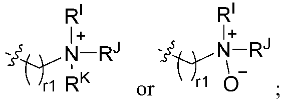

Alternatively, R^{G} and R^{H}, together with the carbon atoms to which they are jointly attached form a C₃₋₁₀ cycloalkyl or 3-10 membered heterocyclyl which is unsubstituted or optionally substituted with one, two or more R^{L};
r and r¹ are each independently selected from any integer from 0 to 10;
R^{I}, R^{J} and R^{K} are each independently selected from H, and the groups consisting of C₁₋₆ alkyl, C₃₋₁₀ cycloalkyl, C₃₋₁₀ cycloalkyl-C₁₋₁₀ alkyl, or ester groups, which are unsubstituted or optionally substituted with one, two or more R^{M};
alternatively, R^{I} and R^{J}, together with the nitrogen atom to which they are jointly attached form a 4-10 membered heterocyclyl which is unsubstituted or optionally substituted with one, two or more R^{L};
R^{M} and R^{L} are identical or different and are selected independently of one another from the group consisting of deuterium, halogen, hydroxy, cyano, nitro, alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, NH₂, alkylamino, dialkylamino, HC(=O)NH-, alkyl-C(=O)NH-, cycloalkyl-C(=O)NH-, heterocyclyl-C(=O)NH-, aryl-C(=O)NH-, or heteroaryl-C(=O)NH-; optionally, two R^{M} or R^{L} attached to the same carbon atom together form a C₃₋₆ cycloalkyl.

Further preferably, L³ is selected from a bivalent peptide group comprising a combination of 2, 3, 4, 5 or 6 amino acid residues, which are optionally substituted, natural or non-natural, and of L- or D-configuration or amino acid residues of AA¹; each of the amino acid residues is identical or different, independently selected from the following amino acid residues: alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), selenocysteine (Sec), pyrrolysine (Pyl), homoserine, homocysteine, desmethylpyrrolysine, or AA¹; e.g. -ValCit-; -ValAA¹-; -CitVal-; -AlaAla-; -AlaCit-; - CitAla-; -AsnCit-; -CitAsn-; -CitCit-; -ValGlu-; -GluVal-; -SerCit-; -CitSer-; -LysCit-; -CitLys-; -AspCit-; -CitAsp-; -AlaVal-; -ValAla-; -PheAla-; -AlaPhe-; -PheLys-; -LysPhe-; -ValLys-; -GlyAA¹-; -LysVal-; -AlaLys-; -LysAla-; -PheCit-; -CitPhe-; -LeuCit-; -CitLeu-; -IleCit-; -CitIle-; -PheArg-; -ArgPhe-; -CitTrp-; -TrpCit-; -AlaAlaAla-; - PhePheLys-; -ValAA¹Gly-; -AlaAA¹Gly-; - GlyAA¹Gly-; -LysPhePhe-; -DPhePheLys-; -DLysPhePhe-; - GlyPheLys-; -LysPheGly-; -GlyPheLeuGly-; -GlyLeuPheGly-; -AlaLeuAlaLeu-; -GlyGlyGly-; -GlyGlyGlyGly-; - GlyPheValGly-; -GlyValPheGly-; -GlyGlyPheGly-or -GlyGlyValGly-.

Preferably, in the amino acid residues of AA¹,
r and r1 are each independently selected from any integer from 0 to 5;
Either of R^{G} and R^{H} is H and the other is selected from:

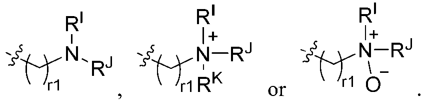

Alternatively, R^{G} and R^{H} together with the carbon atoms to which they are jointly attached form a 5-6 membered heterocyclyl, which is unsubstituted or optionally substituted with one, two or more R^{L};
R^{I}, R^{J} and R^{K} are each independently selected from the group consisting of H, methyl, ethyl, n-propyl, n-butyl, C₃₋₆ cycloalkyl, C₃₋₆ cycloalkyl-C₁₋₆ alkyl, -COOCH₃, COOCH₂CH₃, -COOCH₂CH₂CH₃, -COOCH(CH₃)₂, - COOC(CH₃)₃ and -COOCH₂CH₂CH₂CH₃ unsubstituted or optionally substituted with one, two or more R^{M}.

Alternatively, R^{I} and R^{J} together with the nitrogen atom to which they are jointly attached form a 5-6 membered heterocyclyl that is unsubstituted or optionally substituted with one, two or more R^{L}.

Most preferably,
L³ is selected from -ValAA¹Gly-;
in the amino acid residues of AA¹, r is 0 and r1 is 4;
either of R^{G} and R^{H} is H and the other is selected from:

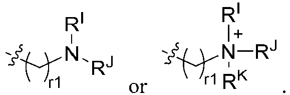

Alternatively, R^{G} and R^{H} together with the carbon atoms to which they are jointly attached to form 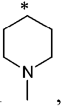 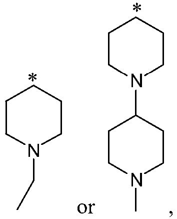 wherein * indicates the carbon atom to which R^{G} and R^{H} are jointly attached;
R^{I}, R^{J} and R^{K} are each independently selected from H, methyl, ethyl, n-propyl, n-butyl, C₃₋₆ cycloalkyl, C₃₋₆ cycloalkyl-C₁₋₄ alkyl, or -COOC(CH₃)₃ unsubstituted or optionally substituted with one, two or more R^{M}.

Alternatively, R^{I} and R^{J} together with the nitrogen atoms to which they are jointly attached form

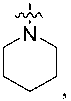

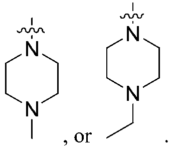

In further embodiments of the present disclosure. AA¹ amino acid residue is selected from one of the following substructures:

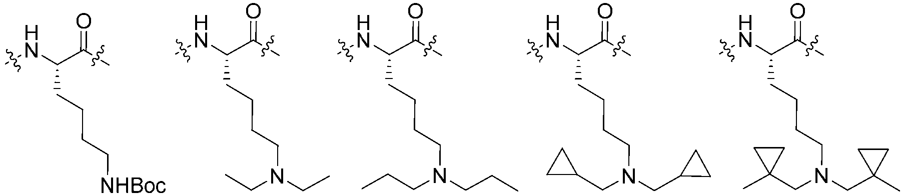

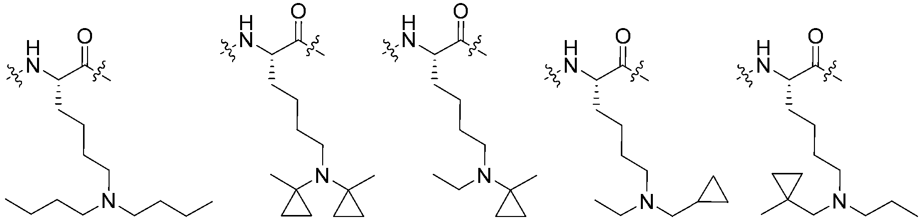

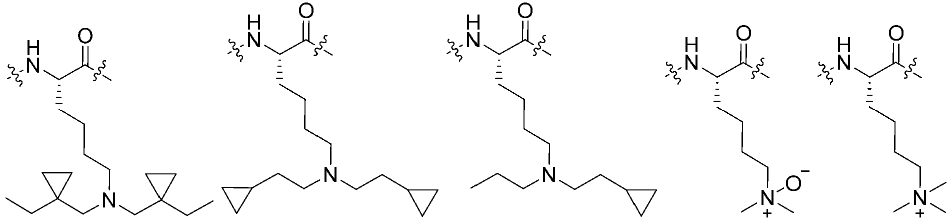

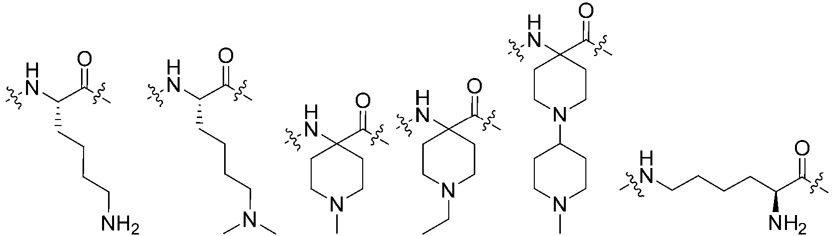

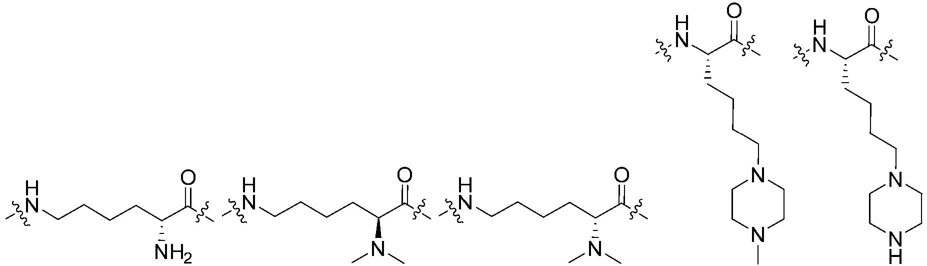

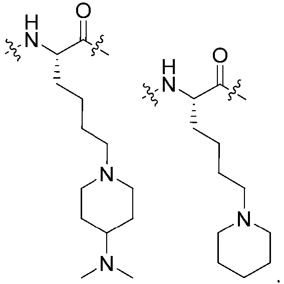

In further embodiments of the present disclosure,
L⁴ is absent or selected from:

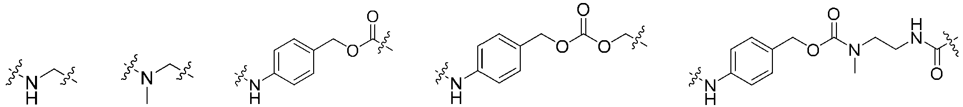

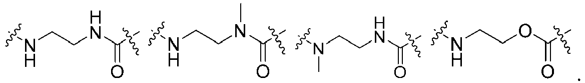

Further preferably, L⁴ is: 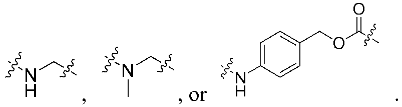 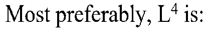 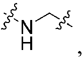 and the corresponding L^{4'} reactive group is in the acetate form:

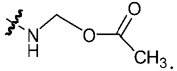

Its reaction with a compound of formula (GH) (B being an oxygen atom) is shown in the following formula:

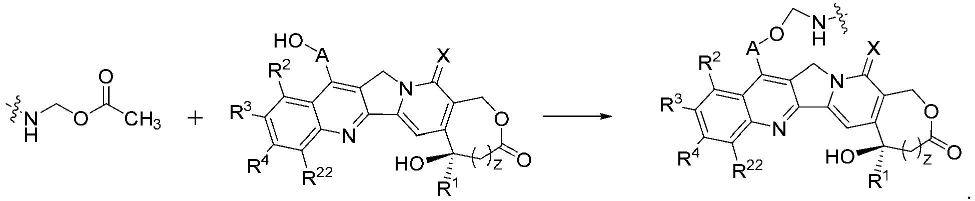

When L⁴ is absent, the corresponding L^{4'} reactive group is formed from an amino acid in the terminal group of L³, such as an active ester of glycine.

In a further embodiment of the present disclosure,
L¹ is generated by coupling Tp with a thiol-reactive group selected from a maleimide group, a substituted maleimide group or Hal-Het-;
a hydrophilic moiety as defined above is contained between L¹ and L², or between L² and L³, or in a form of replacement of L², or in a form of insertion into the L² unit.

In a further embodiment of the present disclosure,
L¹ is generated by coupling Tp with a thiol-reactive group selected from Hal-Het-;
L⁴ is:

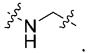

In a further embodiment of the present disclosure,
L¹-L² is formed by coupling Tp with L^{1'}-L² of the following structure: 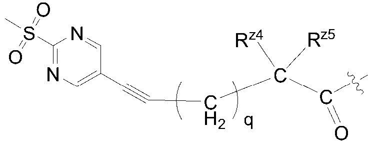
q is an integer selected from 0 to 10, preferably 1, 2, 3, 4, 5, 6, 7 or 8;
R^{z4} and R^{z5} are identical or different and independently selected from H, C₁₋₄ alkyl, C₃₋₆ cycloalkyl, C₃₋₆ cycloalkyl-C₁₋₄ alkyl, or R^{z4} and R^{z5} together form a C₃₋₆ cycloalkyl, wherein at least one of R^{z4} or R^{z5} is not H.

Further preferably, R^{z4} and R^{z5} together form a C₃₋₆ cycloalkyl;
L¹-L² is most preferably generated by coupling 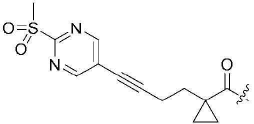 with Tp.

Each of the fragments L¹-L⁴ or the hydrophilic moiety of L as defined above may be linked by any chemical bond, such as a direct bond, an ester bond (-CO-O-), an amide bond (-CO-NH-), an ether bond (-O-), a thioether bond (-S-), a carbamate (-N-CO-O-), or a ureido group (-O-CO-O-);
Preferably, H in each peptide bond or amide bond in L may optionally be substituted by methyl.

The present disclosure further provides a conjugate of the following structure:

Tp-L-D (D)

wherein Tp is a targeting moiety;
D is a biologically active molecular fragment, preferably a molecular fragment having antitumor biological activity;
L is selected from the linker shown by formula (L):

   #L¹-L²-L³-L⁴ * (L)
wherein L¹ is a linking moiety to a targeting moiety Tp, formed from a reactive group L^{1'} and the targeting moiety Tp, # represents a linking site to the Tp moiety; preferably, L¹ is 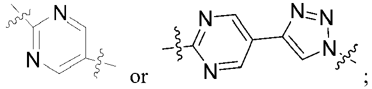
L² is selected from -(C≡C)-(CH₂)_{q}-C(R^{z4}R^{z5})-C(=O)- or -(CH₂)_{q}-C(R^{z4}R^{z5})-C(=O)-;
L^{1'}-L² has the following structure: 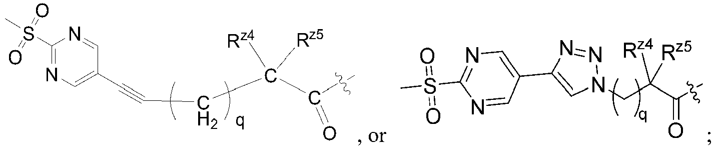
q is an integer selected from 0 to 10, preferably 1, 2, 3, 4, 5, 6, 7 or 8;
R^{z4} and R^{z5} are identical or different and independently of one another are selected from H, C₁₋₄ alkyl, C₃₋₆ cycloalkyl, C₃₋₆ cycloalkyl-C₁₋₄ alkyl, or R^{z4} and R^{z5} together form a C₃₋₆ cycloalkyl, wherein at least one of R^{z4} or R^{z5} is not H;
   or
L² is selected from -(C≡C)-(CH₂)_{q}-C(=O)-, wherein a hydrophilic moiety as defined above is contained between L² and L³: 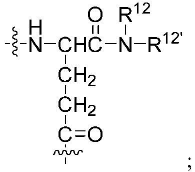
L³ is -ValAA¹Gly-, wherein AA¹ is as defined above;
L⁴ is absent or is a linking moiety of a peptide moiety to a biologically active molecule D, formed by reacting a reactive group L^{4'} with the biologically active molecule, and * represents a linking site to the biologically active molecule D.

The present disclosure also provides an intermediate for the synthesis of the conjugate, which has the structure:

L¹'-L²-L³-L⁴-G(C')

wherein: G, L^{1'}, L², L³, and L⁴ are as defined above.

The present disclosure also provides conjugates of the linker and the drug:

L¹'-L²-L³-L⁴-D

wherein L^{1'}, L², L³, L⁴, and D are as defined above, provided that:
L^{1'} is a reactive group attached to the targeting moiety Tp,
L^{1'}is preferably 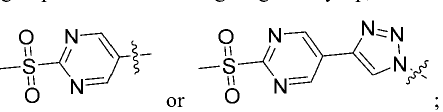
L² is selected from-(C≡C)-(CH₂)_{q}-C(R^{z4}R^{z5})-C(=O)- or -(CH₂)_{q}-C(R^{z4}R^{z5})-C(=O)-;
wherein L^{1'}-L² has the following structure: 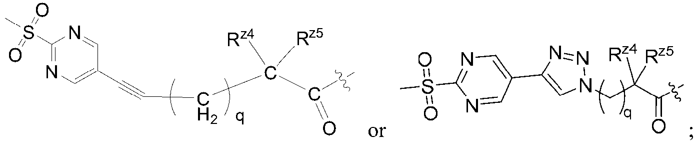
q is an integer selected from 0 to 10, preferably 1, 2, 3, 4, 5, 6, 7 or 8;
R^{z4} and R^{z5} are identical or different and independently of one another are selected from H, C₁₋₄ alkyl, C₃₋₆ cycloalkyl, C₃₋₆ cycloalkyl-C₁₋₄ alkyl, or R^{z4} and R^{z5} together form a C₃₋₆ cycloalkyl, wherein at least one of R^{z4} or R^{z5} is not H;
   or
L² is selected from the group consisting of -(C≡C)-(CH₂)_{q}-C(=O)-, wherein a hydrophilic moiety as defined above is contained between L² and L³: 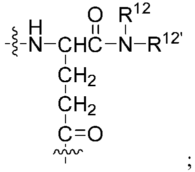
L³ is -ValAA¹Gly-, wherein AA¹ is as defined above;
L⁴ is absent or is a linking moiety of a peptide moiety to a biologically active molecule D, formed by reacting a reactive group L^{4'} with a biologically active molecule, and * represents a linking site to the biologically active molecule D.

It is understood by those skilled in the art that the biologically active molecule D may be a biologically active or potentially biologically active compound as documented in the Chinese Pharmacopoeia, The United States Pharmacopoeia, or European Pharmacopoeia or disclosed in other publications. As an example, the drug may be selected from the group consisting of a cytotoxic drug, a cytostatic drug, and an immunosuppressive drug, for example, an anti-tubulin agent, a tubulin inhibitor, a DNA minor groove binder, a DNA replication inhibitor, an alkylating agent, an antibiotic, an antifolate, an antimetabolite, a chemosensitizer, a topoisomerase inhibitor, a vinca alkaloid, and the like. Examples of such cytotoxic drugs include, for example, auristatin, camptothecin, duocarmycin, etoposide, maytansinoid and a maytansinoid alkaloid, a taxane, a benzodiazepine or a benzodiazepine-containing drug, and a vinca alkaloid.

Tp is a targeting moiety (e.g., a small molecule ligand, a protein, a polypeptide, a non-proteinaceous agent (e.g., a sugar, RNA or DNA)).

In some preferred embodiments, the target of Tp is selected from the group consisting of epidermal growth factor, Trop-2, CD37, HER2, CD70, EGFRvIII, Mesothelin, Folate receptor1, Mucin 1, CD138, CD20, CD19, CD30, SLTRK6, Nectin 4, Tissue factor, Mucinl6, Endothelin receptor, STEAP1, SLC39A6, Guanylylcyclase C, PSMA, CCD79b, CD22, Sodium phosphate cotransporter 2B, GPNMB, Trophoblast glycoprotein, AGS-16, EGFR, CD33, CD66e, CD74, CD56, PD-L1, TACSTD2, DR5, E16, 0772P, MPF, Napi3b, Sema 5b, PSCA hlg, ETBR, MSG783, STEAP2, TrpM4, CRIPTO, CD21, CD79b, FcRH2, NCA, MDP, IL20Rα, Brevican, EphB2R, ASLG659, PSCA, GEDA, BAFF-R, CD79a, CXCR5, HLA-DOB, P2X5, CD72, LY64, FcRH1, IRTA2, TENB2, Integrin α5β6, α4β7, FGF2, FGFR2, Her3, CA6, DLL3, DLL4, P-cadherin, EpCAM, pCAD, CD223, LYPD3, LY6E, EFNA4, ROR1, SLITRK6, 5T4, ENPP3, Claudin18.2, BMPR1B, Tyro7, c-Met, ApoE, CD11c, CD40, CD45(PTPRC), CD49D(ITGA4), CD80, CSF1R, CTSD, GZMB, Ly86, MS4A7, PIK3AP1, PIK3CD, CCR5, IFNG, IL10RA1, IL-6, ACTA2, COL7A1, LOX, LRRC15, MCPT8, MMP10, NOG, SERPINE1, STAT1, TGFBR1, CTSS, PGF, VEGFA, C1QA, C1QB, ANGPTL4, EGLN, EGLN3, BNIP3, AIF1, CCL5, CXCL10, CXCL11, IFI6, PLOD2, KISS1R, STC2, DDIT4, PFKFB3, PGK1, PDK1, AKR1C1, AKR1C2, CADM1, CDH11, COL6A3, CTGF, HMOX1, KRT33A, LUM, WNT5A, IGFBP3, MMP14, CDCP1, PDGFRA, TCF4, TGF, TGFB1, TGFB2, CD11b, ADGRE1, EMR2, TNFRSF21, UPK1B, TNFSF9, MMP16, MFI2, IGF-1R, RNF43, NaPi2b and BCMA.

In some preferred embodiments, Tp is a small molecule ligand, such as a folic acid derivative, a glutamate urea derivative, a somatostatin derivative, an arylsulfonamide derivative (e.g., a carbonic anhydrase IX inhibitor), an ICG dye, a cyanine dye or a derivative thereof.

According to an embodiment of the present disclosure, the preferred ligand is selected from an antibody or antigen-binding fragment thereof, wherein the antibody is selected from a chimeric antibody, a humanized antibody or a fully human antibody; preferably a monoclonal antibody.

According to an exemplary embodiments of the present disclosure, the antibody or antigen-binding fragment thereof is at least one selected from the following antibodies or antigen-binding fragments thereof: anti-CD20 antibody, anti-CD22 antibody, anti-CD30 antibody, anti-CD33 antibody, anti-CD44 antibody, anti-CD56 antibody, anti-CD70 antibody, anti-CD73 antibody, anti-CD105 antibody, anti-CEA antibody, anti-A33 antibody, anti-Cripto antibody, anti-EphA2 antibody, anti-G250 antibody, anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-MUCl antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody or anti-Mesothelin antibody, and the antibody may be a bispecific antibody or a multispecific antibody.

As an example, the antibody or antigen-binding fragment thereof is at least one selected from the following antibodies or antigen-binding fragments thereof: Trastuzumab, Pertuzumab, Nimotuzumab, Enoblituzumab, Emibetuzumab, Inotuzumab, Pinatuzumab, Brentuximab, Gemtuzumab, Bivatuzumab, Lorvotuzumab, cBR96, and Glembatumumab.

According to an embodiment of the present disclosure, the conjugate, linker thereof, or linker-drug may be selected from one of the followings, wherein u is selected from an integer of 0 to 10, G has the definition described above, and LG has the definition of Tp as described above;

| No. | Conjugate, linker or linker-drug thereof |
|---|---|
| 1 | 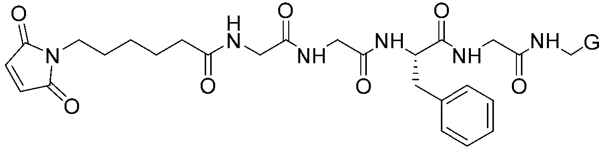 |
| 2 | 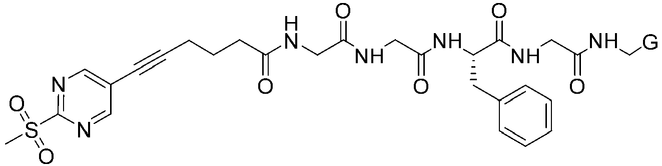 |
| 3 | 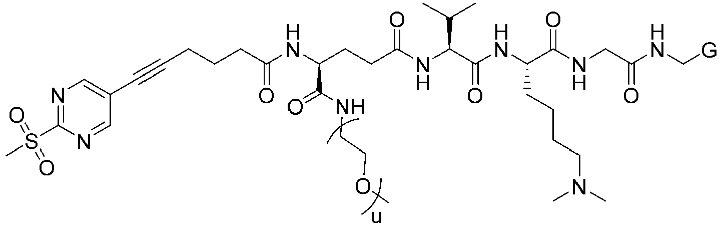 |
| 4 | 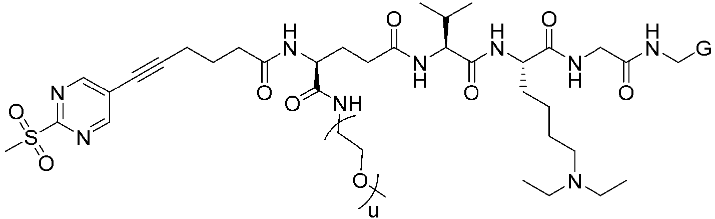 |
| 5 | 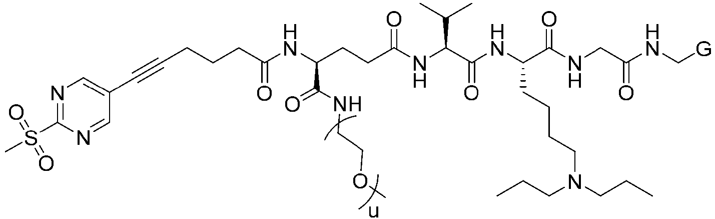 |
| 6 | 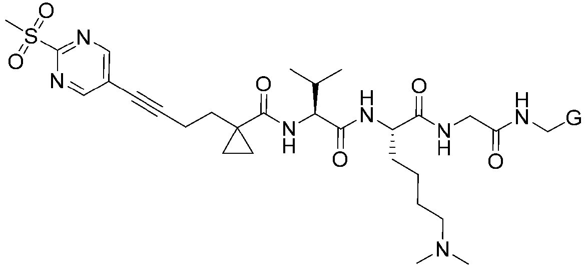 |
| 7 | 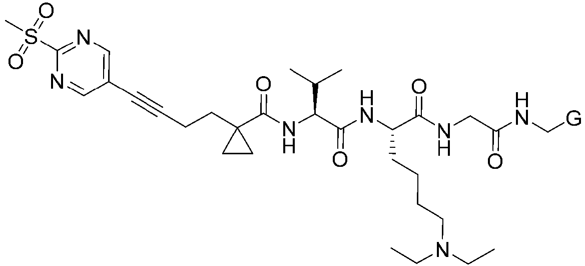 |
| 8 | 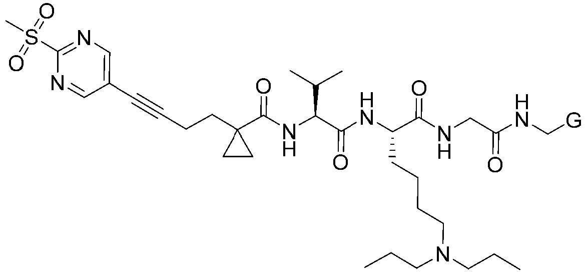 |
| 9 | 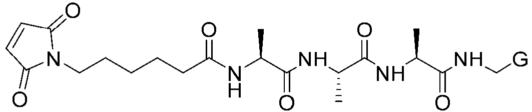 |
| 10 | 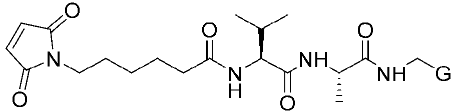 |
| 11 | 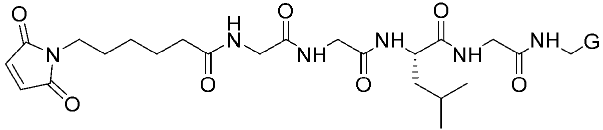 |
| 12 | 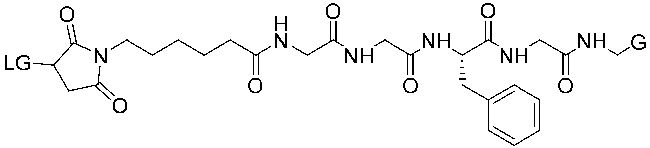 |
| 13 | 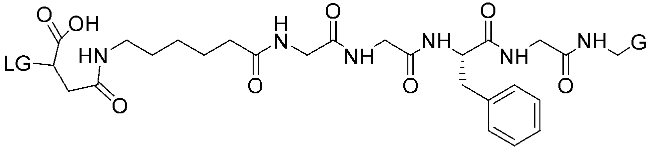 |
| 14 | 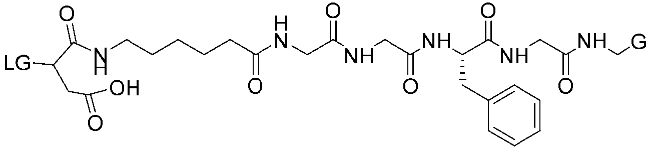 |
| 15 | 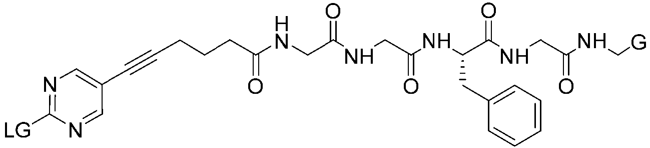 |
| 16 | 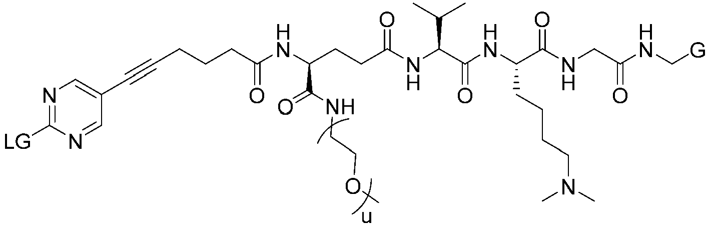 |
| 17 | 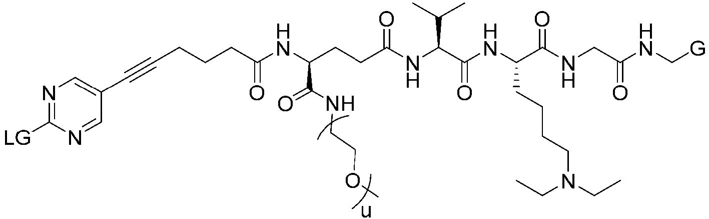 |
| 18 | 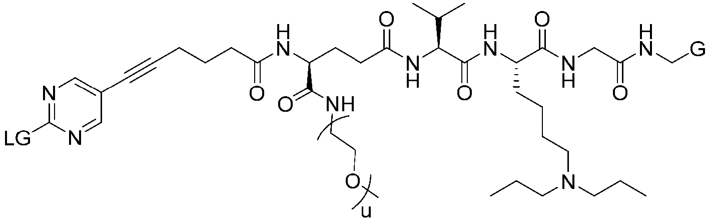 |
| 19 | 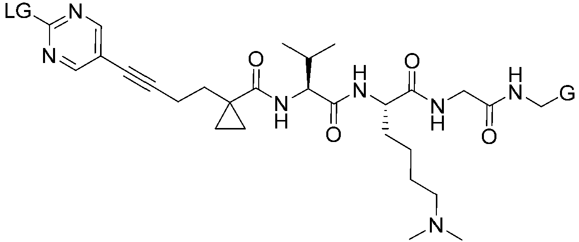 |
| 20 | 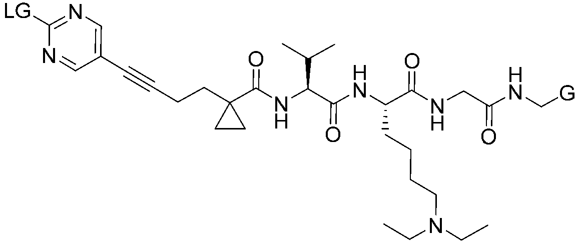 |
| 21 | 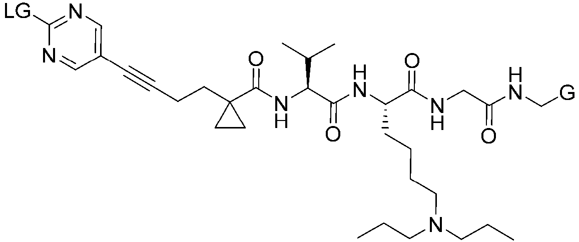 |
| 22 | 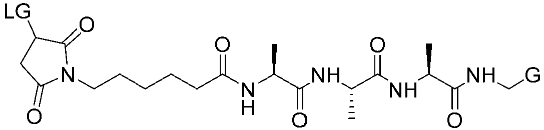 |
| 23 | 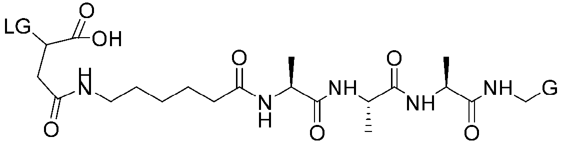 |
| 24 | 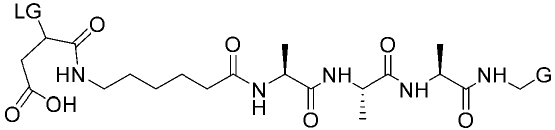 |
| 25 | 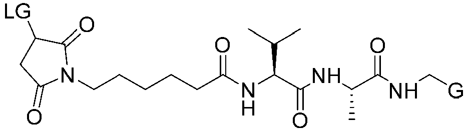 |
| 26 | 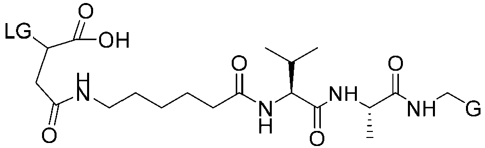 |
| 27 | 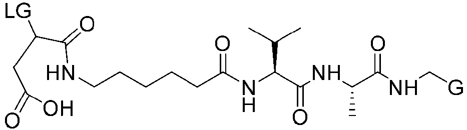 |
| 28 | 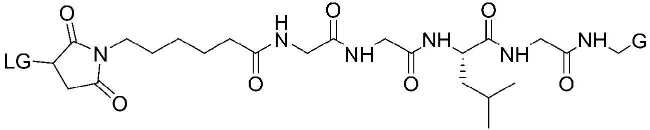 |
| 29 | 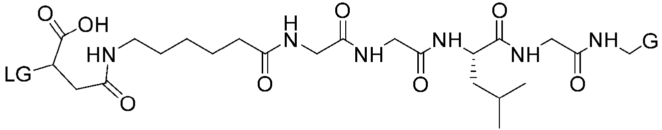 |
| 30 | 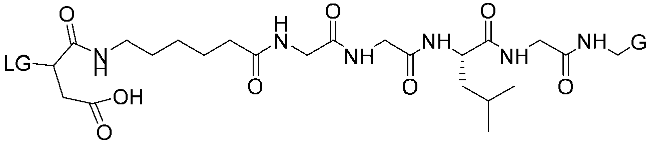 |
| 31 | 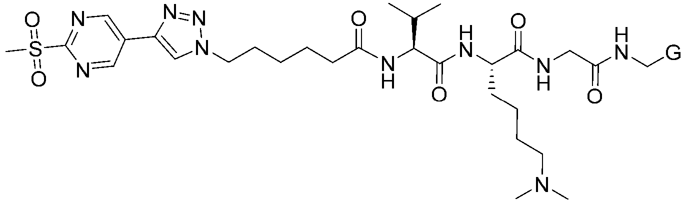 |
| 32 | 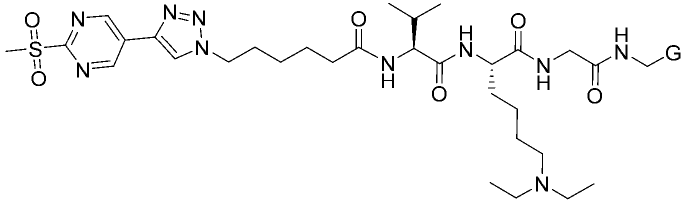 |
| 33 | 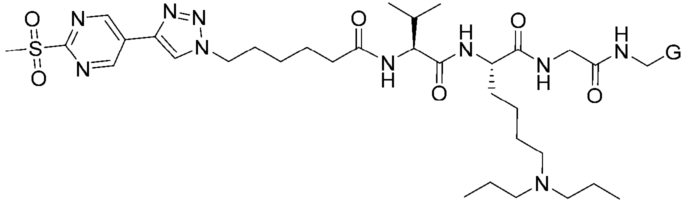 |
| 34 | 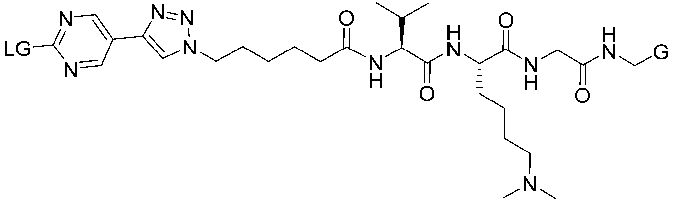 |
| 35 | 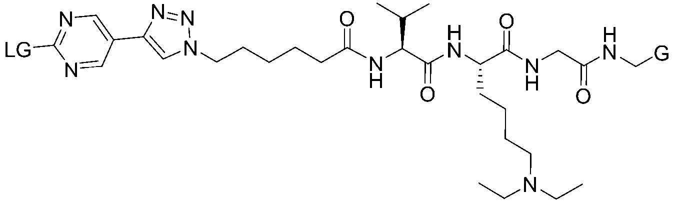 |
| 36 | 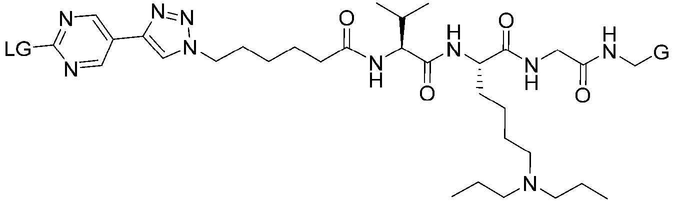 |
| 37 | 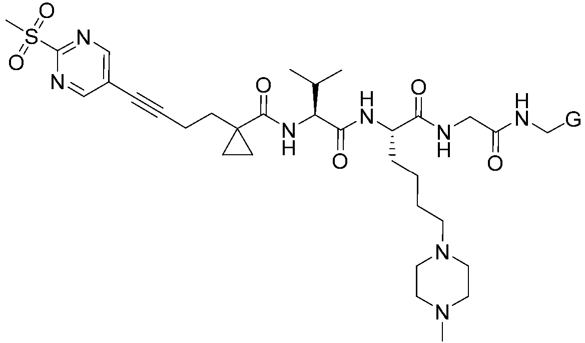 |
| 38 | 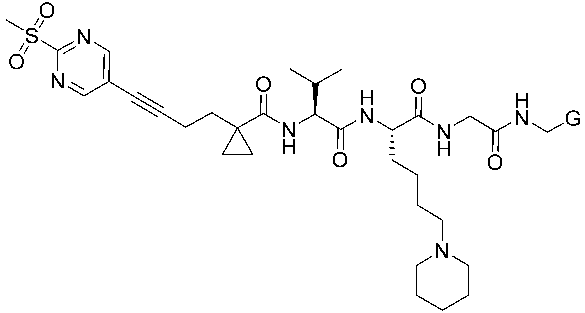 |
| 39 | 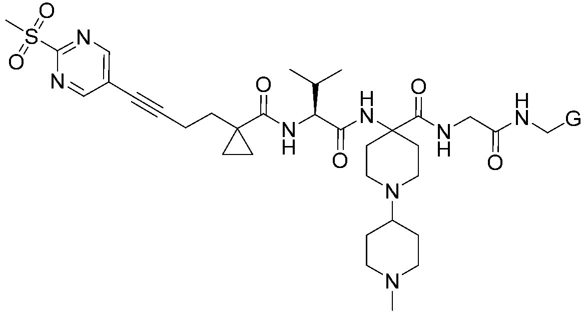 |
| 40 | 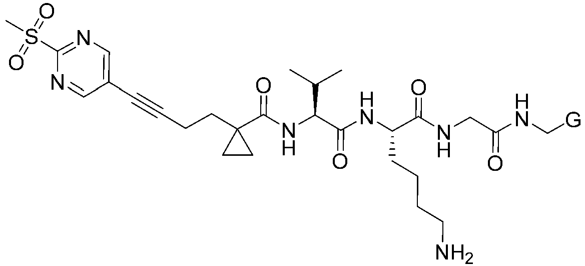 |
| 41 | 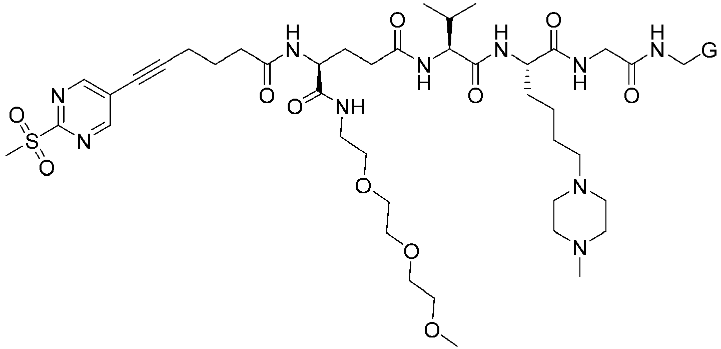 |
| 42 | 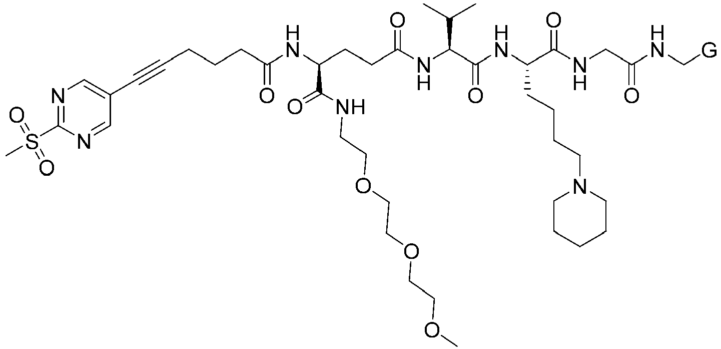 |
| 43 | 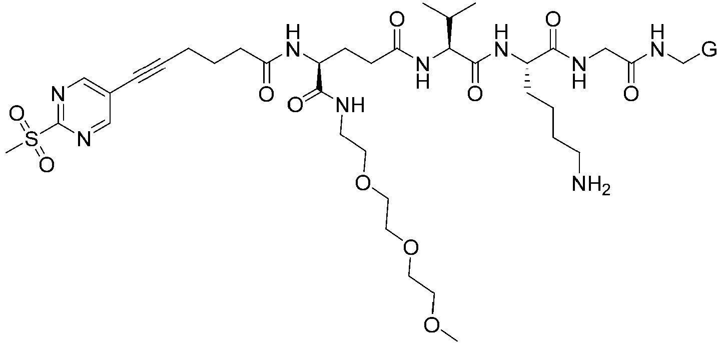 |
| 44 | 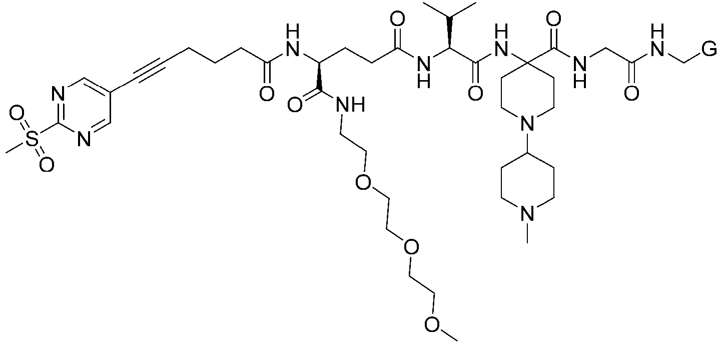 |
| 45 | 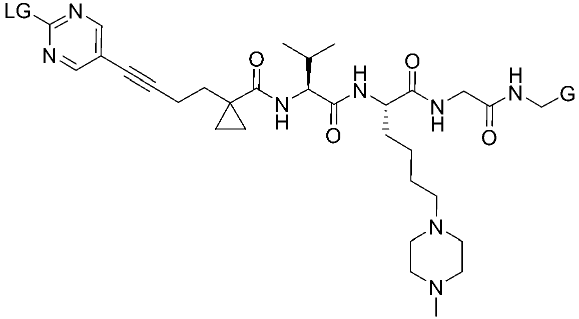 |
| 46 | 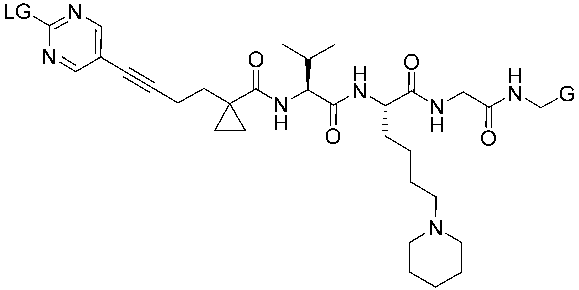 |
| 47 | 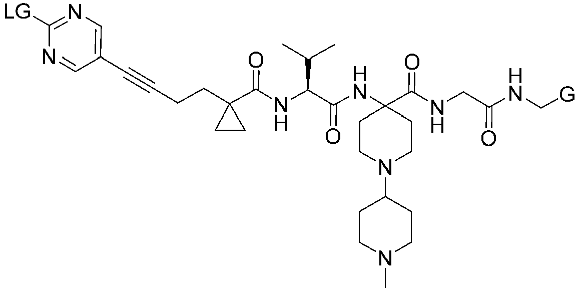 |
| 48 | 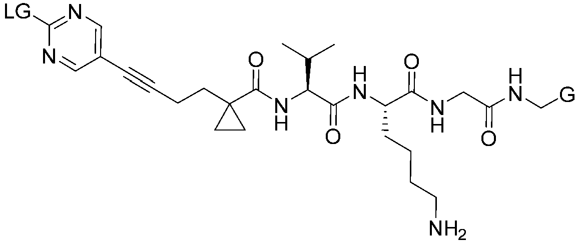 |
| 49 | 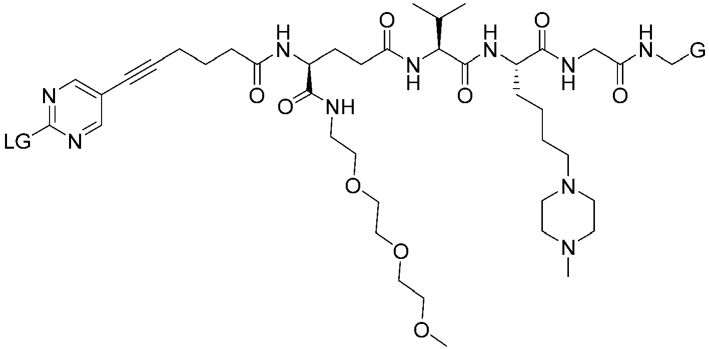 |
| 50 | 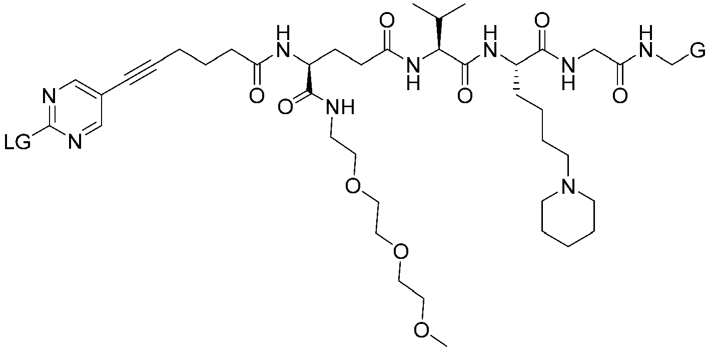 |
| 51 | 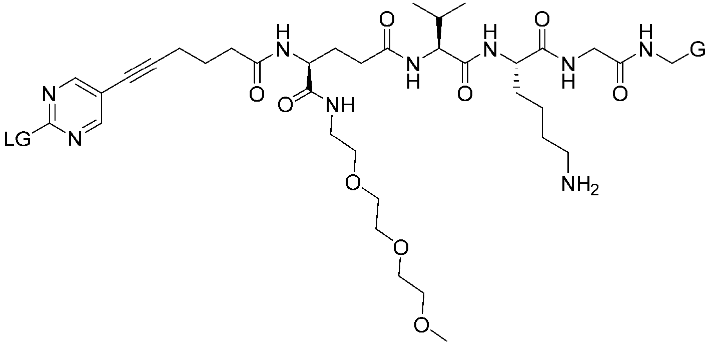 |
| 52 | 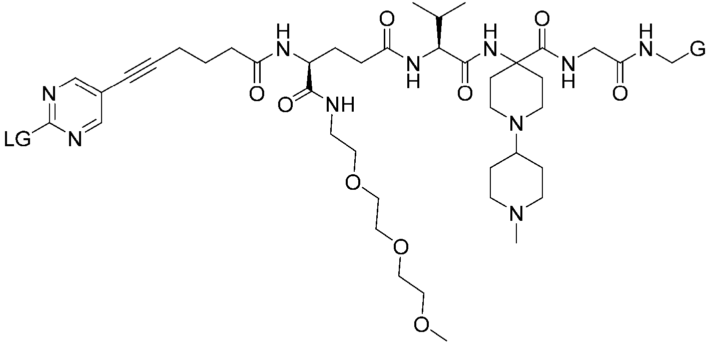 |
| 53 | 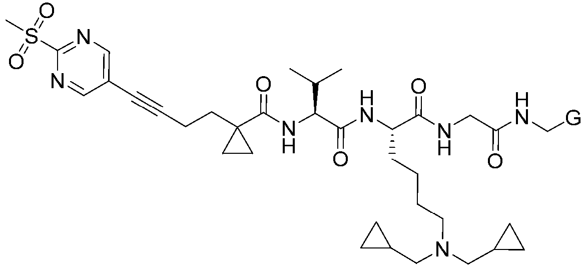 |
| 54 | 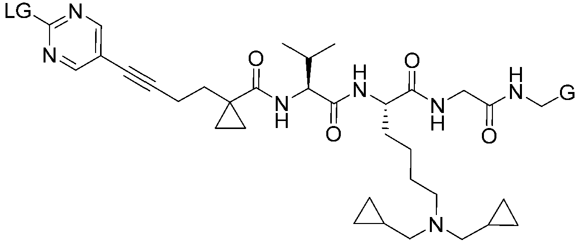 |
| 55 | 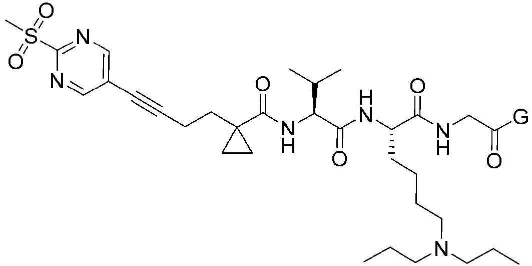 |
| 56 | 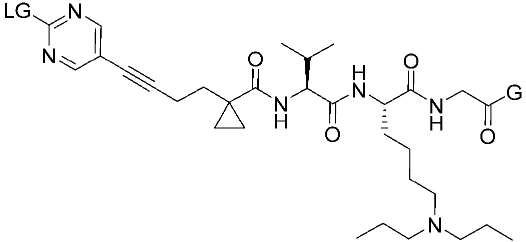 |

According to an embodiment of the present disclosure, the conjugate may have a structure as shown in the following formula: 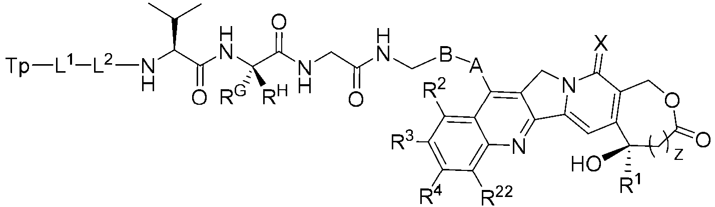 wherein R¹, R², R²², R³, R⁴, R^{G}, R^{H}, A, B, X, Z, L¹, L² and Tp have the definitions as described above.

According to an embodiment of the present disclosure, the conjugate may have a structure as shown in the following formula: 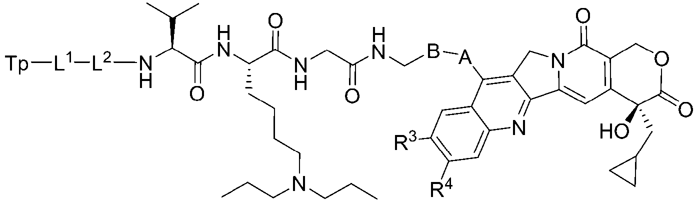 wherein A, B, R³, R⁴, L¹, L² and Tp have the definitions as described above.

According to an embodiment of the present disclosure, the conjugate may have a structure as shown in the following formula: 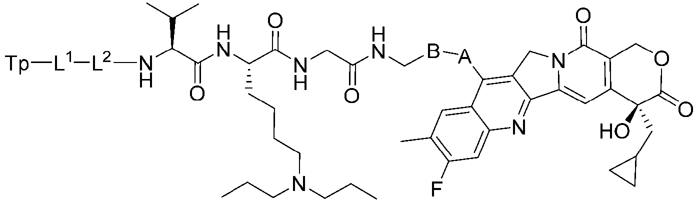 wherein A, B, L¹, L² and Tp have the definitions as described above.

According to an embodiment of the present disclosure, the ligand-drug conjugate represented by formula (C) may be further schematized as the following structure of formula (CA):

Tp-(L-G)y (C_{A})

wherein Tp, L and G are as defined previously;
y represents the average number of small molecule drugs attached to each mAb (DAR), which can be selected from an integer or decimal, e.g., an integer or decimal from 1 to 50, an integer or decimal from 1 to 20, or an integer or decimal from 1 to 10.

According to an embodiment of the present disclosure, the conjugate may have a structure as shown by the following formula: 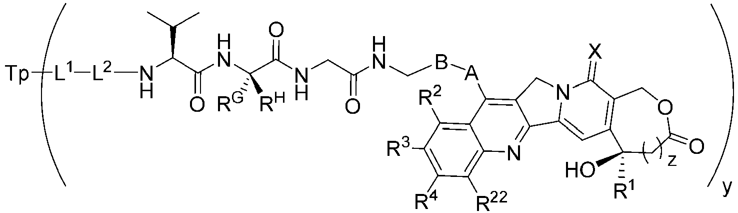 wherein R¹, R², R²², R³, R⁴, R^{G}, R^{H}, A, B, X, Z, L¹, L², Tp and y have the definitions as described above.

According to an embodiment of the present disclosure, the conjugate may have a structure as shown by the following formula: 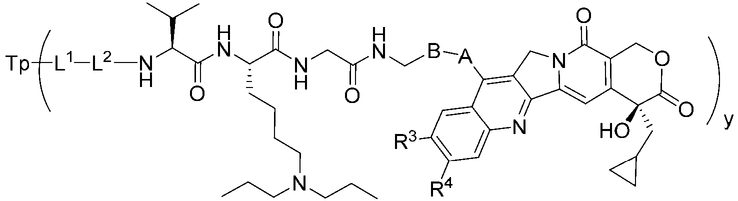 wherein A, B, R³, R⁴, L¹, L², Tp and y have the definitions as described above.

According to an embodiment of the present disclosure, the conjugate may have a structure as shown by the following formula: 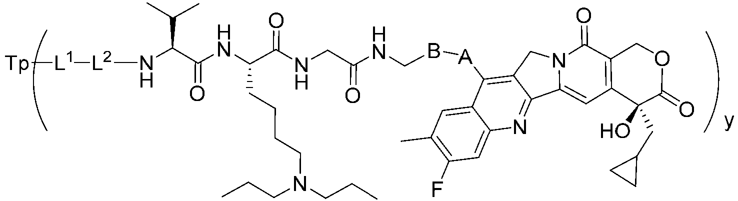 wherein A, B, L1, L², Tp and y have the definitions as described above.

According to an embodiment of the present disclosure, the conjugate may be selected from one of the following: 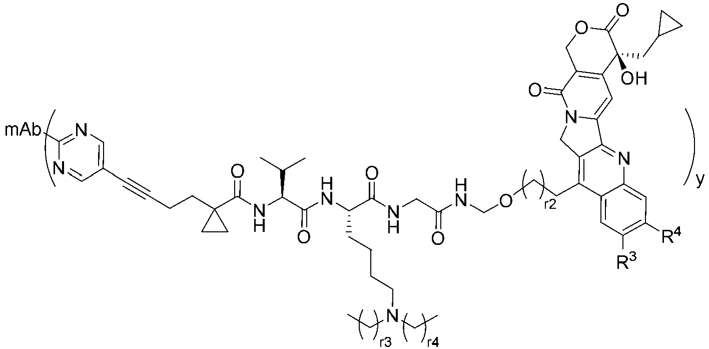 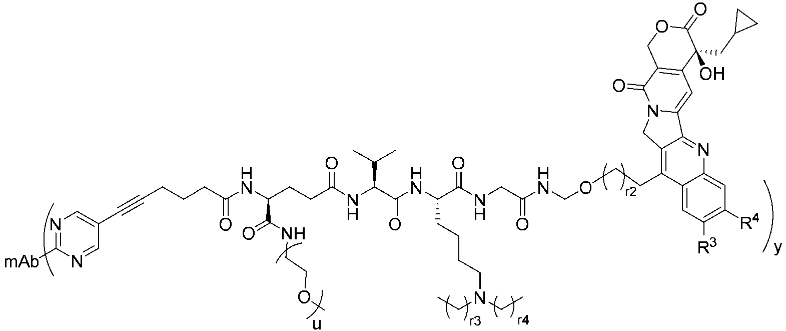 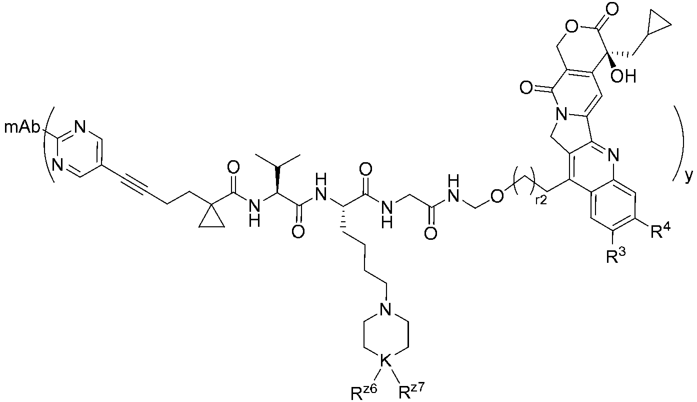 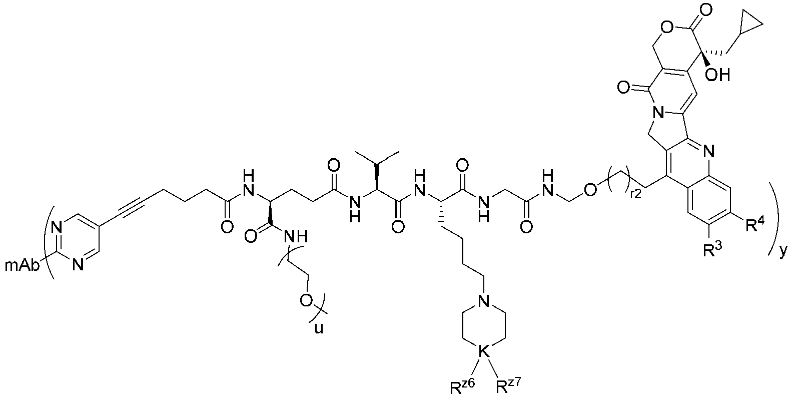 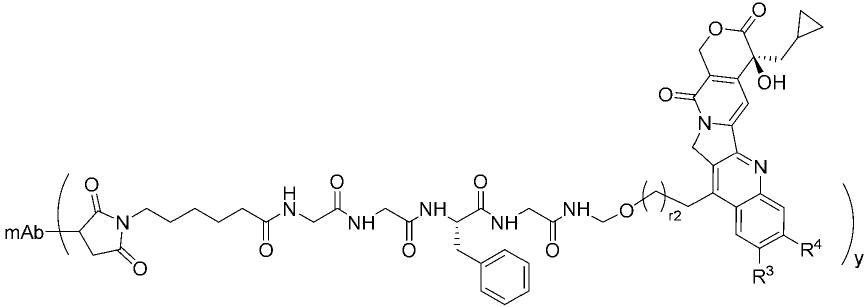 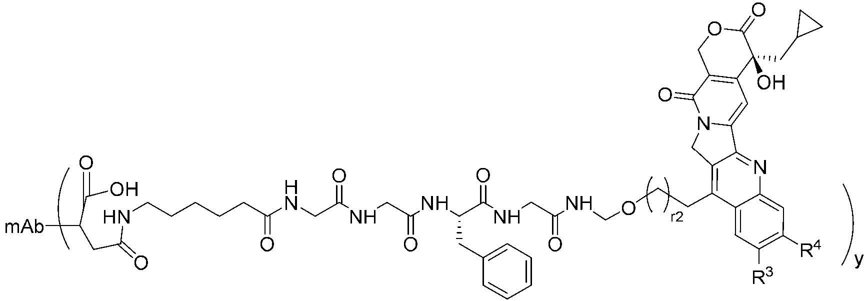 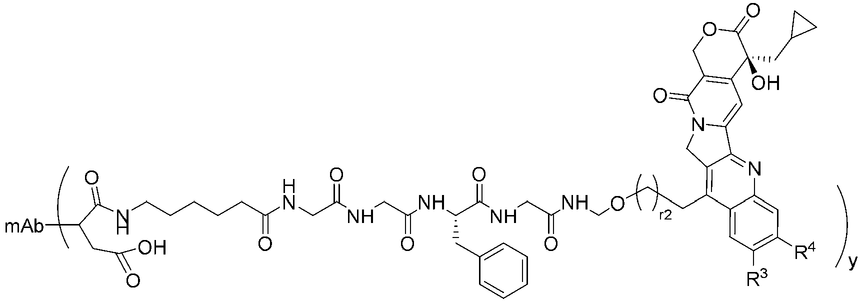 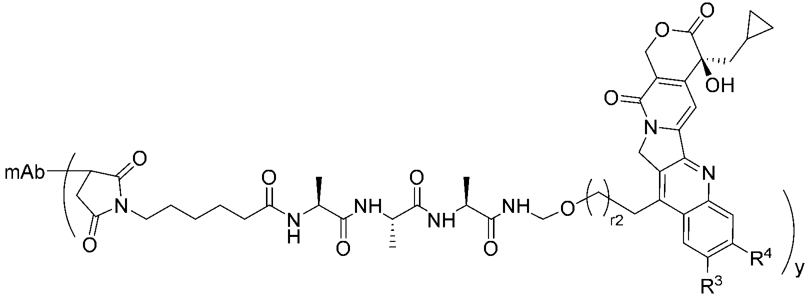 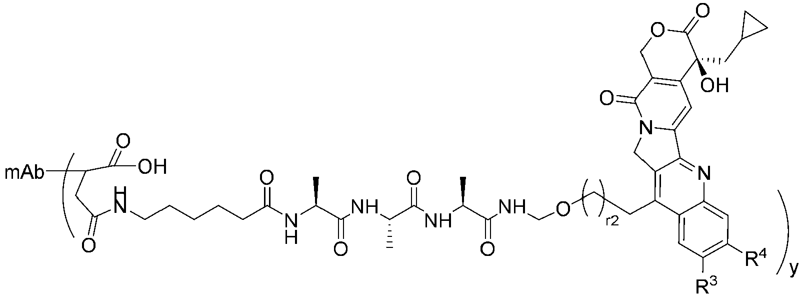 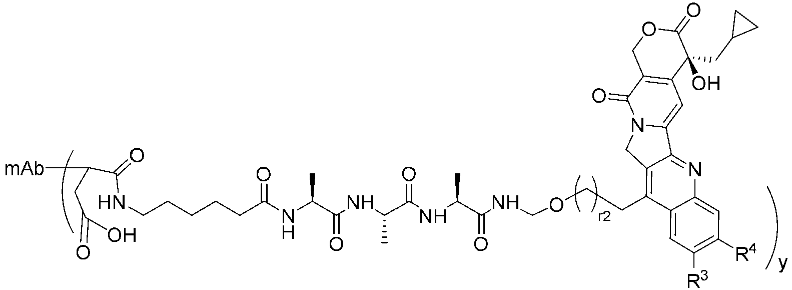 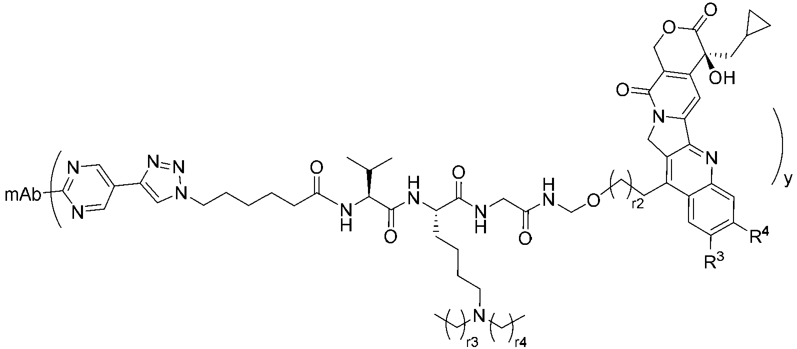 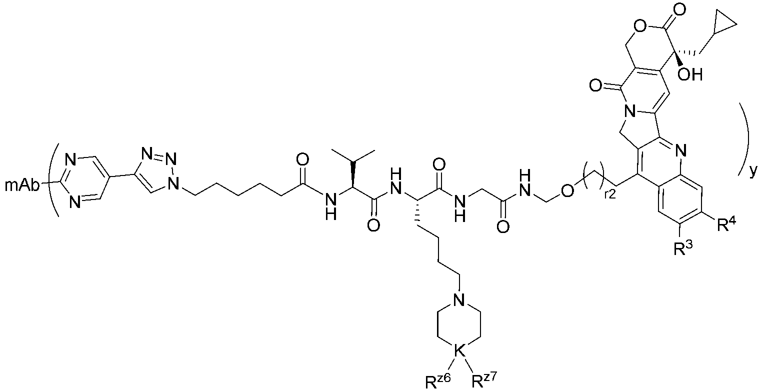 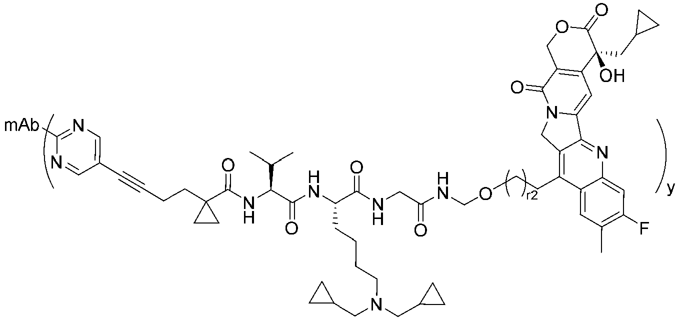
wherein R³ and R⁴ have the definitions as described above;
u is an integer selected from 0 to 10;
r2 is an integer selected from 0 to 4 and may be 0, 1, 2, 3, or 4; r3 and r4 are independently an integer selected from 0 to 3 and may be 0, 1, 2 or 3;
K is C or N.
R^{z6} and R^{z7} are identical or different, independently selected from hydrogen, amino, alkylamino or dialkylamino, alkyl, alkyloxy, cycloalkyl, cycloalkylalkyl or cycloalkyloxy;
alternatively, R^{z6}, R^{z7} together with the atoms to which they are attached form a ring structure; preferably, form a 5-6 membered heterocyclyl optionally substituted with a C₁₋₄ alkyl, said 5-6 membered heterocyclyl preferably being piperidinyl or piperazinyl;
mAb represents a monoclonal antibody;
y represents the average number of small molecule drugs attached to each mAb (DAR), which may be selected from an integer or decimal, e.g., an integer or decimal from 1 to 50, an integer or decimal from 1 to 20, or an integer or decimal from 1 to 10.

According to an embodiment of the present disclosure, an example of the linker-drug conjugate may be selected from one of the following:

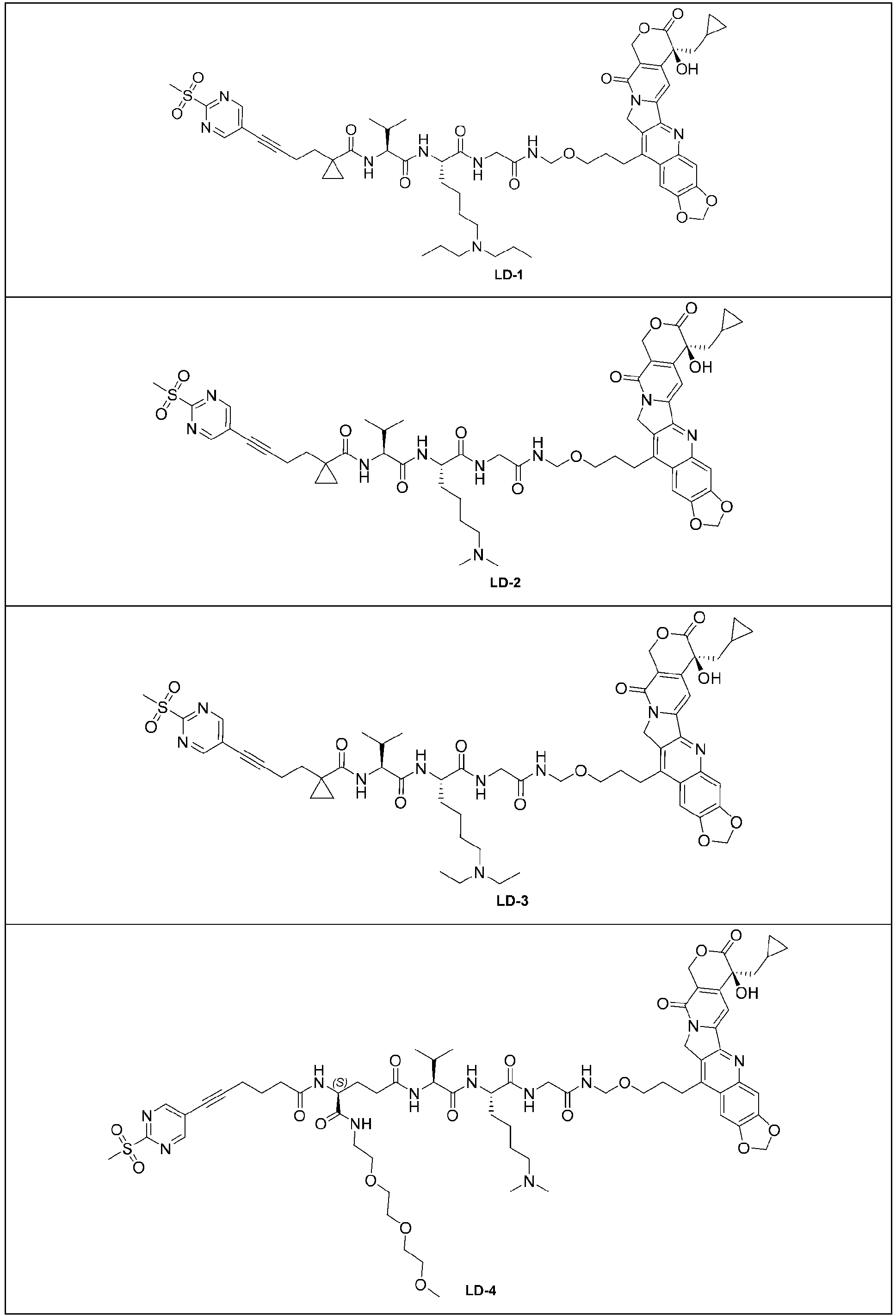

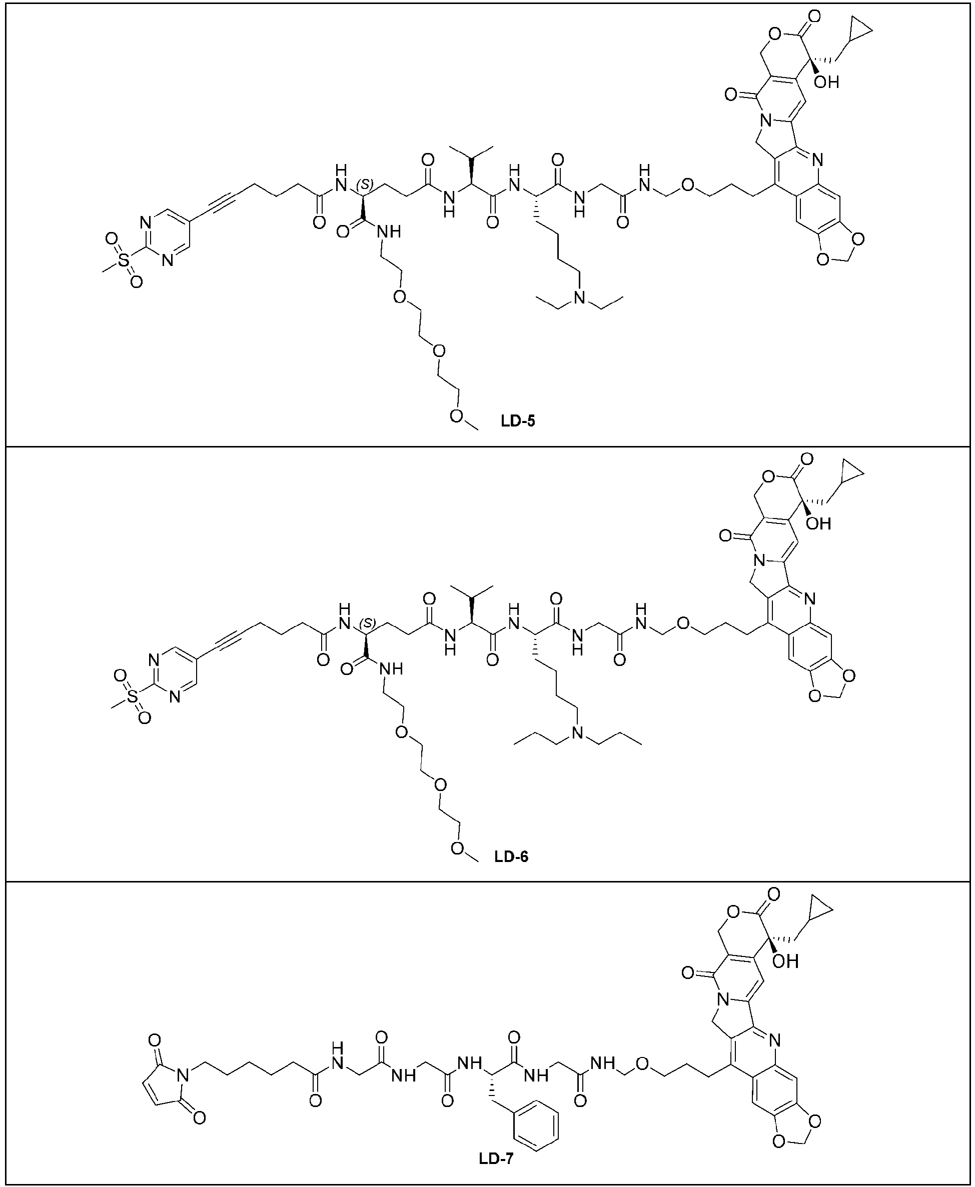

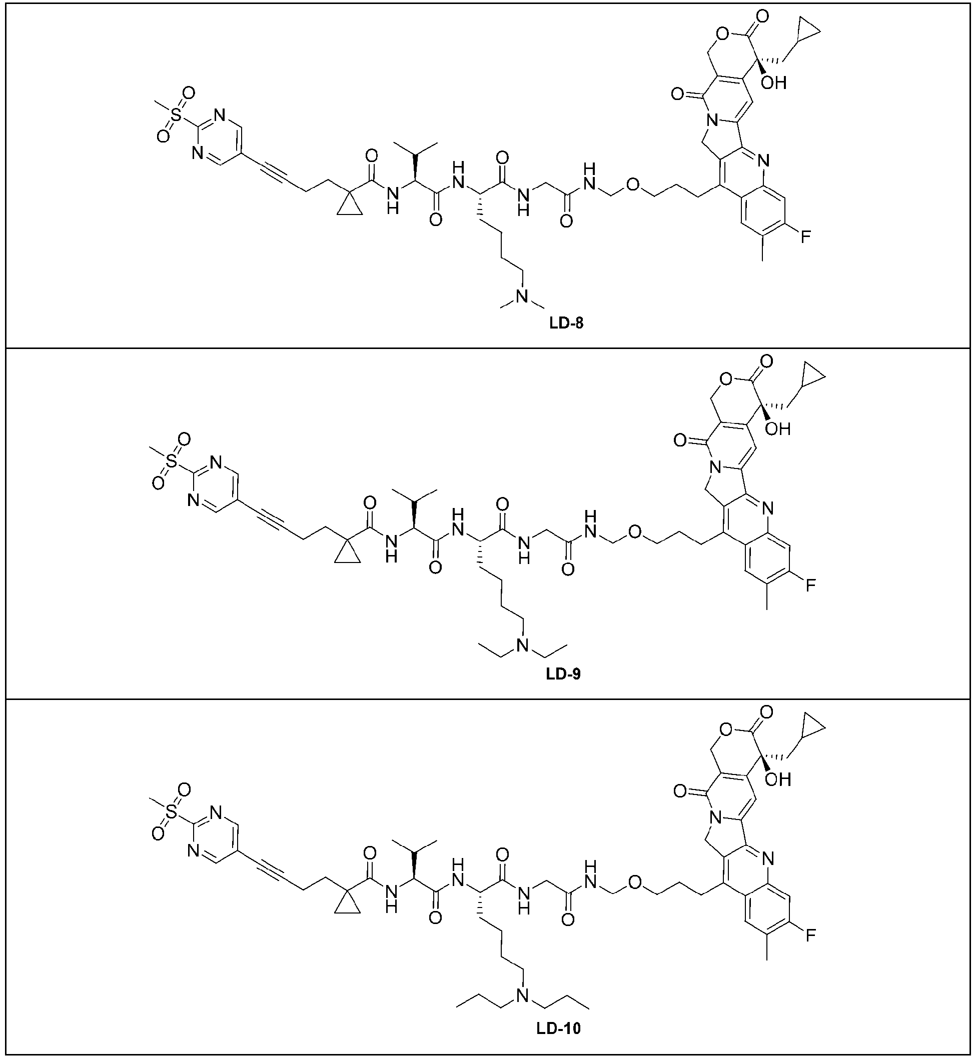

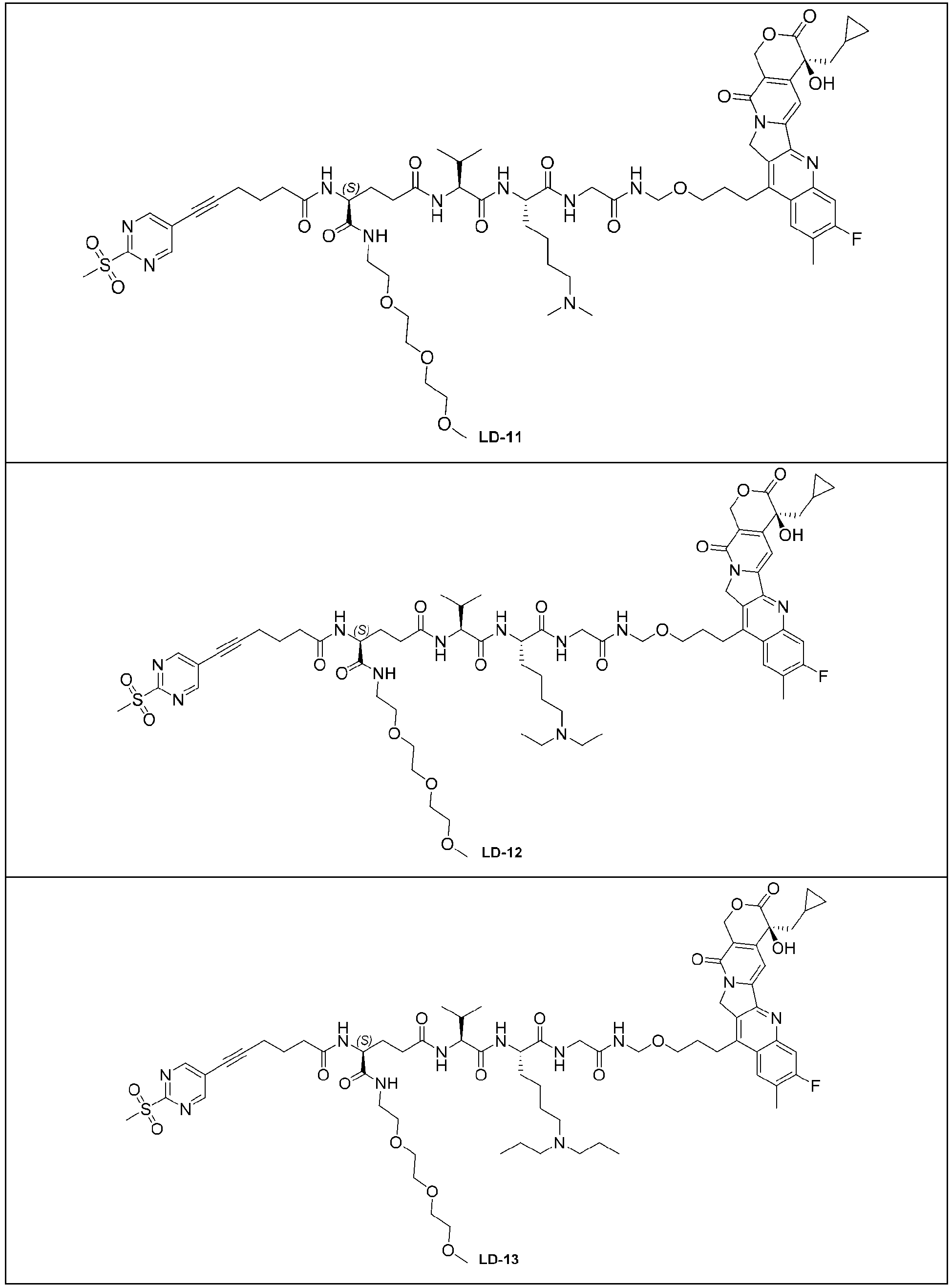

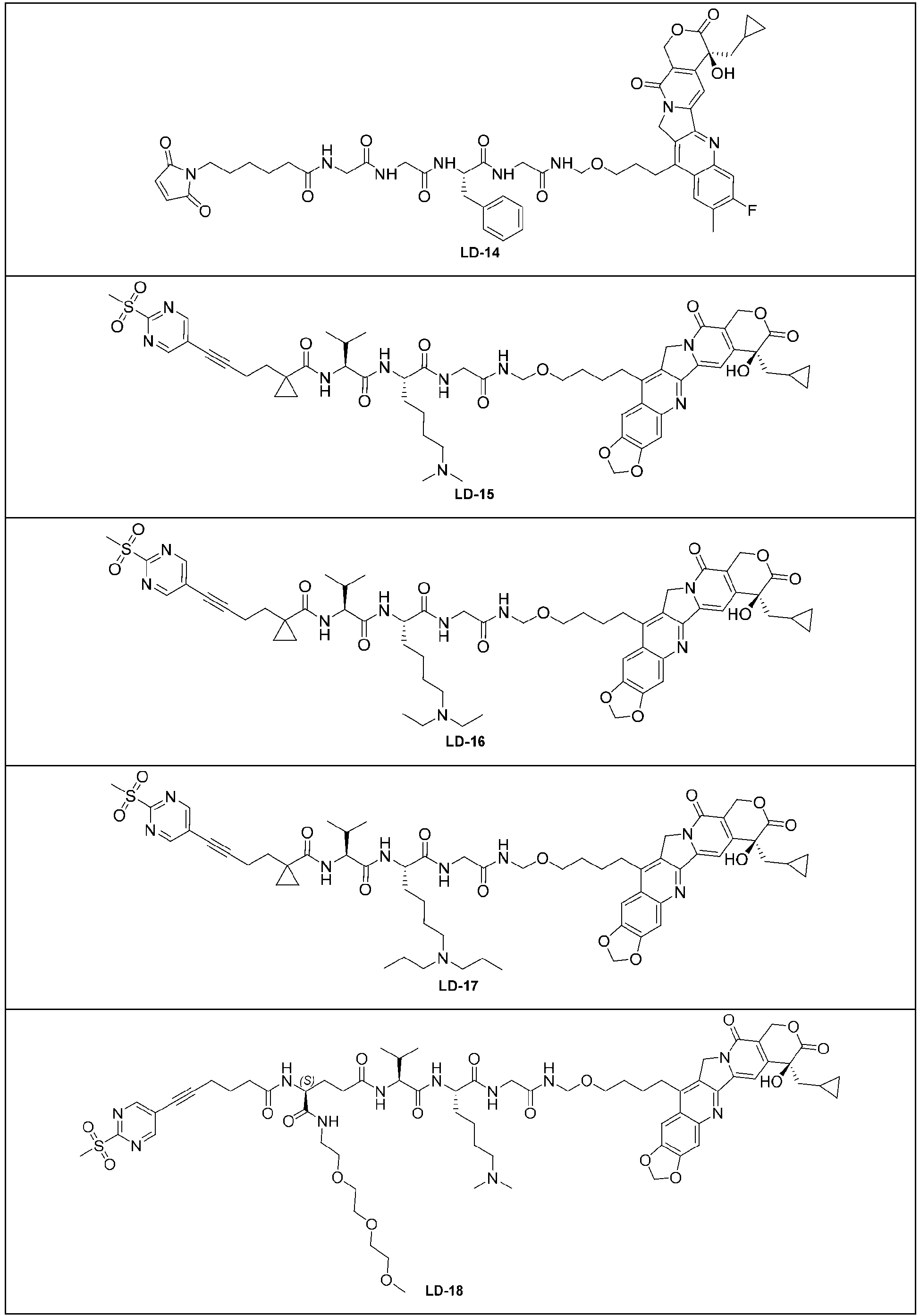

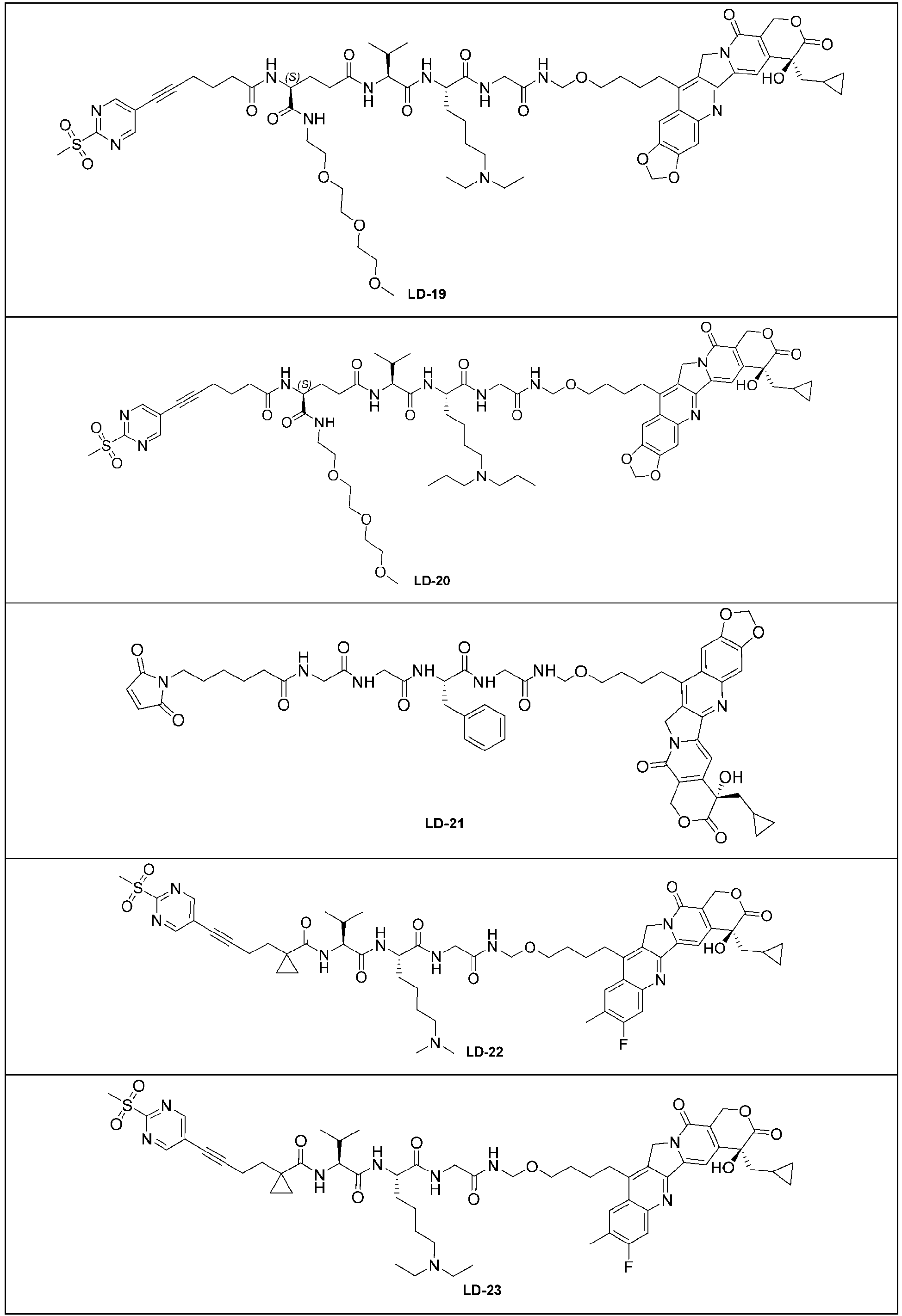

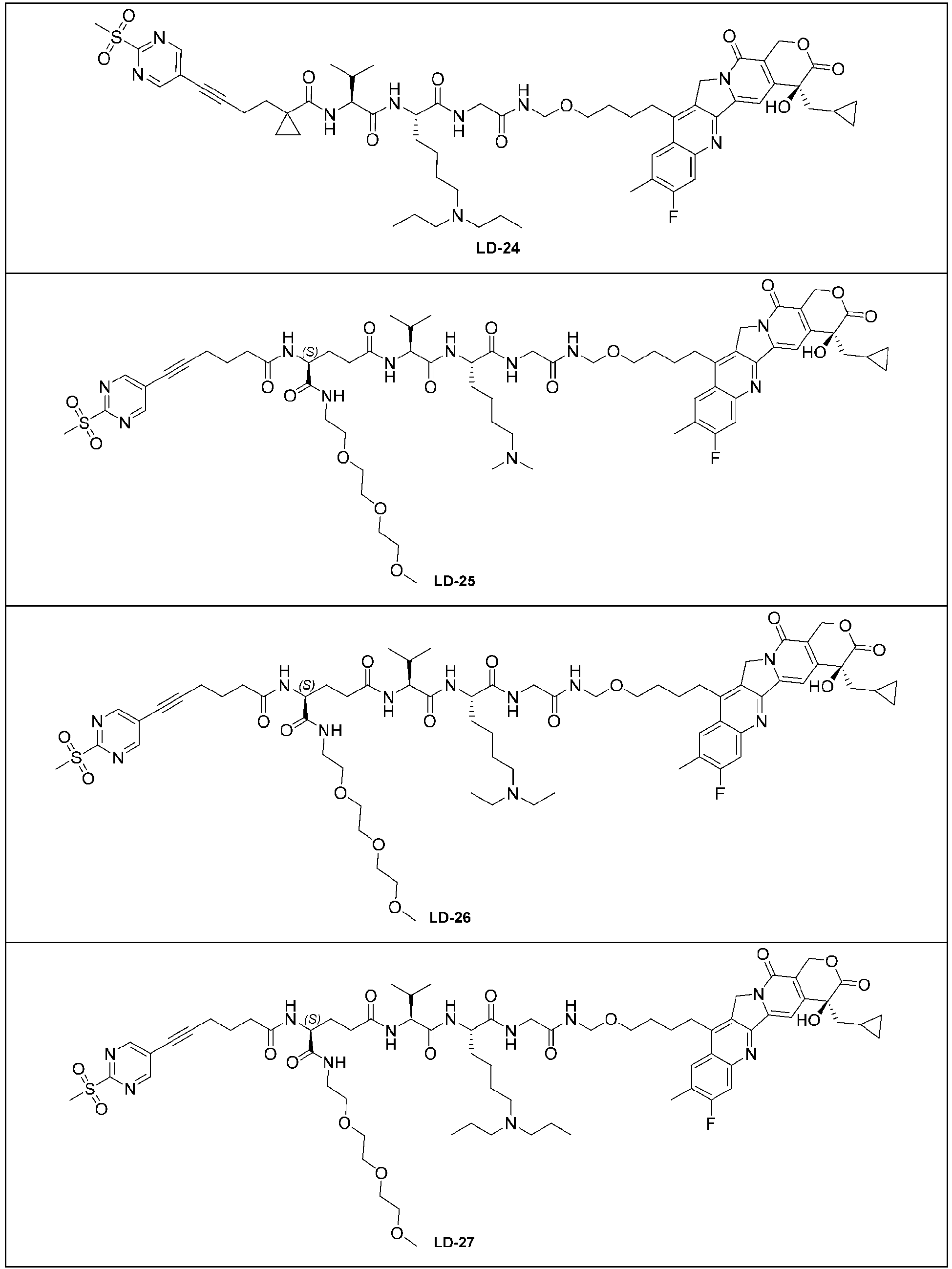

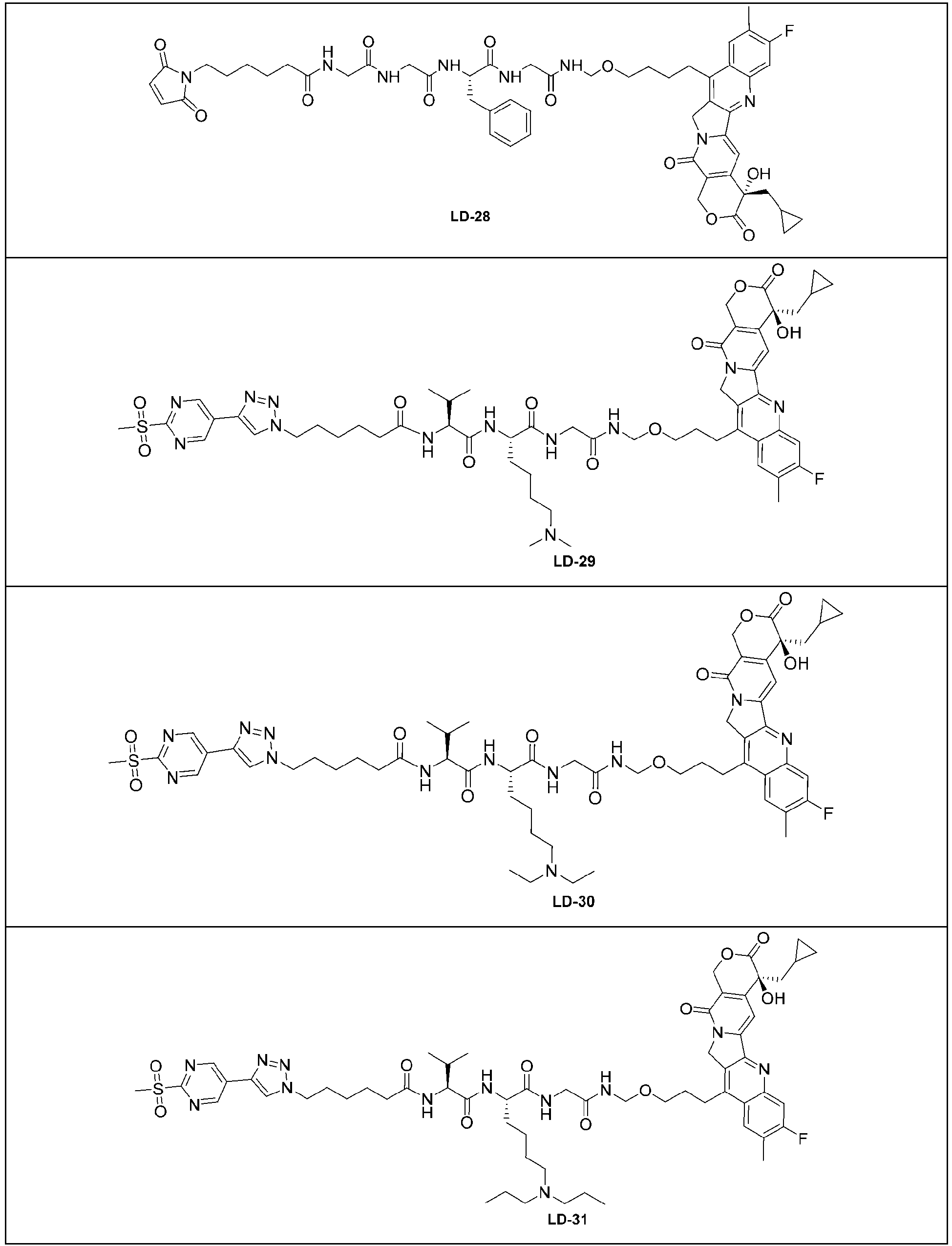

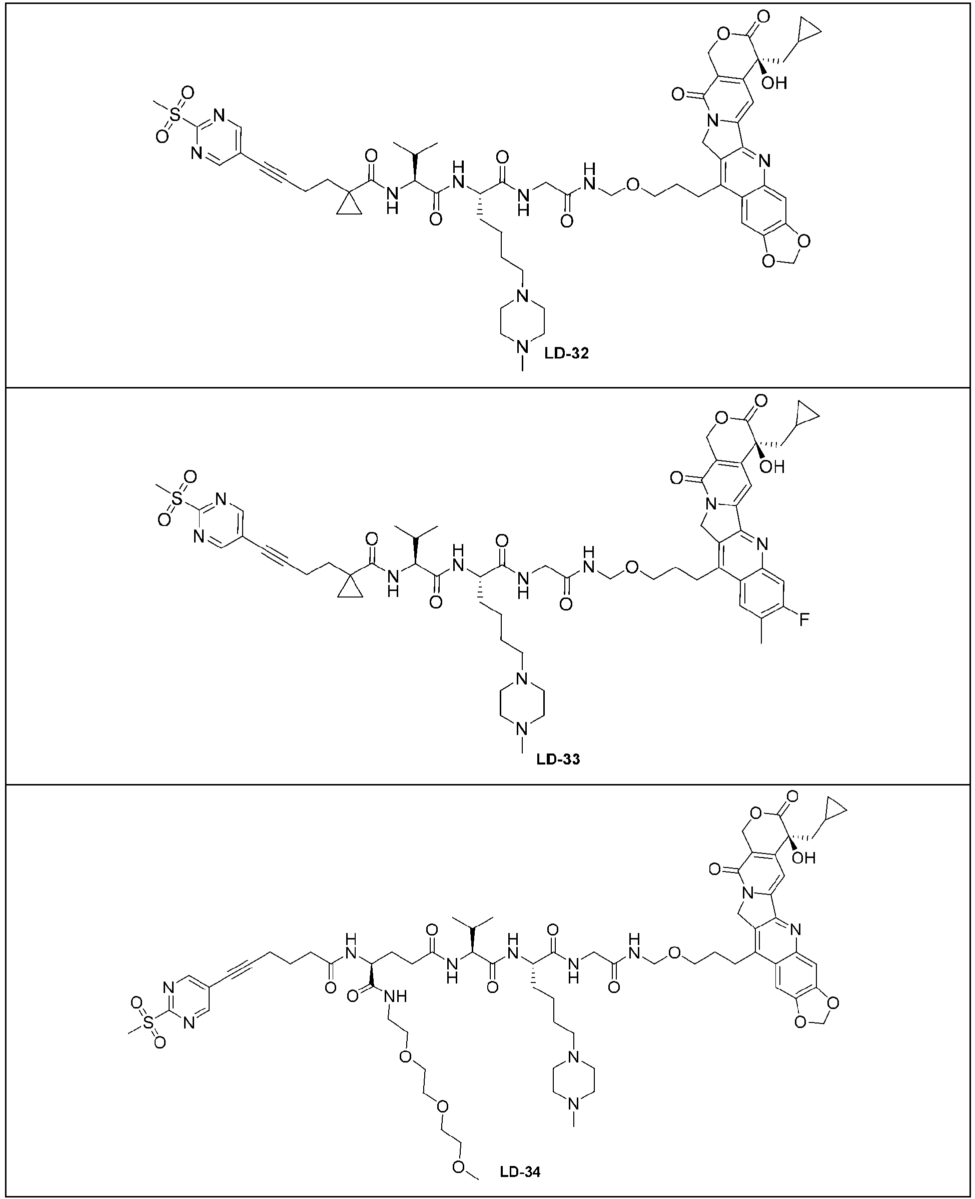

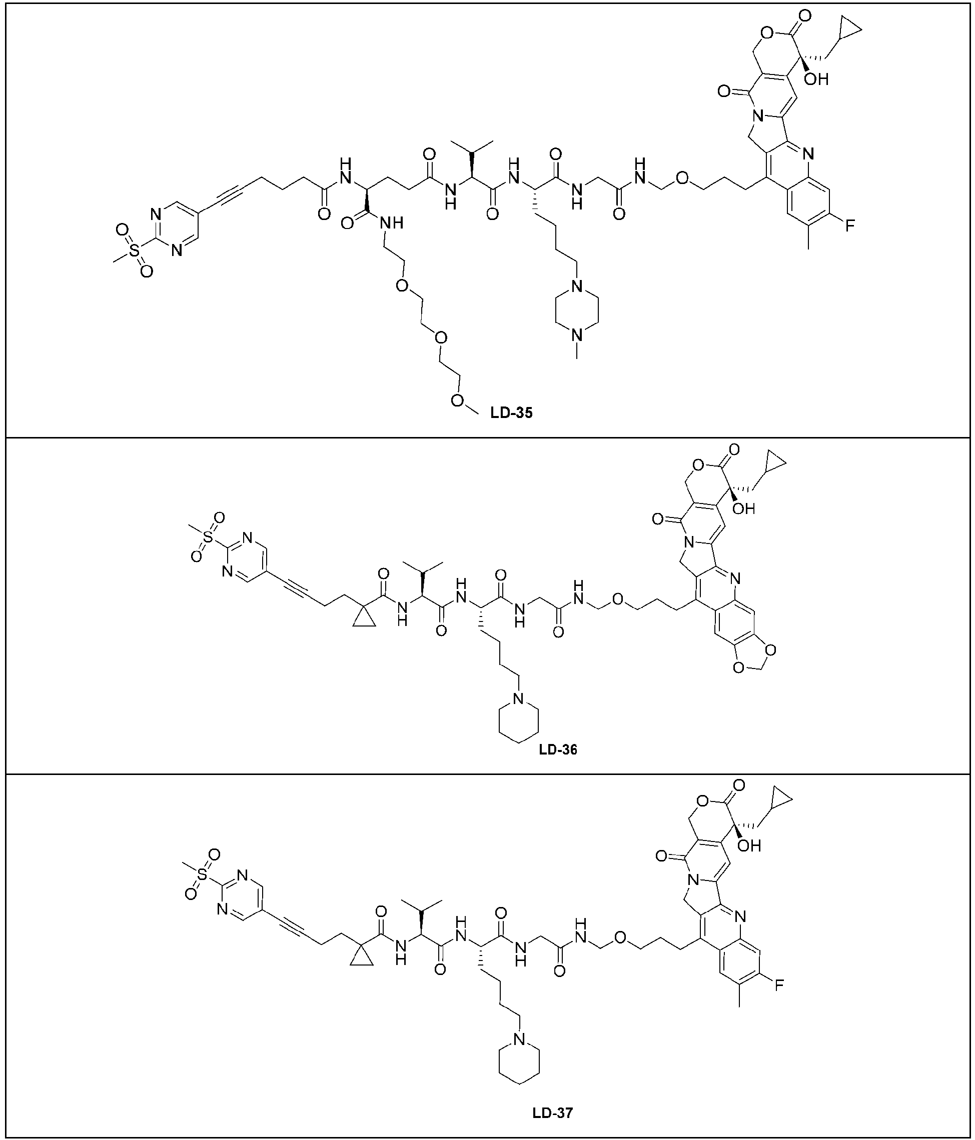

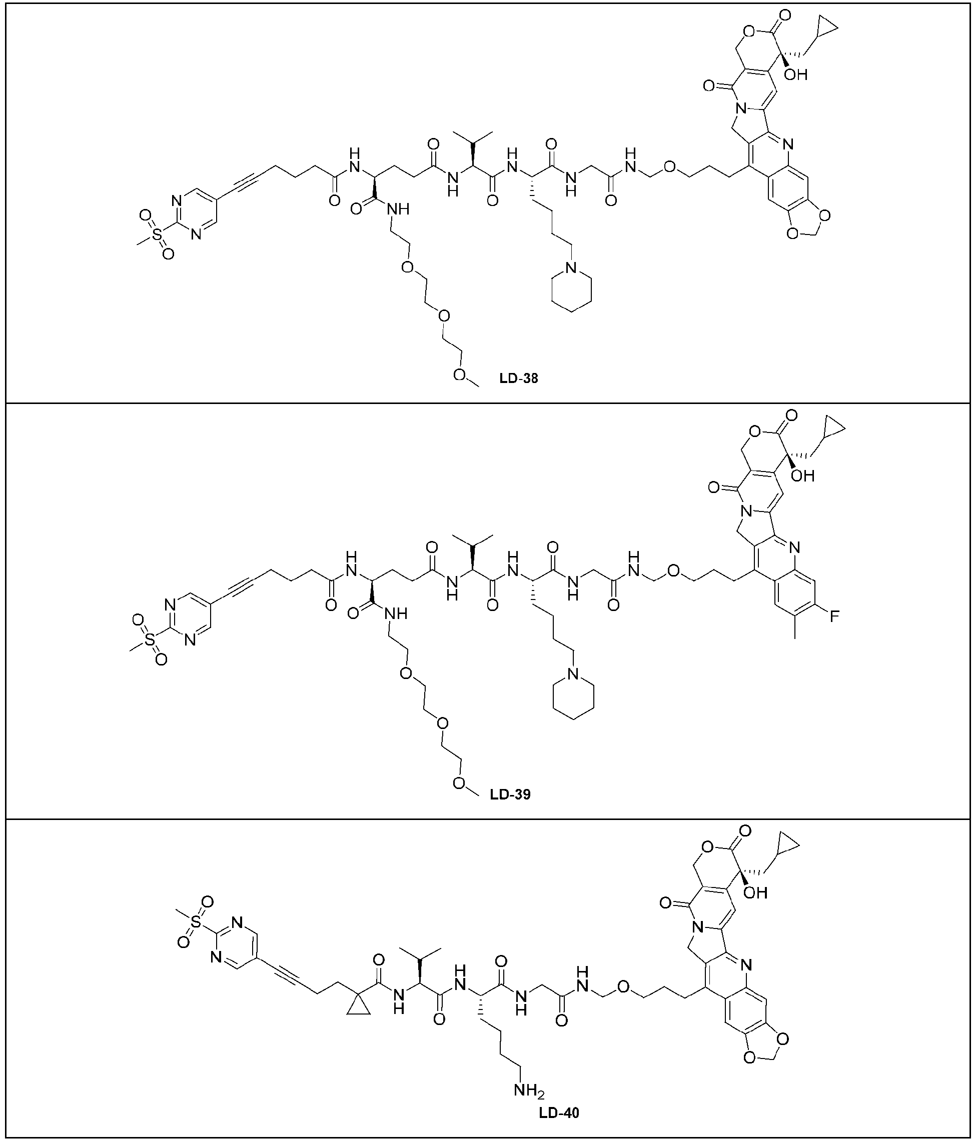

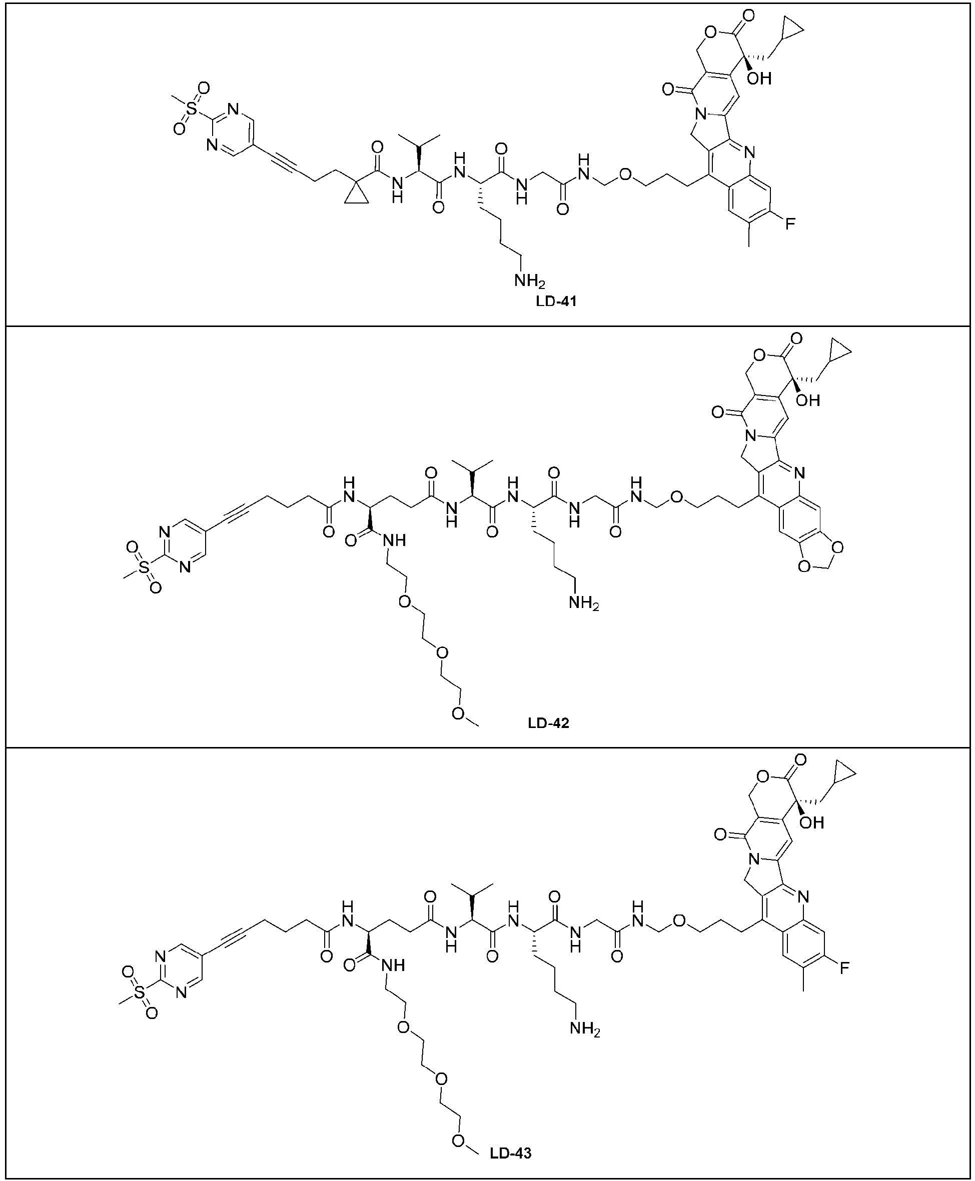

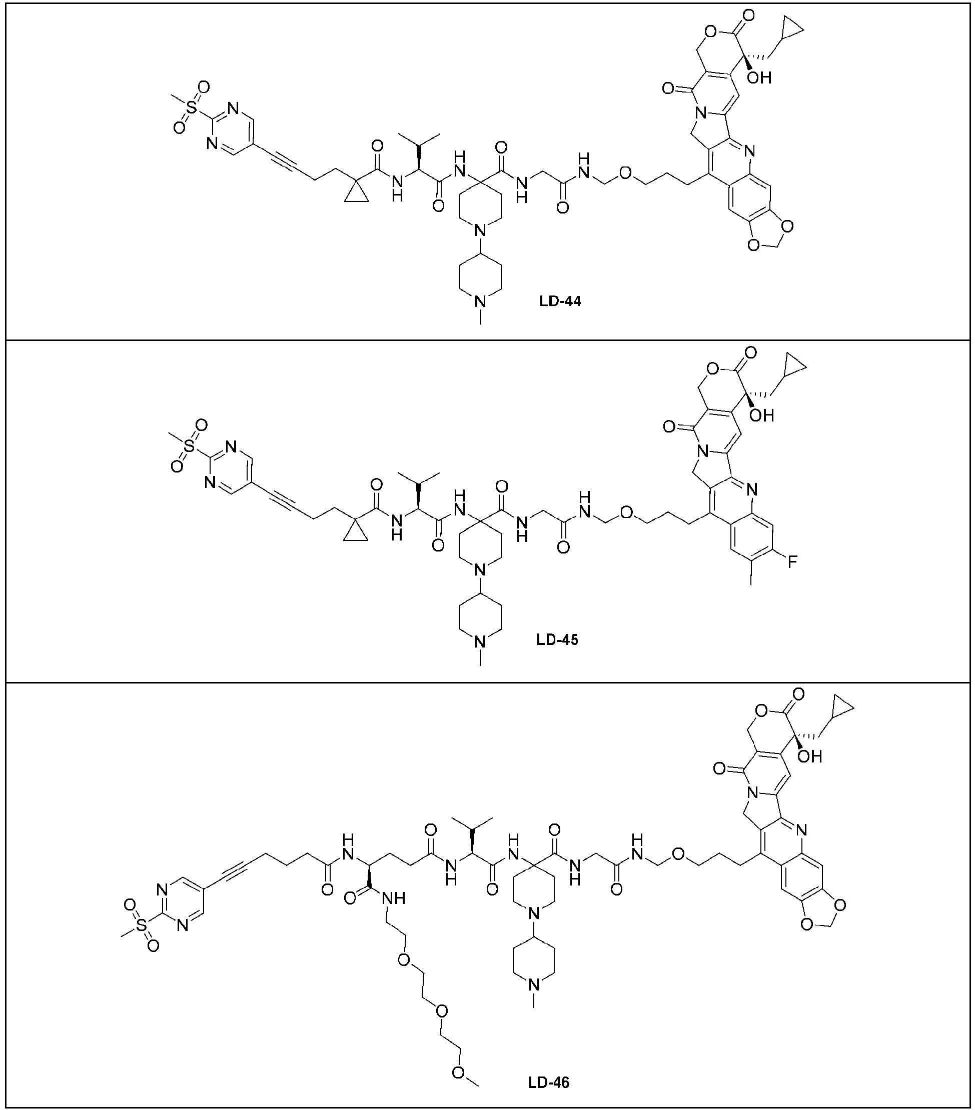

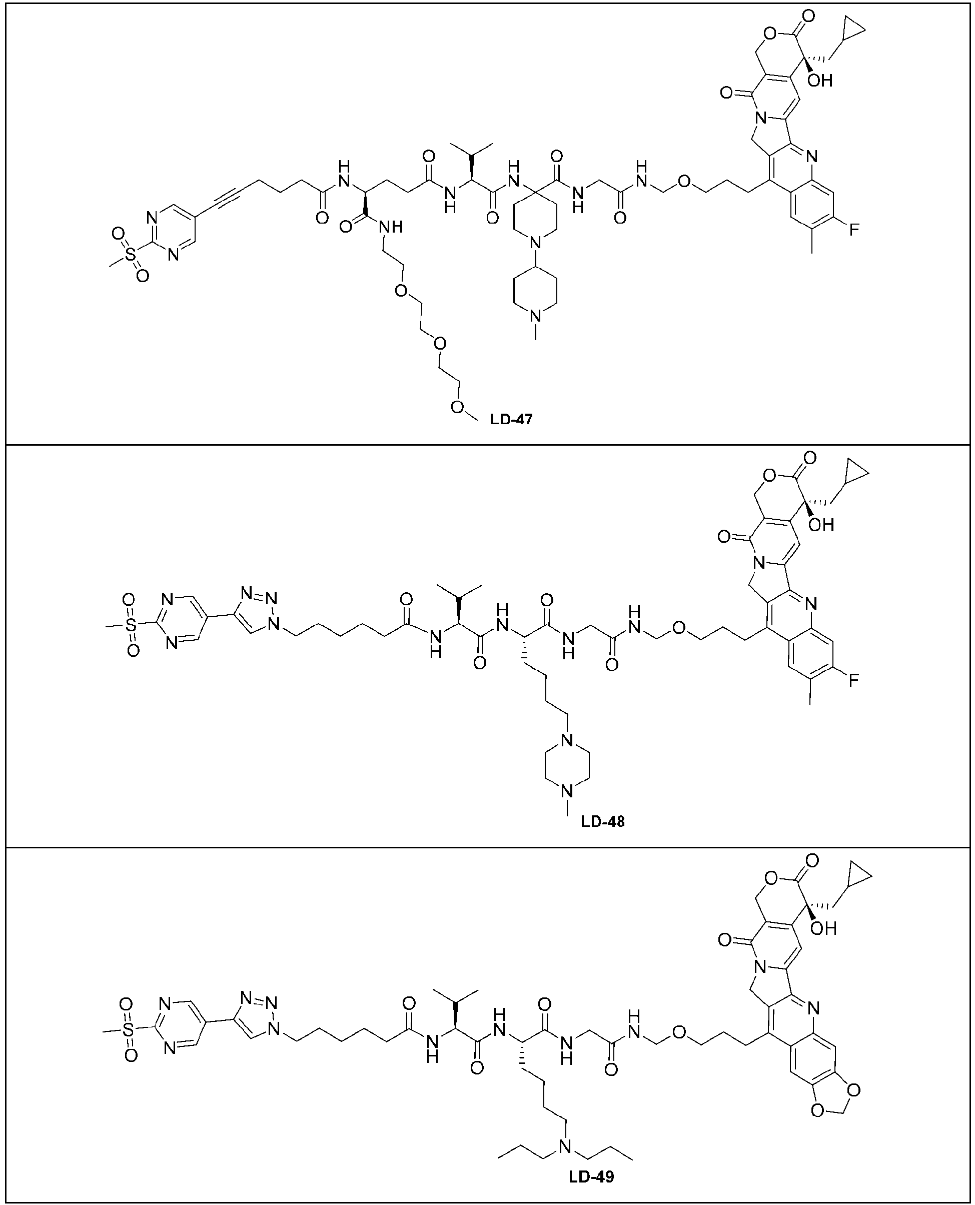

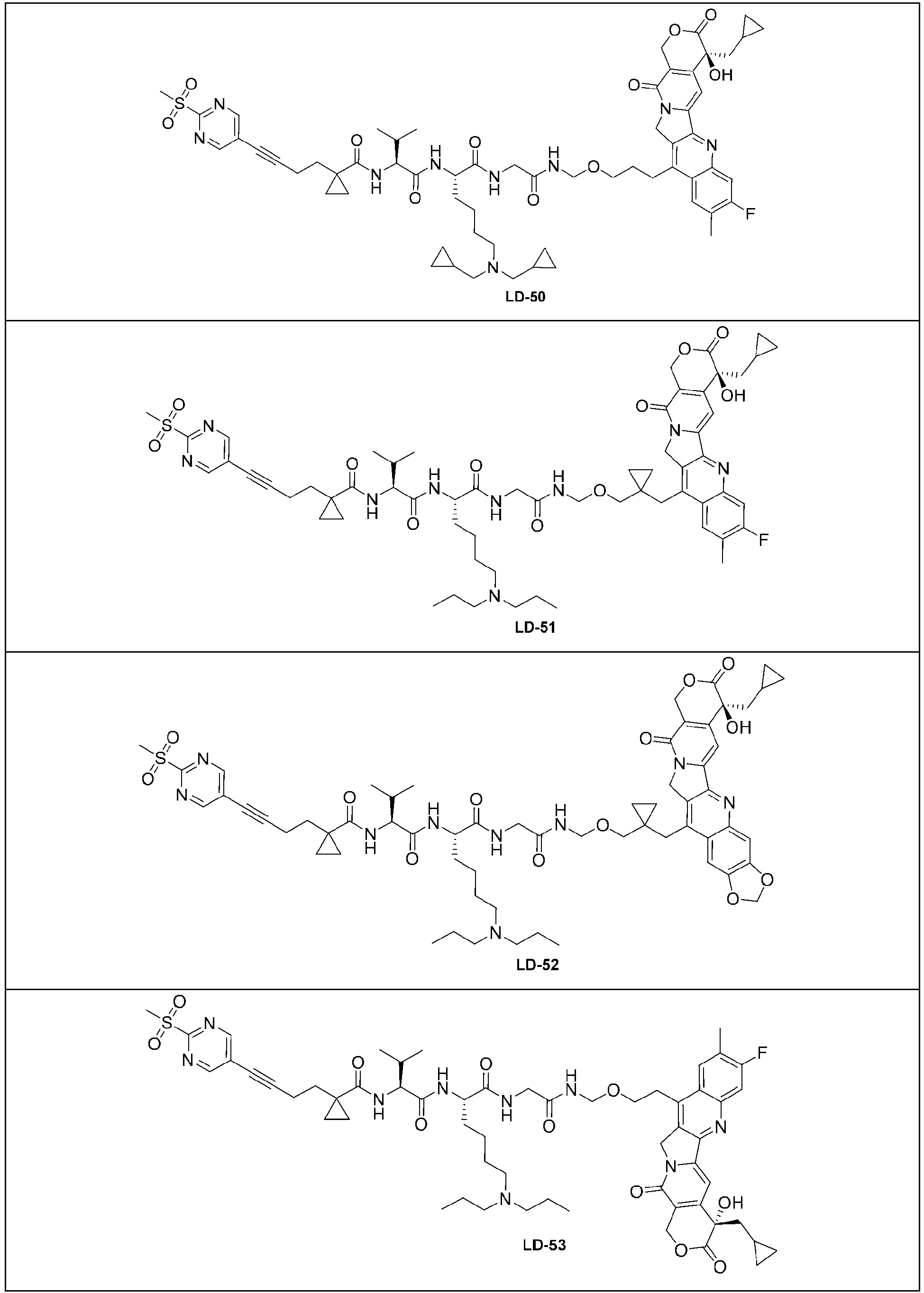

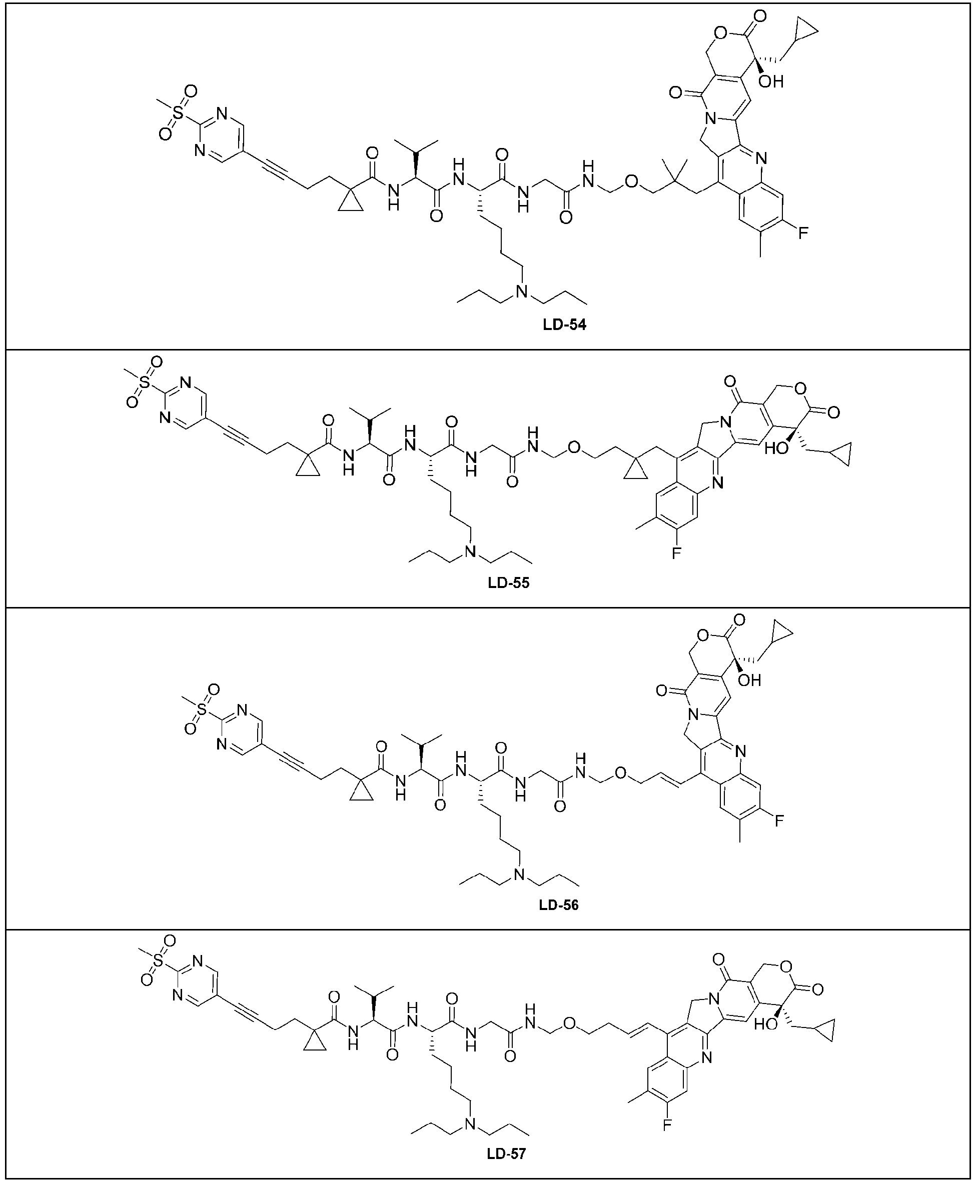

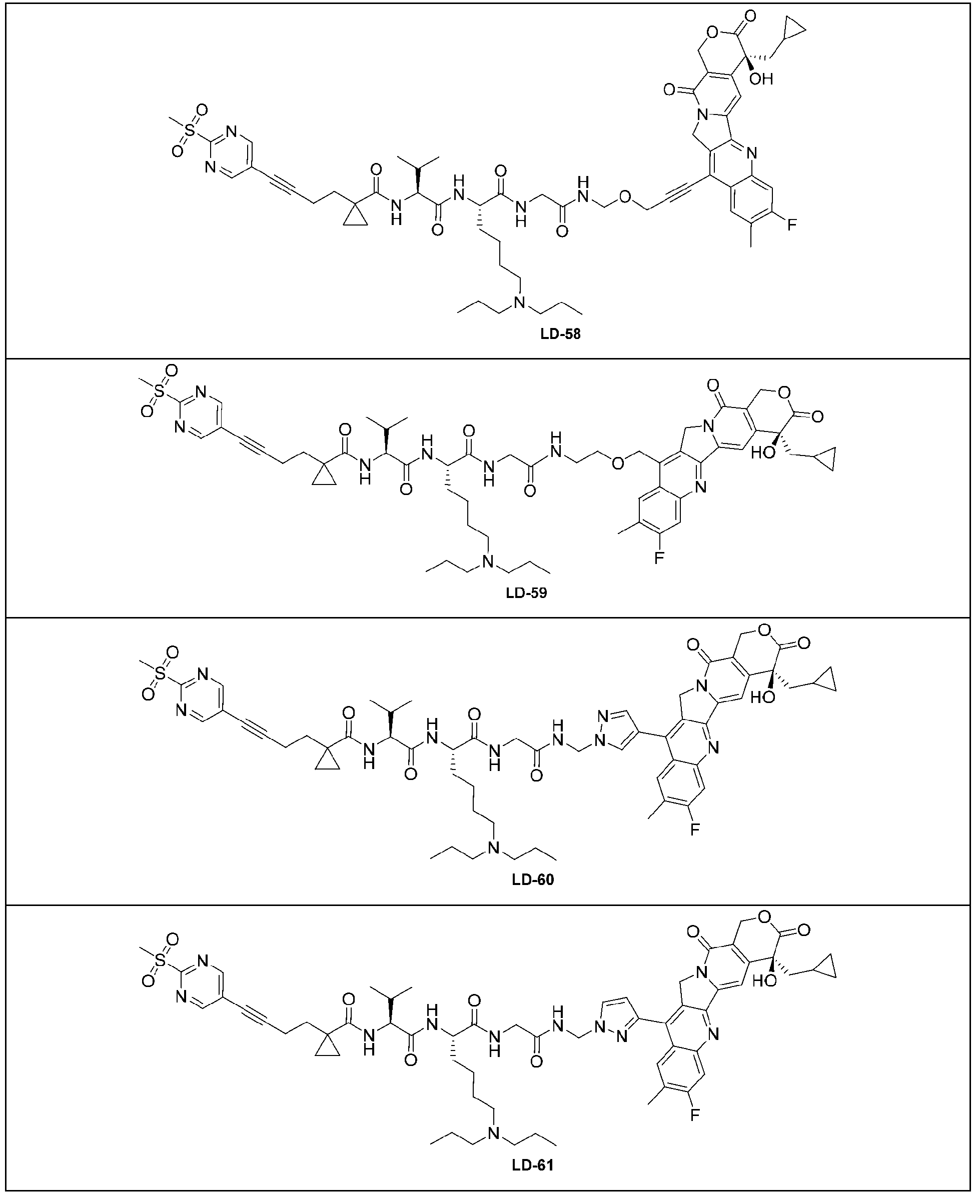

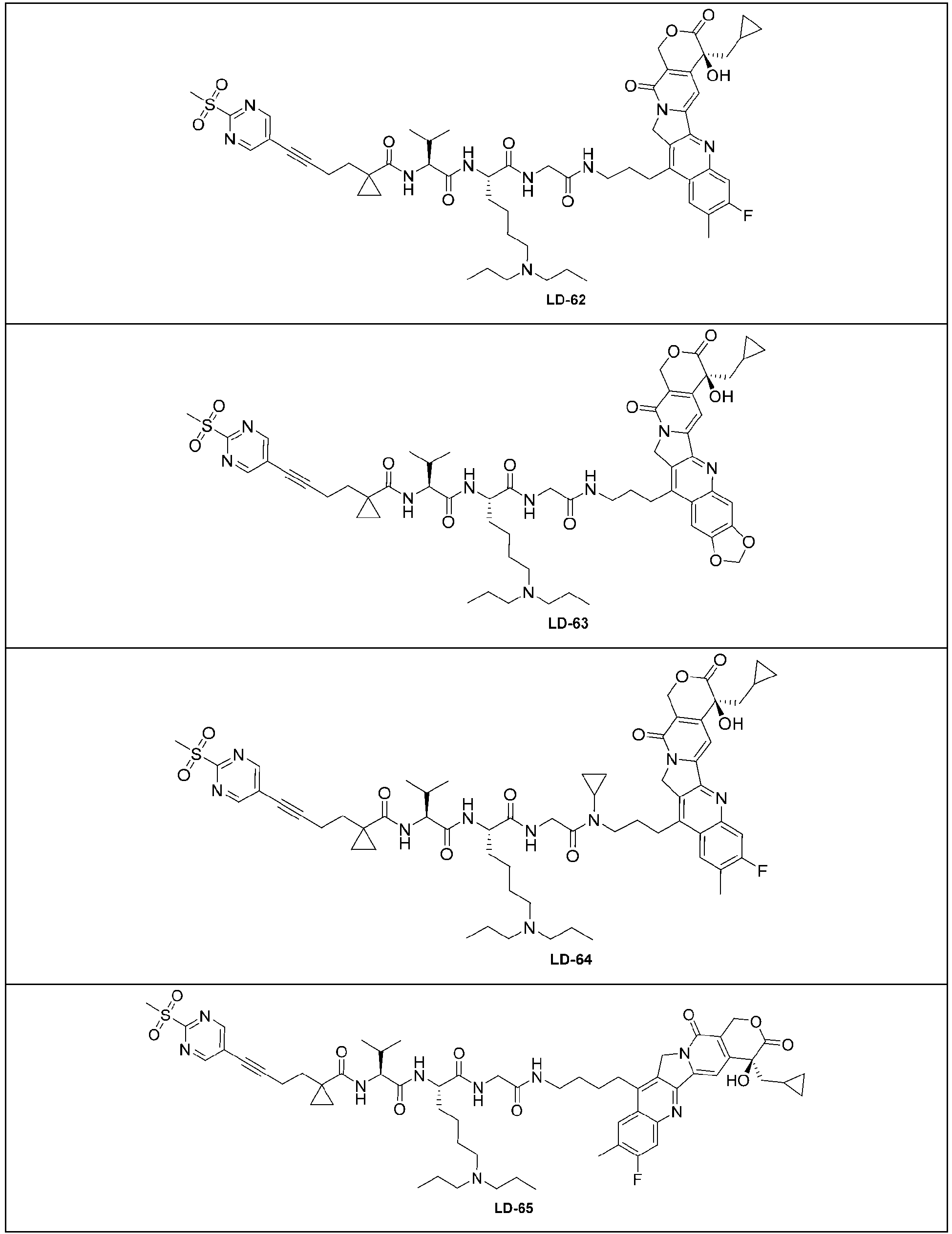

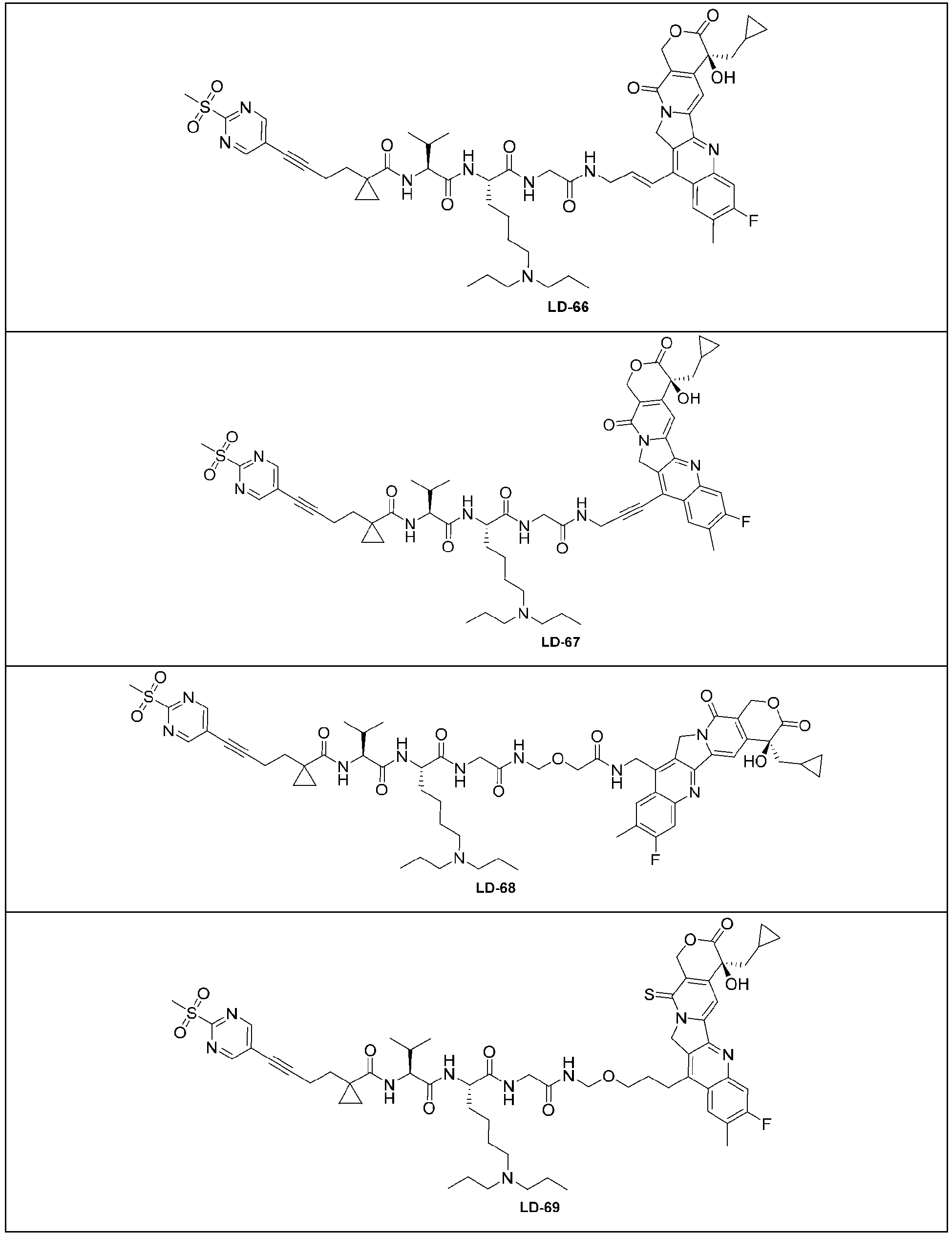

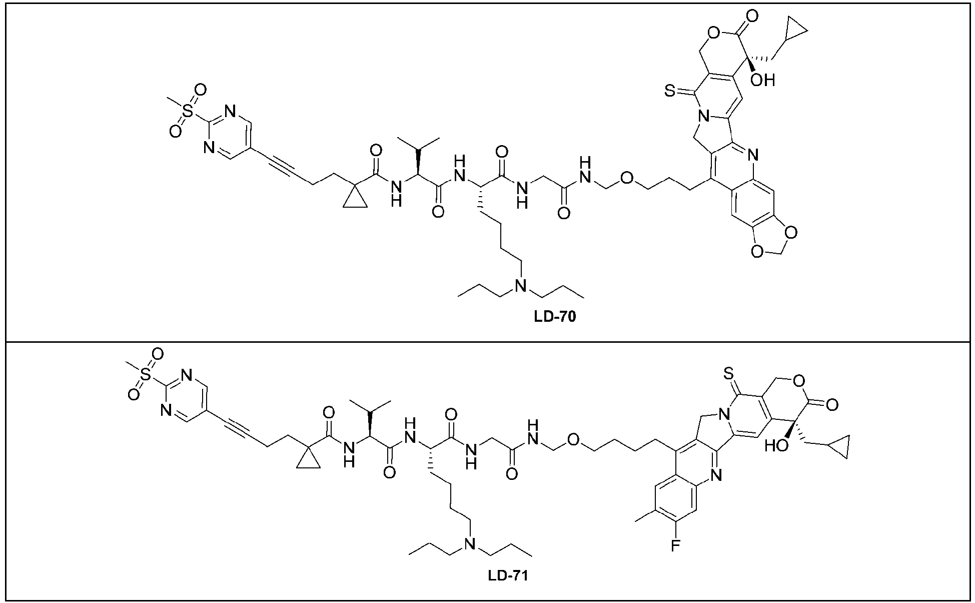

According to an embodiment of the present disclosure, an example of the conjugate may be selected from one of the following, wherein:
y represents the average number of small molecule drugs attached to each monoclonal antibody (DAR), which may be selected from an integer or decimal, e.g. an integer or decimal selected from 1 to 50, an integer or decimal selected from 1 to 20, or an integer or decimal selected from 1 to 10;

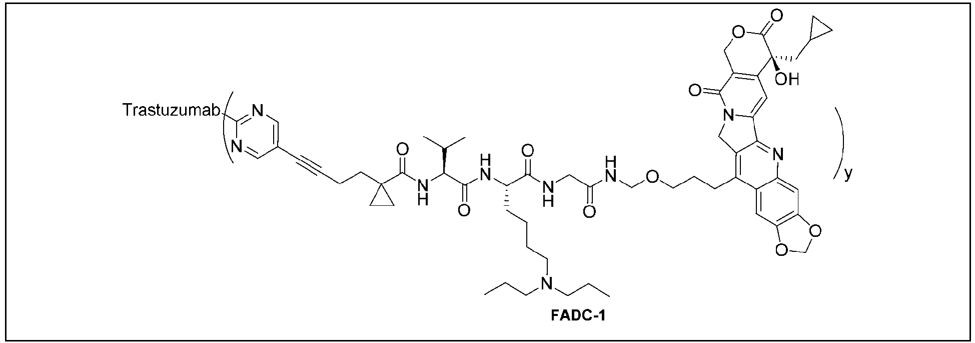

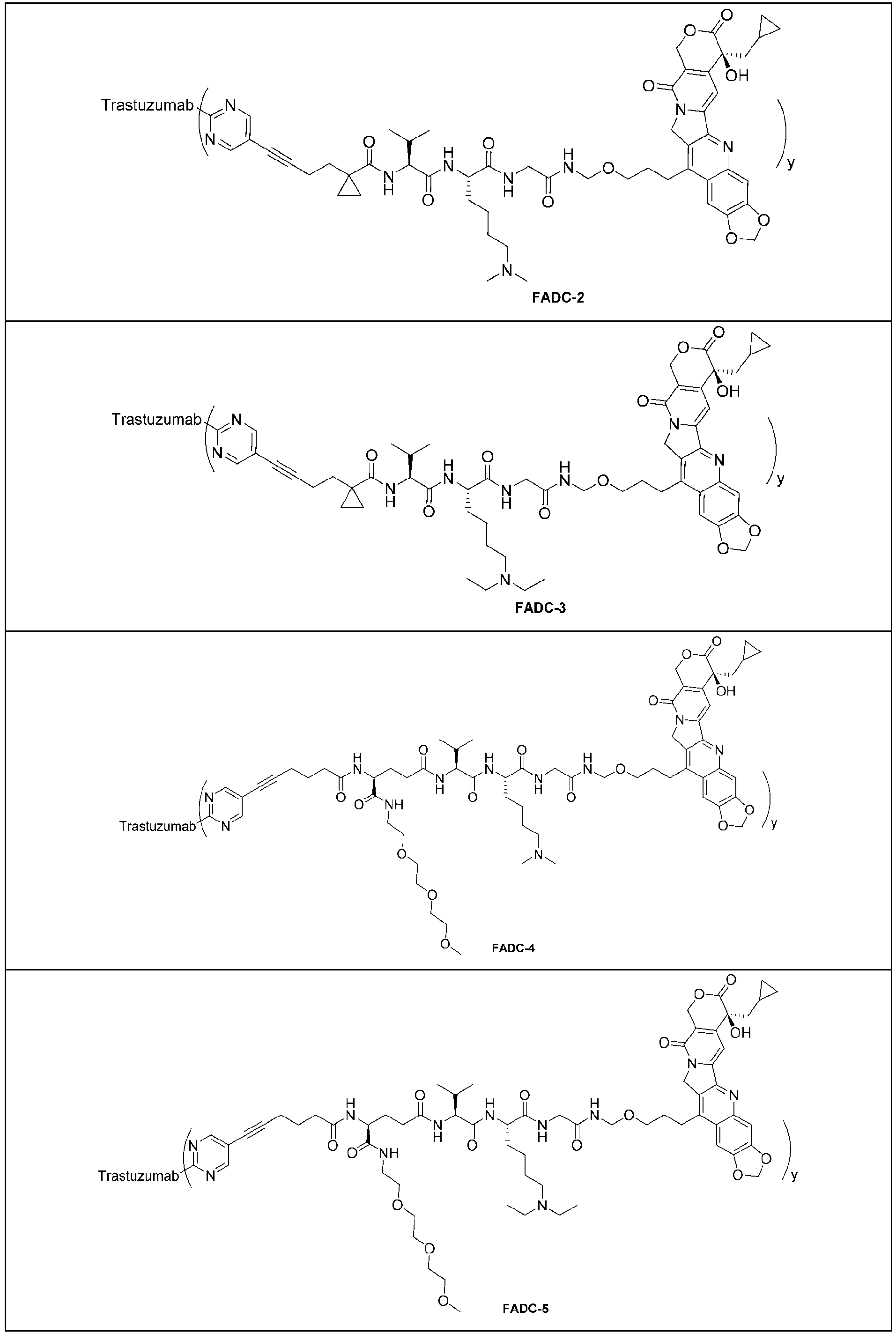

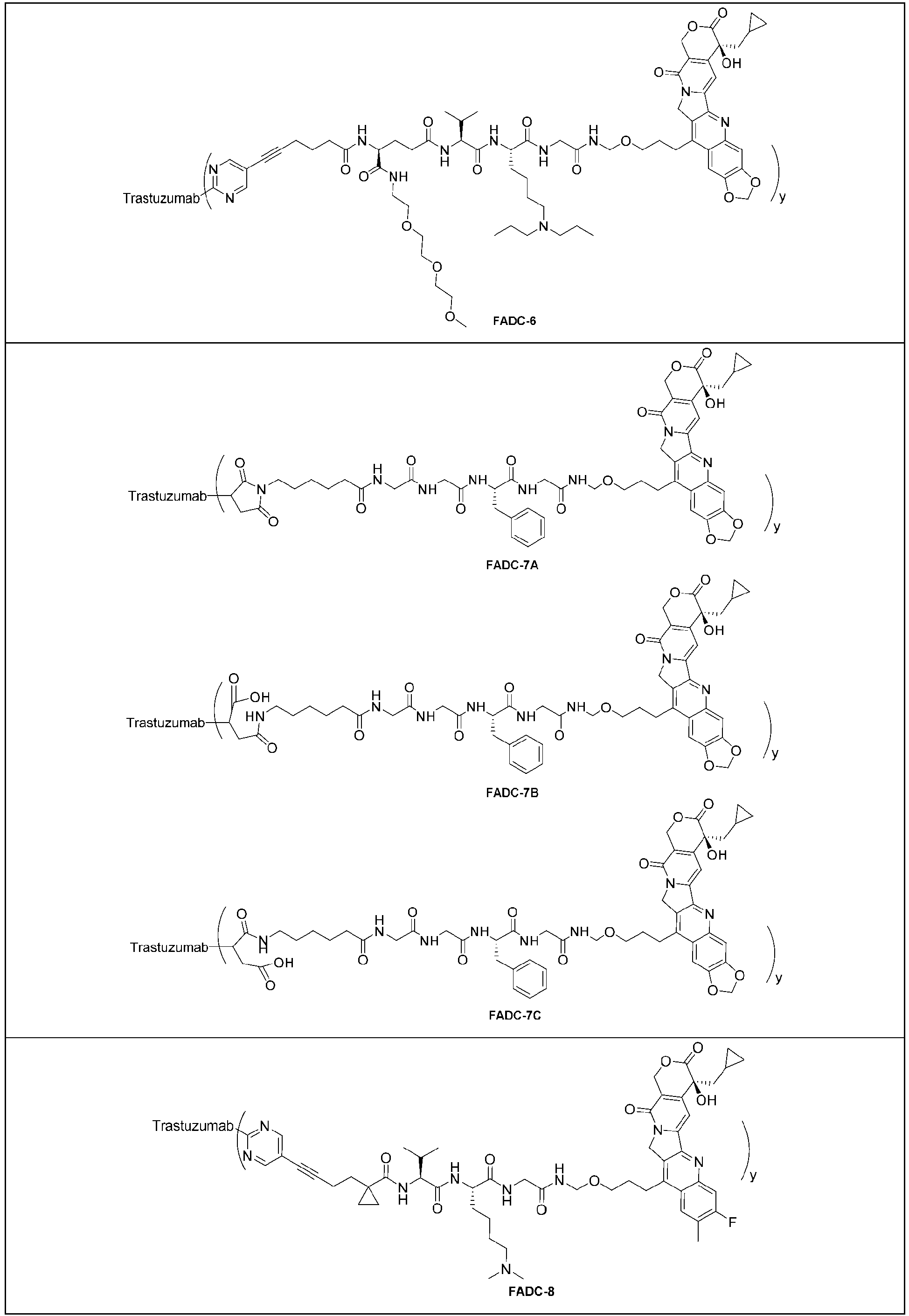

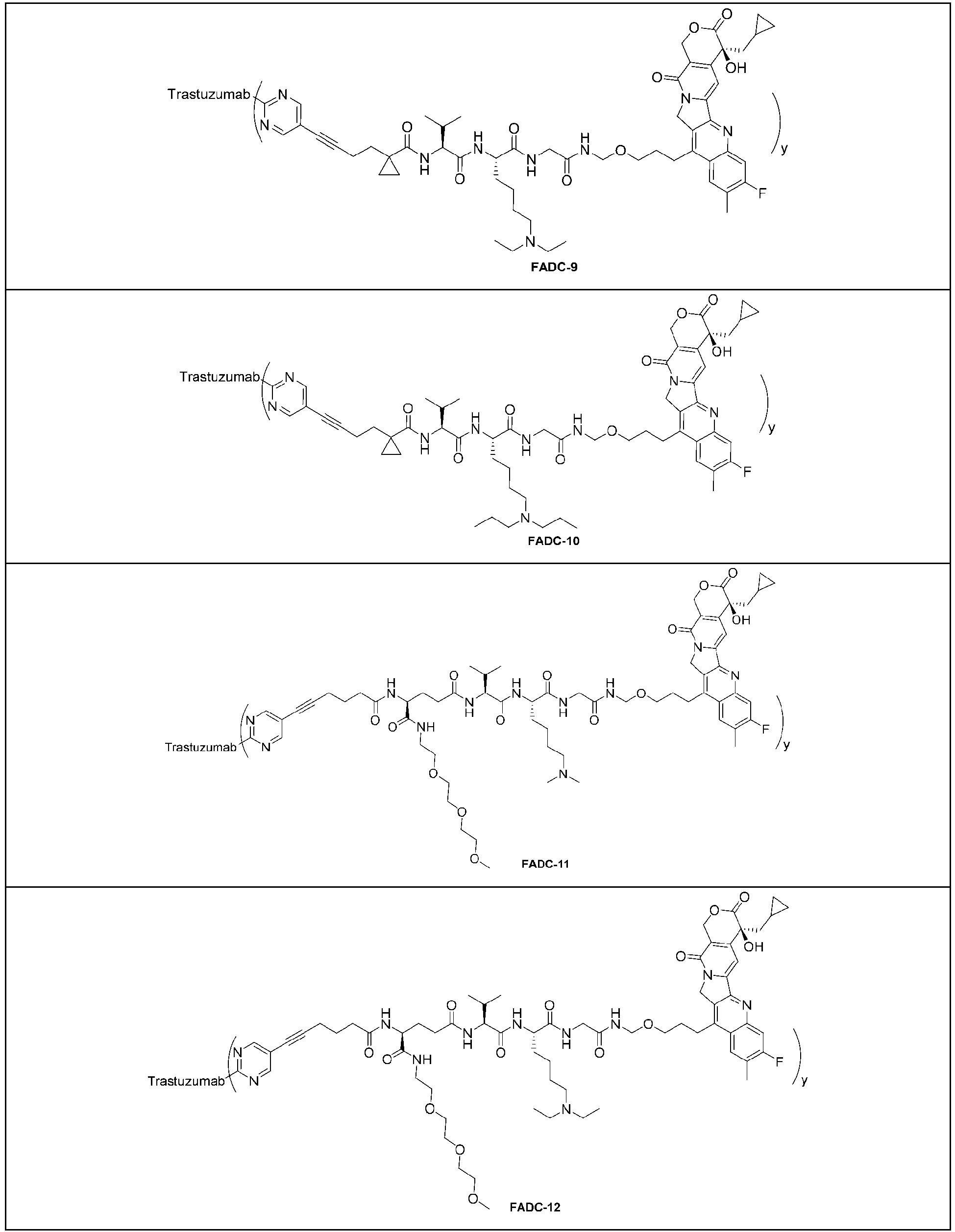

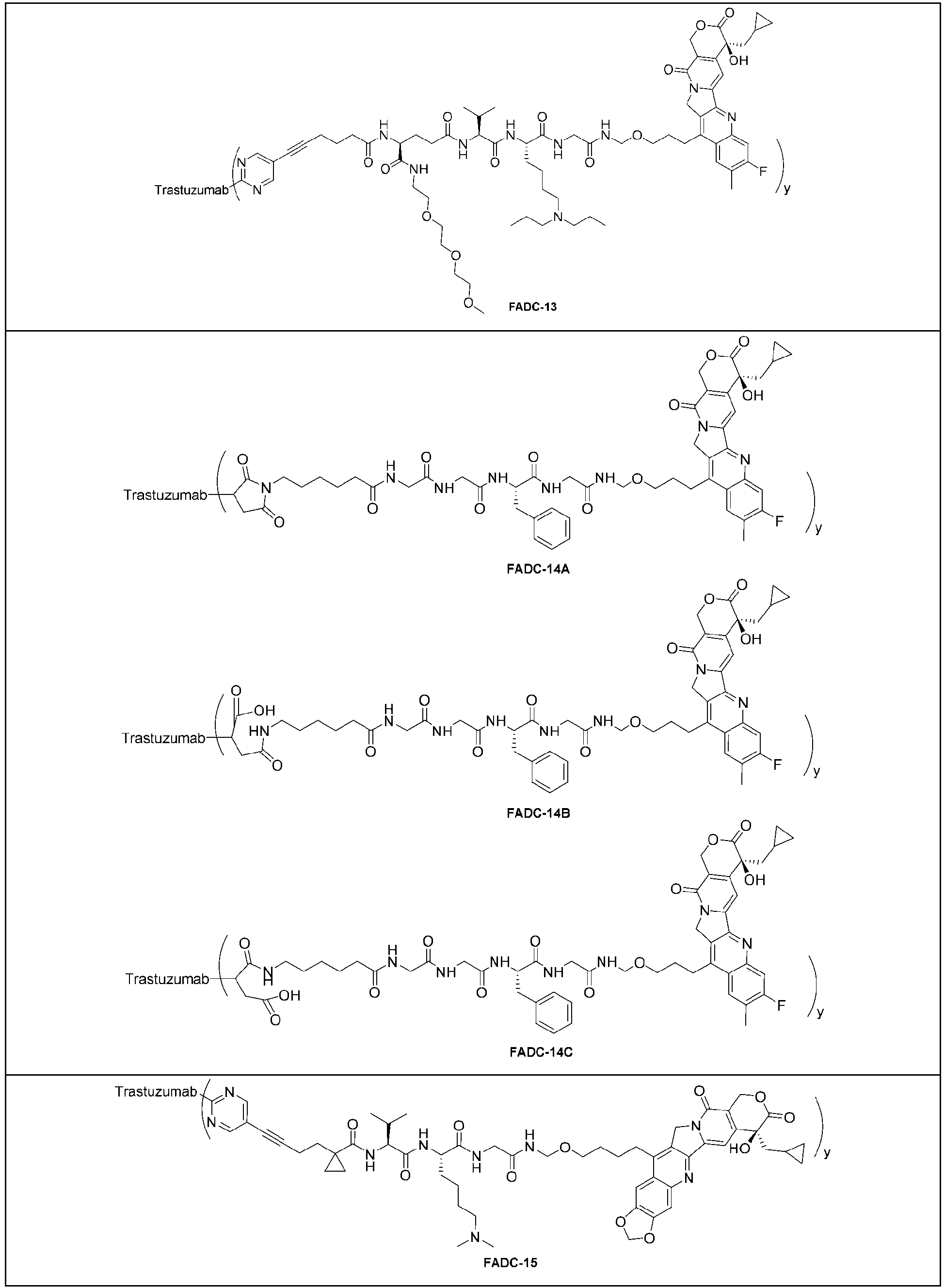

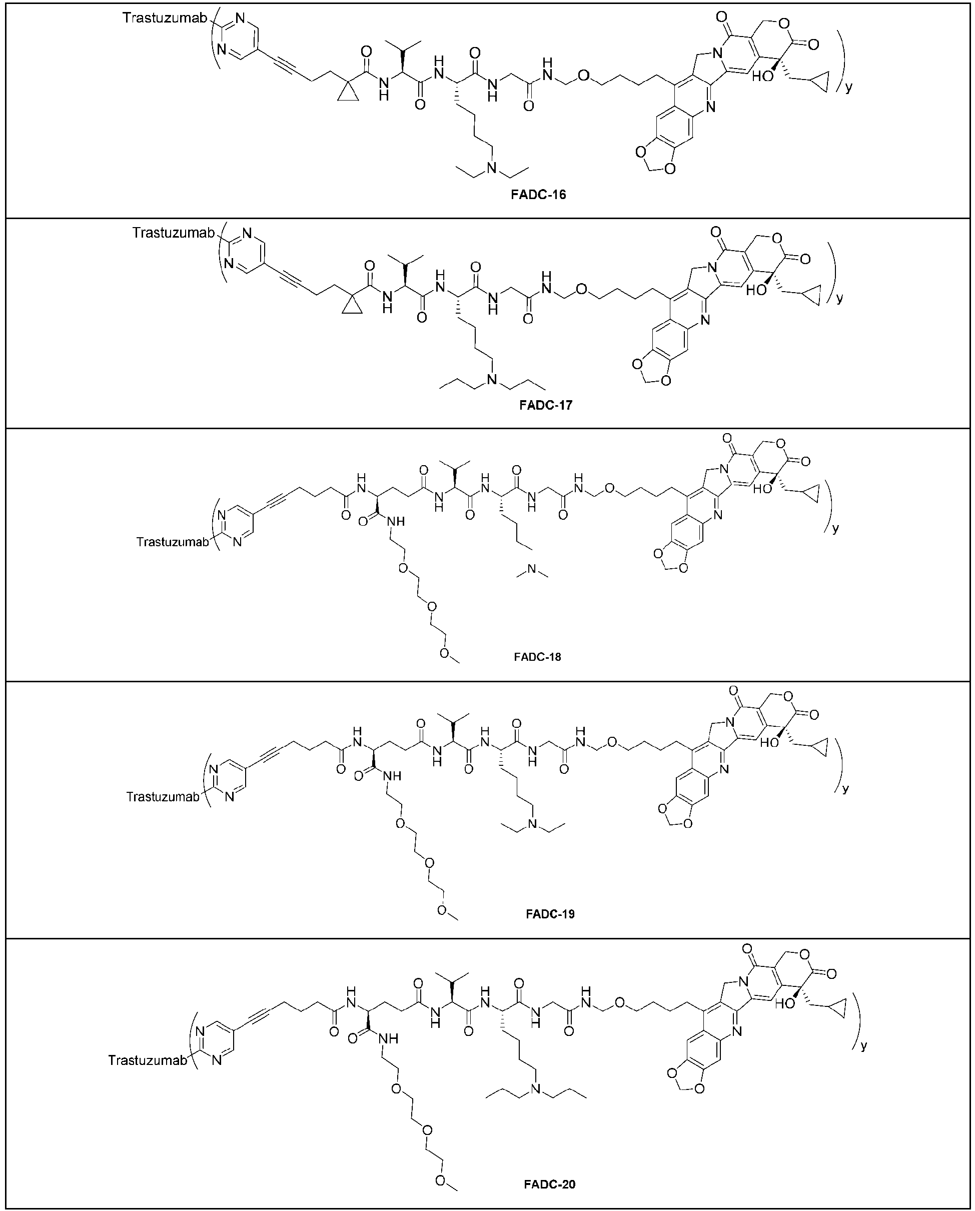

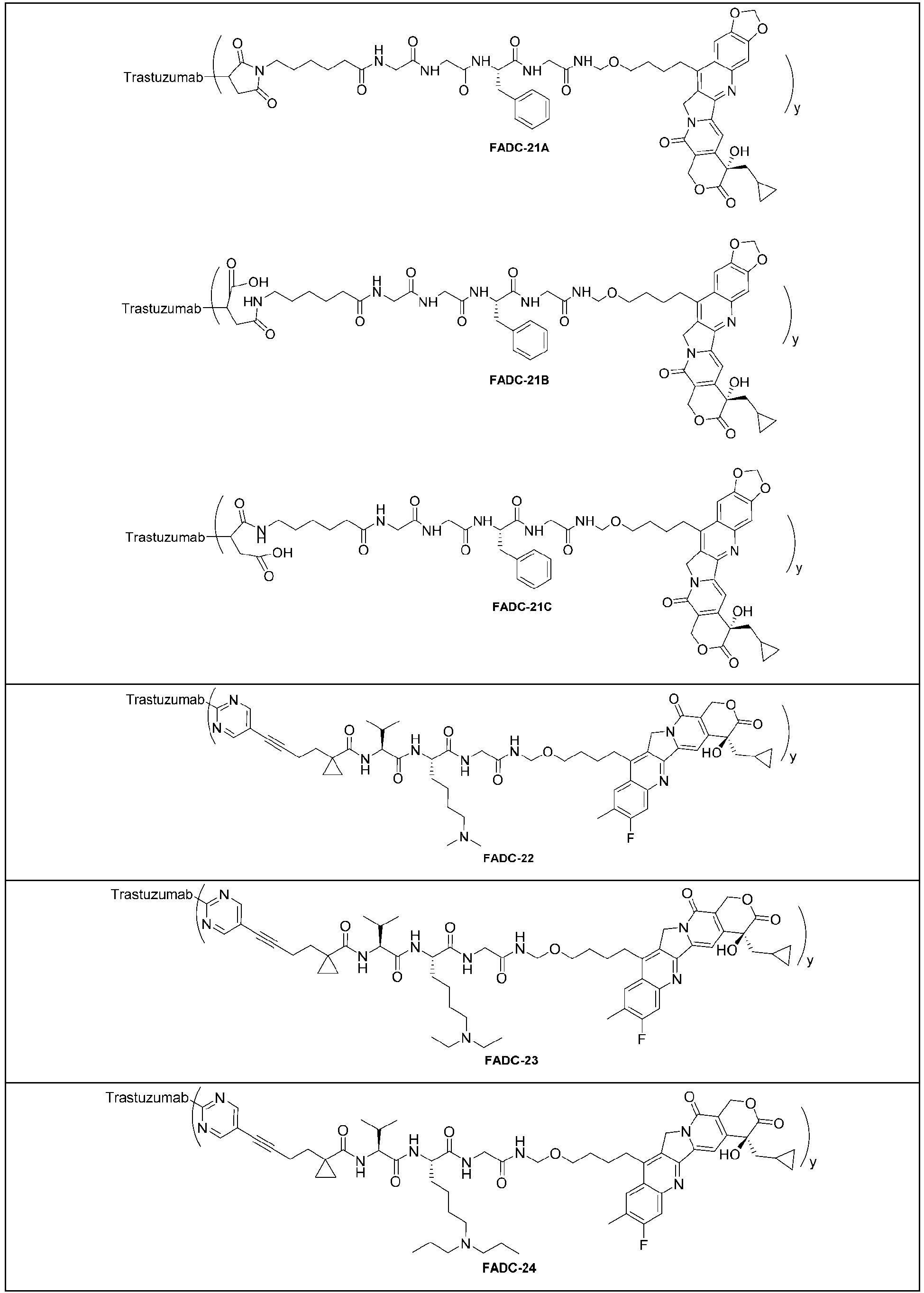

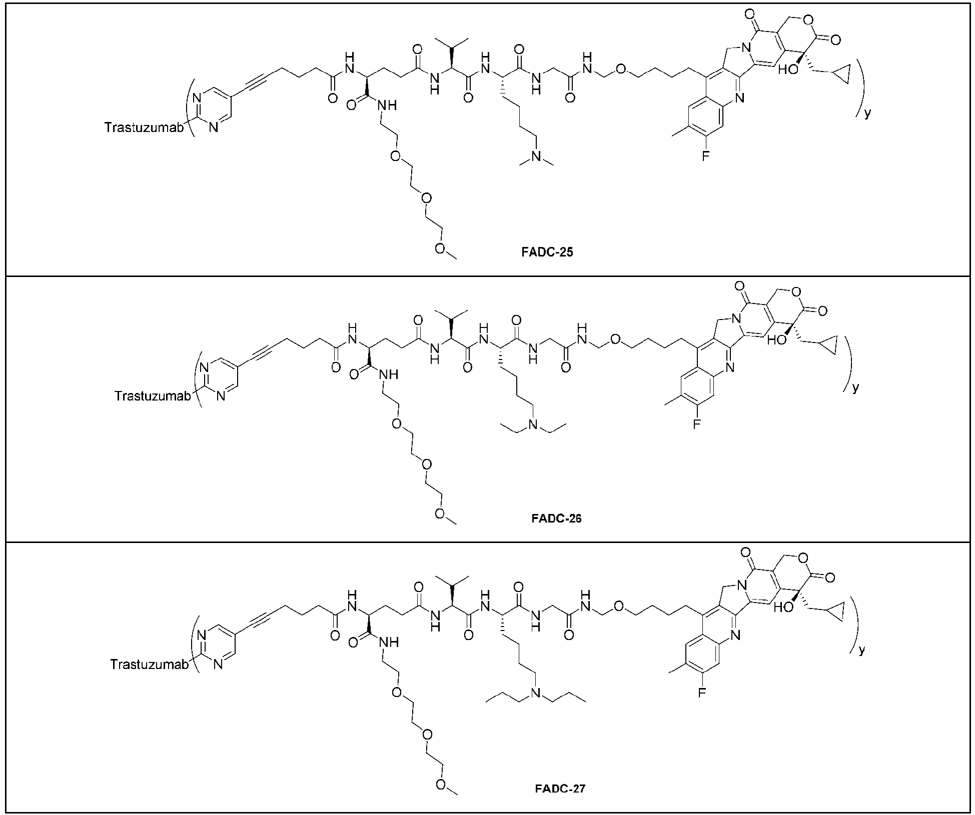

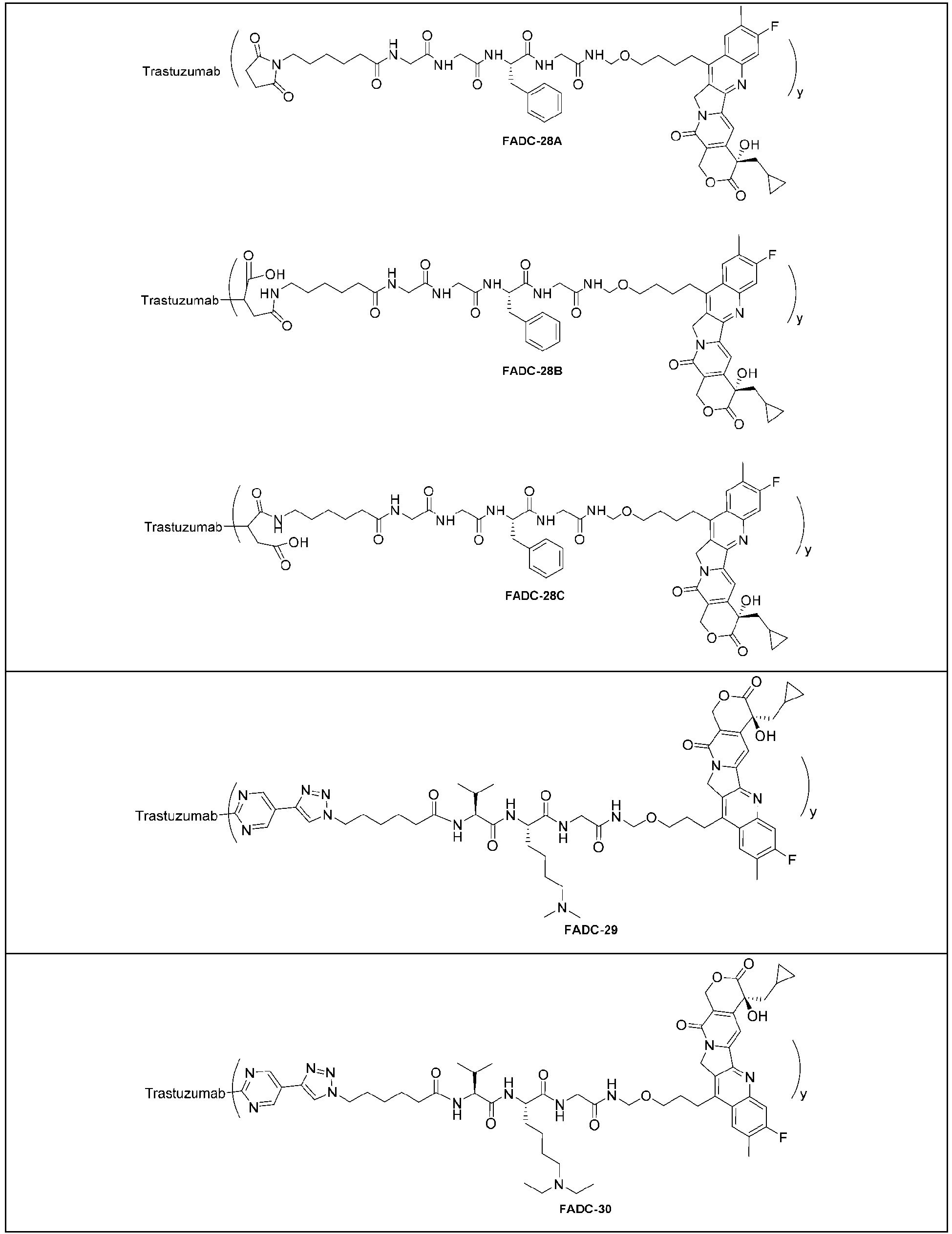

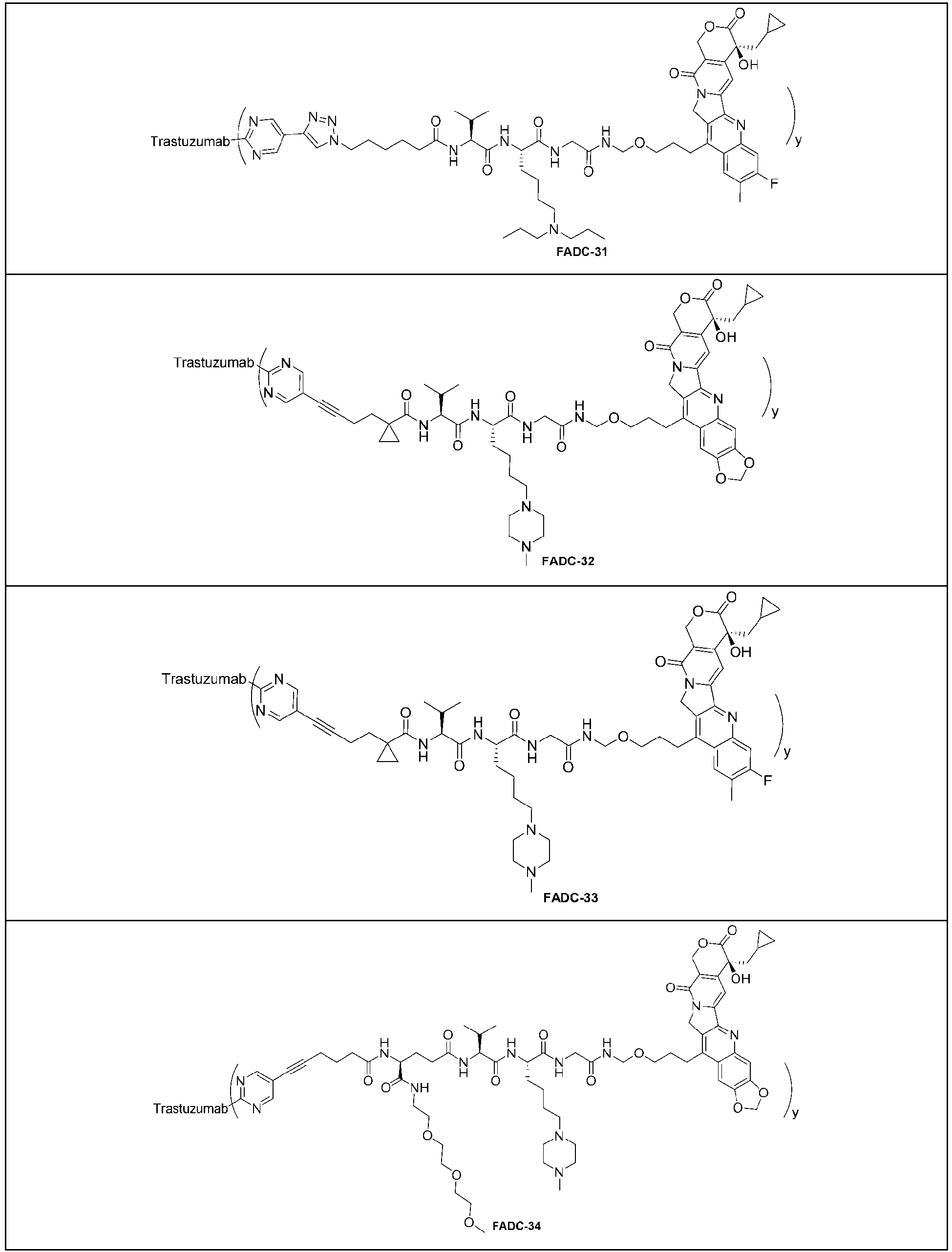

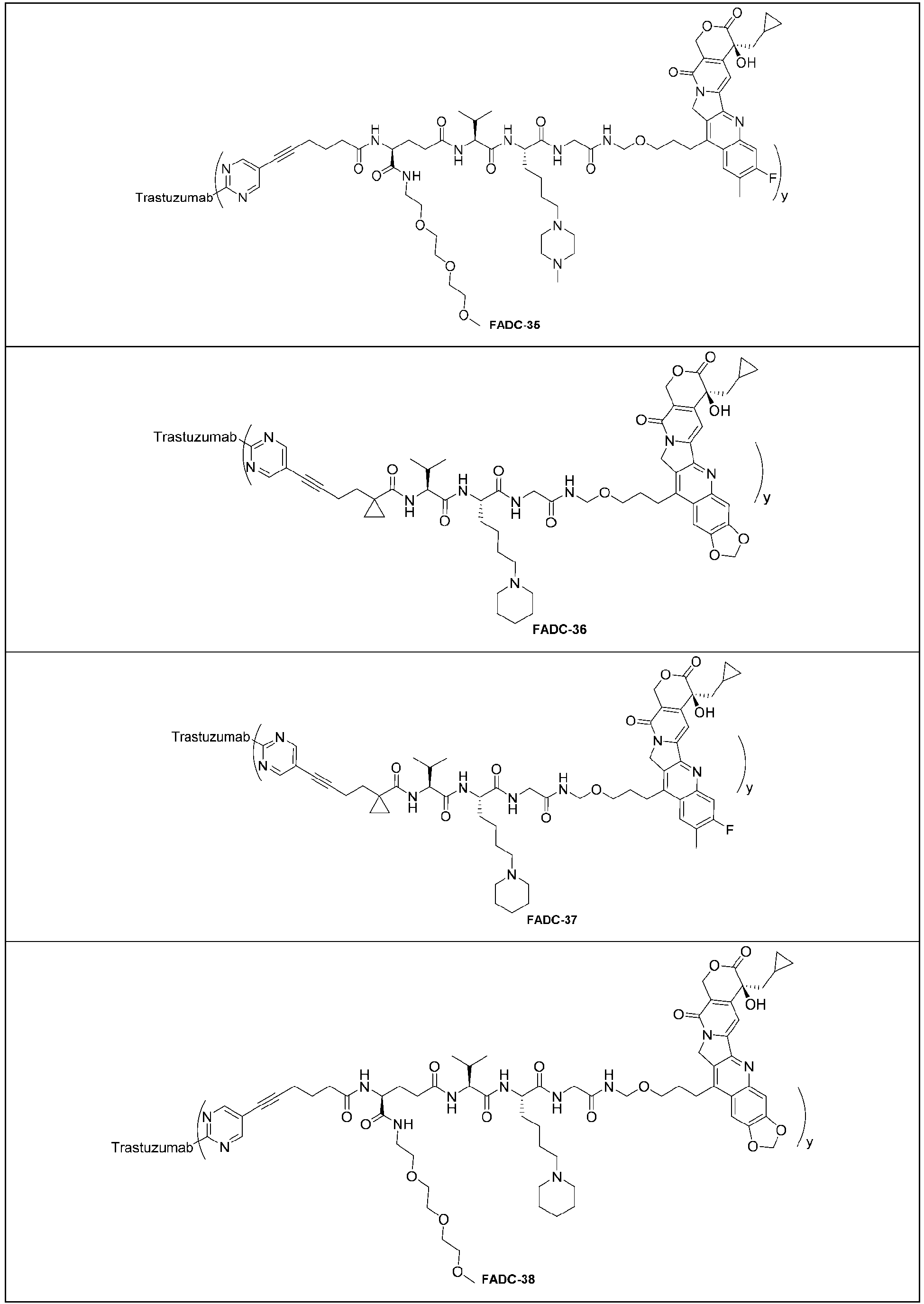

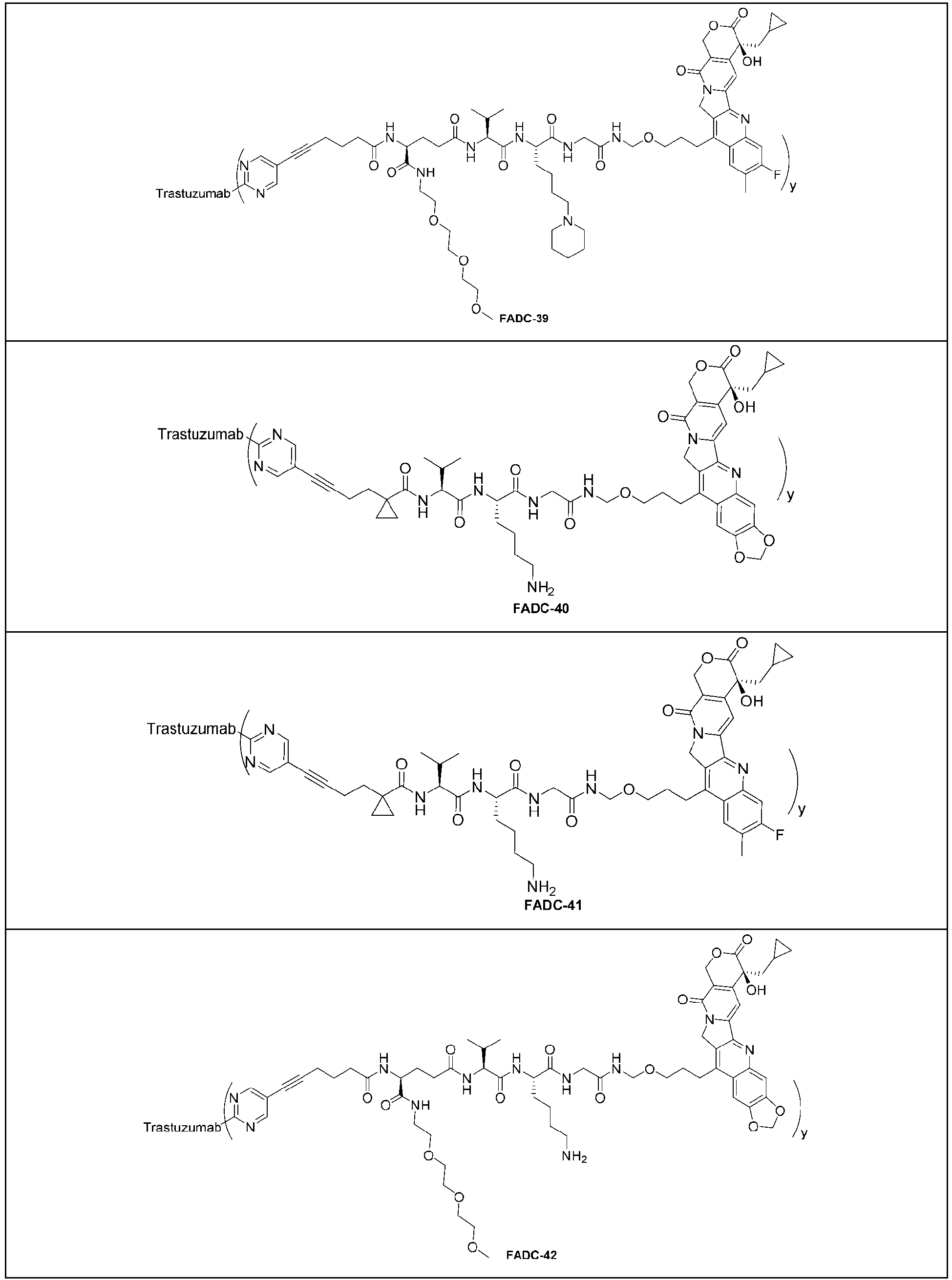

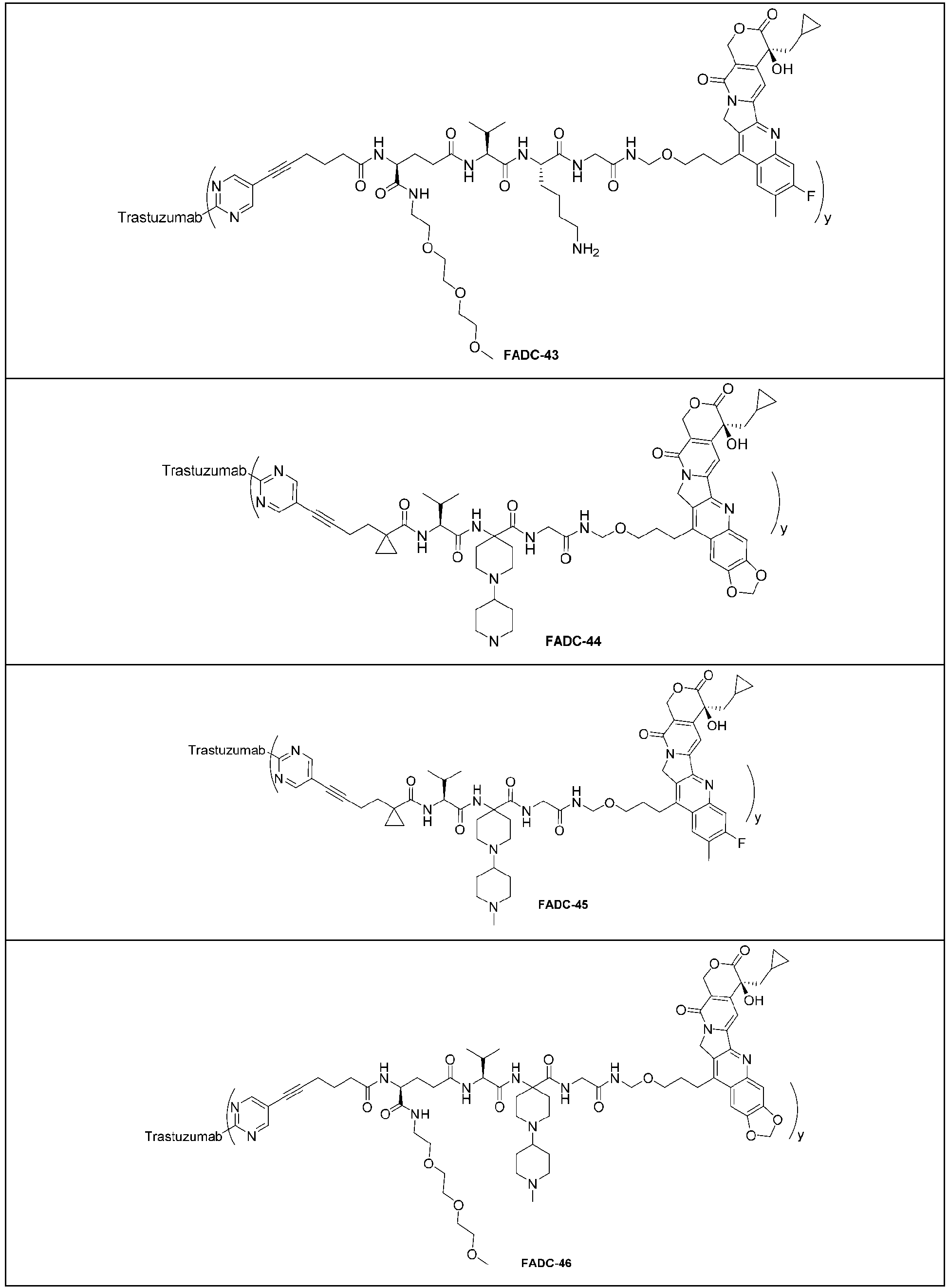

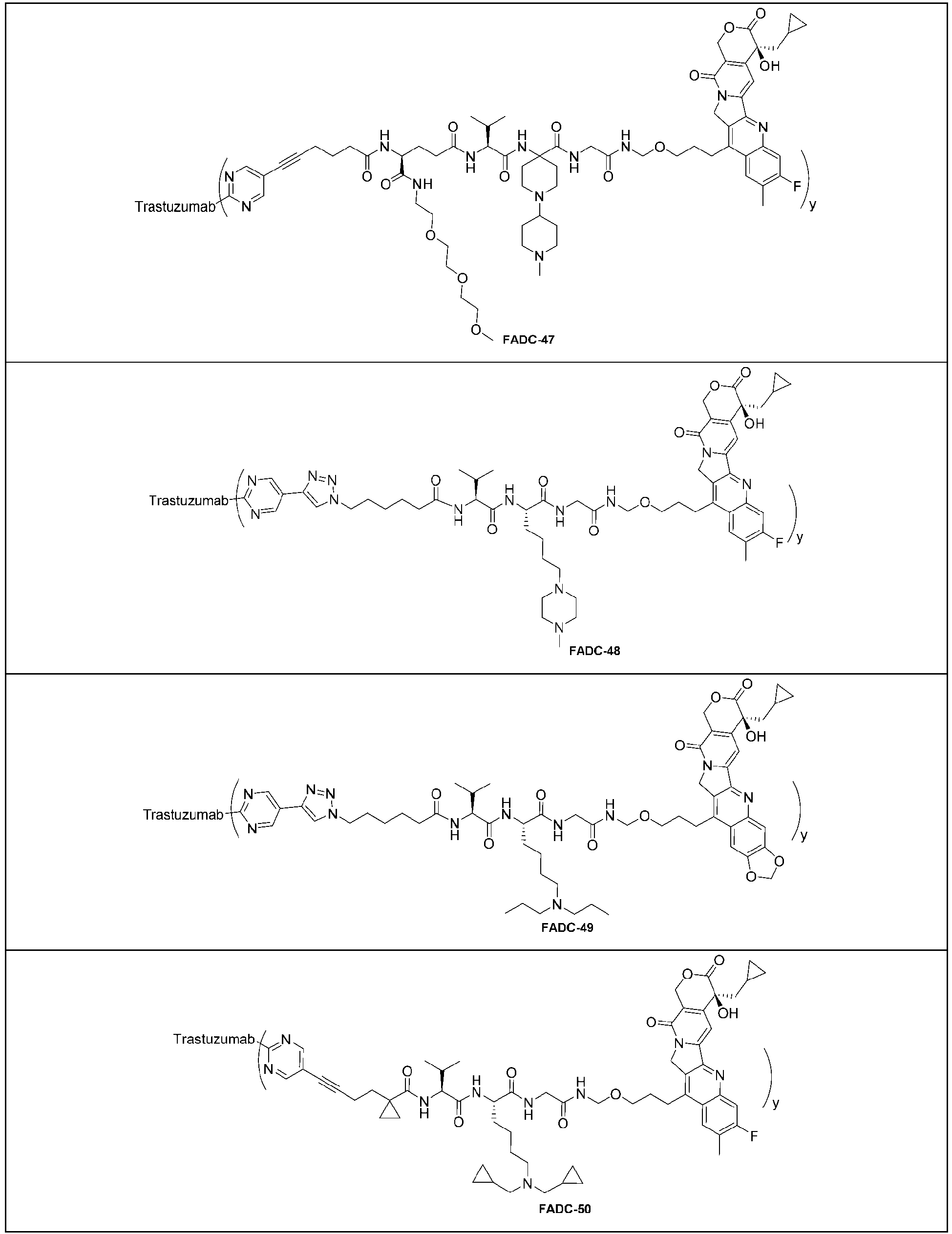

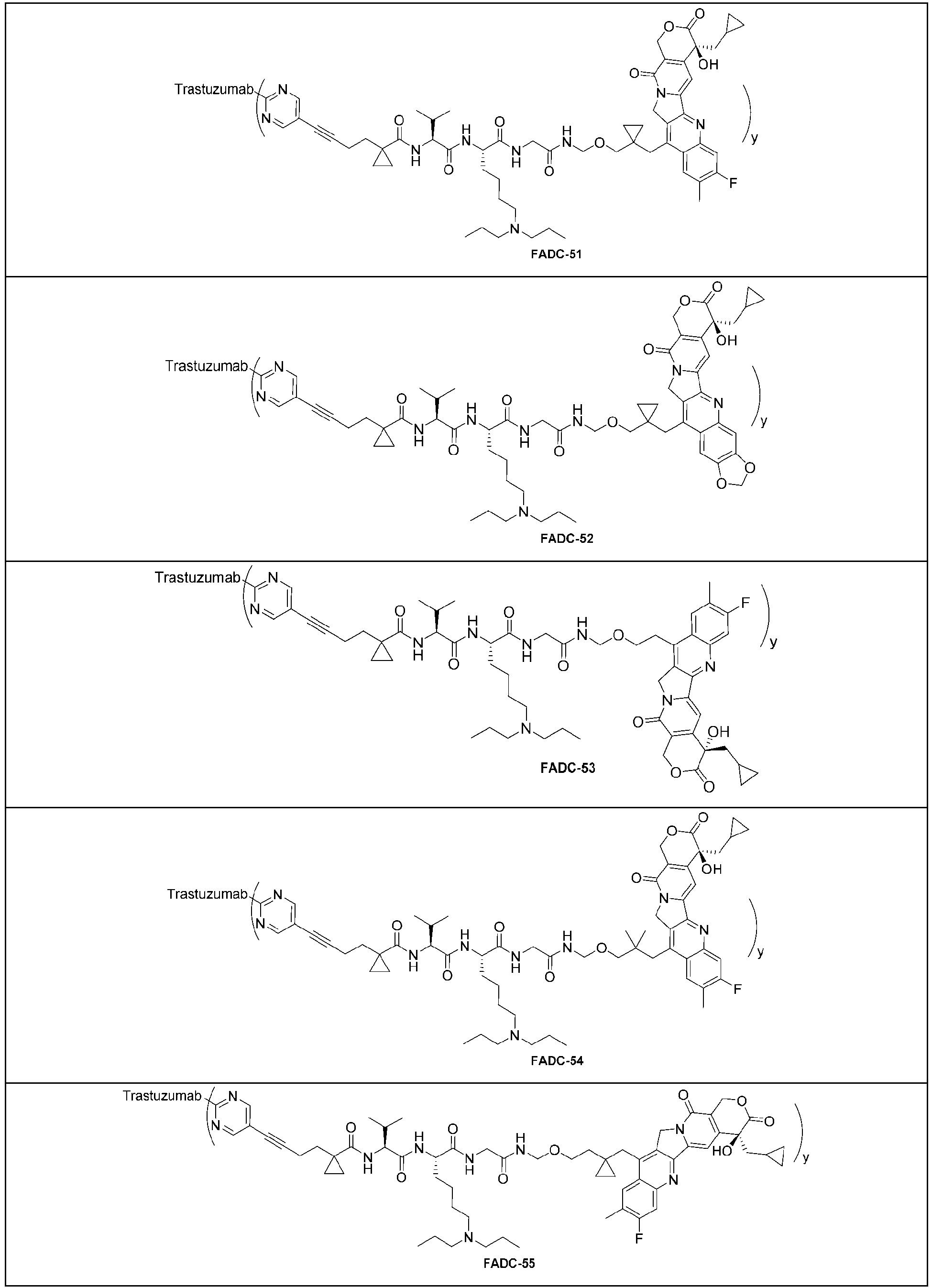

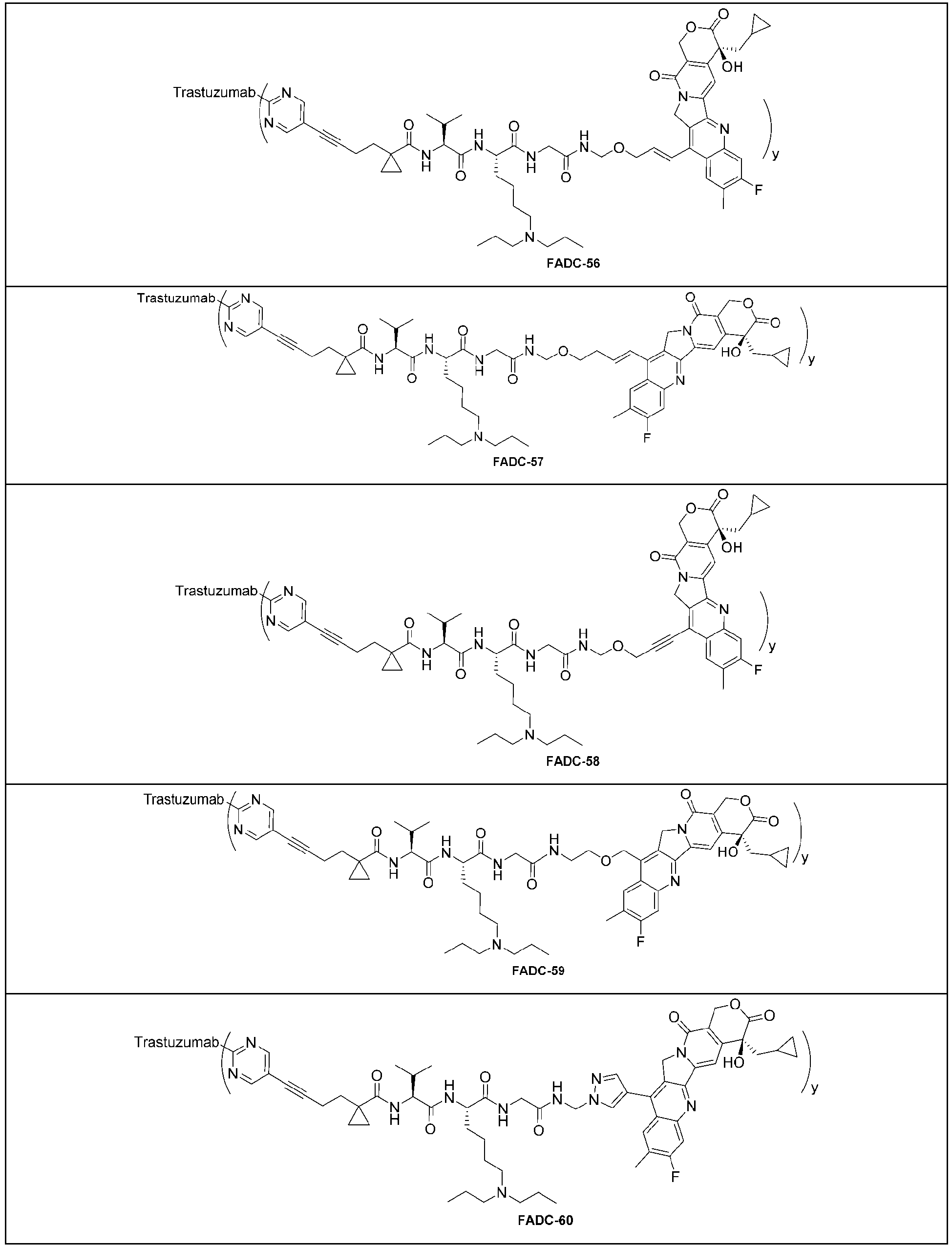

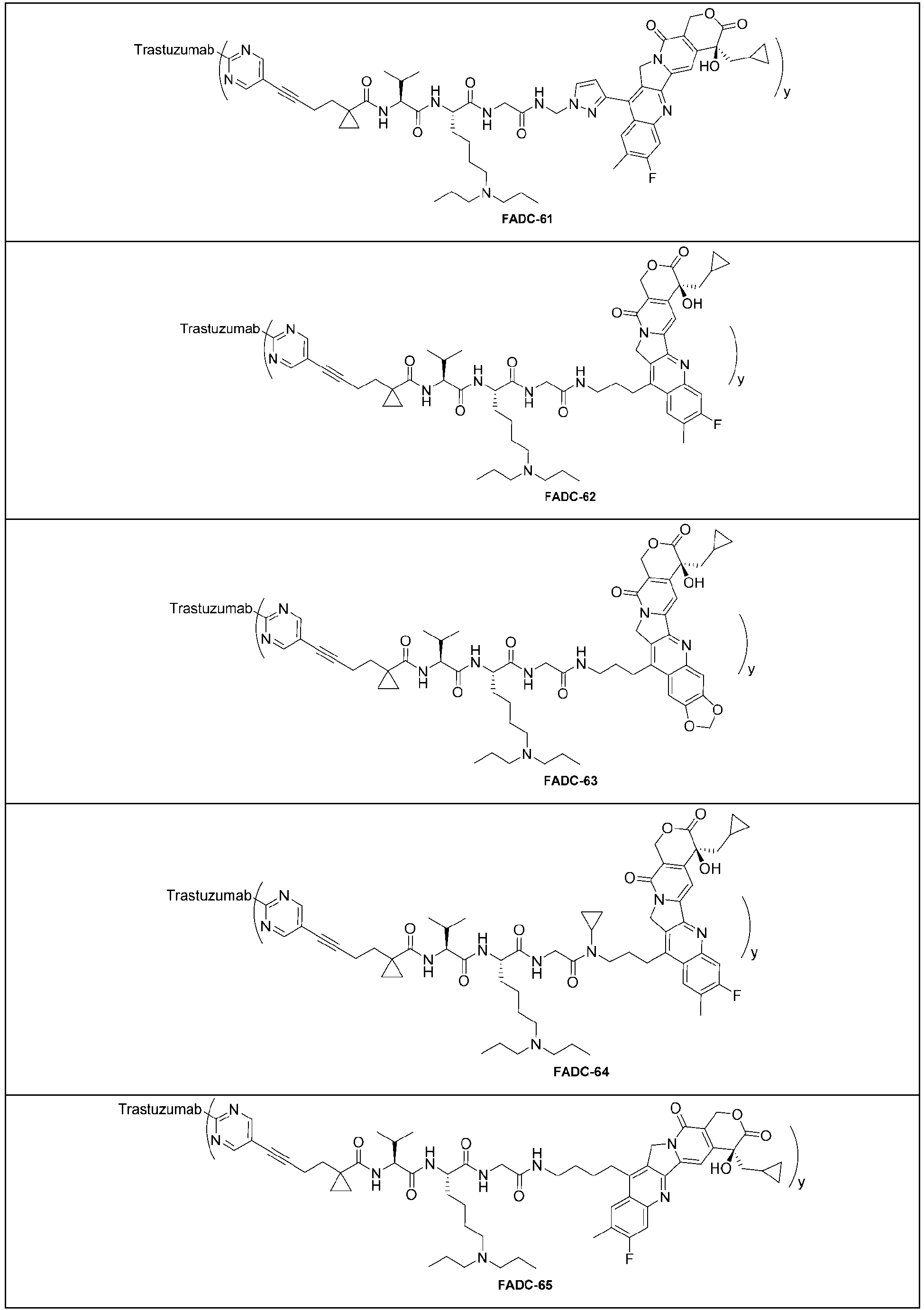

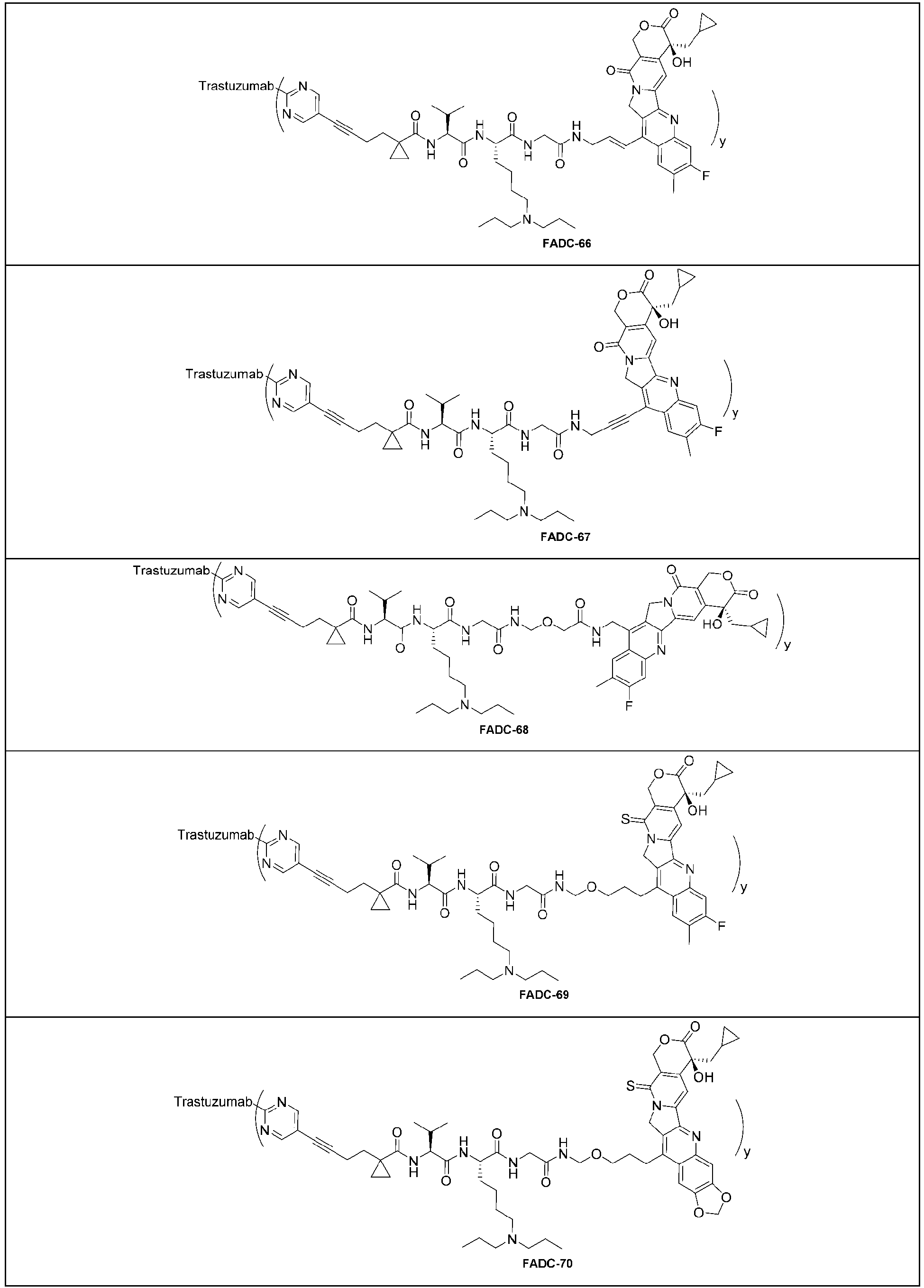

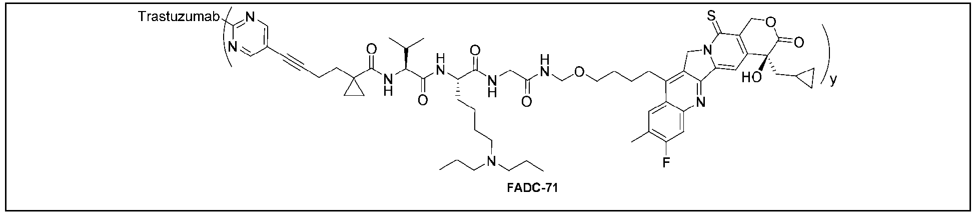

The present disclosure also provides a method for preparing the ligand-drug conjugate, or a stereoisomer, racemate, tautomer, isotopologue, isotope-labeled compound, N-oxide, prodrug, solvate, or pharmaceutically acceptable salt thereof, wherein the preparation method comprises the following steps:
step 1: providing a linker as shown in L¹'-L²-L³-L⁴'(L');
preferably, in the linker,
L^{4'} is a reactive form of: 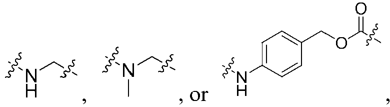 preferably L^{4'} is acetate ester;
further preferably, L^{4'} is a reactive form of 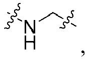 preferably L^{4'} is an acetate ester having the following structure: 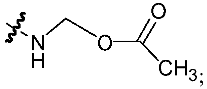
or if L⁴ is absent, L^{4'} is a reactive form of L³ (e.g., the C-terminal glycine gly in L³ is converted into an active ester), and L^{4'} forms an amide linkage directly to GH, with B being N.
step 2: the linker is coupled with the compound of formula (GH) to obtain the coupling intermediate of L¹'-L²-L³-L⁴-G(C');
wherein the structure of formula (GH) is: 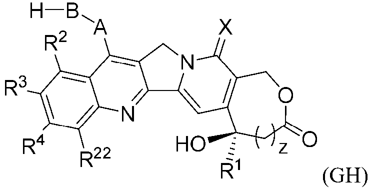
wherein A, B, Z, X, R¹, R², R²², R³, R⁴, L^{1'}, L¹, L², L³, L⁴ and L^{4'} are independently defined as described above.

In a specific embodiment of the present disclosure, the following preparation method is also provided:
step 1: providing a linker fragment comprising L^{4'}, said L^{4'} has the definitions as described above;
step 2: the linker fragment containing L^{4'} is coupled with the compound of formula (GH) to obtain the L^{4'}-containing linker fragment-G intermediate;
step 3: providing another linker fragment to react with the intermediate to form the coupling intermediate of L^{1'}-L²-L³-L⁴-G (C') containing the complete linker;
preferably, the linker fragment comprising L^{4'} is: 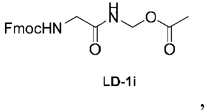 which reacts with GH in which B is -O- to form an ether linkage;
preferably, the other linker fragment is:

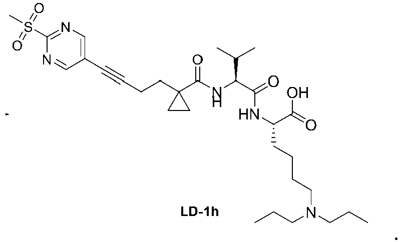

Preferably, the preparation method further comprises a step 4 of coupling the coupling intermediate of formula (C') to a targeting moiety Tp;
optionally, if necessary, the functional groups of the reaction substrate may also be protected, with a protecting group known in the art, to allow the reaction to proceed smoothly and to remove the protecting group after the reaction is completed.

The present disclosure also provides a coupling intermediate of the following structure: 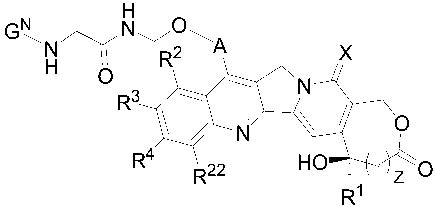 wherein G^{N} is H or an optional amino protecting group, and A, R¹, R², R²², R³, R⁴, X and Z independently have the definitions as described above.

The present disclosure also provides a linker fragment compound of the structure:

L^{1'}-L²-ValAA^{1'}(L")

wherein L^{1'} is a reactive group, preferably 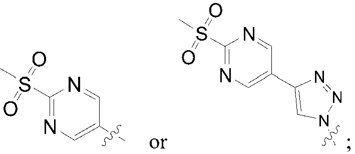
L² is selected from-(C≡C)-(CH₂)_{q}-C(R^{z4}R^{z5})-C(=O)- or -(CH₂)_{q}-C(R^{z4}R^{z5})-C(=O);
wherein L^{1'}-L² has the following structure: 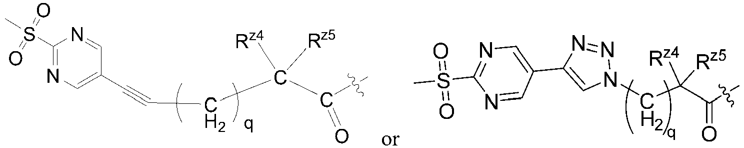
q is an integer selected from 0 to 10, preferably 1, 2, 3, 4, 5, 6, 7 or 8;
R^{z4} and R^{z5} are identical or different and independently selected from H, C₁₋₄ alkyl, C₃₋₆ cycloalkyl, C₃₋₆ cycloalkyl-C₁₋₄ alkyl, or R^{z4} and R^{z5} together form a C₃₋₆ cycloalkyl, wherein at least one of R^{z4} and R^{z5} is not H;
   or
L² is selected from the group consisting of -(C≡C)-(CH₂)_{q}-C(=O)-, wherein a hydrophilic moiety as defined above is contained between L² and L³: 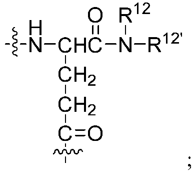
AA^{1'} is AA¹ or a reactive form thereof such as an active ester, wherein AA¹ is as defined above.

The present disclosure also provides a linker:

L¹'-L²-L³-L⁴'(L')

Wherein
L^{1'}-L² is 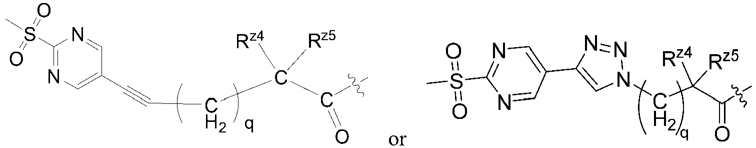 as defined above;
or
L² is selected from the group consisting of -(C≡C)-(CH₂)_{q}-C(=O)-, wherein a hydrophilic moiety as defined above is contained between L² and L³: 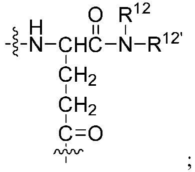 and
L³ is: -ValAA¹Gly-, wherein AA¹ is as defined above.
L' is preferably:

| |
|---|
| 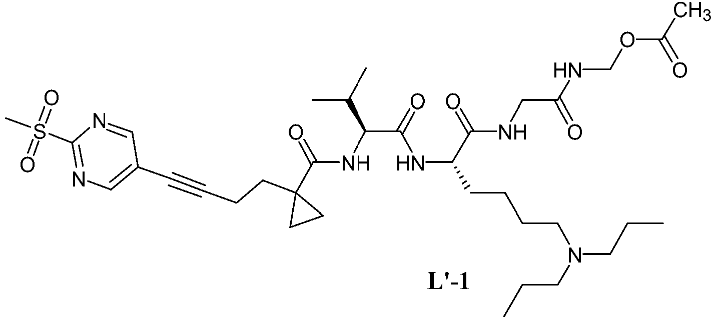 |
| 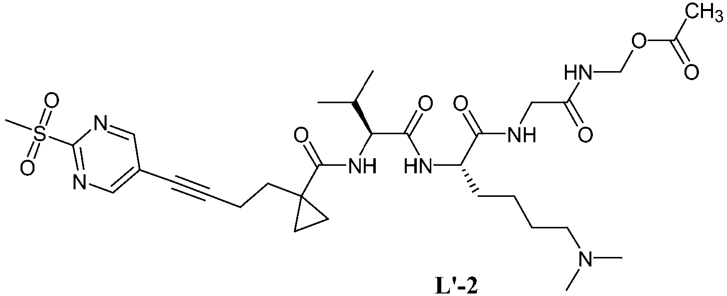 |
| 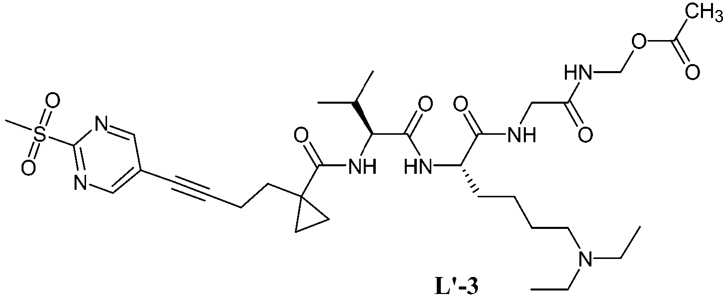 |
| 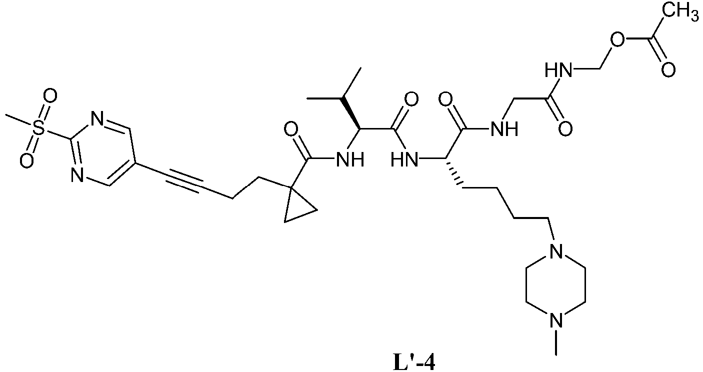 |
| 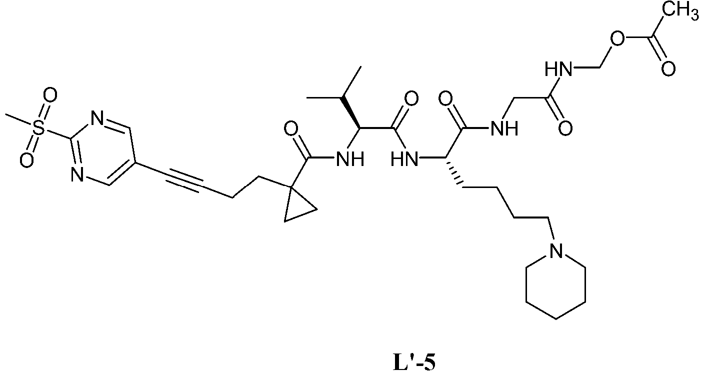 |
| 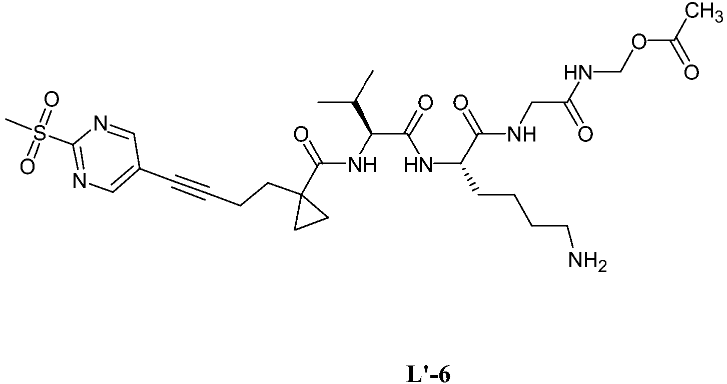 |
| 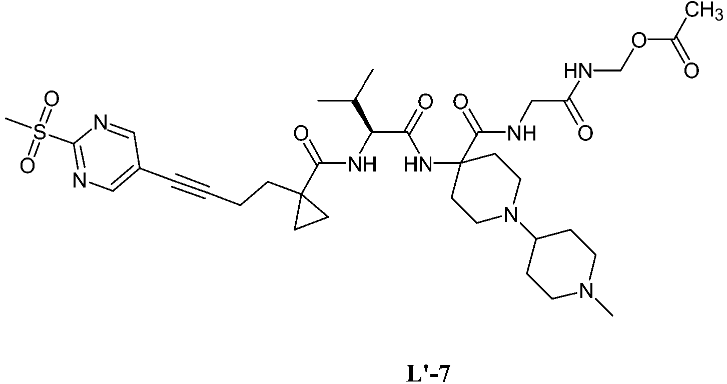 |
| 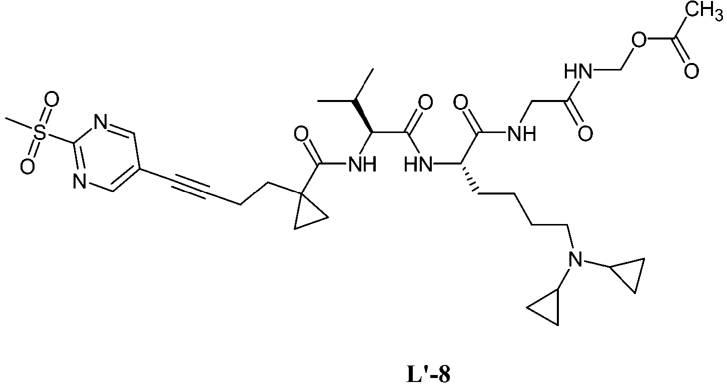 |
| 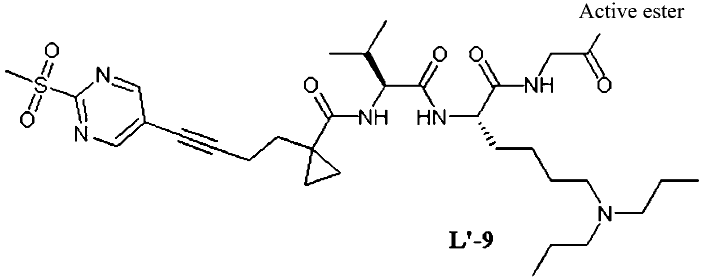 |
| 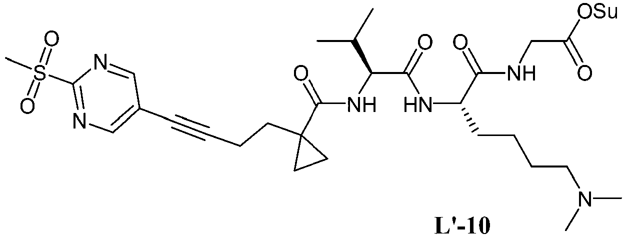 |
| Wherein OSu represents N-OH succinimide active ester |
| 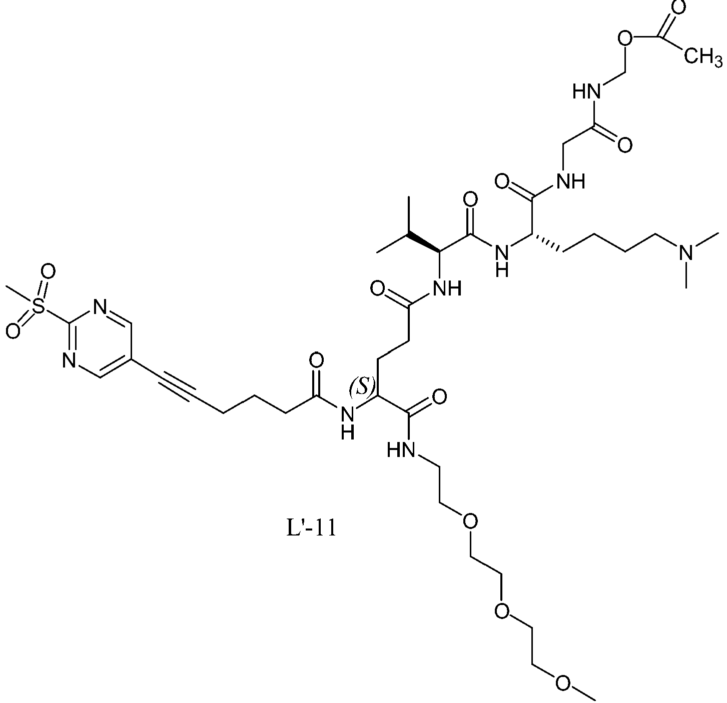 |

The present disclosure also provides a compound obtained by attaching the above group G to a hydrogen atom, wherein the group B is attached to a hydrogen atom.

The present disclosure also provides a compound represented by the following formula (GH), or a stereoisomer, racemate, tautomer, isotopologue, isotope-labeled compound, N-oxide, prodrug, solvate, or pharmaceutically acceptable salt thereof: 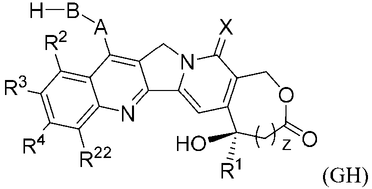 wherein A, B, X, Z, R¹, R², R²², R³ and R⁴ independently of each other have the definitions as described above.

According to an embodiment of the present disclosure, in Formula (GH) as described in the context of the specification, hydrogen, carbon or other atoms in the structural formula are optionally substituted by their isotopes, whether the group is defined as selected from the above definitions or is directly depicted in the structural formula. For example, any hydrogen atom on any group of formula (GH) may be selected from ¹H, ²H or ³H. As an example, in a compound of Formula (GH), any hydrogen atom on an unsubstituted alkyl, alkylene, alkylidyne, phenyl, pyridyl or lactone group or on a substituent of a substituted alkyl, alkylene, alkylene, phenyl, pyridyl or lactone group or a ring-forming atom thereof (if any) may be independently selected from ¹H, ²H or ³H.

According to an embodiment of the present disclosure, the compound represented by formula (GH) may be selected from a compound represented by the following formula (GH-1): 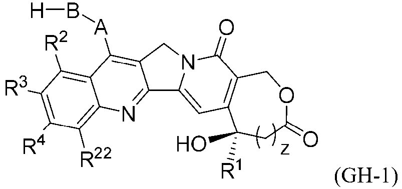 wherein A, B, Z, R¹, R², R²², R³ and R⁴, independently from one another, have definitions as described above.

According to an embodiment of the present disclosure, the compound represented by formula (GH) may be selected from a compound represented by the following formula (GH-2): 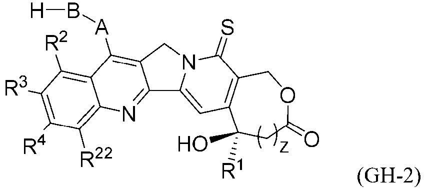 wherein A, B, Z, R¹, R², R²², R³ and R⁴, independently from one another, have definitions as described above.

According to an embodiment of the present disclosure, the compound represented by formula (GH) may be selected from the following compounds:

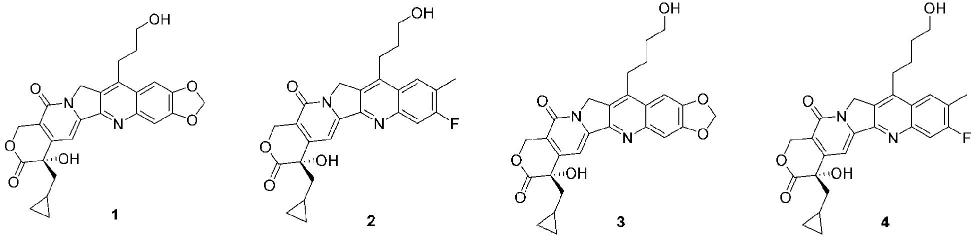

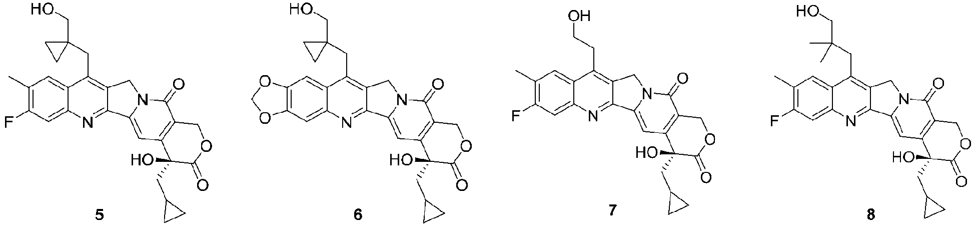

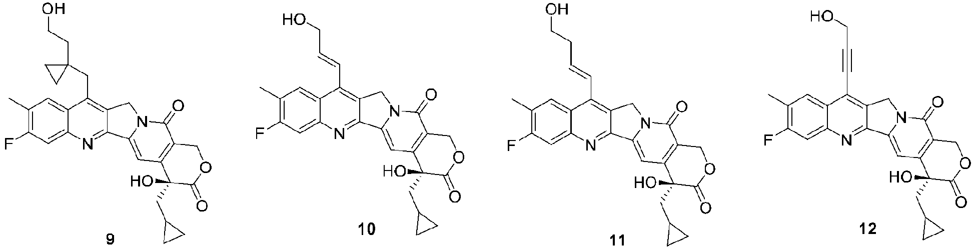

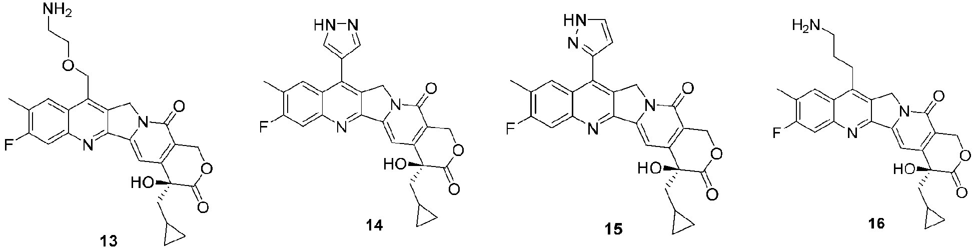

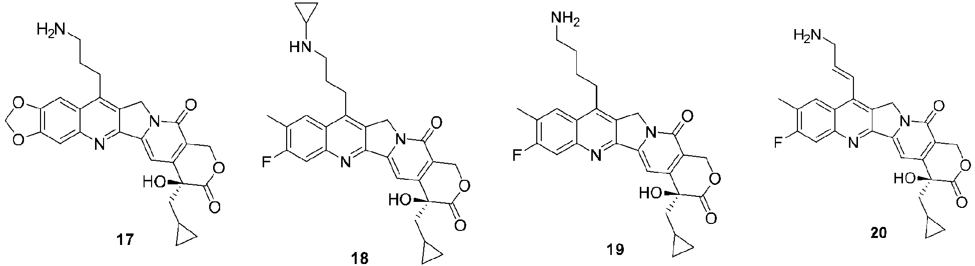

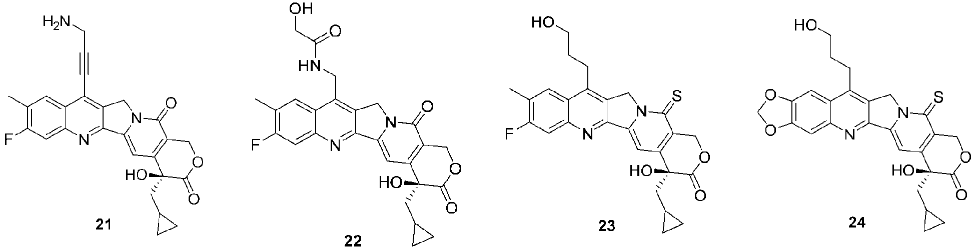

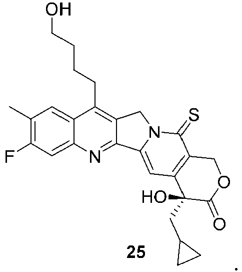

The present disclosure also provides a method for preparing the compound represented by formula (GH), said method comprising the step of reacting a compound of formula (i) with a compound of formula (ii) to obtain a compound of the formula (G '): 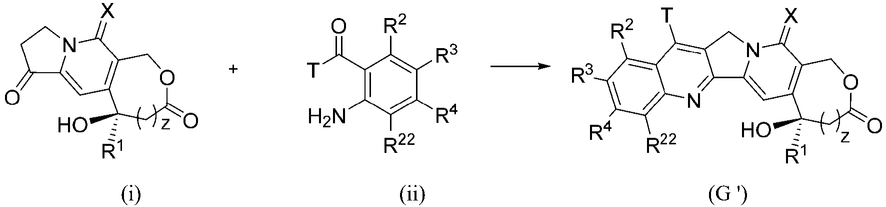
wherein, T is 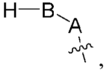 or is a group which can be converted into 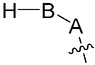 by reaction methods such as oxidation or reduction. Those skilled in the art will understand that a group containing an active functional group such as an alkenyl, aldehyde, carbonyl, or nitro group, and the like can generally be selected as T for introduction of 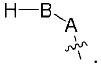 Specifically, when the active functional group is a carbon-containing structure such as an alkenyl, aldehyde, or carbonyl, the residue obtained from T by removing the active functional group is a structure with one fewer carbon atom than A; when the active functional group is a nitro group, the residue obtained from T upon removal the nitro group is A, which, for example, may be selected from the following group forms: NO₂-C(R¹⁵R¹⁶)ₘ-(CR¹⁷=CR¹⁸)ₙ-C(R¹⁹R²⁰)ₒ-, (CR¹⁷=CR¹⁸)ₙ-C(R¹⁹R²⁰)ₒ-, O=C R²¹-C(R¹⁵R¹⁶)ₘ-(CR¹⁷=CR¹⁸)ₙ-C(R¹⁹R²⁰)ₒ-;
wherein m, n and o are independently from one another selected from any integer from 0 to 6;
each of R¹⁵, R¹⁶, R¹⁷, R¹⁸, R¹⁹, R²⁰ and R²¹ is identical or different and independently of one another is selected from hydrogen, halogen, cyano, the following groups unsubstituted or optionally substituted with one, two or more R^{A}: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy;
alternatively, R¹⁵ and R¹⁶ together with the atoms to which they are attached form a 3-10 membered ring structure which is unsubstituted or optionally substituted with one, two or more R^{A};
alternatively, R¹⁹ and R²⁰ together with the atoms to which they are attached form a 3-10 membered ring structure which is unsubstituted or optionally substituted with one, two or more R^{A};
wherein R¹, R², R²², R³, R⁴, X and Z independently have the definitions as described above.

The present disclosure also provides a method for preparing a compound represented by formula (GH), which comprises the step of reacting a compound of the formula (G') to obtain a compound of formula (GH): 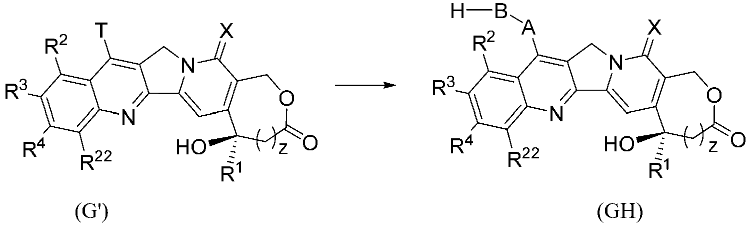
wherein A, B, R¹, R², R²², R³, R⁴, X and Z independently have the definitions as described above;
wherein T is a group which can be converted into 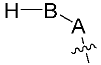 by reaction methods such as oxidation, reduction, and the like, and it is understood by those skilled in the art, for example, that a group containing an active functional group such as an alkenyl, an aldehyde, a carbonyl, a nitro, and the like, can generally be selected as T to introduce 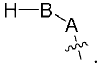 Specifically, when the active functional group is a carbon-containing structure such as an alkenyl, an aldehyde, or a carbonyl, the residue obtained from T by removing the active functional group is a structure with one fewer carbon atom than A; when the active functional group is a nitro group, the residue obtained from T by removing the nitro group is A, which, for example, may be selected from the following group forms: preferably NO₂-C(R¹⁵R¹⁶)ₘ-(CR¹⁷=CR¹⁸)ₙ-C(R¹⁹R²⁰)ₒ-, (CR¹⁷=CR¹⁸)ₙ-C(R¹⁹R²⁰)ₒ-, O=C R²¹-C(R¹⁵R¹⁶)ₘ-(CR¹⁷=CR¹⁸)ₙ-C(R¹⁹R²⁰)ₒ-;
wherein m, n, o, R¹⁵, R¹⁶, R¹⁷, R¹⁸, R¹⁹, R²⁰ and R²¹ independently have the definitions as described above.

The present disclosure also provides a compound represented by formula (i):

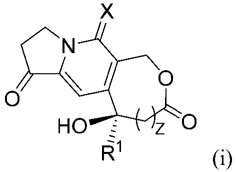

Wherein R¹, X and Z independently have the definitions as described above.

The present disclosure also provides a compound represented by formula (G'):

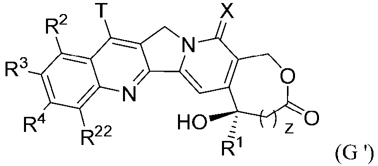

Wherein R¹, R², R²², R³, R4, X, Z and T independently have the definitions as described above.

The present disclosure also provides a pharmaceutical composition comprising at least one selected from the group consisting of a ligand-drug conjugate represented by formula (C), or a stereoisomer, racemate, tautomer, isotopologue, isotope-labeled compound, N-oxide, prodrug, solvate, or pharmaceutically acceptable salt thereof; a compound represented by the formula (GH), or a stereoisomer, racemate, tautomer, isotopologue, isotope-labeled compound, N-oxide, prodrug, solvate, or pharmaceutically acceptable salt thereof;

Preferably, said compound represented by formula (GH), or a stereoisomer, racemate, tautomer, isotopologue, isotope-labeled compound, N-oxide, prodrug, solvate, or pharmaceutically acceptable salt thereof, or said ligand-drug conjugate represented by formula (C), or a stereoisomer, racemate, tautomer, isotopologue, isotope-labeled compound, N-oxide, prodrug, solvate, or pharmaceutically acceptable salt thereof in the pharmaceutical composition is present in a therapeutically effective amount.

According to an embodiment of the present disclosure, the pharmaceutical composition comprises a therapeutically effective amount of at least one selected from the group consisting of a compound represented by formula (GH), or a stereoisomer, racemate, tautomer, isotopologue, isotope-labeled compound, N-oxide, prodrug, solvate, or pharmaceutically acceptable salt thereof, and a ligand-drug conjugate represented by formula (C), or a stereoisomer, prodrug, or pharmaceutically acceptable salt or solvate thereof.

The present disclosure also provides use of a compound represented by the formula (GH), or a stereoisomer, racemate, tautomer, isotopologue, isotope-labeled compound, N-oxide, prodrug, solvate, or pharmaceutically acceptable salt thereof, or a ligand-drug conjugate represented by the formula (C), or a stereoisomer, racemate, tautomer, isotopologue, isotope-labeled compound, N-oxide, prodrug, solvate, or pharmaceutically acceptable salt thereof, for preventing and/or treating a disease or condition and/or manufacturing a medicament.

According to an embodiment of the present disclosure, the medicament is used for preventing and/or treating a disease or disorder.

The present disclosure also provides a method of preventing and/or treating a disease or condition comprising administering to a patient a therapeutically effective amount of at least one selected from the group consisting of a compound represented by Formula (GH), or a stereoisomer, racemate, tautomer, isotopologue, isotope-labeled compound, N-oxide, prodrug, solvate, or pharmaceutically acceptable salt thereof; a ligand-drug conjugate represented by Formula (C), or a stereoisomer, racemate, tautomer, isotopologue, isotope-labeled compound, N-oxide, prodrug, solvate, or pharmaceutically acceptable salt thereof.

According to an embodiment of the present disclosure, the disease or disorder can be selected from a tumor, such as a solid tumor or a hematological cancers.

Examples of the solid tumor include malignancies of various organ systems, e.g., sarcomas, adenocarcinomas, blastomas, and carcinomas, such as those affecting liver, lung, breast, lymphoid, biliary bowel (e.g., colon), genitourinary tract (e.g., kidney, urothelial cells), prostate, and pharynx. Adenocarcinomas include malignancies such as most of colon cancers, rectum cancers, renal cell cancers, liver cancers, small cell lung cancers, non-small cell lung cancers, small intestine cancers, and esophagus cancers. In one embodiment, the cancer is melanoma, e.g., advanced melanoma. Examples of other cancers that can be treated include: bone cancer, pancreatic cancer, skin cancer, head and neck cancer, malignant melanoma of skin or eye, uterine cancer, ovarian cancer, rectal cancer, colorectal cancer, cancer of the anal region, peritoneal cancer, gastric cancer, esophageal cancer, salivary gland cancer, testicular cancer, fallopian tube cancer, endometrial cancer, cervical cancer, vaginal cancer, vulvar cancer, penile cancer, malignant glioma, neuroblastoma, Hodgkin's disease, non-Hodgkin's lymphoma, esophageal cancer, small intestine cancer, endocrine system cancer, thyroid cancer, parathyroid cancer, adrenal cancer, soft tissue sarcoma, urethral cancer, chronic or acute leukemia (including acute myeloid leukemia, chronic myeloid leukemia, acute lymphoblastic leukemia, chronic lymphocytic leukemia), childhood solid tumors, lymphocytic lymphomas, bladder cancer, renal or ureteral cancer, renal pelvis cancer, central nervous system (CNS) tumors, primary CNS lymphomas, tumor angiogenesis, spinal axis tumors, brain stem gliomas, pituitary adenomas, Kaposi's sarcoma, neuroendocrine tumors (including carcinoid tumors, gastrinomas, and islet cell carcinomas), mesotheliomas, schwannomas (including acoustic neuromas), meningiomas, epidermoid carcinomas, squamous cell carcinomas, T-cell lymphomas, environmentally induced cancers (including cancers induced by asbestos), and combinations of the cancers.

Examples of the hematological cancer include leukemias, lymphomas, and malignant lymphoproliferative disorders affecting blood, bone marrow, and lymphatic system. Leukemias can be classified into acute and chronic leukemias. Acute leukemias can be further classified into acute myelogenous leukemias (AMLs) and acute lymphoblastic leukemias (ALLs). Chronic leukemia includes chronic myelogenous leukemia (CML) and chronic lymphocytic leukemia (CLL). Other associated conditions include myelodysplastic syndromes (MDS, formerly known as "preleukemia"), a diverse collection of hematological conditions combined by the ineffective production (or dysplasia) of bone marrow blood cells and the risk of transformation to AML. Lymphomas are a group of blood cell tumors that develop from lymphocytes. Exemplary lymphomas include non-Hodgkin's lymphoma and Hodgkin's lymphoma.

According to an embodiment of the present disclosure, the pharmaceutical composition may further comprise a pharmaceutically acceptable excipient, such as a carrier or excipient. The pharmaceutically acceptable excipient is preferably non-chemically reactive or inert to the active ingredient. For example, the pharmaceutically acceptable excipient is selected from at least one of the following excipients including but not limited to: fillers, disintegrants, binders, lubricants, surfactants, flavoring agents, humectants, matrix and the like.

According to an embodiment of the present disclosure, the route of administration of the pharmaceutical composition includes, but is not limited to, gastrointestinal or parenteral administration; wherein the gastrointestinal administration may be oral administration; the non-gastrointestinal administration may be topical administration, percutaneous administration, injection administration, or the like.

In one embodiment, the route of administration of the pharmaceutical composition can be topical administration combined with oral administration.

According to an embodiment of the present disclosure, the dosage form of the pharmaceutical composition may be selected from capsules, tablets, patches, films, pills, powders, lozenges, sachets, cachets, elixirs, suspensions, emulsions, solutions, syrups, aerosols, ointments, creams, suppositories, or injections.

### Definition and explanation of terms

Unless otherwise specified, the definitions of terms set forth in the specification and claims, including definitions thereof as examples, exemplary definitions, preferred definitions, definitions set forth in tables, definitions of specific compounds in embodiments, and the like, may be arbitrarily combined and incorporated with one another. Such combinations and incorporations shall fall within the scope of the specification of this disclosure.

The term "antibody-drug conjugate" (ADC) refers to the attachment of a targeting ligand, such as an antibody (e.g., a monoclonal antibody), or antibody fragment to a biologically active small molecule drug via a stable chemical linker compound. Those skilled in the art will appreciate that in the ADC, the antibody may be attached to different numbers of small molecule drugs. The average number of small molecule drugs attached to an antibody is usually denoted by DAR.

Unless otherwise stated, the numerical range contained in this specification and in the claims is equivalent to at least reciting every and each specific integer values therein. For example, the numerical range "0-10" is equivalent to reciting every and each integer value in the numerical range "0-10", i.e. 0, 1, 2, 3, 4, 5, 6, 7, 8, 9, or 10. It is understood that in the context of "one, two, or more" used herein in describing substituents, "more" should refer to an integer of ≥ 3, such as 3, 4, 5, 6, 7, 8, 9, or 10. When the expression "one or more" is used herein to describe substituents, then "more" can be understood as an integer > 1, such as 2, 3, 4, 5, 6, 7, 8, 9, or 10. In addition, when certain numerical ranges are defined as "numbers", it should be understood that the two endpoints of the range, each integer within the range, and each decimal within the range are recited. For example, "0-10" should be understood to mean not only reciting every and each of the integers 0, 1, 2, 3, 4, 5, 6, 7, 8, 9 and 10, but also reciting at least the sum of each of the integers with 0.1, 0.2, 0.3, 0.4, 0.5, 0.6, 0.7, 0.8, 0.9, respectively. Similarly, the numerical ranges "0 to 5", "0 to 4", "0 to 3", "0 to 6" and "an integer or decimal of 1 to 50", "an integer or decimal of 1 to 20", "an integer or decimal of 1 to 10" should be understood to mean that the two endpoints of the above numerical ranges are recited, as well as each integer within each range and each decimal within that range.

The term "isotopologue" includes the replacement of an atom in a compound or conjugate of the present disclosure with another isotope having the same atomic number but different atomic mass or mass number. For example, the "hydrogen" in the compound or conjugate of the present disclosure may be selected from the group consisting of protium (¹H), deuterium (²H) and tritium (³H); the "carbon" may be selected from the group consisting of ¹²C, ¹³C and ¹⁴C. The compound or conjugate of the present disclosure should therefore be understood at least as comprising those in various deuterated forms. For example, one, two, or more of each available hydrogen atom (e.g., a hydrogen atom of a C₁₋₁₀ alkyl) attached to a carbon atom may be independently replaced by a deuterium atom. those skilled in the art is able to synthesize the deuterated form of the compound or conjugate with reference to the relevant literatures. In the preparation of the deuterated form of the compound or conjugate, commercially available deuterated starting substances may be used or synthesized using conventional techniques with deuterated reagents. The possible deuterated reagents include, but are not limited to, deuterated borane, trideuterated borane tetrahydrofuran solution, deuterated lithium aluminum hydride, deuterated iodoethane, deuterated iodomethane, and the like.

The term "isotope-labeled compound" includes, but is not limited to, the compound or conjugate of the present disclosure labelled using at least one of the following elements: ¹¹¹In, ¹⁷⁷Lu, ²¹²Bi, ²¹³Bi, ²¹At, ⁶²Cu, ⁶⁷Cu, ⁹⁰Y, ¹²⁵I, ¹³¹I, ³²P, ³³P, ⁴⁷Sc, ¹¹¹Ag, ⁶⁷Ga, ¹⁴²Pr, ¹⁵³Sm, ¹⁶¹Tb, ¹⁶⁶Dy, ¹⁶⁶Ho, ¹⁸⁶Re, ¹⁸⁸Re, ¹⁸⁹Re, ²¹²Pb, ²²³Ra, ²²⁵Ac, ⁵⁹Fe, ⁷⁵Se, ⁷⁷As, ⁸⁹Sr, ⁹⁹Mo, ¹⁰⁵Rh, ¹⁰⁹Pd, ¹⁴³Pr, ¹⁴⁹Pm, ¹⁶⁹Er, ¹⁹⁴Ir, ¹⁹⁸Au, ¹⁹⁹Au, ^{227Th} and ²¹¹Pb.

The term "prodrug" represents the in vivo conversion of a compound to an active compound represented by the general formula. Such a conversion is influenced by hydrolysis of the prodrug in blood or enzymatic conversion to the parent structure in blood or tissues. The prodrug compound of the present disclosure may be an ester, and in the present invention the ester which may act as a prodrug includes a phenyl ester, an aliphatic (C₁₋₂₄) ester, an acyloxymethyl ester, a carbonate, a carbamate and an amino acid ester. For example, a compound in the present disclosure contains a hydroxy, this means that it can be acylated to obtain a compound in a prodrug form. Other prodrug forms include a phosphate ester, which are obtained by phosphorylation of the hydroxy on the parent. A complete discussion of prodrugs can be made with reference to the following: T. Higuchi and V. Stella, Pro-drugs as Novel Delivery Systems, Vol.14 of the A.C.S. Symposium Series, Edward B. Roche, ed., Bioreversible Carriers in Drug Design, American Pharmaceutical Association and Pergamon Press, 1987, J. Rautio et al., Prodrugs: Design and Clinical Applications, Nature Review Drug Discovery, 2008, 7, 255-270, and S. J. Hecker et al., Prodrugs of Phosphates and Phosphonates, Journal of Medical Chemistry, 2008, 51, 2328-2345.

The term "N-oxide" means that when a compound contains several amine functional groups, one or more nitrogen atoms can be oxidized to form an N-oxide. Special examples of N-oxides are N-oxides of tertiary amines or N-oxides of nitrogen-containing heterocyclic nitrogen atoms. The corresponding amine may be treated with an oxidizing agent, such as hydrogen peroxide, or a peracid, such as a peroxycarboxylic acid, to form an N-oxide (see Advanced Organic Chemistry, Wiley Interscience, 4th edition, Jerry March, pages). In particular, the N-oxide can be prepared by the process of L. W. Deady (Syn. Comm. 1977, 7, 509-514), wherein an amine compound is reacted with m-chloroperoxybenzoic acid (MCPBA), for example in an inert solvent, e.g. methylene chloride.

The term "halogen" denotes fluorine, chlorine, bromine, and iodine.

The term "alkyl" should be understood to mean preferably a linear or branched saturated monovalent hydrocarbon radical, preferably "C₁₋₁₀ alkyl". "C₁₋₁₀ alkyl" should be understood to preferably denote a linear or branched saturated monovalent hydrocarbon radical having 1 to 10 carbon atoms. For example, "C₁₋₆ alkyl" denotes a linear and branched alkyl having 1, 2, 3, 4, 5 or 6 carbon atoms. "C₁₋₄ alkyl" denotes a linear and branched alkyl having 1, 2, 3 or 4 carbon atoms. Said alkyl is, for example, methyl, ethyl, propyl, butyl, pentyl, hexyl, isopropyl, isobutyl, sec-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl or the like or isomers thereof.

The term "cycloalkyl" should be understood to mean a saturated monovalent monocyclic hydrocarbon or bicyclic (fused, bridged or spiro) hydrocarbon, preferably a "C₃₋₁₀ cycloalkyl". The term "C₃₋₁₀ cycloalkyl" should be understood to mean a saturated monovalent monocyclic, bicyclic (fused, bridged, or spiro) hydrocarbon having 3 to 10 carbon atoms, such as 3, 4, 5, 6, 7, 8, 9, or 10 carbon atoms. The term "C₃₋₆ cycloalkyl" has 3 to 6 carbon atoms, such as 3, 4, 5 or 6 carbon atoms. The cycloalkyl may be a monocyclic hydrocarbon, such as cyclopropyl, cyclobutyl, cyclopentyl, cyclohexyl, cycloheptyl, cyclooctyl, cyclononyl or cyclodecyl, or a bicyclic hydrocarbon, such as decalin ring.

The term "ring structure" should be understood to mean a group having a monocyclic, bicyclic (fused, bridged or spiro) or more ring structure, such as a monocyclic hydrocarbon, a bicyclic hydrocarbon, a tricyclic hydrocarbon, a hetero-monocyclic hydrocarbon, a hetero-bicyclic hydrocarbon, a hetero-tricyclic hydrocarbon, etc.. The ring structure may be saturated or unsaturated. The term "ring structure" may preferably be a 4-10 membered ring structure having 4, 5, 6, 7, 8, 9 or 10 ring atoms or preferably a 3-10 membered ring structure having 3, 4, 5, 6, 7, 8, 9 or 10 ring atoms. The hetero-monocyclic hydrocarbon, hetero-bicyclic hydrocarbon, hetero-tricyclic hydrocarbon may comprise 1-10, preferably 1-5, heteroatoms independently selected from N, O and S, for example, 1, 2, 3, 4 or 5 heteroatoms independently selected from N, O and S. The bicyclic ring may be the following ring structure containing no heteroatom or containing 1, 2 or 3 heteroatoms independently selected from N, O and S: one or two of aryl, heteroaryl, cycloalkyl and heterocyclyl alone or fused to each other, spiro [2.5] ring, spiro [3.3] ring, spiro [4.2] ring, spiro [4.3] ring, spiro [5.2] ring, spiro [5.4] ring, bicyclo [2.1.1], bicyclo [2.2.1], bicyclo [2.2.2], bicyclo [3.2.1], bicyclo [4.1.0] or the like. Alternatively, the ring structure may have 1, 2, 3, 4 or 5 double bonds, such as a carbon-carbon double bond or a carbon-nitrogen double bond.

In various parts of the present disclosure, linking substituents are described. When a linker group is clearly required for the structure, the Markush variables enumerated for that group should be understood as a linker group. For example, if the structure requires a linking group and the Markush group definition for that variable enumerates "alkyl" or "aryl", it should be understood that this "alkyl" or "aryl" represents a linked alkylene or arylene, respectively.

The term "alkylene" should be understood to mean a saturated linear or branched aliphatic hydrocarbon having a residue derived from the removal of two hydrogen atoms from the same carbon atom or two different carbon atoms of the parent alkane, and is a linear or branched group comprising 1 to 20 carbon atoms, preferably an alkylene comprising 1 to 10 carbon atoms (e.g. 1, 2, 3, 4, 5, 6, 7, 8, 9 or 10 carbon atoms), more preferably 1 to 6, or 1 to 4 carbon atoms. Non-limiting examples of alkylene include, but are not limited to, methylene (-CH₂-), 1,1-ethylidene (-CH(CH₃)-), 1,2-ethylidene (-CH₂CH₂-), 1,1-propylidene (-CH(CH₂CH₃)-), 1,2-propylidene (-CH₂CH(CH₃)-), 1,3-propylidene (-CH₂CH₂CH₂-), 1,4-butylidene (-CH₂CH₂CH₂CH₂-), and 1,5-butylidene (-CH₂CH₂CH₂CH₂-). The alkylene may be substituted or unsubstituted, and when substituted, the substituents may be substituted at any available attachment site, said substituents independently preferably being substituted by one or more substituents selected from alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio, and oxo.

The term "alkenylene" should be understood to mean a linear or branched group of 1 to 20 carbon atoms and containing at least one carbon-carbon double bond, preferably containing 2 to 10 carbon atoms (e.g. 2, 3, 4, 5, 6, 7, 8, 9 or 10 carbon atoms) and containing at least one carbon-carbon double bond, more preferably containing 2 to 6 carbon atoms and containing at least one carbon-carbon double bond. Non-limiting examples of alkenylene include, but are not limited to -CH=CH-, -C(CH₃)=CH-, -CH=CHCH₂CH₂-, -CH=CHCH₂-, -CH=CHCH₂CH=CH-, - CH=CHCH₂CH₂CH=CH- or -CH=CHCH₂CH=CHCH₂CH=CH-. The alkenylene group may be substituted or unsubstituted, and when substituted, the substituents may be substituted at any available attachment site, said substituents independently preferably being substituted by one or more substituents selected from alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio, and oxo.

The term "alkynylene" should be understood to mean a linear or branched group of 1 to 20 carbon atoms and containing at least one carbon-carbon triple bond, preferably containing 1 to 10 carbon atoms (e.g. 1, 2, 3, 4, 5, 6, 7, 8, 9 or 10 carbon atoms) and containing at least one carbon-carbon triple bond, more preferably containing 1 to 6 carbon atoms or 1 to 4 carbon atoms and containing at least one carbon-carbon triple bond. Non-limiting examples of alkynylene include, but are not limited to -CH≡CH-, -C(CH₃)≡CH-, -CH≡CHCH₂CH₂-, -CH≡CHCH₂-, - CH≡CHCH₂CH≡CH-, -CH≡CHCH₂CH₂CH≡CH- or -CH≡CHCH₂CH≡CHCH₂CH≡CH-. The alkynylene group may be substituted or unsubstituted, and when substituted, the substituents may be substituted at any available attachment site, said substituents independently preferably being substituted by one or more substituents selected from the group consisting of alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio, and oxo.

It should be understood that when a group of a compound described in the present disclosure forms a fused ring, spiro ring or bicyclic ring with the original ring structure of the compound after forming the above ring structure, and an unsaturated bond exists in the original ring structure of the compound, the ring structure formed by the group preferably retains the unsaturated bond in the original ring structure.

The term "heterocyclyl" means a saturated monovalent monocyclic, bicyclic (fused, bridged or spiro) hydrocarbon comprising 1 to 5 heteroatoms independently selected from N, O and S, preferably "3 to 20 membered heterocyclyl". The term "3-20 membered heterocyclic group" means a saturated monovalent monocyclic, bicyclic (fused, bridged or spiro) hydrocarbon comprising a non-aromatic cyclic group having 1-5 heteroatoms independently selected from N, O and S and having an overall ring atom number of 3-20 (e.g., atom number of 3, 4, 5, 6, 7, 8, 9, 10, etc.), preferably a "3-10 membered heterocyclic group". The term "3-10 membered heterocyclyl" means a saturated monovalent monocyclic, bicyclic (fused, bridged or spiro) hydrocarbon comprising 1-5, preferably 1-3, heteroatoms independently selected from N, O and S, e.g. 1, 2 or 3 heteroatoms independently selected from N, O and S. The heterocyclyl may be linked to the remainder of the molecule via any one of the carbon atoms or a nitrogen atom (if present). In particular, the heterocyclyl may include, but is not limited to, a 4-membered ring, such as azetidinyl, oxetidinyl (e.g., an azetidin-1-yl); a 5-membered ring, such as tetrahydrofuranyl, dioxolenyl, pyrrolidinyl, imidazolidinyl, pyrazolidinyl, pyrrolidinyl; or a 6-membered ring, such as tetrahydropyranyl, piperidinyl, morpholinyl, dithianyl, thiomorpholinyl, piperazinyl or trithianyl; or a 7-membered ring, such as diazacycloheptyl. Optionally, the heterocyclyl may be benzo-fused. The heterocyclyl may be bicyclic, such as, but not limited to, a 5,5-membered ring, such as hexahydrocyclopenta [c] pyrrol-2 (1H)-yl ring, or a 5, 6-membered bicyclic ring, such as hexahydropyrrolo [1,2-a] pyrazin-2 (1H)-yl ring. The nitrogen atom-containing ring may be partially unsaturated, i.e., it may comprise one or more double bonds, such as, but not limited to, 2,5-dihydro-1H-pyrrolyl, 4H-[1,3,4] thiadiazinyl, 4,5-dihydrooxazolyl or 4H-[1,4] thiazinyl, or, it may be benzo-fused, such as, but not limited to, dihydroisoquinolinyl. According to the present disclosure, the heterocyclyl are aromaticity-free. When the heterocyclyl is linked to other groups to constitute the compound of the present disclosure, the carbon atom on the heterocyclyl may be linked to the other groups, or the heterocyclic atom on the heterocyclyl ring may be linked to other groups. For example, when the heterocyclyl is selected from piperazinyl, the nitrogen atom on the piperazinyl may be linked to other groups. Or when the heterocyclyl is selected from piperidinyl, the nitrogen atom on the piperidinyl ring and the carbon atom in the para position thereof may be linked to other groups.

The term "aryl" should be understood as a monovalent aromatic or partially aromatic monocyclic, bicyclic or tricyclic hydrocarbon ring group, preferably "C₆₋₂₀ aryl". The term "C₆₋₂₀ aryl" should be understood to denote preferably a monovalent aromatic or partially aromatic monocyclic, bicyclic or tricyclic hydrocarbon ring having 6 to 20 carbon atoms, preferably "C₆₋₁₄ aryl" or "C₆₋₁₂ aryl". The term "C₆₋₁₄ aryl" should be understood to preferably denote a monovalent aromatic or partially aromatic monocyclic, bicyclic or tricyclic hydrocarbon ring having 6, 7, 8, 9, 10, 11, 12, 13 or 14 carbon atoms ("C₆₋₁₄ aryl"), in particular a ring having 6 carbon atoms ("C₆ aryl"), such as phenyl, or biphenyl, or a ring with 9 carbon atoms ("C₉ aryl"), such as indanyl or indenyl, or a ring with 10 carbon atoms ("C₁₀ aryl"), such as tetrahydronaphthyl, dihydronaphthyl or naphthyl, or a ring with 13 carbon atoms ("C₁₃ aryl"), such as fluorenyl, or a ring with 14 carbon atoms ("C₁₄ aryl"), such as anthracenyl. When the aryl is substituted, it may be mono-or multi-substituted. Furthermore, there is no restriction on the site of substitution thereof, for example, it may be ortho-, para-or meta-substitution.

The term "heteroaryl" should be understood as a monovalent monocyclic, bicyclic or tricyclic aromatic ring system group comprising 1-5 heteroatoms independently selected from N, O and S, preferably "5-20 membered heteroaryl". The term "5-20 membered heteroaryl" should be understood to include such a monovalent monocyclic, bicyclic or tricyclic aromatic ring system group having 5-20 ring atoms and containing 1-5 heteroatoms independently selected from N, O and S, such as "5-14 membered heteroaryl" or "5-12 membered heteroaryl". The term "5-14-membered heteroaryl" should be understood to include such a monovalent monocyclic, bicyclic or tricyclic aromatic ring system groups having 5, 6, 7, 8, 9, 10, 11, 12, 13 or 14 ring atoms, in particular 5, 6, 9 or 10 carbon atoms, and containing 1-5, preferably 1, 2 or 3 heteroatoms independently selected from N, O and S and, additionally, can be benzo-fused in each case. In particular, the heteroaryl is selected from thienyl, furyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl and the like and benzo derivatives thereof, such as benzofuranyl, benzothienyl, benzoxazolyl, benzisoxazolyl, benzimidazolyl, benzotriazolyl, indazolyl, indolyl, isoindolyl and the like; or pyridyl, pyridazinyl, pyrimidinyl, pyrazinyl, triazinyl, or the like, and their benzo derivatives, such as quinolyl, quinazolinyl, isoquinolinyl, or the like; or azocinyl, indazinyl, purinyl and the like and their benzo derivatives; or cinolinyl, phthalazinyl, quinazolinyl, quinoxalinyl, naphthyridinyl, pteridinyl, carbazolyl, acridinyl, phenazinyl, phenothiazinyl, phenoxazinyl, etc. When the heteroaryl is attached to other groups to constitute the compounds of the present disclosure, the carbon atom on the heteroaryl ring may be attached to the other groups, or the heteroatom on the heteroaryl ring may be attached to other groups. When the heteroaryl is substituted, it may be mono-or multi-substituted. Furthermore, there is no restriction on the site of substitution thereof, for example, it may be that a hydrogen attached to a carbon atom on the heteroaryl ring is substituted, or a hydrogen attached to a heteroatom on the heteroaryl ring is substituted.

Unless otherwise specified, heterocyclyl, heteroaryl or heteroarylidene groups include all possible isomeric forms thereof, such as positional isomers thereof. Thus, for some illustrative and non-limiting examples, this may include forms substituted at one, two, or more of its 1-, 2-, 3-, 4-, 5-, 6-, 7-, 8-, 9-, 10-, 11-, 12-positions, or the like (if present) or bonded to other groups, including pyridin-2-yl, pyridyli-2-dene, pyridin-3-yl, pyridyli-3-dene, pyridin-4-yl, and pyridyli-4-dene; thienyl or thienylidene groups, including thien-2-yl, thienyli-2-dene, thien-3-yl and thienyli-3-dene; pyrazol-1-yl, pyrazol-3-yl, pyrazol-4-yl, or pyrazol-5-yl.

The term "oxo" refers to the substitution (= O) of a carbon, nitrogen, or sulfur atom in the substituent by an oxy group formed by oxidation. It should be understood that carbonyl -C(=O)-is formed when a carbon atom is replaced by an oxy group.

Unless otherwise stated, the definitions of terms herein apply equally to groups containing the term, for example, the definition of alkyl also applies to alkyl in alkyloxy or cycloalkylalkyl; the definition of cycloalkyl also applies to cycloalkyl in cycloalkyloxy or cycloalkylalkyl. For another example, the definition of C₁₋₁₀ alkyl also applies to C₁₋₁₀ alkyl in C₁₋₁₀ alkyloxy or C₃₋₁₀ cycloalkyl-C₁₋₁₀ alkyl; the definition of C₃₋₁₀ cycloalkyl also applies to C₃₋₁₀ cycloalkyl in C₃₋₁₀ cycloalkyloxy or C₃₋₁₀ cycloalkyl-C₁₋₁₀ alkyl.

The term "thiol reactive group" refers to any group that can react with the thiol groups contained in Tp (e.g., an antibody), such as maleimide group, substituted maleimide group, halogen, OMs, OTs, OTf, nitro group, alkyl thioether group, aryl thioether group, heteroaryl thioether group, alkyl sulfoxide group, aryl sulfoxide group, heteroaryl sulfoxide group, alkyl sulfonyl, aryl sulfonyl, or heteroaryl sulfonyl.

The term "amino reactive group" refers to any group that can react with amino contained in Tp (e.g., an antibody), such as a succinimidyl ester (NHS), a nitrophenyl ester (NPC), a pentafluorophenyl ester (PFP), an aldehyde (CHO) such as an acetaldehyde or a propionaldehyde, an epoxy (EPO) or an isothiocyanate (ISC).

The term "carboxyl reactive group" refers to any group that can react with a carboxyl contained in Tp (e.g., an antibody), such as hydroxyl, amino, thiol, halogen, amidinoor guanidino.

The term "dithiol bridging group" refers to any bifunctional linker that can react with two free thiol/sulfhydryl contained in Tp (e.g., an antibody) to form a bridging, e.g., a bismaleimide reagent.

The term "click chemical reaction group" refers to a group that can undergo a "click chemical" reaction, such as ketone, hydrazine or hydrazide, azide, an alkyne, cyclopropene, or diene. A typical click chemical reaction is, for example, the reaction of an azide with an alkyne to form a 5-membered heteroatom ring. Those skilled in the art may introduce click chemical groups into an antibody or other polypeptide targeting group, Tp, by techniques such as codon expansion.

It is appreciated by those skilled in the art that the compounds of the present disclosure may be present in the form of various pharmaceutically acceptable salts. If these compounds have a basic center, they can form acid addition salts; if these compounds have an acidic center, they can form base addition salts; if these compounds have both an acidic center (e.g., carboxyl) and a basic center (e.g., amino), they can also form inner salts.

The compounds of the present disclosure may exist as solvates (e.g. hydrates), wherein the compounds of the present disclosure comprise a polar solvent, particularly, for example, water, methanol or ethanol, as a structural element of the crystal lattice of the compound. The amount of the polar solvent, particularly water, may be present stoichiometrically or non-stoichiometrically.

Depending on their molecular structure, the compounds or groups of the present disclosure may be chiral or have a chiral carbon atom, and thus may be present in various enantiomeric forms. These compounds or groups may thus exist in racemate form or in optically active form. For example, when group A in the general formula (G), (G') or (GH) is C-R^{A} and the groups to which the carbon atom is attached are not the same, the carbon atom is a chiral carbon atom, which may have a chiral configuration of R or S. The compounds of the present disclosure, or intermediates thereof, may be separated as enantiomeric compounds by chemical or physical methods well known to those skilled in the art or used in synthesis in this form. In the case of racemic amines, diastereomers can be prepared from the mixture by reaction with optically active resolution reagents. Examples of suitable resolution reagents are optically active acids such as tartaric acid, diacetyltartaric acid, dibenzoyltartaric acid, mandelic acid, malic acid, lactic acid, suitably N-protected amino acids such as N-benzoylproline or N-benzenesulfonylproline in R and S forms, or various optically active camphorsulfonic acids. Chromatographic enantiomeric resolution can also be advantageously carried out with the aid of optically active resolution reagents, such as derivatives of dinitrobenzoylphenylglycine, cellulose triacetate or other carbohydrates immobilized on silica gel or chirally derivatized isobutenate polymers. Suitable eluents for this purpose are water-containing or alcohol-containing solvent mixtures, e.g. Hexane/isopropanol/acetonitrile.

The term "tautomer" refers to an isomer of a functional group resulting from the rapid movement of an atom in a molecule in two positions. The compounds of the disclosure may exhibit tautomerism. A tautomeric compound may exist in two or more interconvertible species. Proton shift tautomers arise from the migration of a hydrogen atom covalently bonded between two atoms. Tautomers generally exist in equilibrium, and attempts to separate a single tautomer usually yield a mixture whose physicochemical properties are consistent with the mixture of compounds. The position of the equilibrium depends on the chemical properties within the molecule. For example, in many aliphatic aldehydes and ketones such as acetaldehyde, the ketone form predominates; in phenols, the enol form predominates . The present disclosure comprises all tautomeric forms of the compounds.

The corresponding stable isomers can be separated according to known methods, such as by extraction, filtration or column chromatography.

The term "patient" means any animal, including mammals, preferably mice, rats, other rodents, rabbits, dogs, cats, pigs, cattle, sheep, horses or primates, and most preferably humans.

The phrase "therapeutically effective amount" as used herein refers to the amount of an active compound or drug that is being sought by a researcher, veterinarian, physician, or other clinician in a tissue, system, animal, individual, or person to elicit a biological or medical response and includes one or more of the following: (1) prevention of a disease: e.g. prevention of a disease, disorder, or condition in an individual susceptible to a disease, disorder, or condition but who has not yet experienced or developed the pathology or symptoms of the disease; (2) inhibition of a disease: for example, inhibition of a disease, disorder, or condition in an individual who is experiencing or developing a pathology or symptom of the disease, disorder, or condition (i.e., prevention of further development of the pathology and/or symptom); (3) alleviating a disease: for example, alleviating a disease, disorder, or condition (i.e., reversing the pathology and/or symptom) in an individual who is experiencing or developing a pathology or symptom of the disease, disorder, or condition.

The term "ligand" is a macromolecular compound that recognizes and binds to an antigen or receptor associated with a target cell. The role of ligands is to present the drug to the target cell population to which the ligand binds, such ligands include, but are not limited to, proteinaceous hormones, lectins, growth factors, antibodies, or other molecules capable of binding to the cell. In an embodiment of the present disclosure, the ligand is denoted LG and the ligand may form a linking bond with a linking unit via a heteroatom on the ligand, preferably with an antibody or an antigen-binding fragment thereof, the antibody being selected from a chimeric antibody, a humanized antibody, a fully human antibody or a murine antibody, preferably a monoclonal antibody.

The term "drug" refers to any compound having a desired biological activity and a reactive functional group that can be used to incorporate the drug into the conjugate of the present disclosure. Desired biological activities include the diagnosis, curing, alleviation, treatment, or prevention of diseases in humans or other animals. Said reactive functional group forms a bond with the functional group L³ or L⁴. In some embodiments, the drug has a nitrogen atom or a hydroxyl that can form a bond with a functional group L³ or L⁴.

The term "antibody" refers to an immunoglobulin and includes a tetrapeptide chain structure consisting of two identical heavy chains and two identical light chains linked by an interchain disulfide bond (also referred to as a "monoclonal antibody" or a "monoepitope antibody"), or a tetrapeptide chain structure consisting of two different heavy chains and two different light chains linked by an interchain disulfide bond (also referred to as a "bi-antibody" or a "bi-epitope antibody"). The constant region of immunoglobulin heavy chain has different amino acid composition and sequence, so its antigenicity is also different. From this, immunoglobulins can be divided into five classes, or isotypes of immunoglobulins, that is, IgM, IgD, IgG, IgA, and IgE, the corresponding heavy chains of which are µ, δ, γ, α, and ε chains, respectively. The same class of Ig can be divided into different subclasses according to the amino acid composition in the hinge region and the number and position of disulfide bonds in the heavy chain. For example, IgG can be divided into IgG1, IgG2, IgG3 and IgG4. Light chains are classified into κ or λ chains by differences in the constant region. Each of the five classes of Ig can have either a κ chain or a λ chain. The antibody described in the present disclosure is preferably an antibody specific for a cell surface antigen on a target cell, with non-limiting examples being one or more of the following: anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-CD20 antibody, anti-CD22 antibody, anti-CD30 antibody, anti-CD33 antibody, anti-CD44 antibody, anti-CD56 antibody, anti-CD70 antibody, anti-CD73 antibody, anti-CD105 antibody, anti-CEA antibody, anti-A33 antibody, anti-Cripto antibody, anti-EphA2 antibody, anti-G250 antibody, anti-MUCl antibody, anti-Lewis Y antibody, anti-VEGFR antibody, anti-GPNMB antibody, anti-Integrin antibody, anti-PSMA antibody, anti-Tenascin-C antibody, anti- SLC44A4 antibody or anti-Mesothelin antibody; preferably, trastuzumab (trade name Herceptin), pertuzumab (also known as 2C4, trade name Perjeta), nimotuzumab (trade name Taixinsen), Enoblituzumab, Emibetuzumab, Inotuzumab, Pinatuzumab, Brentuximab, Gemtuzumab, Bivatuzumab, Lorvotuzumab, cBR96 or Glembatumumab.

The sequence of about 110 amino acids near the N-terminal of the heavy and light chains of the antibody is very variable, which is a variable region (Fv region); the rest of the amino acid sequence near the C-terminal is relatively stable, which is a constant region. The variable region includes three hypervariable regions (HVR) and four conserved backbone regions (FR). The specificity of the antibody is determined by the three hypervariable regions, also known as complementarity determining regions (CDRs). Each light chain variable region (LCVR) and heavy chain variable region (HCVR) is composed of 3 CDR regions and 4 FR regions, with the sequence from N-terminal to C-terminal being: FR1, CDR1, FR2, CDR2, FR3, CDR3, and FR4. The three CDR regions of the light chain refer to LCDR1, LCDR2, and LCDR3; the three CDR regions of the heavy chain refer to HCDR1, HCDR2, and HCDR3.

The antibody of the present disclosure includes a murine antibody, a chimeric antibody, a humanized antibody, and a fully human antibody, preferably a humanized antibody and a fully human antibody.

The term "murine antibody" in this disclosure is an antibody prepared from mice in accordance with the knowledge and skill in the art. A test object is injected with a specific antigen at the time of preparation, and then hybridomas expressing an antibody having a desired sequence or functional property are isolated.

The term "chimeric antibody" refers to an antibody obtained by fusing the variable regions of a murine antibody with the constant regions of a human antibody, which can alleviate the immune response induced by the murine antibody. To establish a chimeric antibody, hybridoma secreting mouse specific monoclonal antibody should be established firstly, then variable region genes should be cloned from hybridoma cells of mouse, then constant region genes of a human antibody is cloned according to need, then mouse variable region genes and human constant region genes are linked into chimeric gene and inserted into an expression vector, finally chimeric antibody molecules are expressed in eukaryotic system or prokaryotic system.

The term "humanized antibody", also known as CDR-grafted antibody, refers to an antibody produced by grafting murine CDR sequences into human antibody variable region frameworks, i.e., different types of human germline antibody framework sequences. The heterologous response induced by the chimeric antibody, which carries a large amount of murine protein components, can be overcome. Such framework sequences may be obtained from public DNA databases including germline antibody gene sequences or from published references. For example, germline DNA sequences of human heavy and light chain variable region genes can be found in the "VBase" human germline sequence database (available on the Internet at www.mrccpe.com.ac.uk/vbase), as well as in Kabat, E.A., et al., 1991Sequences of Proteins of Immunological Interest, 5th edition. In order to avoid the decrease of immunogenicity and the resultant decrease of activity, the human antibody variable region framework sequence can be subjected to a minimum of reverse mutation or reverse mutation to maintain activity. The humanized antibodies of the present disclosure also include humanized antibodies after further affinity maturation of CDRs by phage display. Literatures further describing methods involved in humanizing of usable mouse antibodies include, for example, the methods of Queen et al., Proc., Natl. Acad. Sci. USA, 88, 2869, 1991 and Winter and colleagues [Jones et al., Nature, 321, 522 (1986), Riechmann, et al., Nature, 332, 323-327 (1988), Verhoeyen, et al., Science, 239, 1534 (1988)].

The terms "fully human antibody", also referred to as "fully human monoclonal antibody", has a variable region and a constant region of human origin, so as to eliminate immunogenicity and toxic and side effects. The development of monoclonal antibody has experienced four stages: murine monoclonal antibody, chimeric monoclonal antibody, humanized monoclonal antibody and fully human monoclonal antibody. A fully human monoclonal antibody is used in the present disclosure. The techniques related to the preparation of fully human antibody mainly include human hybridoma technique, EBV transformed B lymphocyte technique, phage display technique, transgenic mouse antibody preparation technique and single B cell antibody preparation technique, and so on.

The term "antigen-binding fragment" refers to one or more fragments of an antibody that retain the ability to bind specifically to an antigen. It has been shown that fragments of the full-length antibody can be utilized to carry out the antigen binding function of the antibody. Examples of binding fragments contained in "antigen-binding fragments" include (i) a Fab fragment, which is a monovalent fragment consisting of VL, VH, CL and CH1 domains; (ii) an F (ab') 2 fragment comprising a bivalent fragment of two Fab fragments linked by a disulfide bridge in the hinge region, (iii) an Fd fragment consisting of VH and CH1 domains; (iv) an Fv fragment consisting of VH and VL domains of a single arm of an antibody; (v) a single domain or dAb fragment (Ward et al., (1989) Nature 341: 544-546) consisting of VH domains; and (vi) an isolated complementarity determining region (CDR) or (vii) a combination of two or more isolated CDRs which may optionally be linked by a synthetic linker. In addition, although the two domains of the Fv fragment, VL and VH, are encoded by separate genes, recombinant methods can be used to join them by a synthetic linker, thereby enabling it to produce a single protein chain in which the VL and VH regions pair to form a monovalent molecule (referred as single chain Fv (scFv); See, for example, Bird et al. (1988) Science 242: 423-426; and Huston et al. (1988) Proc. Natl. Acad. Sci USA 85: 5879-5883). Such single chain antibodies are also intended to be included in the term "antigen-binding fragment" of an antibody. Such antibody fragments are obtained using conventional techniques known to those skilled in the art, and the fragments are screened for functionality in the same manner as for intact antibodies. The antigen-binding moiety can be produced by recombinant DNA technology or by enzymatic or chemical cleavage of an intact immunoglobulin. The antibodies may be antibodies of different isotypes, for example, IgG (e.g., IgG1, IgG2, IgG3 or IgG4 subtypes), IgA1, IgA2, IgD, IgE or IgM antibodies.

A Fab is an antibody fragment having a molecular weight of about 50,000 and having antigen-binding activity in a fragment obtained by treating an IgG antibody molecule with the protease of the protease papain (cleaving the amino acid residue at position 224 of the H chain), wherein about half of the N-terminal side of the H chain and the entire L chain are bonded together by a disulfide bond.

F(ab') 2 is an antibody fragment having a molecular weight of about 100,000 and having antigen-binding activity and comprising two Fab regions joined at the hinge position, obtained by digesting the portion below the two disulfide bonds in the hinge region of IgG with the enzyme pepsin.

Fab' is an antibody fragment having a molecular weight of about 50,000 and having an antigen-binding activity, obtained by cleaving the disulfide bond of the hinge region of the above F(ab') 2.

Further, said Fab' can be produced by inserting a DNA encoding a Fab' fragment of an antibody into a prokaryotic expression vector or an eukaryotic expression vector and introducing the vector into a prokaryotic or eukaryotic organism to express the Fab'.

The term "single chain antibody", "single chain Fv" or "scFv" means a molecule comprising an antibody heavy chain variable domain (or region; VH) and an antibody light chain variable domain (or regions; VL) linked by a linker. Such scFv molecules may have the general structure: NH 2-VL-linker-VH-COOH or NH 2-VH-linker-VL-COOH. Suitable prior art linkers consist of repetitive GGGGS amino acid sequences or variants thereof, e.g. using 1-4 repetitive variants (Holliger et al. (1993), Proc. Natl. Acad. Sci. USA 90: 6444-6448). Other linkers useful in this disclosure are described by Alfthan et al. (1995), Protein Eng. 8: 725-731, Choi et al. (2001), Eur. J. Immuno. 31: 94-106, Hu et al. (1996), Cancer Res. 56: 3055-3061, Kipriyanov et al. (1999), J. Mol. Biol. 293: 41-56, and Roovers et al. (2001), Cancer Immunol.

The term "CDR" refers to one of the six hypervariable regions within the variable domain of an antibody that primarily contribute to antigen binding. One of the most commonly used definitions of the six CDRs was provided by Kabat E. A. et al., (1991) Sequences of proteins of immunological interest. NIH Publication 91-3242. As used herein, the Kabat definition of CDRs applies only to CDR1, CDR2, and CDR3 of the light chain variable domain (CDR L1, CDR L2, CDR L3 or L1, L2, L3), and to CDR2 and CDR3 of the heavy chain variable domain (CDR H2, CDR H3 or H2, H3).

The term "antibody framework" refers to a portion of a variable domain VL or VH, which serves as a scaffold for the antigen binding loop (CDR) of that variable domain. In essence, it is a variable domain that does not have CDRs.

The term "epitope" or "antigenic determinant " refers to the site on an antigen where immunoglobulin or antibody specifically binds. The epitope typically includes at least 3, 4, 5, 6, 7, 8, 9, 10, 11, 12, 13, 14 or 15 consecutive or non-consecutive amino acids in a unique spatial conformation. See, for example, Epitope Mapping Protocols in Methods in Molecular Biology, vol. 66, G. E. Morris, Ed. (1996).

The terms "specific binding", "selective binding", "selectively binding", and "specifically binding" refer to the binding of an antibody to an epitope on a predetermined antigen. Typically, the antibody binds with an affinity (KD) of about less than 10⁻⁷M, such as about less than 10⁻⁸M, 10⁻⁹M, or 10⁻¹⁰M or less.

The term "nucleic acid molecule" refers to a DNA molecule and a RNA molecule. The nucleic acid molecule may be single-stranded or double-stranded, but is preferably double-stranded DNA. The nucleic acid is "operatively linked" when placed in functional relationship with another nucleic acid sequence. For example, if the promoter or enhancer affects transcription of a coding sequence, then the promoter or enhancer is operatively linked to said coding sequence.

The term "carrier" refers to a nucleic acid molecule capable of transporting another nucleic acid to which it has been linked. In one embodiment, the vector is a "plasmid", which refers to a circular double-stranded DNA circle into which additional DNA segments can be linked. In another embodiment, the vector is a viral vector in which additional DNA segments can be linked into the viral genome. The vectors disclosed herein are capable of autonomous replication in a host cell into which they have been introduced (e.g., bacterial vectors having a bacterial origin of replication and episomal mammalian vectors) or can integrate into the genome of a host cell after introduction into the host cell, thereby replicating with the host genome (e.g., non-episomal mammalian vectors).

Methods for producing and purifying antibodies and antigen-binding fragments are well known in the prior art, such as Using Antibodies: A Laboratory Manual, published by Cold Spring Harbor Laboratory Press, chapters 5-8 and 15. Antigen-binding fragments can likewise be prepared by conventional methods. The antibody or antigen-binding fragment of the invention is genetically engineered by adding one or more human FR regions to a non-human CDR region. Human FR germline sequences are obtained from the ImMunoGeneTics (IMGT) website at http://imgt.cines.fr by aligning the IMGT Human Antibody Variable Region Germline Gene Database and MOE software, or from the Journal of Immunoglobulins, 2001ISBN012441351.

The term "host cell" refers to a cell into which an expression vector has been introduced. The host cells may include bacterial, microbial, plant or animal cells. Bacteria susceptible to transformation include members of the enterobacteriaceae, such as strains of Escherichia coli or Salmonella; Bacillaceae such as Bacillus subtilis; Pneumococcus; Streptococcus and Haemophilus influenzae. Suitable microorganisms include Saccharomyces cerevisiae and Pichia pastoris. Suitable animal host cell lines include CHO (Chinese hamster ovary cell line) and NS0 cells.

The antibodies or antigen-binding fragments engineered in the disclosure can be prepared and purified by conventional methods. For example, cDNA sequences encoding heavy and light chains can be cloned and recombined into a GS expression vector. The recombinant immunoglobulin expression vector can stably transfect CHO cells. As a more recommendable existing technique, mammalian expression systems result in glycosylation of antibodies, particularly at the highly conserved N-terminal site in the Fc region. Positive clones are expanded in serum-free medium in a bioreactor for antibody production. The culture fluid that secretes the antibody can be purified by conventional techniques. For example, purification is performed using A or G Sepharose FF columns containing an adjusted buffer. Nonspecifically bound components were washed away. The bound antibody was eluted by a pH gradient method, and the antibody fragments are detected by SDS-PAGE and collected. Antibodies can be filtered and concentrated using conventional methods. Soluble mixtures and polymers can also be removed by conventional methods, such as molecular sieves, or ion exchange. The resulting product should be immediately frozen, e.g.-70 °C, or lyophilized.

The term "peptide" refers to a compound fragment between amino acid and protein, which is composed of two or more amino acid molecules connected by peptide bond. It is the structural and functional fragment of a protein, such as hormones and enzymes, which are essentially peptides.

The term "sugar" refers to a biological macromolecule composed of C, H and O elements, which can be divided into monosaccharide, disaccharide and polysaccharide.

The term "amino acid analog" (e.g., "arginine analog", "lysine analog", or "histidine analog") refers to an amino acid variant that retains at least one function of that amino acid, such as a variant that retains side chain polarity or electrostatic interactions. Such variants may have elongated or shorter side chains, such as retaining side-chain polarity or electrostatic interactions through one or more-CH₂-groups. For example, the arginine analog may include an additional methylene or ethylene group between the backbone and the guanidine/guanidinium group.

### Beneficial effects

The compound and conjugate of the present disclosure have excellent tumor cell inhibiting activity, stability and in vivo drug effect in animals, and can be used as effective drugs for inhibiting tumor cell and preventing and/or treating cancer.

### Brief description of drawings

FIG. 1 illustrates the results of the bystander killing test of the antibody-drug conjugates of the present disclosure on HER2-targeted tumor cells;
FIG. 2 illustrates the results of the bystander killing test of antibody-drug conjugates of the present disclosure on EGFR-targeted tumor cells.

### DETAILED DESCRIPTION

The technical solution of the present disclosure will be further described in detail hereinafter in connection with specific examples. It will be understood that the following examples are only exemplary illustration and explanation of the disclosure and should not be construed as limiting the scope of the disclosure. All technologies realized based on the above contents of the disclosure are covered by the scope of the disclosure which is intended to be protected.

Unless otherwise specified, the starting materials and reagents used in the following examples are commercially available or can be prepared by known methods.

### I. Antibody Examples

The following antibodies were prepared according to conventional antibody methods, for example, after vector construction, eukaryotic cells such as HEK293 cells (Life Technologies Cat. No. 11625019) can be transfected
Exemplary antibody sequences are as follows:
The following is the sequence of Trastuzumab
Light chain 
DIQMTQSPSSLSASVGDRVTITCRASQDVNTAVAWYQQKPGKAPKLLIYSASFLYSGVPSRFSGSRS
GTDFTLTISSLQPEDFATYYCQQHYTTPPTFGQGTKVEIKRTVAAPSVFIFPPSDEQLKSGTASVVCLLNNF
YPREAKVQWKVDNALQSGNSQESVTEQDSKDSTYSLSSTLTLSKADYEKHKVYACEVTHQGLSSPVTKS
FNRGEC
SEQ ID NO: 1
Heavy chain

EVQLVESGGGLVQPGGSLRLSCAASGFNIKDTYIHWVRQAPGKGLEWVARIYPTNGYTRYADSVK
GRFTISADTSKNTAYLQMNSLRAEDTAVYYCSRWGGDGFYAMDYWGQGTLVTVSSASTKGPSVFPLAP
SSKSTSGGTAALGCLVKDYFPEPVTVSWNSGALTSGVHTFPAVLQSSGLYSLSSVVTVPSSSLGTQTYICN
VNHKPSNTKVDKKVEPKSCDKTHTCPPCPAPELLGGPSVFLFPPKPKDTLMISRTPEVTCVVDVSHEDPE
VKFNWYVDGVEVHNAK
SEQ ID NO: 2

The following is the sequence of Pertuzumab
Light chain 
DIQMTQSPSSLSASVGDRVTITCKASQDVSIGVAWYQQKPGKAPKLLIYSASYRYTGVPSRFSGSGS
GTDFTLTISSLQPEDFATYYCQQYYIYPYTFGQGTKVEIKRTVAAPSVFIFPPSDEQLKSGTASVVCLLNNF
YPREAKVQWKVDNALQSGNSQESVTEQDSKDSTYSLSSTLTLSKADYEKHKVYACEVTHQGLSSPVTKS
FNRGEC
SEQ ID NO.3
Heavy chain

EVQLVESGGGLVQPGGSLRLSCAASGFTFTDYTMDWVRQAPGKGLEWVADVNPNSGGSIYNQRFK
GRFTLSVDRSKNTLYLQMNSLRAEDTAVYYCARNLGPSFYFDYWGQGTLVTVSSASTKGPSVFPLAPSS
KSTSGGTAALGCLVKDYFPEPVTVSWNSGALTSGVHTFPAVLQSSGLYSLSSVVTVPSSSLGTQTYICNV
NHKPSNTKVDKKVEPKSCDKTHTCPPCPAPELLGGPSVFLFPPKPKDTLMISRTPEVTCVVVDVSHEDPE
VKFNWYVDGVEVHNAK
SEQ ID NO.4

The following is the sequence of Nimotuzumab
Light chain 
DIQMTQSPSSLSASVGDRVTITCRSSQNIVHSNGNTYLDWYQQTPGKAPKLLIYKVSNRFSGVPSRFS
GSGSGTDFTFTISSLQPEDIATYYCFQYSHVPWTFGQGTKLQITREVAAPSVFIFPPSDEQLKSGTASVVCL
LNNFYPREAKVQWKVDNALQSGNSQESVTEQDSKDSTYSLSSTLTLSKADYEKHKVYACEVTHQGLSSP
VTKSFNRGEC
SEQ ID NO: 5
Heavy chain

QVQLQQSGAEVKKPGSSVKVSCKASGYTFTNYYIYWVRQAPGQGLEWIGGINPTSGGSNFNEKFKT
RVTITVDESTNTAYMELSSLRSEDTAFYFCARQGLWFDSDGRGFDFWGQGSTVTVSSASTKGPSVFPLAP
SSKSTSGGTAALGCLVKDYFPEPVTVSWNSGALTSGVHTFPAVLQSSGLYSLSSVVTVPSSSLGTQTYICN
VNHKPSNTKVDKKVEPKSCDKTHTCPPCPAPELLGGPSVFLFPPKPKDTLMISRTPEVTCVVDVSHEDPE
VKFNWYVDG
SEQ ID NO: 6

### II. Examples of compounds

The structures of the compounds were determined by Nuclear Magnetic Resonance (NMR) or Mass Spectrometry (MS). The chemical shifts δ were given in a unit of 10⁻⁶ (ppm). NMR was measured by Bruker instrument with deuterated dimethyl sulfoxide (DMSO-*d₆*), deuterated chloroform (CDCl₃) and deuterated methanol (CD₃OD) as solvents and tetramethylsilane (TMS) as internal standard.

For LCMS determination: Agilent 1260 Infinity II (ESI) mass spectrometer, Waters UPLC H Class plus (ESI) or Shimadzu LCMS-2020 (ESI).

High performance liquid chromatography (HPLC) analysis used Agilent 1260 or Shimadzu LC-20AD.

GILSON GX-281 or Agilent 1260 Infinity II was used for Preparative High Performance Liquid Chromatography (pre-HPLC) to prepare the liquid phase.

Supercritical fluid chromatography (SFC) with Shimadzu LC-30Adsf or Shimadzu LC-20AD instrumentation was used for chiral preparation.

GF254 acrylic adhesive silica gel plate of Anhui Liangchen Silicon Source Material Co., Ltd. was used for Thin-Layer Chromatography (TLC). The specification of silica gel plate used for TLC was 0.2 mm silica gel plate, and the specification of TLC separation and purification of products was 0.5 mm silica gel plate.

For the column chromatography, 200-300 mesh silica gel of Anhui Liangchen Silicon Source Material Co., Ltd. was used as a carrier.

The mean kinase inhibition rate and IC₅₀ value were determined with SpectraMax i3X microplate reader (MD corporation, USA).

Known starting materials of the present disclosure may be synthesized by or according to methods known in the art, or may be purchased from Bid Pharmaceuticals, LEYAN, Shaoyuan Chemical Technology, Anneji Chemicals, and the like.

In the following examples, the reactions were carried out under an argon or nitrogen atmosphere, unless otherwise specified.

An argon or nitrogen atmosphere means that the reactor is connected to an argon or nitrogen balloon of about 1L volume.

Hydrogen atmosphere refers to the reactor connected to a hydrogen balloon of about 1L volume.

The hydrogenation reaction is usually evacuated to vacuum and filled with hydrogen gas and repeated three times.

The oxygen atmosphere means that the reactor is connected to an oxygen balloon with a volume of about 1L.

Unless otherwise specified in the examples hereinafter, the solution refers to an aqueous solution, and the temperature of the reaction is room temperature, 20 °C-30 °C.

In the examples, the reaction process was monitored by thin-layer chromatography (TLC), the developing agent used for the reaction, the eluent system for column chromatography used for purifying the compound and the developing agent system for thin-layer chromatography included: A: dichloromethane/methanol system, B: petroleum ether/ethyl acetate system, the volume ratio of the solvents was adjusted according to the polarity of the compound, a small amount of basic or acidic reagent such as triethylamine and acetic acid could be added for adjustment.

### Example 2-1: Preparation of Compound 1

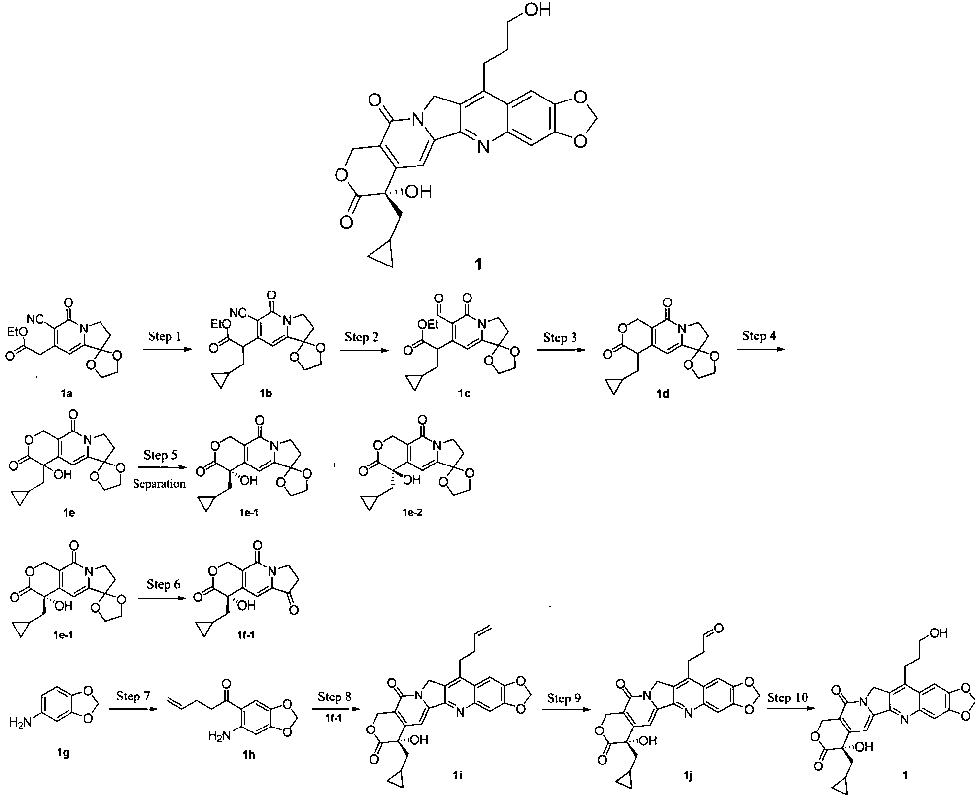

### Step 1

### Ethyl 2-(6-cyano-5-oxo-2, 3-dihydro-5H-spiro [indolizine-1, 2'-[1, 3] dioxolane]-7-yl)-3-cyclopropylpropanoate 1b

**1a** (1.01 g, 3.31 mmol, prepared according to the method disclosed in example 30 on page 28 of patent application "WO2019238046") was dissolved in 15 mL of acetonitrile, bromomethylcyclopropane (894.93 mg, 6.63 mmol) and potassium carbonate (916.16 mg, 6.63 mmol) were added and stirred at 80 °C for 13 hours. 10 mL of water was added, the diluted reaction solution was extracted with ethyl acetate (15 mL × 2), the organic phase was washed with saturated sodium chloride solution (10 mL × 2), then dried over anhydrous sodium sulfate, filtered, the filtrate was concentrated by distillation under reduced pressure, and the residue obtained was purified by silica gel column chromatography with developing system A to afford the title product **1b** (1.12 g, yield: 92%) as a yellow solid.
MS m/z (ESI): 359.1 [M+1].

### Step 2

### Ethyl 3-cyclopropyl-2-(6-formyl-5-oxo-2, 3-dihydro-5H-spiro [indolizine-1, 2'-[1, 3] dioxolan]-7-yl) propanoate 1c

**1b** (1.12 g, 3.05 mmol) was dissolved in a mixture of 5 mL of water, 5 mL of acetonitrile and 5 mL of formic acid under nitrogen atmosphere, Raney nickel (261.72 mg) was added, the atmosphere was exchanged by hydrogen for three times, and the reaction solution was stirred under hydrogen (15 Psi) at 60 °C for 4 hours. The reaction solution was filtered with Celite, the filter cake was washed with dichloromethane (50 mL × 3), the filtrate was washed with aqueous hydrochloric acid (4 M, 20 mL), then washed with aqueous sodium carbonate (12 M, 50 mL), the organic phase was dried over anhydrous sodium sulfate, filtered, the filtrate was concentrated by distillation under reduced pressure, and the residue obtained was purified by reverse-phase liquid chromatography (separation conditions: column: 120 g Flash Coulmn Welch Ultimate XB_C18 20-40 µm; mobile phase: A-water: B-acetonitrile, gradient elution, flow rate: 85 mL/min, instrument: ISCO) to afford the title product **1c** (680 mg, yield: 60%) as a yellow solid.
MS m/z (ESI): 362.1 [M+1].

### Step 3

### 4-(Cyclopropylmethyl)-1, 4, 7, 8-tetrahydro-3H, 10H-spiro [pyrano [3, 4-f] indolizine-6, 2'-[1, 3] dioxolane]-3, 10-dione 1d

**1c** (680 mg, 1.85 mmol) was dissolved in 10 mL of dichloromethane, cool to 0 °C in an ice-water bath under nitrogen atmosphere, sodium borohydride (108.02 mg, 2.86 mmol) was added in portions, and the reaction solution was stirred at 0 °C for 30 minutes. Acetic acid (133.15 mg, 2.22 mmol) was added dropwise at 25 °C to produce gas and stirring was continued for 2 hours. 30 mL of water was added dropwise at 15 °C to generate gas and stirred at 15 °C for 1.5 hours. The reaction solution was washed with water (50 mL) at 15 °C, and p-toluenesulfonic acid monohydrate (35.15 mg, 184.78 µmol) was added to the washed organic layer and stirred at 15 °C for 12 hours. 50 mL of water was added, the reaction liquid was extracted with dichloromethane (45 mL × 3), the organic phase was dried over anhydrous sodium sulfate, filtered, the filtrate was concentrated by distillation under reduced pressure, and the residue obtained was purified by silica gel column chromatography with developing system B to afford the title product **1d** (200 mg, yield: 32%) as a yellow oil.
MS m/z (ESI): 318.1 [M+1].

### Step 4

### 4-(Cyclopropylmethyl)-4-hydroxy-1, 4, 7, 8-tetrahydro-3H, 10H-spiro [pyrano [3, 4-f] indolizine-6, 2'-[1, 3] dioxolane]-3, 10-dione 1e

**1d** (202.13 mg, 598.74 µmol) was dissolved in 0.5 mL of methanol, cool to 0 °C in an ice-water bath, potassium carbonate (82.75 mg, 598.74 µmol) was added in portions, and stirred at 0 °C for 5 hours under bubbling with oxygen (15 psi). The reaction solution was poured into 10 mL saturated ammonium chloride aqueous solution, and methanol was removed by vacuum distillation. The reaction solution was extracted with dichloromethane (20 mL × 3). The organic phase was dried with anhydrous sodium sulfate, filtered, and the filtrate was concentrated by vacuum distillation. The crude title product **1e** (140 mg) was obtained as a yellow oil. The product was directly used in the next step without purification.
MS m/z (ESI): 334.1 [M+1].

### Step 5

### (S)-4-(Cyclopropylmethyl)-4-hydroxy-1, 4, 7, 8-tetrahydro-3H, 10H-spiro [3, 4-f] indolizine-6, 2'-[1, 3] dioxolane]-3, 10-dione 1e-1

### (R)-4-(cyclopropylmethyl)-4-hydroxy-1, 4, 7, 8-tetrahydro-3H, 10H-spiro [3, 4-f] indolizine-6, 2'-[1, 3] dioxolane]-3, 10-dione 1e-2

**1e** (1.30 g, 3.88 mmol) was separated by SFC (separation conditions: column: DAICEL CHIRALPAK AS 250 mm × 50 mm, 10 µm; mobile phase: A-carbon dioxide: B-methanol (0.1% NH₃·H₂O), isocratic elution: B: 20%, flow rate: 120 mL/min, instrument: Shimadzu LC-30ADsf) to afford the title product **1e-1** (301 mg, yield: 22.1%) as a yellow solid and the title product **1e-2** (285 mg, yield: 19.4%) as a yellow solid.

### Single Configuration Compound 1e-1

SFC analysis: retention time 1.392 minutes. (Column: Chiralpak AS-3 50 × 4.6 mm I.D., 3 µm, mobile phase: A-carbon dioxide, B-methanol (0.05% diethylamine), gradient elution: B%: 5%-40%, flow rate: 3 mL/min, instrument: Shimadzu LC-30ADsf).
MS m/z (ESI): 334.0 [M+1].

### Single Configuration Compound le-2

SFC analysis: retention time 1.762 minutes. (Column: Chiralpak AS-3 50 × 4.6 mm I.D., 3 µm, mobile phase: A-carbon dioxide, B-methanol (0.05% diethylamine), gradient elution: B%: 5%-40%, flow rate: 3 mL/min, instrument: Shimadzu LC-30ADsf).
MS m/z (ESI): 333.9 [M+1].

### Step 6

### (S)-4-(cyclopropylmethyl)-4-hydroxy-7, 8-dihydro-1H-pyrano [3, 4-f] indolizine-3, 6, 10 (4H)-trione 1f-1

**1e-1** (301 mg, 857 µmol) was dissolved in a mixture of 2 mL of trifluoroacetic acid and 0.5 mL of water and stirred at 25 °C for 4 hours. The reaction solution was concentrated by distillation under reduced pressure, and the residue was obtained as a yellow solid without purification to afford the crude title product **1f-1** (209 mg), which was directly used in the next step without purification.
MS m/z (ESI): 290.1 [M+1].

### Step 7

**1g** (800 mg, 5.83 mmol) was dissolved in 20 mL of 1,2-dichloroethane, cooled to 0 °C in an ice-water bath under nitrogen atmosphere, a solution of boron trichloride in dichloromethane (1 M, 4.08 mL) and 4-pentenenitrile (709 mg, 8.75 mmol) were added dropwise, and stirred at 80 °C for 2 hours under nitrogen atmosphere. The reaction solution was cooled to room temperature, aqueous hydrochloric acid (2 M, 50 mL) was added, and stirred at 80 °C for 0.5 h. The reaction solution was extracted with dichloromethane (30 mL × 3). The organic phase was dried over anhydrous sodium sulfate, filtered, the filtrate was concentrated under reduced pressure, and the residue obtained was purified by silica gel column chromatography with developing system B to afford the title product **1h** as a yellow oil (220 mg, yield: 17%).
MS m/z (ESI): 220.2 [M+1].

### Step 8

**1f-1** (288 mg, 991 µmol) and **1h** (220 mg, 991 µmol) were dissolved in 10 mL of toluene, pyridinium 4-methylbenzenesulfonate (124 mg, 495 µmol) and o-cresol (214 mg, 1.98 mmol) were added and stirred at 120 °C for 2 hours under nitrogen atmosphere. 50 mL of water was added to the reaction solution, extracted with dichloromethane (45 mL × 3), the organic phase was dried over anhydrous sodium sulfate, filtered, the filtrate was concentrated under reduced pressure, and the residue obtained was purified by silica gel column chromatography with developing system A to afford the title product 1i (202 mg, yield: 41%) as a yellow solid.
MS m/z (ESI): 473.0 [M+1].

### Step 9

**1i** (200 mg, 406 µ mol) was dissolved in 10 mL of dichloromethane, cooled to-78 °C, bubbled with ozone for 0.5 h (15 Psi). Excess ozone was purged with nitrogen, triphenylphosphine (213 mg, 812 µ mol) was added at - 78 °C, and stirred at-78 °C for 0.5 h. 50 mL of water was added to the reaction solution, extracted with dichloromethane (45 mL × 3), the organic phase was dried over anhydrous sodium sulfate, filtered, the filtrate was concentrated under reduced pressure, and the residue obtained was purified by silica gel column chromatography with a dichloromethane/tetrahydrofuran developing system to afford the title product **1j** (70 mg, yield: 36%) as a yellow solid.
MS m/z (ESI): 475.3 [M+1].

### Step 10

**1j** (70.0 mg, 147 µmol) was dissolved in 5 mL of tetrahydrofuran under nitrogen atmosphere, sodium borohydride (2.79 mg, 73.7 µmol) was added, stirred at-78 °C for 1 hour, 10 mL of ammonium chloride solution was added to the reaction solution, extracted with ethyl acetate (8 mL × 3), the organic phase was dried over anhydrous sodium sulfate, filtered, the filtrate was concentrated under reduced pressure, and the residue obtained was purified by preparative high performance liquid chromatography (separation conditions: column: Phenomenex luna C18 150 × 25 mm × 10 µm; mobile phase: A-water (0.225% formic acid): B-acetonitrile, gradient elution: B%: 22%-42%) to afford the title product 1 as a yellow solid (8.95 mg, yield: 21%).
MS m/z (ESI): 477.0 [M+1].

¹H NMR (400 MHz, CD₃OD) δ 7.64 - 7.61 (m, 1H), 7.55 - 7.51 (m, 1H), 7.41 - 7.37 (m, 1H), 6.24 - 6.19 (m, 2H), 5.62 - 5.55 (m, 1H), 5.40 - 5.34 (m, 1H), 5.26 - 5.21 (m, 2H), 3.72 - 3.64 (m, 2H), 3.25 - 3.19 (m, 2H), 1.99 - 1.90 (m, 3H), 1.89 - 1.81 (m, 1H), 0.97 - 0.88 (m, 1H), 0.52 - 0.38 (m, 2H), 0.15 - 0.09 (m, 1H), 0.07 - 0.00 (m, 1H).

### Example 2-2: Preparation of Compound 2

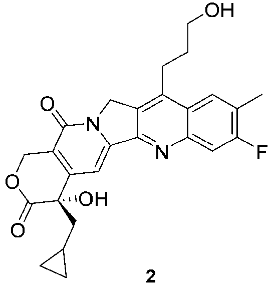

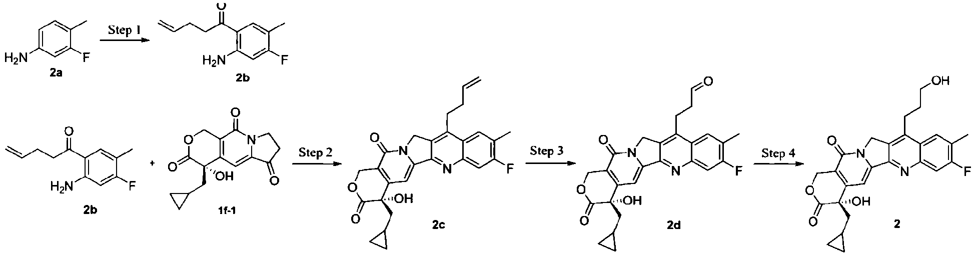

### Step 1

**2a** (1.00 g, 7.99 mmol) was dissolved in 20 mL of 1,2-dichloroethane, cooled to 0° C in an ice-water bath under nitrogen atmosphere, a solution of boron trichloride in dichloromethane (1 M, 10.4 mL) and 4-pentenenitrile (777 mg, 9.59 mmol) were added dropwise and stirred at 80 °C for 5 hours under nitrogen atmosphere. The reaction solution was cooled to room temperature, aqueous hydrochloric acid (2 M, 9 mL) was added and stirred at 80 °C for 0.5 h. The reaction solution was extracted with dichloromethane (30 mL × 3), the organic phase was dried over anhydrous sodium sulfate, filtered, the filtrate was concentrated under reduced pressure, and the residue obtained was purified by silica gel column chromatography with developing system B to afford the title product **2b** (315 mg, yield: 19%) as a yellow solid.
MS m/z (ESI): 208.1 [M+1].

### Step 2

**1f-1** (400 mg, 1.37 mmol) and **2b** (313 mg, 1.51 mmol) were dissolved in 50 mL of toluene, pyridinium 4-methylbenzenesulfonate (207 mg, 823 µmol) and o-cresol (1.19 g, 11.0 mmol) were added and stirred at 135 °C for 3 hours. The reaction solution was concentrated by distillation under reduced pressure, and the residue obtained was purified by silica gel column chromatography with developing system A to afford the title product **2c** (420 mg, yield: 60%) as a yellow solid.
MS m/z (ESI): 461.3 [M+1].

### Step 3

**2c** (100 mg, 197 µmol) was dissolved in 2 mL of dichloromethane, cooled to-78 °C, bubbled with ozone for 0.5 h (15 Psi). Excess ozone was purged with nitrogen, triphenylphosphine (51.7 mg, 197 µmol) was added at -78 ° C, and stirred at-78 °C for 0.5 h. The reaction solution was concentrated by distillation under reduced pressure, and the residue obtained was purified by silica gel column chromatography in developing system A to afford the title product **2d** (53.0 mg, yield: 54%) as a yellow solid.
MS m/z (ESI): 463.2 [M+1].

### Step 4

**2d** (40.0 mg, 80.1 µmol) was dissolved in a mixture of 2 mL of tetrahydrofuran and 0.1 mL of N,N-dimethylacetamide under nitrogen atmosphere, sodium borohydride (1.52 mg, 40.1 µmol) was added, stirred at - 78 °C for 1 hour, 1 mL of ammonium chloride solution was added to the reaction solution, extracted with ethyl acetate (2 mL × 3), the organic phase was dried over anhydrous sodium sulfate, filtered, and the filtrate was concentrated under reduced pressure, and the residue obtained was purified by preparative high performance liquid chromatography (separation conditions: column: Phenomenex luna C18 150 × 25 mm × 10 µm; Mobile phase: A-water (0.225% formic acid): B-acetonitrile, gradient elution: B%: 27%-57%), then purified by supercritical fluid chromatography, column: Phenomenex-Cellulose-2 (250 mm × 30 mm, 10 µ m), mobile phase: A-carbon dioxide, B-methanol and acetonitrile, isocratic elution: B%: 55%, to obtain the title product 2 (8.66 mg, yield: 45%) as a light yellow solid.
MS m/z (ESI): 464.9 [M+1].

¹H NMR (400 MHz, CD₃OD) δ 8.24 - 8.17 (m, 1H), 7.80 - 7.73 (m, 1H), 7.73 - 7.67 (m, 1H), 5.64 - 5.57 (m, 1H), 5.44 - 5.33 (m, 3H), 3.75 - 3.66 (m, 2H), 3.40 - 3.34 (m, 2H), 2.60 - 2.52 (m, 3H), 2.04 - 1.96 (m, 2H), 1.94 - 1.82 (m, 2H), 0.93 - 0.89 (m, 1H), 0.52 - 0.38 (m, 2H), 0.17 - 0.08 (m, 1H), 0.07 - -0.04 (m, 1H).

### Example 2-3: Preparation of Compound 3

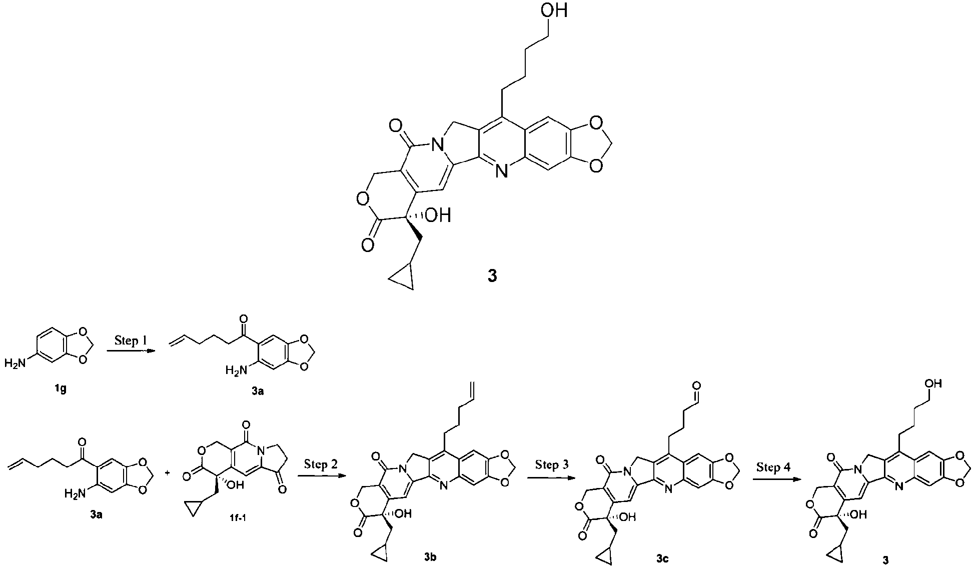

### Step 1

**1g** (1.5 g, 10.94 mmol) was dissolved in 20 mL of 1,2-dichloroethane, cooled to 0 °C in an ice-water bath under nitrogen atmosphere, a solution of boron trichloride in dichloromethane (1 M, 8.75 mL) and 5-hexenenitrile (1.56 g, 16.4 mmol) were added dropwise, and stirred at 80 °C for 2 hours under nitrogen atmosphere. The reaction solution was cooled to room temperature, aqueous hydrochloric acid (2 M, 50 mL), was added and stirred at 80 °C for 0.5 h. The reaction solution was extracted with dichloromethane (50 mL × 3), the organic phase was dried over anhydrous sodium sulfate, filtered, the filtrate was concentrated under reduced pressure, and the residue obtained was purified by silica gel column chromatography with developing system B to afford the title product **3a** (502 mg, yield: 19%) as a yellow oil.
MS m/z (ESI): 234.0 [M+1].

### Step 2

**If-1** (249 mg, 857 µmol) and **3a** (200 mg, 780 µmol) were dissolved in 5 mL of toluene, pyridinium 4-methylbenzenesulfonate (150 mg, 600 µmol) and o-cresol (185 mg, 1.71 mmol) were added and stirred at 120 °C for 2 hours under nitrogen atmosphere. 50 mL of water was added to the reaction solution, extracted with dichloromethane (45 mL × 3), the organic phase was dried over anhydrous sodium sulfate, filtered, the filtrate was concentrated under reduced pressure, and the residue obtained was purified by silica gel column chromatography with developing system A to afford the title product **3b** (310 mg, yield: 53%) as a yellow solid.
MS m/z (ESI): 487.3 [M+1].

### Step 3

**3b** (250 mg, 513 µmol) was dissolved in 5 mL dichloromethane, cooled to-78 °C, bubbled with ozone for 0.5 h (15 Psi). Excess ozone was purged with nitrogen, triphenylphosphine (674 mg, 2.57 mmol) was added at -78 °C, and stirred at -78 ° C for 0.5 h. The reaction solution was concentrated by distillation under reduced pressure, and the residue obtained was purified by silica gel column chromatography with developing system of dichloromethane/tetrahydrofuran to afford the title product 3c (65.0 mg, yield: 26%) as a yellow solid.
MS m/z (ESI): 489.2 [M+1].

### Step 4

**3c** (65.0 mg, 133 µmol) was dissolved in 15 mL of tetrahydrofuran under nitrogen atmosphere, sodium borohydride (2.5 mg, 66 µmol) was added, stirred at-78 °C for 1 hour, 20 mL of ammonium chloride solution was added to the reaction solution, extracted with ethyl acetate (20 mL × 3), the organic phase was dried over anhydrous sodium sulfate, filtered, the filtrate was concentrated under reduced pressure, and the residue was purified by preparative high performance liquid chromatography (separation conditions: column: Phenomenex luna C18 150 × 25 mm × 10 µ m; mobile phase: A-water (0.225% formic acid): B-acetonitrile, gradient elution: B%: 25%-45%) to afford the title product 3 (5.69 mg, yield: 9%) as a yellow solid.
MS m/z (ESI): 491.0 [M+1].

¹H NMR (400 MHz, CD₃OD) δ 7.62 - 7.58 (m, 1H), 7.50 - 7.46 (m, 1H), 7.37 - 7.34 (m, 1H), 6.24 - 6.18 (m, 2H), 5.62 - 5.54 (m, 1H), 5.42 - 5.32 (m, 1H), 5.21 - 5.16 (m, 2H), 3.69 - 3.62 (m, 2H), 3.17 - 3.10 (m, 2H), 1.98 - 1.90 (m, 1H), 1.88 - 1.79 (m, 3H), 1.78 - 1.70 (m, 2H), 0.96 - 0.85 (m, 1H), 0.52 - 0.40 (m, 2H), 0.18 - 0.08 (m, 1H), 0.07 - -0.00 (m, 1H).

### Example 2-4: Preparation of Compound 4

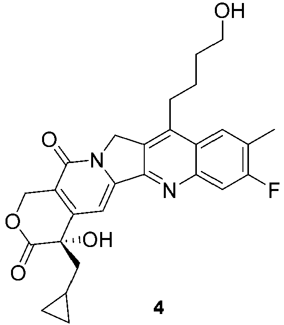

### Example 2-5: Preparation of Compound 5

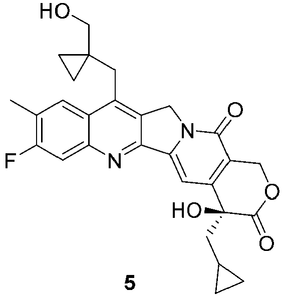

### Example 2-6: Preparation of Compound 6

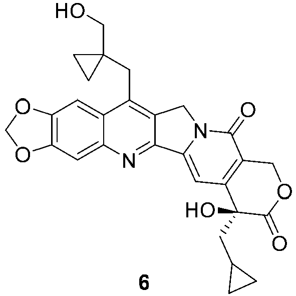

### Example 2-7: Preparation of Compound 7

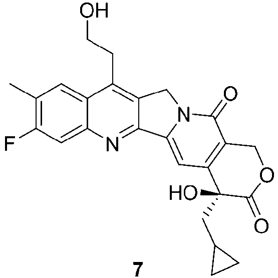

### Example 2-8: Preparation of Compound 8

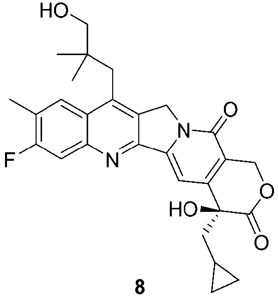

### Example 2-9: Preparation of Compound 9

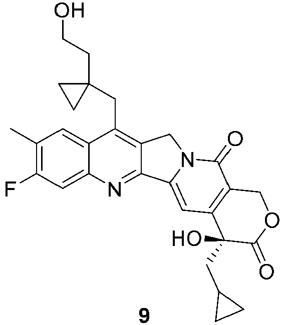

### Example 2-10: Preparation of Compound 10

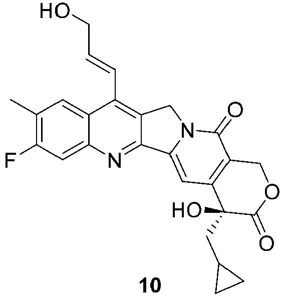

### Example 2-11: Preparation of Compound 11

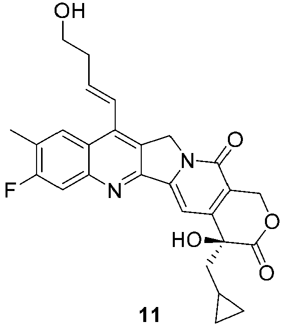

### Example 2-12: Preparation of Compound 12

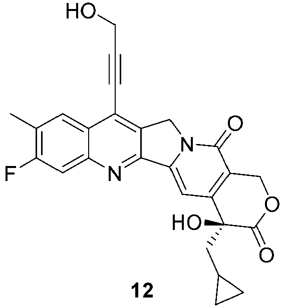

### Example 2-13: Preparation of Compound 13

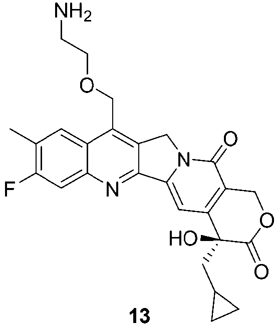

### Example 2-14: Preparation of Compound 14

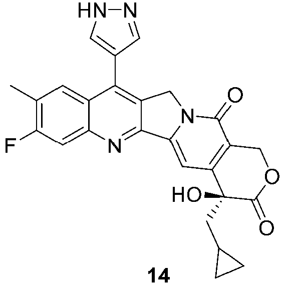

### Example 2-15: Preparation of Compound 15

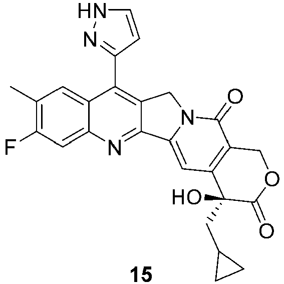

### Example 2-16: Preparation of Compound 16

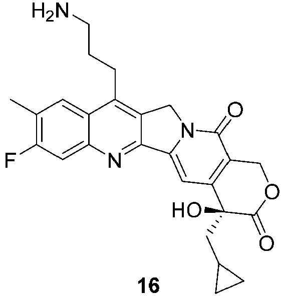

### Example 2-17: Preparation of Compound 17

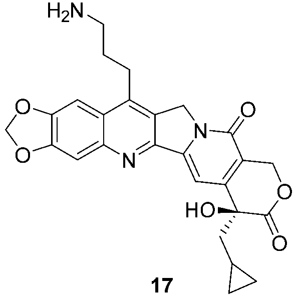

### Example 2-18: Preparation of Compound 18

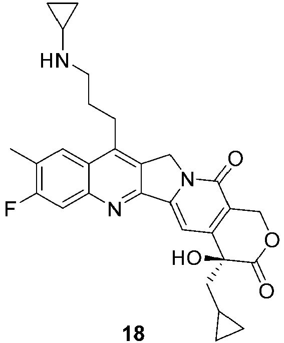

### Example 2-19: Preparation of Compound 19

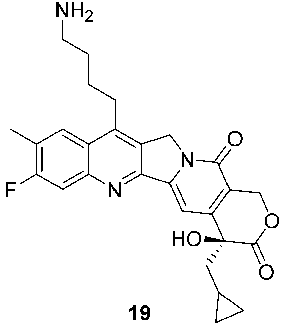

### Example 2-20: Preparation of Compound 20

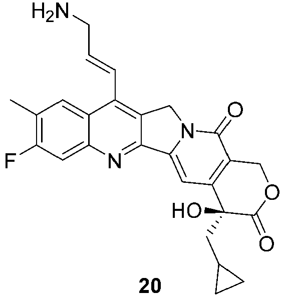

### Example 2-21: Preparation of Compound 21

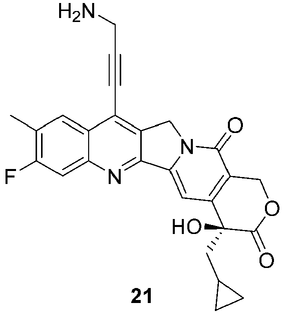

### Example 2-22: Preparation of Compound 22

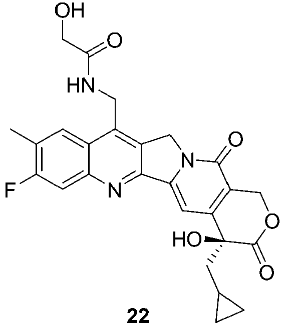

### Example 2-23: Preparation of Compound 23

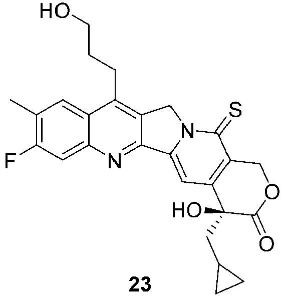

### Example 2-24: Preparation of Compound 24

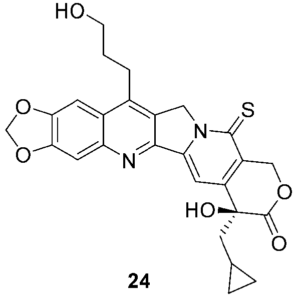

### Example 2-25: Preparation of Compound 25

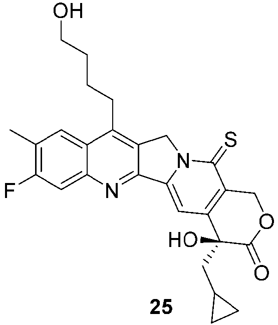

### Example 2-26: Preparation of Reference 1

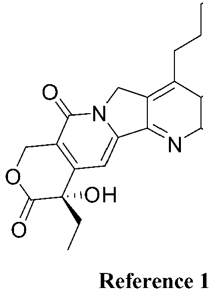

It was prepared according to the method disclosed in Example A1.9, page 207, of Patent Application "WO2022170971 Al".

### III. Preparation Examples of Conjugate Intermediate Linker-Drugs

### Example 3-1 LD-1

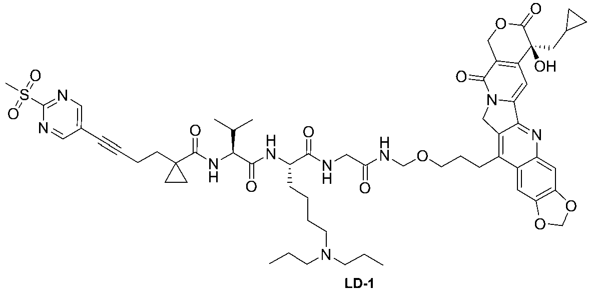

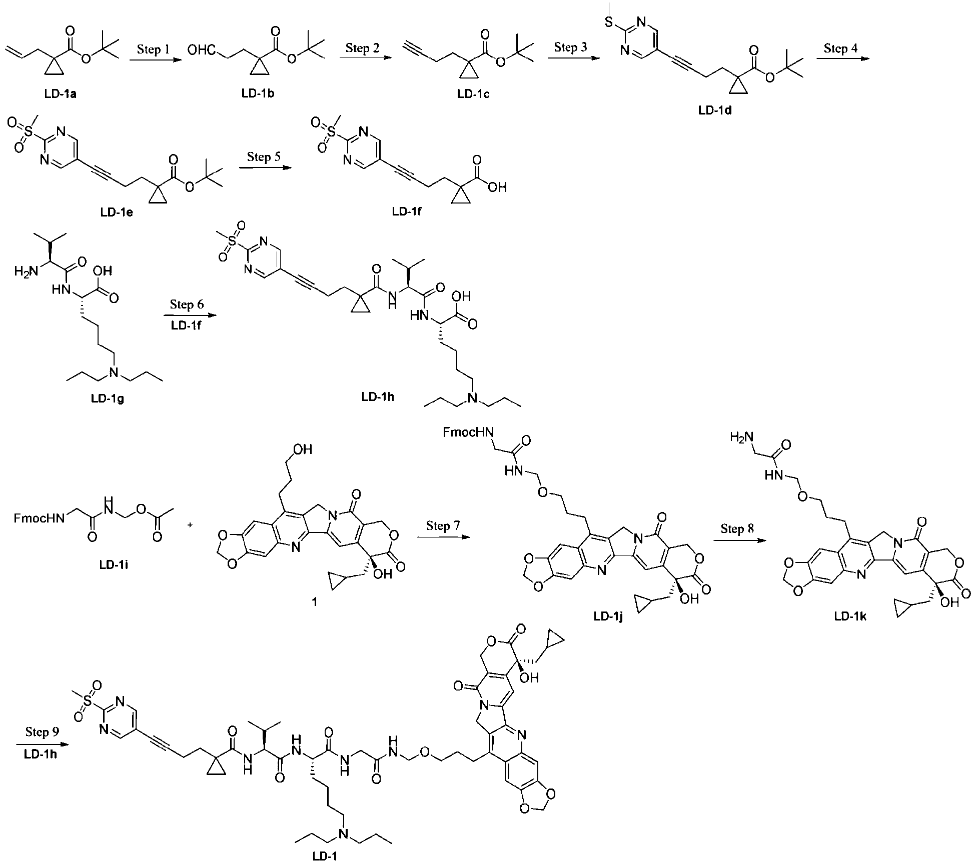

### Step 1

**LD-1a** (2.20 g, 12.1 mmol, prepared according to the method disclosed in example 4 on page 62 of patent application "WO2020077038 A1"), silver nitrite (111 mg, 724 µmol), bis (benzonitrile) palladium (II) chloride (555 mg, 1.45 mmol), nitromethane (4.90 g, 80.3 mmol) and copper (II) chloride dihydrate (247 mg, 1.45 mmol) were dissolved in 44 mL of tert-butanol, the atmosphere was exchanged with oxygen for three times and the reaction solution was stirred at 25 °C under oxygen for 12 hours. At 25 °C, 150 mL of water was added to the reaction solution, extracted with dichloromethane (50 mL × 3), the organic phase was dried over anhydrous sodium sulfate, filtered, the filtrate was concentrated by distillation under reduced pressure, and the residue obtained was purified by silica gel column chromatography with developing system B to afford the title product **LD-1b** (800 mg, yield: 33%) as a white solid.

### Step 2

**LD-1b** (800 mg, 4.04 mmol) was dissolved in 24 mL of methanol, then potassium carbonate (1.12 g, 8.07 mmol) and dimethyl (1-diazo-2-oxopropyl) phosphonate (930 mg, 4.84 mmol) were added and stirred at 25 °C for 12 hours. 50 mL of water was added to the reaction solution, extracted with ethyl acetate (20 mL × 3), the organic phase was dried over anhydrous sodium sulfate, filtered, the filtrate was concentrated by distillation under reduced pressure, and the residue obtained was purified by silica gel column chromatography with developing system B to afford the title product **LD-1c** (650 mg, yield: 83%) as a white solid.

### Step 3

**LD-1c** (650 mg, 3.35 mmol), 5-bromo-2-methiopyrimidine (686 mg, 3.35 mmol), triethylamine (3.39 g, 33.4 mmol), cuprous iodide (63.7 mg, 334 µmol) and bis-triphenylphosphine palladium dichloride (244 mg, 349 µmol) were dissolved in 10 mL of tetrahydrofuran, the atmosphere was exchanged with nitrogen for three times and the reaction solution was stirred at 60 °C for 2 hours under nitrogen atmosphere. The reaction solution was concentrated under reduced pressure, and the residue was purified by silica gel column chromatography with developing system B to afford the title product **LD-1d** (770 mg, yield: 68%) as a white solid.
MS m/z (ESI): 319.6 [M+1].

### Step 4

**LD-1d** (1.80 g, 5.65 mmol) was dissolved in 20 mL of dichloromethane, then m-chloroperoxybenzoic acid (2.87 g, 14.1 mmol, purity 85.0%) was added and stirred at 25 °C for 2 hours. The reaction solution was concentrated under reduced pressure, and the residue obtained was purified by silica gel column chromatography with developing system B to afford the title product **LD-1e** (1.70 g, yield: 85%) as a white solid.
MS m/z (ESI): 351.1 [M+1].

### Step 5

**LD-1e** (500 mg, 1.43 mmol) was dissolved in 6 mL of dichloromethane, then trifluoroacetic acid (2.30 g, 20.2 mmol) was added and stirred at 25 °C for 2 hours. The reaction solution was concentrated under reduced pressure to afford the crude title product **LD-1f** (418 mg) as a white solid. The product was used in the next step without purification.
MS m/z (ESI): 295.2 [M+1].

### Step 6

**LD-1f** (10 mg, 34.0 µmol) was dissolved in 1 mL of N,N-dimethylformamide, followed by the addition of 2-(7-azobenzotriazole)-N,N,N',N'-tetramethyluronium hexafluorophosphate (13 mg, 34.0 µmol) and N,N-diisopropylethylamine (13 mg, 100.9 µmol), stirred at 25 °C for 0.5 hours, and then **LD-1g** (14.5 mg, 44.0 µmol, prepared according to the method disclosed in example C1.22, page 241 of patent application "WO2022170971 A1") was added and stirred at 25 °C for 3 hours. The reaction solution was purified without treatment by pre-HPLC (column: InfinityLab Poroshell 120 SB-C18 21.2 × 150 mm, 4 µm; mobile phase: A-water (0.1% formic acid), B-acetonitrile; gradient elution: B%: 10%-70%) to afford the title product **LD-1h** (8.10 mg, yield: 39%) as a white solid.
MS m/z (ESI): 606.5 [M+1].

### Step 7

**LD-1i** (325 mg, 882 µmol) and 1 (42 mg, 88.1 µmol) were dissolved in 4 mL of N,N-dimethylformamide, then hydrochloric acid in ethyl acetate (4 mol/L, 100 µ L) was added and stirred at 25 °C for 15 hours. The reaction solution was purified without treatment by reverse-phase liquid chromatography (mobile phase: A-water (0.1% formic acid), B-acetonitrile; gradient elution: B%: 20%-80%) to afford the title product **LD-1j** (34 mg, yield: 48%) as a yellow solid.
MS m/z (ESI): 785.6 [M+1].

### Step 8

**LD-1j** (38 mg, 48.4 µmol) was dissolved in 1 mL of N,N-dimethylformamide, then diethylamine (142 mg, 1.93 mmol) was added and stirred at 25 °C for 0.5 hr. The reaction solution was concentrated under reduced pressure and the resulting residue was purified by pre-HPLC (column: InfinityLab Poroshell 120 SB-C18 21.2 × 250 mm, 4 µ m; mobile phase: A-water (0.1% formic acid), B-acetonitrile; gradient elution: B%: 5%-50%) to afford the title product **LD-1k** (11 mg, yield: 40%) as a yellow solid.
MS m/z (ESI): 282.2 [1/2 (M+2)].

### Step 9

**LD-1k** (11 mg, 19.6 µmol), **LD-1h** (12 mg, 19.8 µmol) and 2-(7-azobenzotriazole)-N,N,N',N'-tetramethyluronium hexafluorophosphate (9 mg, 23.6 µmol) were dissolved in 1 mL of N,N-dimethylformamide, then N,N-diisopropylethylamine (8 mg, 61.9 µmol) was added and stirred at 25 °C for 1 hour. The reaction solution was purified without treatment by pre-HPLC (column: InfinityLab Poroshell 120 SB-C18 21.2 × 150 mm, 4 µm; mobile phase: A-water (0.1% formic acid), B-acetonitrile; gradient elution: B%: 20%-55%) to afford the title product **LD-1** (4.3 mg, yield: 19%) as an off-white solid.
MS m/z (ESI): 576.2 [1/2 (M+2)].

¹H NMR (400 MHz, DMSO-*d*₆) δ 9.10 - 9.00 (m, 2H), 8.69 - 8.58 (m, 1H), 8.29 - 8.20 (m, 1H), 8.02 - 7.93 (m, 1H), 7.63 - 7.55 (m, 1H), 7.53 - 7.47 (m, 1H), 7.36 - 7.24 (m, 2H), 6.59 - 6.52 (m, 1H), 6.31 - 6.21 (m, 2H), 5.49 - 5.35 (m, 2H), 5.31 - 5.20 (m, 2H), 4.66 - 4.53 (m, 2H), 4.48 - 4.36 (m, 4H), 4.27 - 4.18 (m, 1H), 4.11 - 4.03 (m, 1H), 3.79 - 3.67 (m, 2H), 3.54 - 3.47 (m, 2H), 3.41 - 3.39 (m, 3H), 3.14 - 3.05 (m, 2H), 2.64 - 2.56 (m, 2H), 2.35 - 2.31 (m, 1H), 2.05 - 1.82 (m, 7H), 1.76 - 1.48 (m, 4H), 1.44 - 1.36 (m, 5H), 1.30 - 1.20 (m, 2H), 1.03 - 0.93 (m, 2H), 0.87 - 0.76 (m, 13H), 0.75 - 0.68 (m, 2H), 0.39 - 0.28 (m, 2H), 0.11 - 0.03 (m, 1H), -0.03 - -0.10 (m, 1H).

### Example 3-2 LD-2

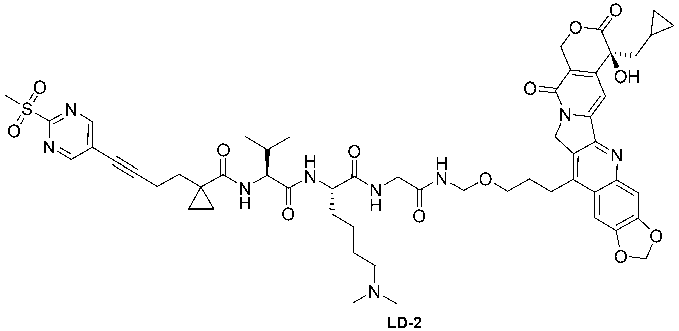

### Example 3-3 LD-3

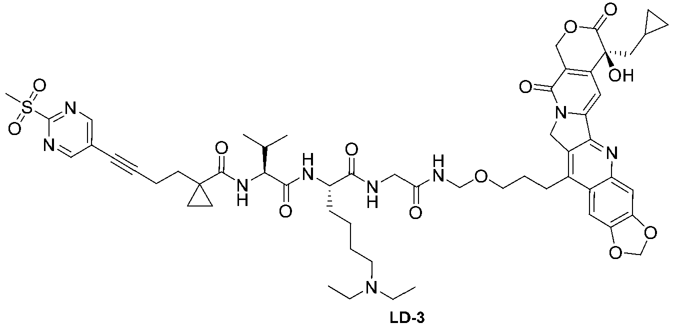

### Example 3-4 LD-4

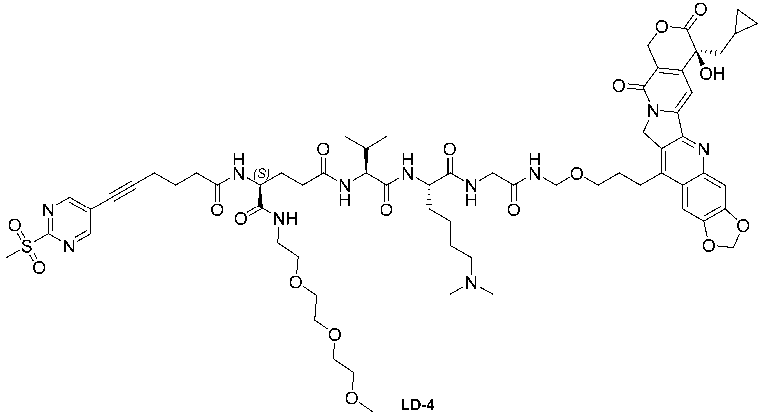

### Example 3-5 LD-5

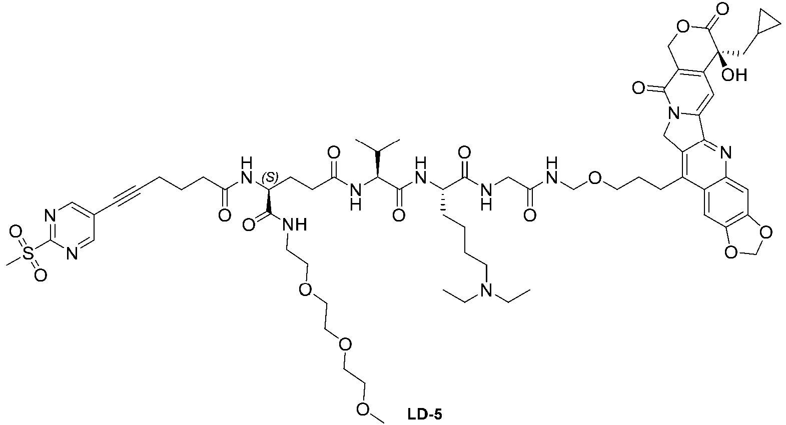

### Example 3-6 LD-6

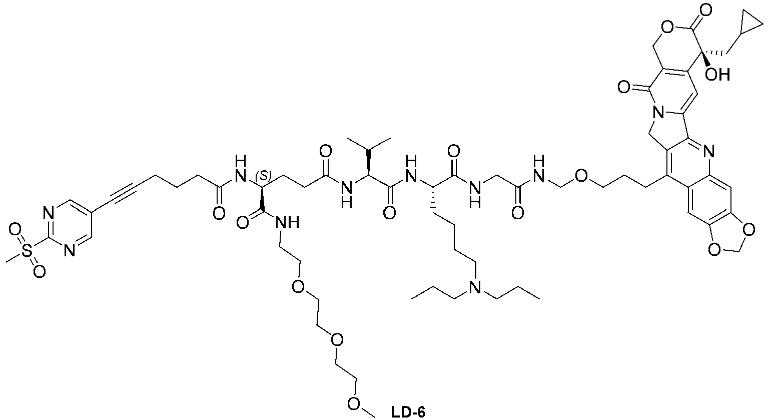

### Example 3-7 LD-7

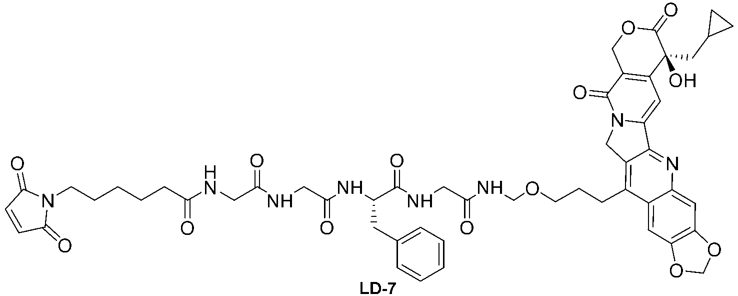

### Example 3-8 LD-8

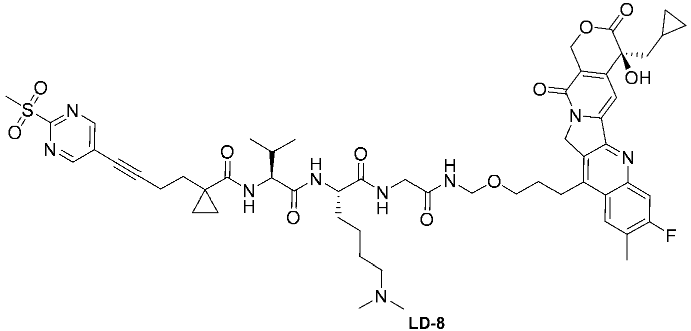

### Example 3-9 LD-9

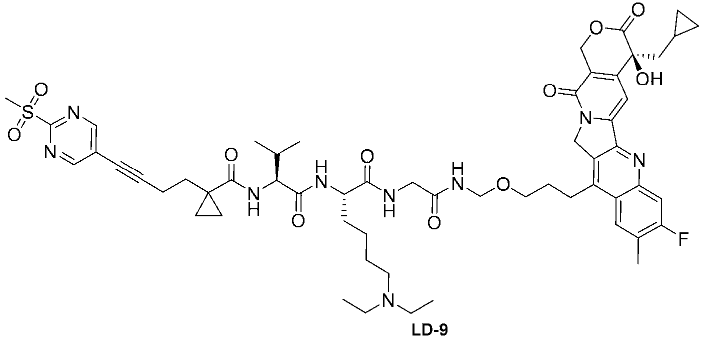

### Example 3-10 LD-10

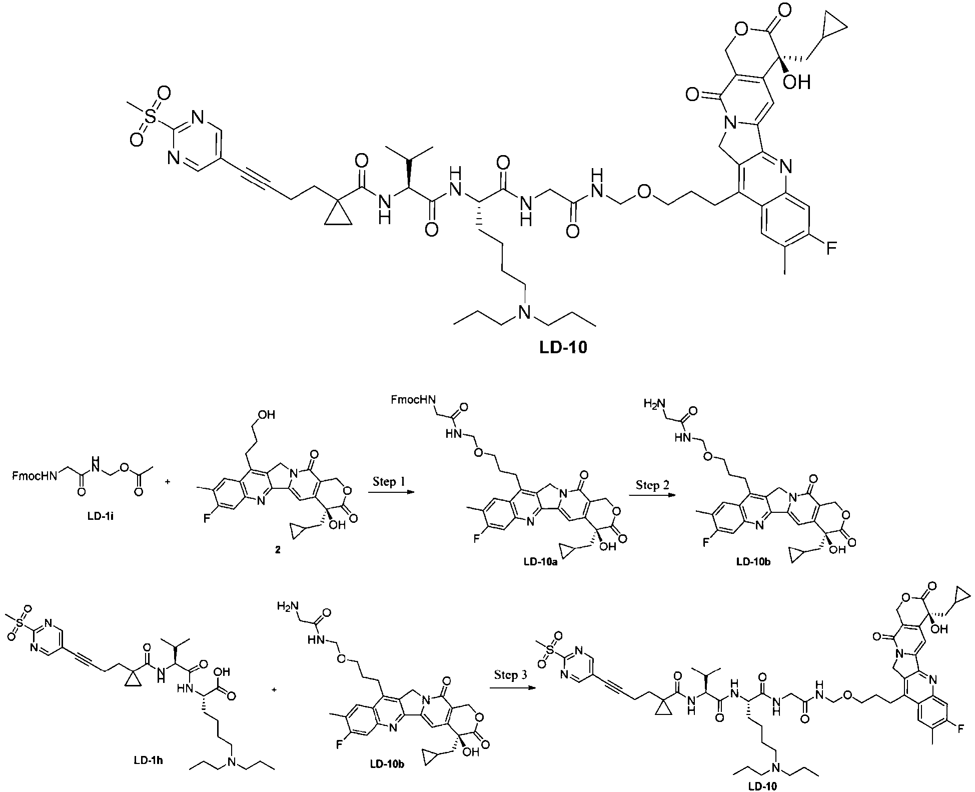

### Step 1

**LD-1i** (333 mg, 904 µmol) and **2** (42 mg, 90.4 µmol) were dissolved in 4 mL of N,N-dimethylformamide, then hydrochloric acid in ethyl acetate (4 mol/L, 100 µL) was added and stirred at 25 °C for 15 hours. The reaction solution was purified without treatment by pre-HPLC (column: InfinityLab Poroshell 120 SB-C18 21.2 × 150 mm, 4 µm; mobile phase: A-water (0.1% formic acid), B-acetonitrile; gradient elution: B%: 20%-90%) to afford the title product **LD-10a** (35 mg, yield: 49%) as a yellow solid.
MS m/z (ESI): 773.6 [M+1].

### Step 2

**LD-10a** (35 mg, 44.6 µmol) was dissolved in 1 mL of N, N-dimethylformamide, followed by addition of diethylamine (142 mg, 1.94 mmol) and stirring at 25 °C for 0.5 hr. The reaction solution was concentrated under reduced pressure and the resulting residue was purified by pre-HPLC (column: InfinityLab Poroshell 120 SB-C18 21.2 × 250 mm, 4 µm; mobile phase: A-water (0.1% formic acid), B-acetonitrile; gradient elution: B%: 5%-70%) to afford the title product **LD-10b** (17 mg, yield: 66%) as a yellow solid.
MS m/z (ESI): 551.4 [M+1].

### Step 3

**LD-10b** (17 mg, 30.0 µmol), **LD-1h** (19 mg, 30.5 µmol) and 2-(7-azobenzotriazole)-N,N,N',N'-tetramethyluronium hexafluorophosphate (13 mg, 30.5 µmol) were dissolved in 1 mL of N,N-dimethylformamide, then N,N-diisopropylethylamine (12 mg, 92.8 µmol) was added and stirred at 25 °C for 1 hour. The reaction solution was purified without treatment by pre-HPLC (column: InfinityLab Poroshell 120 SB-C18 21.2 × 150 mm, 4 µm; mobile phase: A-water (0.1% formic acid), B-acetonitrile; gradient elution: B%: 20%-60%) to afford the title product **LD-10** (7.2 mg, yield: 20%) as a white solid.
MS m/z (ESI): 570.3 [1/2 (M+2)].

¹H NMR (400 MHz, DMSO-*d*₆) δ 9.10 - 9.02 (m, 2H), 8.67 - 8.61 (m, 1H), 8.26 - 8.18 (m, 2H), 8.02 - 7.96 (m, 1H), 7.91 - 7.85 (m, 1H), 7.40 - 7.35 (m, 1H), 7.33 - 7.26 (m, 1H), 6.62 - 6.57 (m, 1H), 5.48 - 5.38 (m, 2H), 5.34 - 5.26 (m, 2H), 4.64 - 4.55 (m, 2H), 4.26 - 4.19 (m, 1H), 4.09 - 4.04 (m, 1H), 3.75 - 3.70 (m, 2H), 3.54 - 3.46 (m, 2H), 3.42 - 3.37 (m, 3H), 3.23 - 3.17 (m, 2H), 2.69 - 2.65 (m, 1H), 2.63 - 2.57 (m, 2H), 2.54 - 2.51 (m, 3H), 2.34 - 2.31 (m, 1H), 2.03 - 1.83 (m, 8H), 1.77 - 1.64 (m, 3H), 1.57 - 1.49 (m, 2H), 1.46 - 1.37 (m, 5H), 1.31 - 1.19 (m, 3H), 1.01 - 0.95 (m, 2H), 0.87 - 0.75 (m, 13H), 0.73 - 0.68 (m, 2H), 0.38 - 0.30 (m, 2H), 0.11 - 0.05 (m, 1H), -0.04 - -0.09 (m, 1H).

### Example 3-11 LD-11

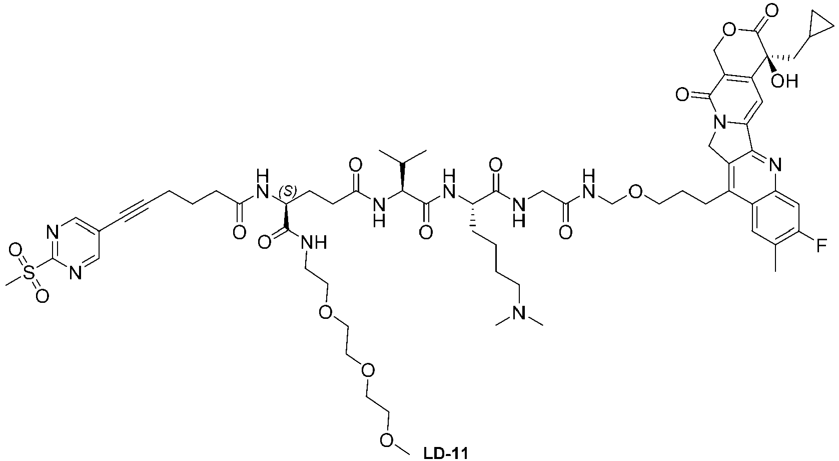

### Example 3-12 LD-12

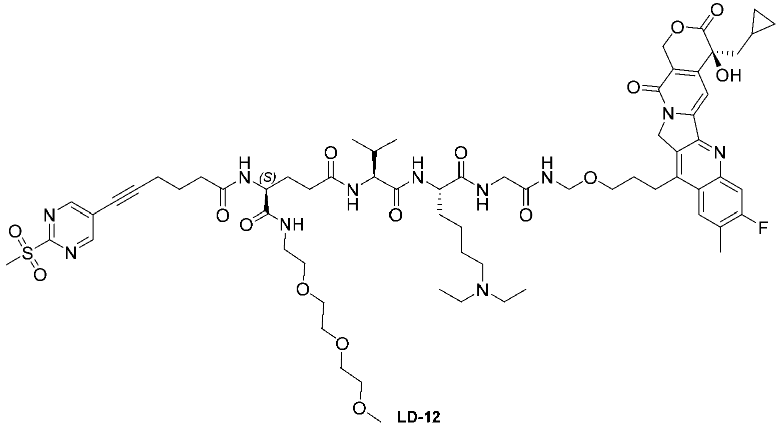

### Example 3-13 LD-13

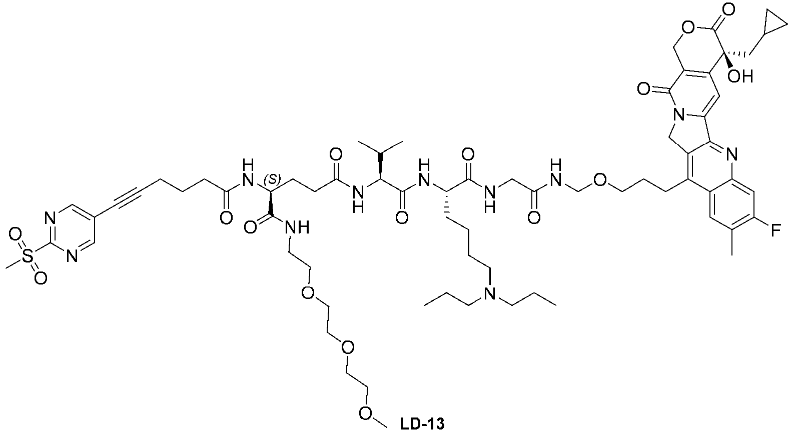

### Example 3-14 LD-14

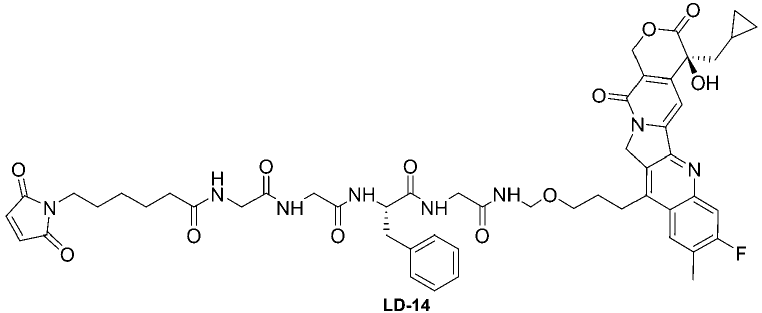

### Example 3-15 LD-15

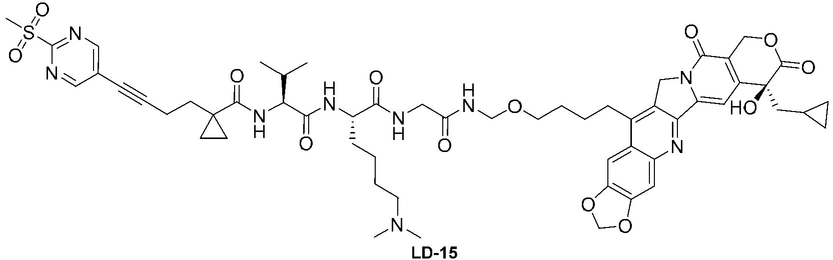

### Example 3-16 LD-16

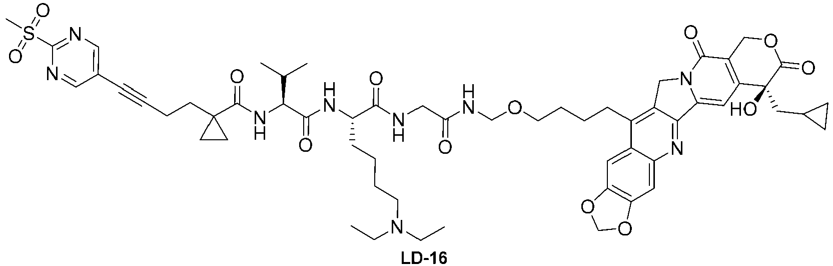

### Example 3-17 LD-17

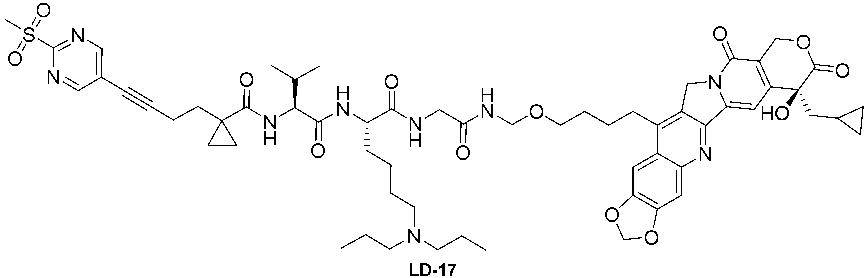

### Example 3-18 LD-18

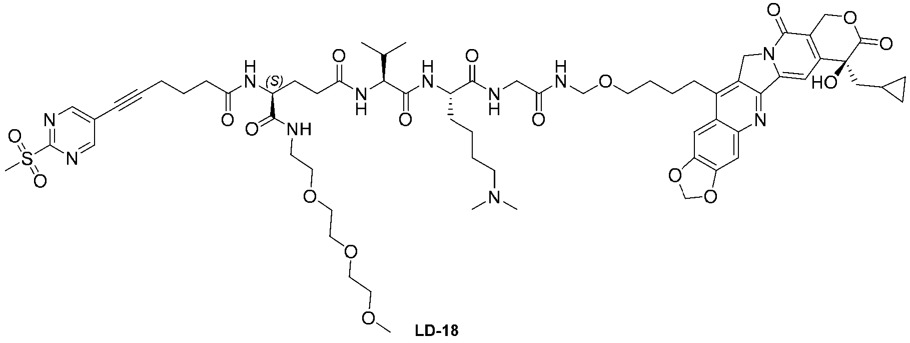

### Example 3-19 LD-19

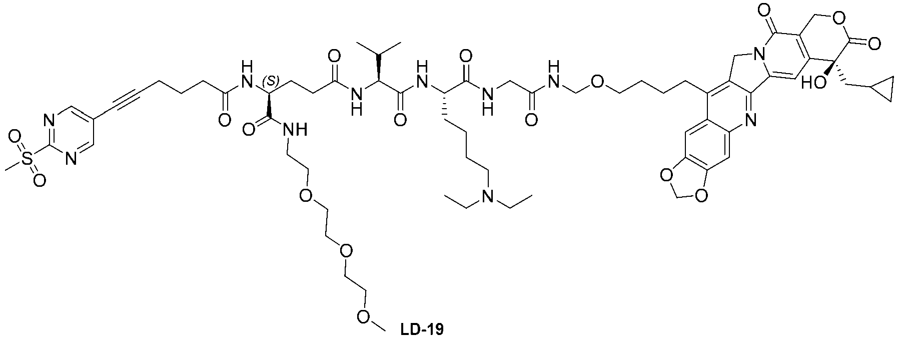

### Example 3-20 LD-20

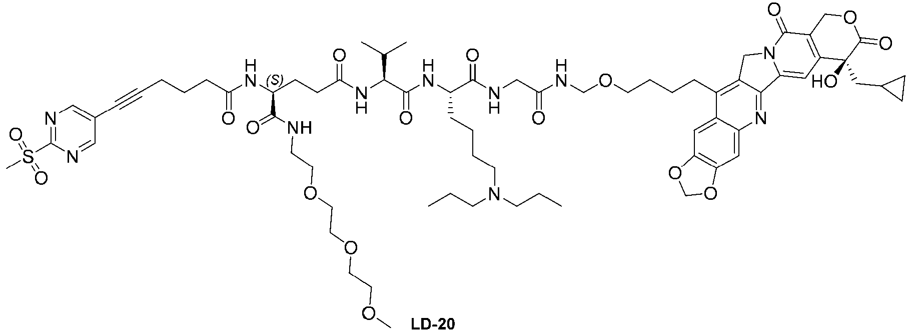

### Example 3-21 LD-21

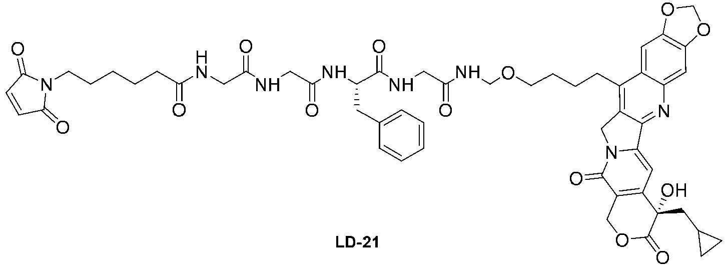

### Example 3-22 LD-22

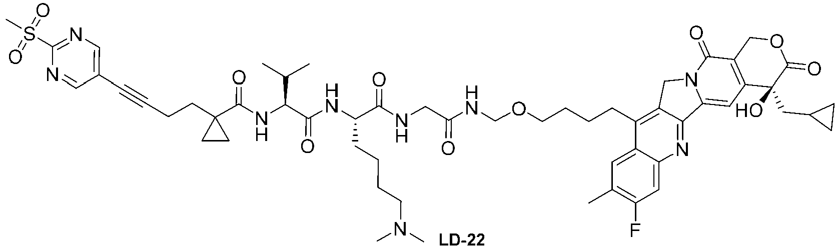

### Example 3-23 LD-23

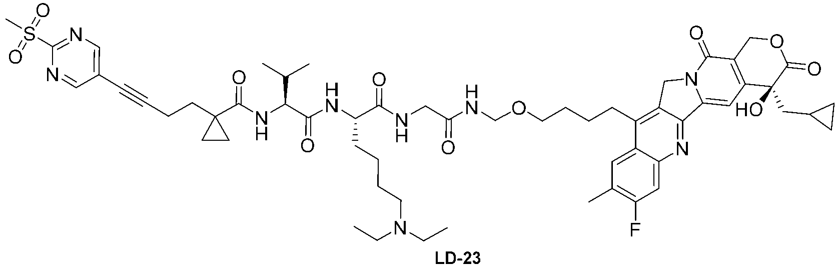

### Example 3-24 LD-24

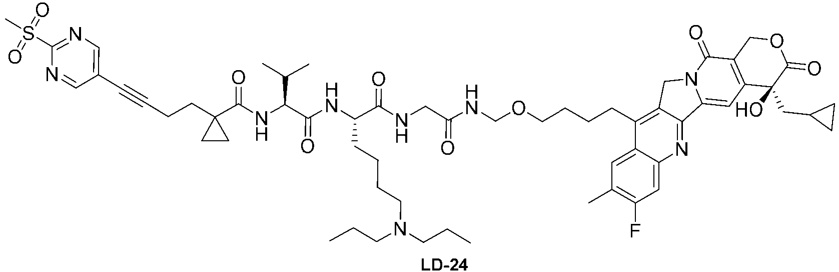

### Example 3-25 LD-25

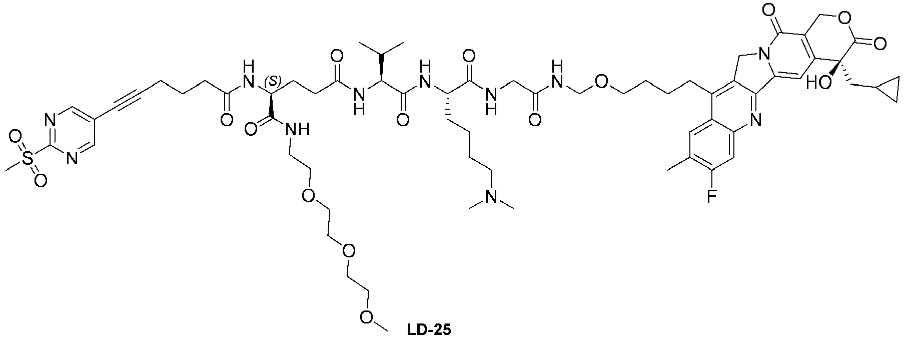

### Example 3-26 LD-26

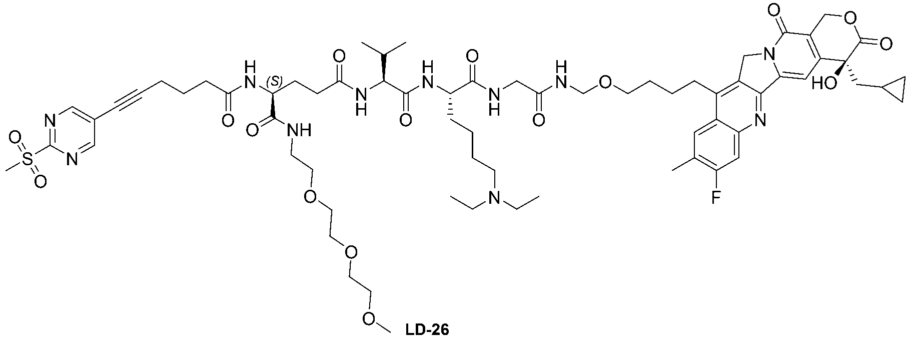

### Example 3-27 LD-27

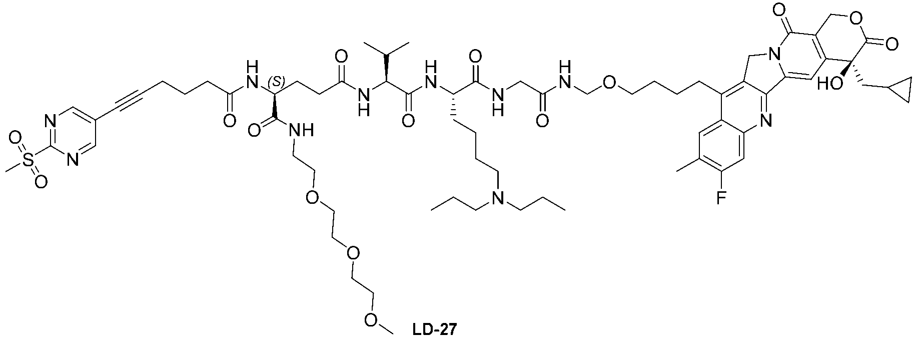

### Example 3-28 LD-28

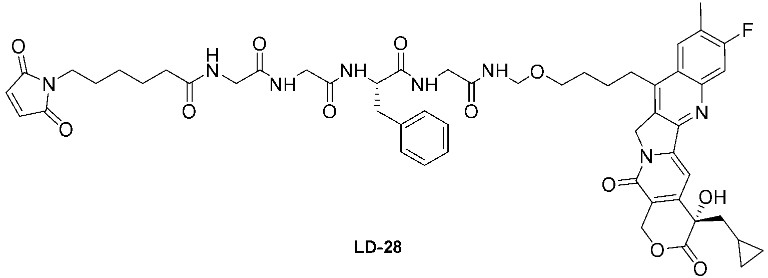

### Example 3-29 LD-29

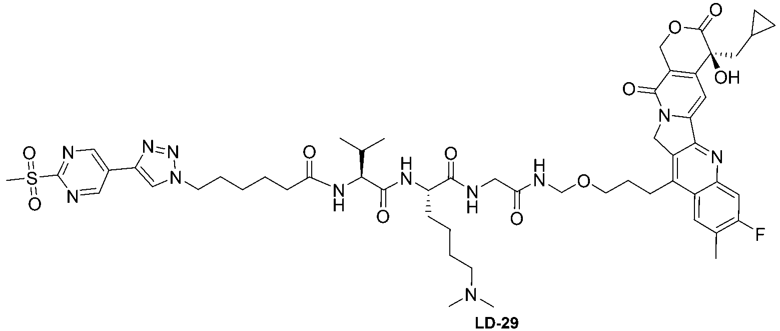

### Example 3-30 LD-30

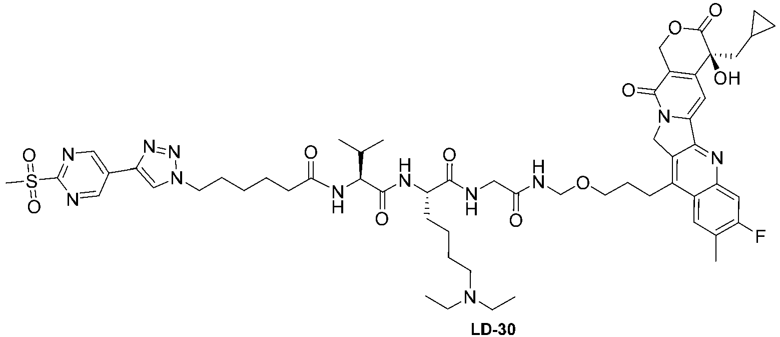

### Example 3-31 LD-31

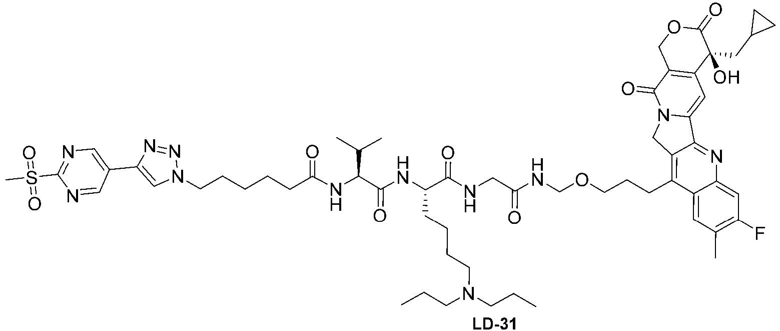

### Example 3-32 LD-32

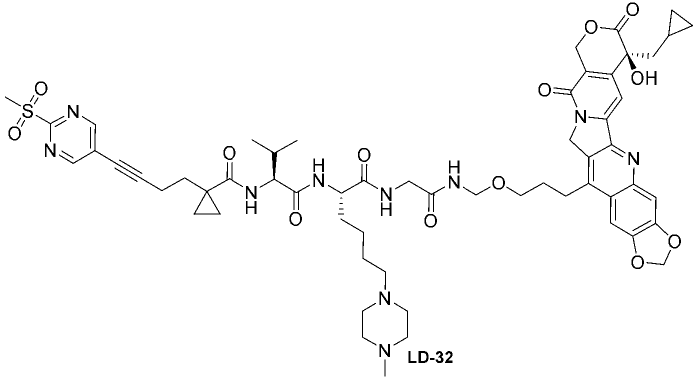

### Example 3-33 LD-33

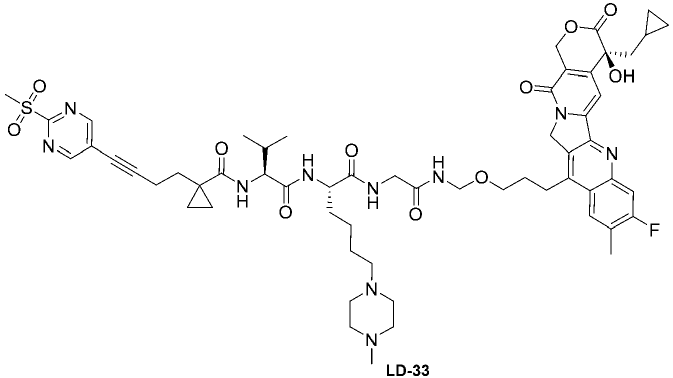

### Example 3-34 LD-34

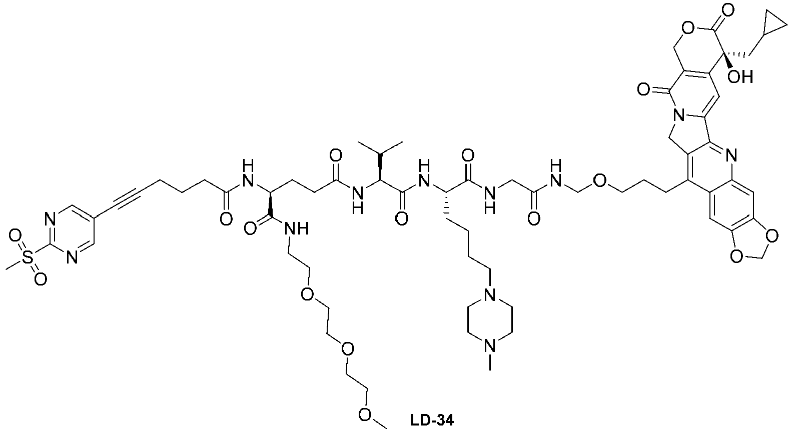

### Example 3-35 LD-35

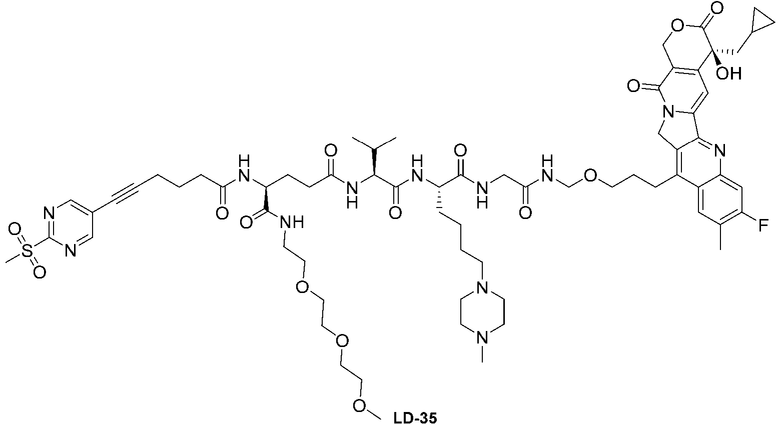

### Example 3-36 LD-36

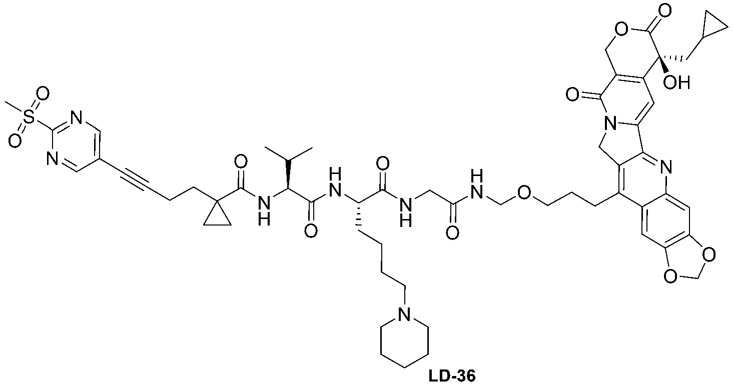

### Example 3-37 LD-37

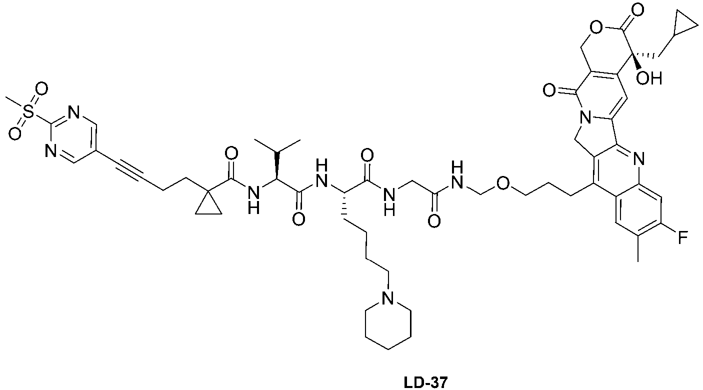

### Example 3-38 LD-38

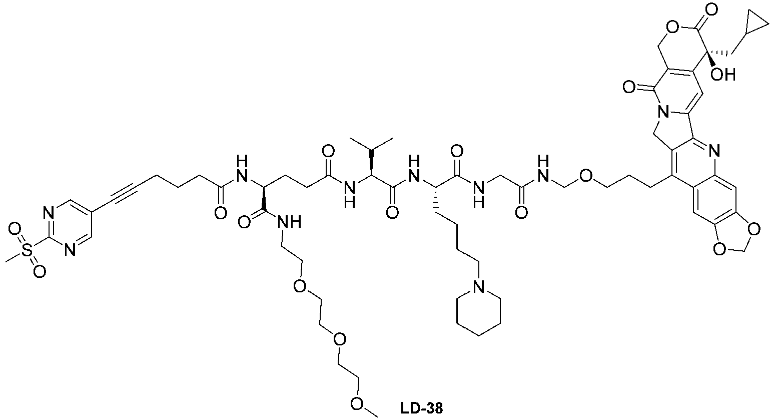

### Example 3-39 LD-39

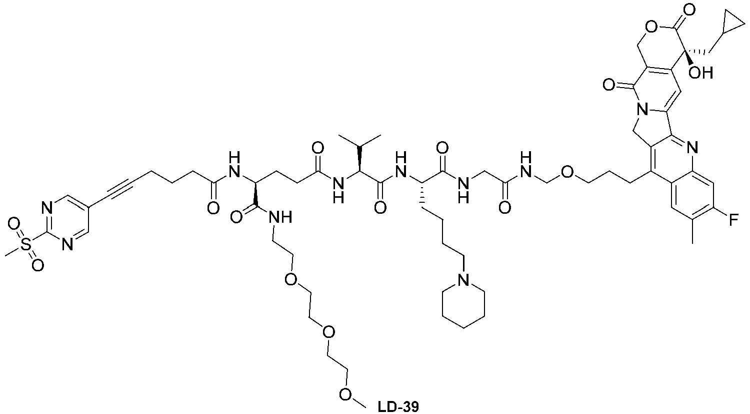

### Example 3-40 LD-40

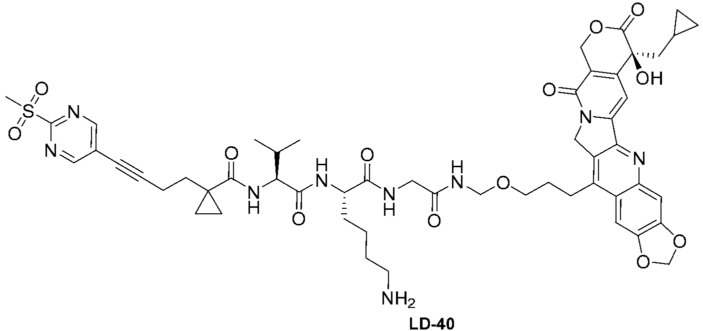

### Example 3-41 LD-41

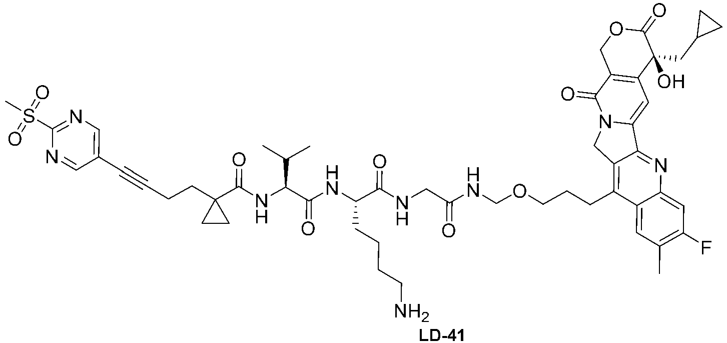

### Example 3-42 LD-42

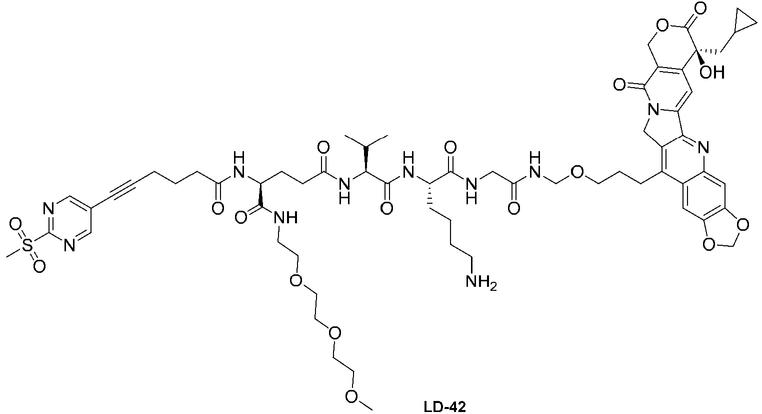

### Example 3-43 LD-43

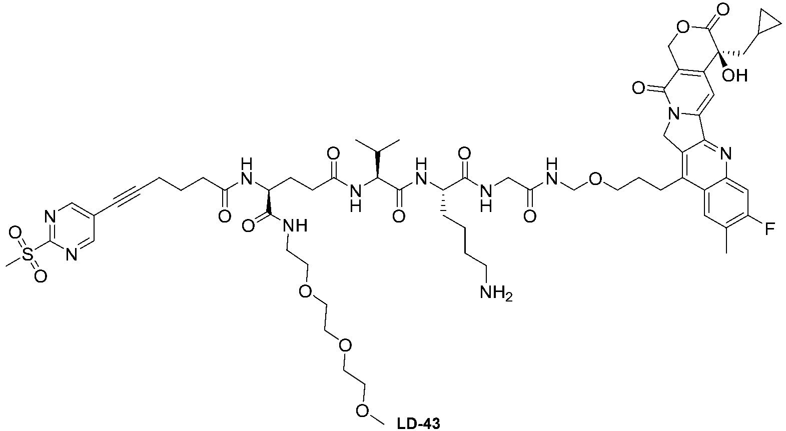

### Example 3-44 LD-44

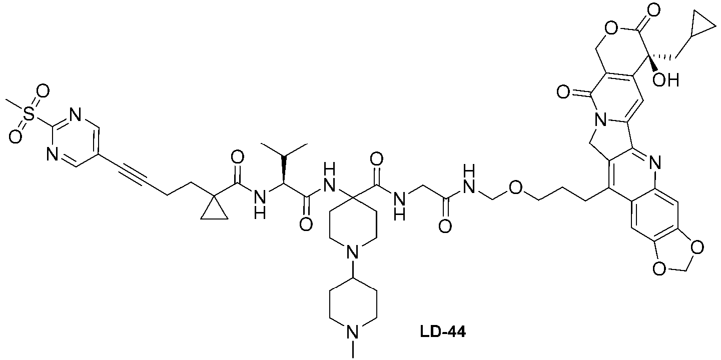

### Example 3-45 LD-45

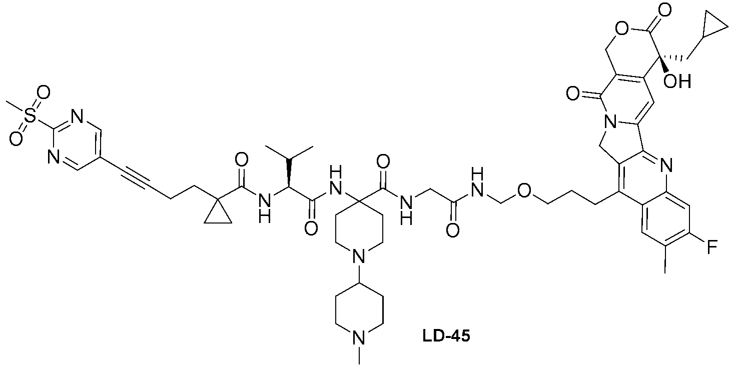

### Example 3-46 LD-46

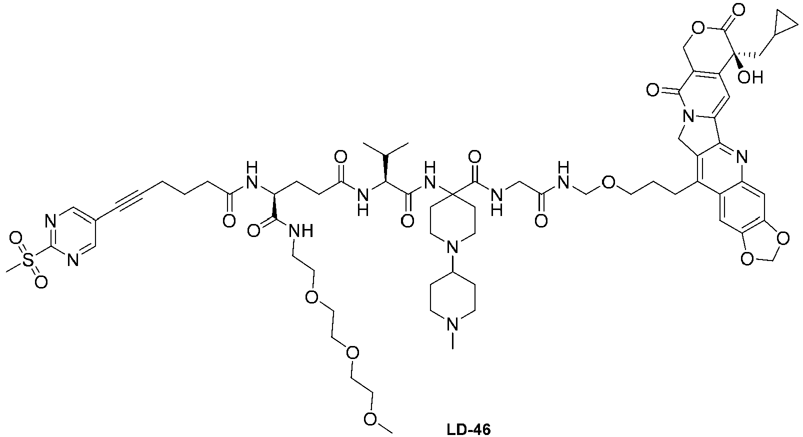

### Example 3-47 LD-47

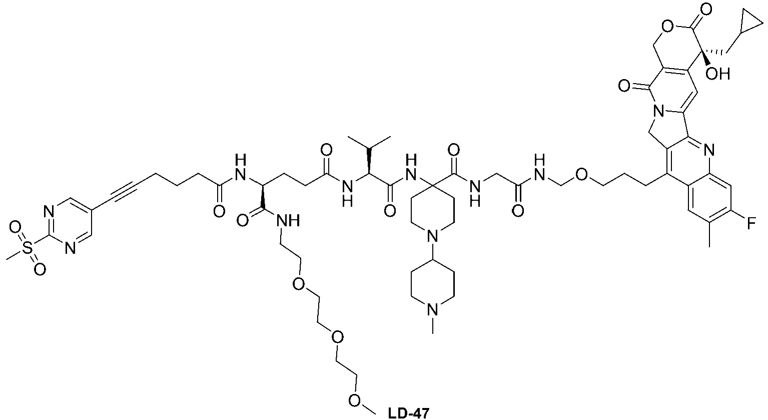

### Example 3-48 LD-48

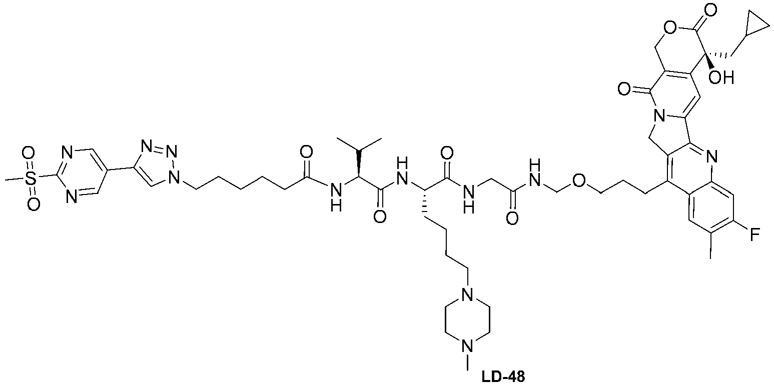

### Example 3-49 LD-49

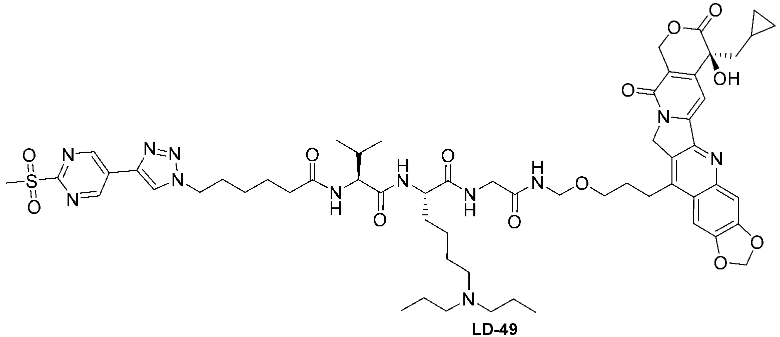

### Example 3-50 LD-50

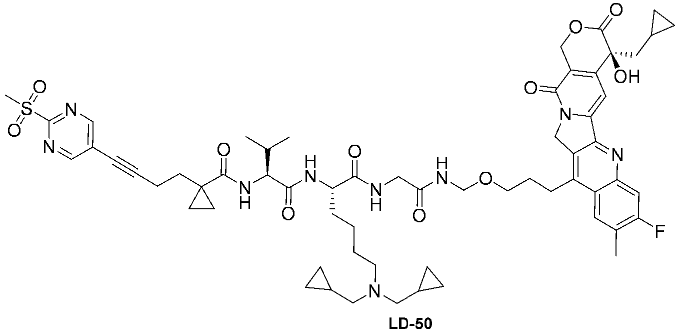

### Example 3-51 LD-51

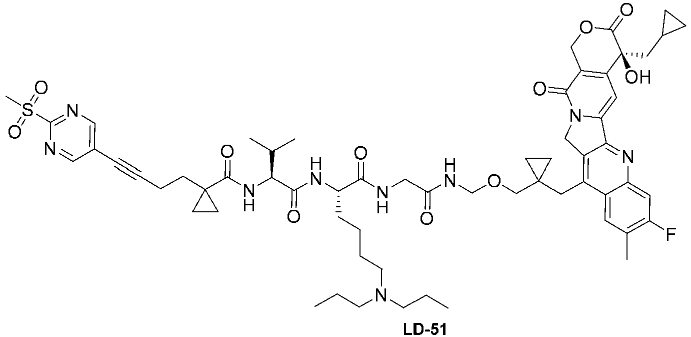

### Example 3-52 LD-52

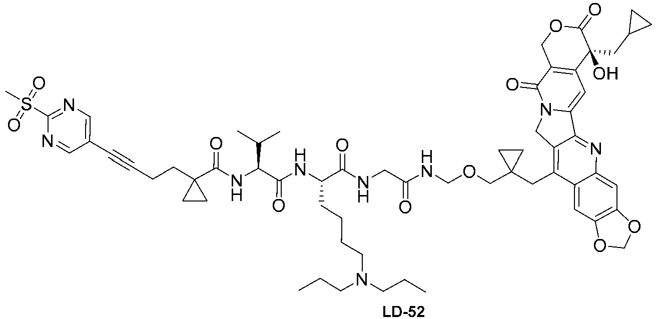

### Example 3-53 LD-53

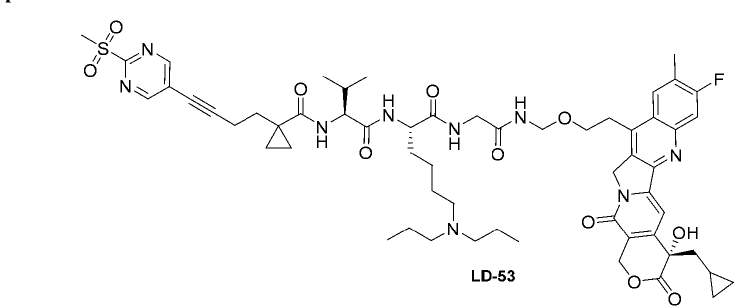

### Example 3-54 LD-54

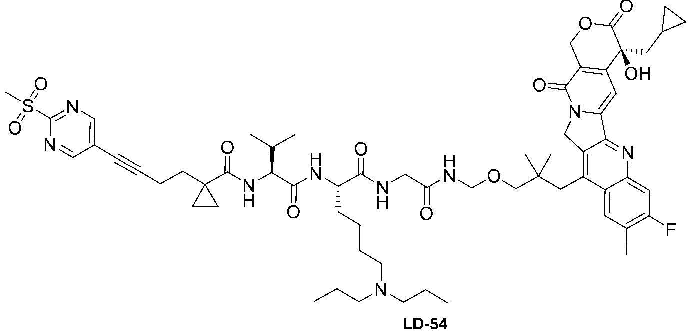

### Example 3-55 LD-55

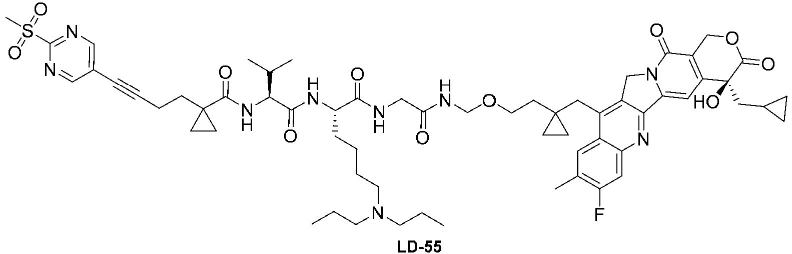

### Example 3-56 LD-56

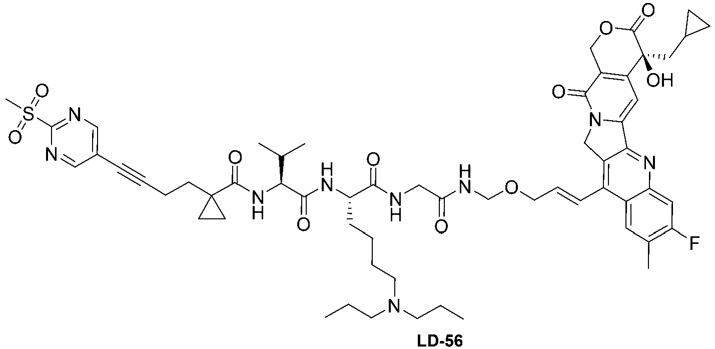

### Example 3-57 LD-57

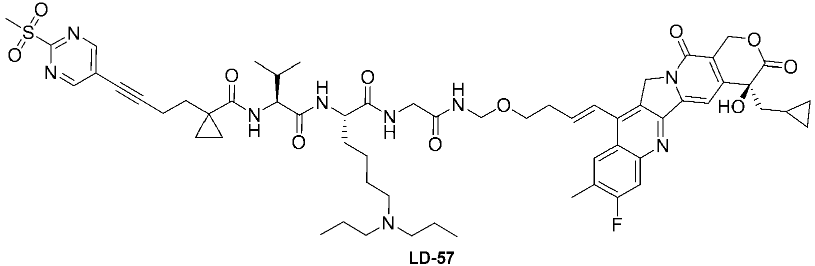

### Example 3-58 LD-58

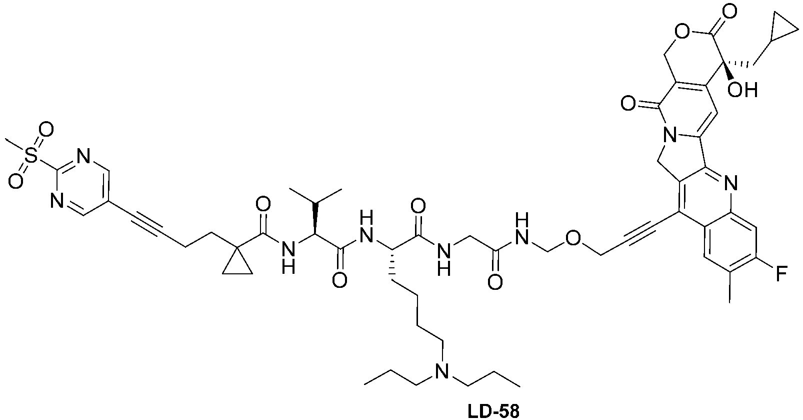

### Example 3-59 LD-59

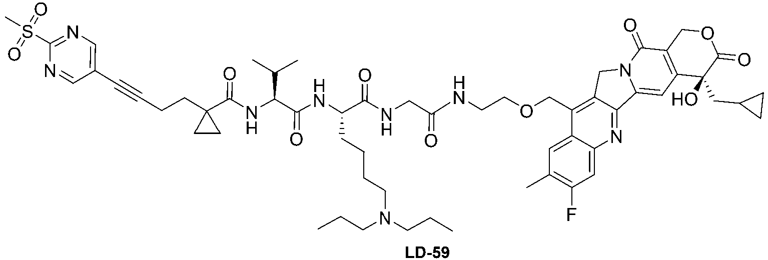

### Example 3-60 LD-60

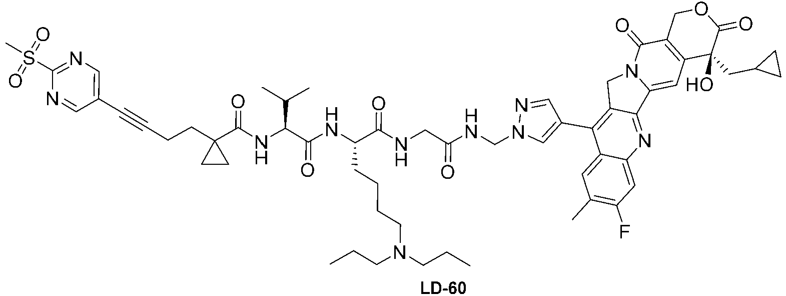

### Example 3-61 LD-61

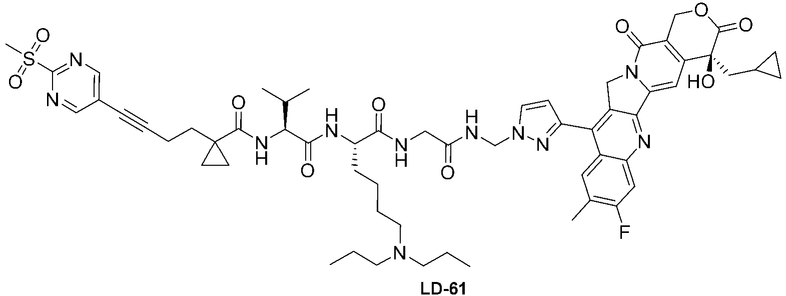

### Example 3-62 LD-62

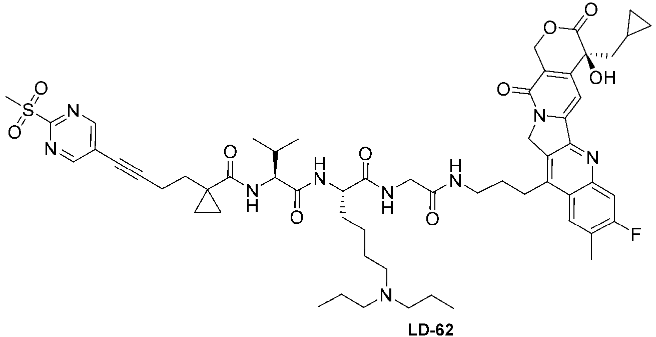

### Example 3-63 LD-63

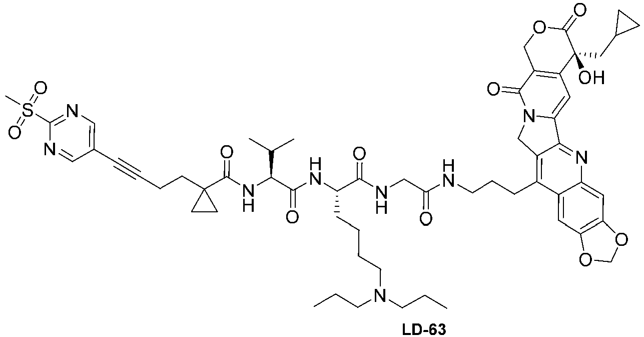

### Example 3-64 LD-64

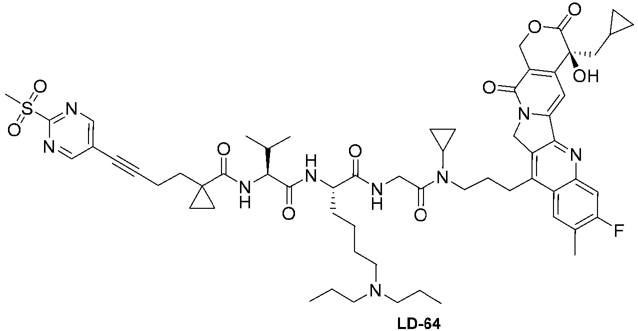

### Example 3-65 LD-65

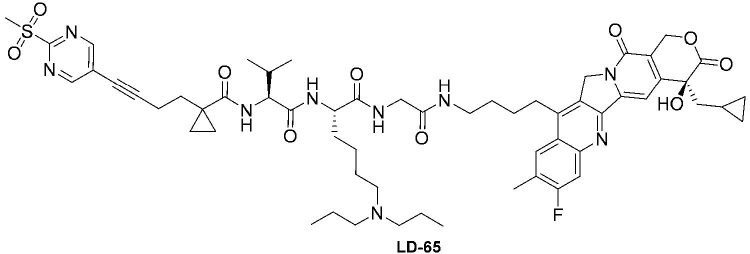

### Example 3-66 LD-66

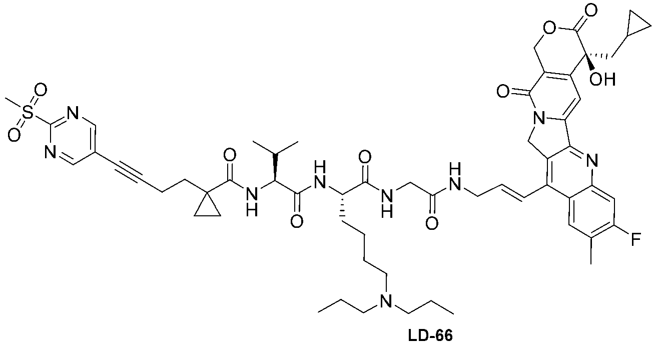

### Example 3-67 LD-67

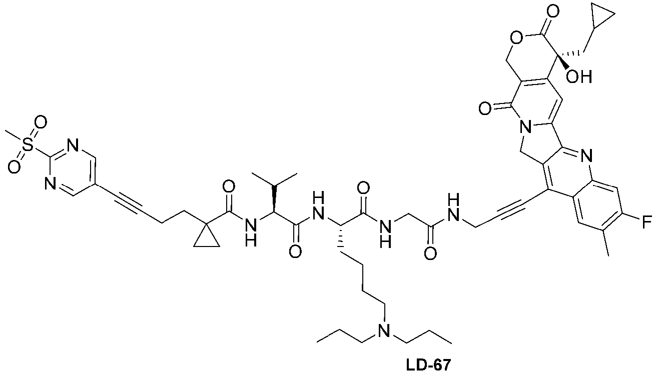

### Example 3-68 LD-68

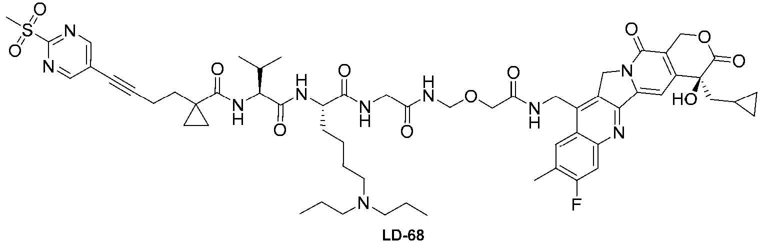

### Example 3-69 LD-69

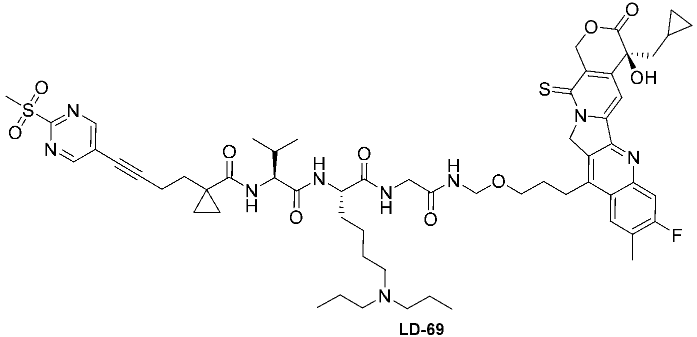

### Example 3-70 LD-70

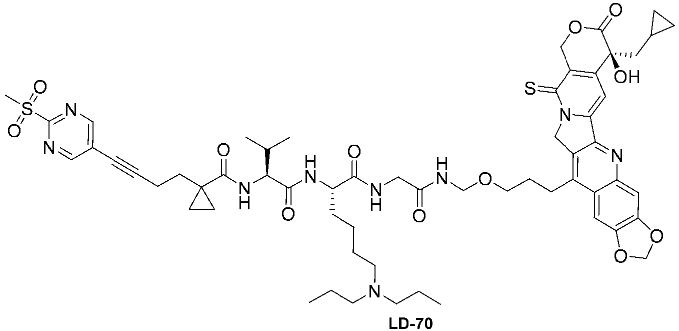

### Example 3-71 LD-71

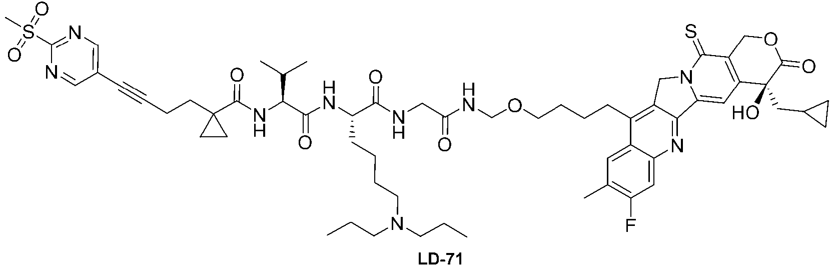

### IV. Examples of ADC Conjugates

### Preparation Procedures of Antibody Conjugate

**A:** The antibody was replaced into 50 mM PBS/1. 0 mM EDTA buffer (adjusted to pH 7.4 with sodium hydroxide solution) using an ultrafiltration tube with a molecular weight cutoff of 50 kD, 5-10 equivalents of 10 mM TCEP in water were added, and shaken at 25 °C for 3 hours. The linker-drug conjugate was dissolved in DMSO, from which 10-20 equivalents of the linker-drug conjugate were taken and added to the reduced antibody solution, vortexed to mix well, and shaken at 25° C for 2 hours to complete the reaction. 40 equivalents of 100 mM NAC in water were added, and the linker reaction was stopped by shaking at 25° C for 20 minutes. Excess small molecules were removed using a 50 kD molecular weight cutoff ultrafiltration centrifuge tube or Sephadex G-25 desalting column, and the antibody-drug conjugate was displaced into 50 mM PBS buffer (pH 6.0), and the sample was filtered using a 0.22 µm membrane to obtain the antibody-drug conjugate, which was stored in a 4 °C refrigerator. The DAR value of the conjugate was determined using reverse-phase high performance liquid chromatography or mass spectrometry. **B:** The antibody was replaced with a 50 kD molecular weight cutoff ultrafiltration tube into 50 mM PBS/1. 0 mM EDTA buffer (pH 6.5 adjusted with sodium hydroxide solution), 5 to 10 equivalents of 10 mM TCEP in water were added, and shaken at 25 °C for 3 hours. The linker-drug conjugate was dissolved in DMSO, from which 10-20 equivalents of the linker-drug conjugate were taken and added dropwise to the reduced antibody solution, vortexed to mix well, and shaken at 25 °C for 2 hours to complete the reaction. 40 equivalents of 100 mM NAC aqueous solution were added, and shaken at 25 °C for 20 minutes to stop the linker reaction. The excess small molecules were removed using a 50 kD molecular weight cutoff ultrafiltration tube or Sephadex G-25 desalting column, and the antibody-drug conjugate was displaced into 50 mM PBS buffer (pH 6.0). The sample was purified by ultrafiltration and filtered with 0.22 µm membrane to obtain the antibody-drug conjugate, which was stored in a refrigerator at 4 °C. The DAR value of the conjugate was determined using reverse-phase high performance liquid chromatography or mass spectrometry.

### Example 4-1 ADC-1

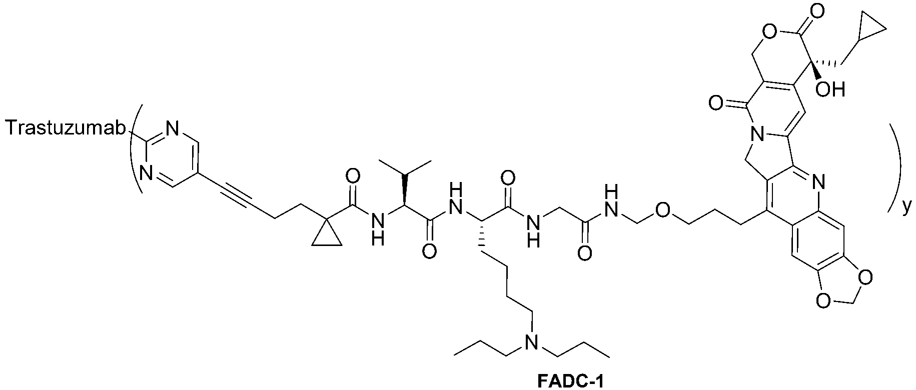

Using antibody Trastuzumab and **LD-1** as starting materials, and following Step A, an example product **ADC-1** of the conjugate mixture **FADC-1** was obtained in PBS buffer and stored at 4 °C. This is designated as Example 4-1.

The average drug loading was calculated by mass spectrometry: y = 8.00.

### Example 4-2 ADC-2

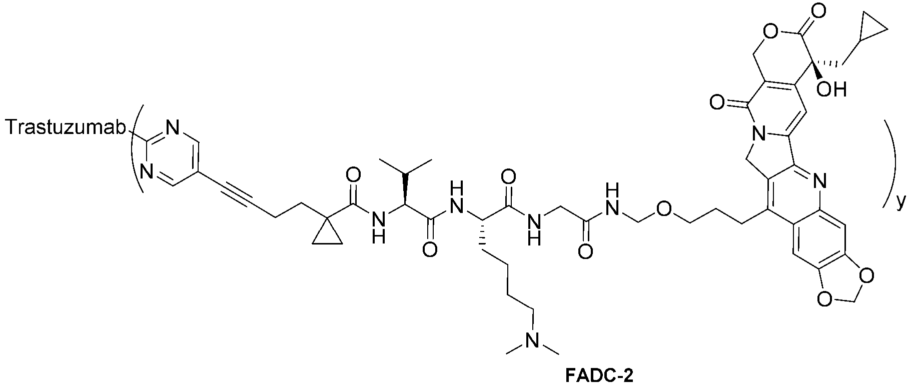

Using antibody Trastuzumab and **LD-2** as starting materials, and following Step A, an example product **ADC-2** of the conjugate mixture **FADC-2** was obtained in PBS buffer and stored at 4 °C. This is designated as Example 4-2.

### Example 4-3 ADC-3

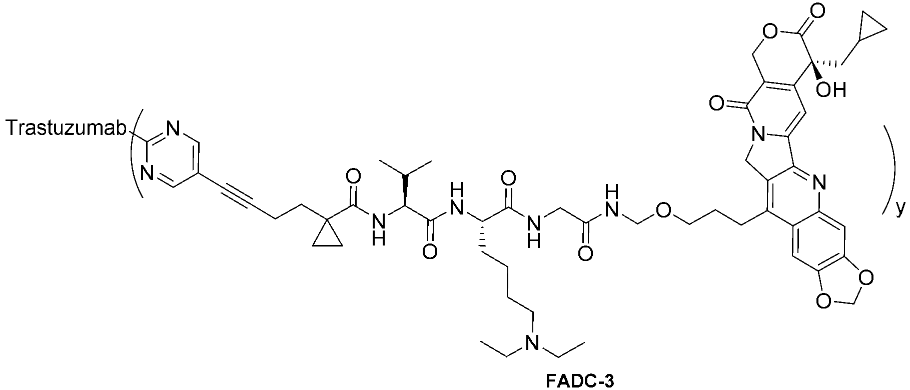

Using antibody Trastuzumab and **LD-3** as starting materials, and following Step A, an example product **ADC-3** of the conjugate mixture **FADC-3** was obtained in PBS buffer and stored at 4 °C. This is designated as Example 4-3.

### Example 4-4 ADC-4

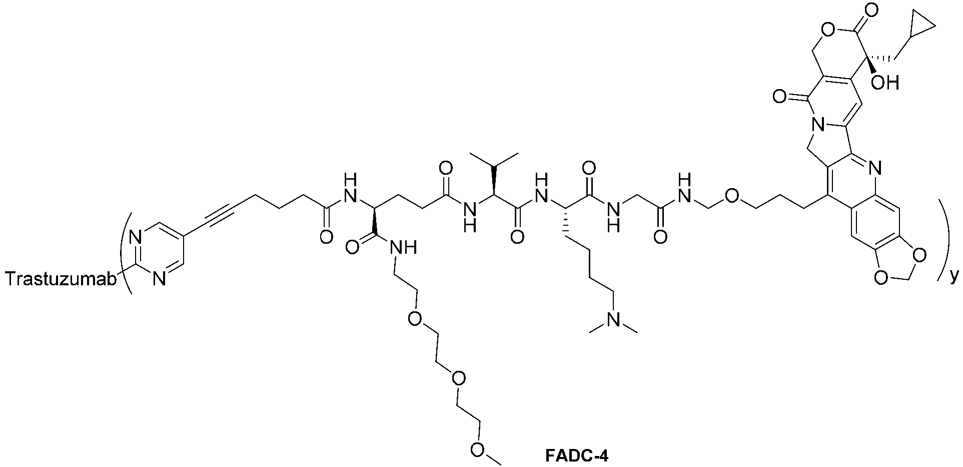

Using antibody Trastuzumab and **LD-4** as starting materials, and following Step A, an example product **ADC-4** of the conjugate mixture **FADC-4** was obtained in PBS buffer and stored at 4 °C. This is designated as Example 4-4.

### Example 4-5 ADC-5

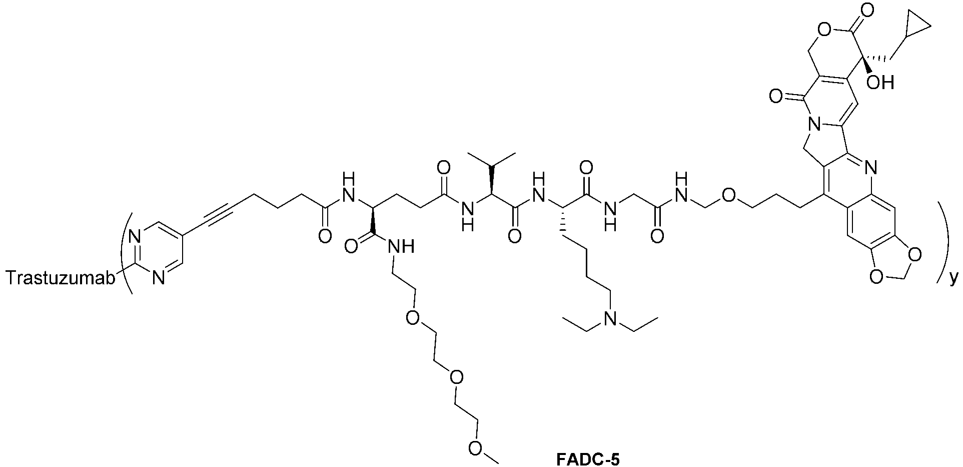

Using antibody Trastuzumab and **LD-5** as starting materials, and following Step A, an example product **ADC-5** of the conjugate mixture **FADC-5** was obtained in PBS buffer and stored at 4 °C. This is designated as Example 4-5.

### Example 4-6 ADC-6

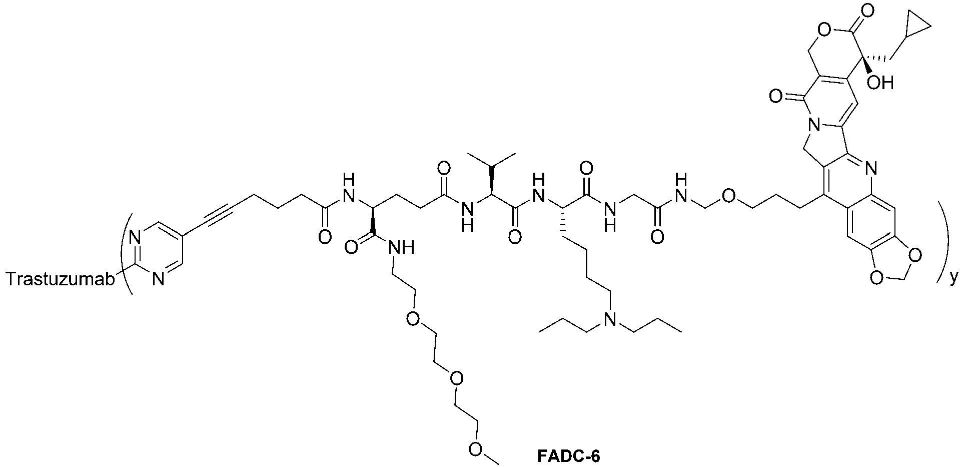

Using antibody Trastuzumab and **LD-6** as starting materials, and following Step A, an example product **ADC-6** of the conjugate mixture **FADC-6** was obtained in PBS buffer and stored at 4 °C. This is designated as Example 4-6.

### Example 4-7 ADC-7

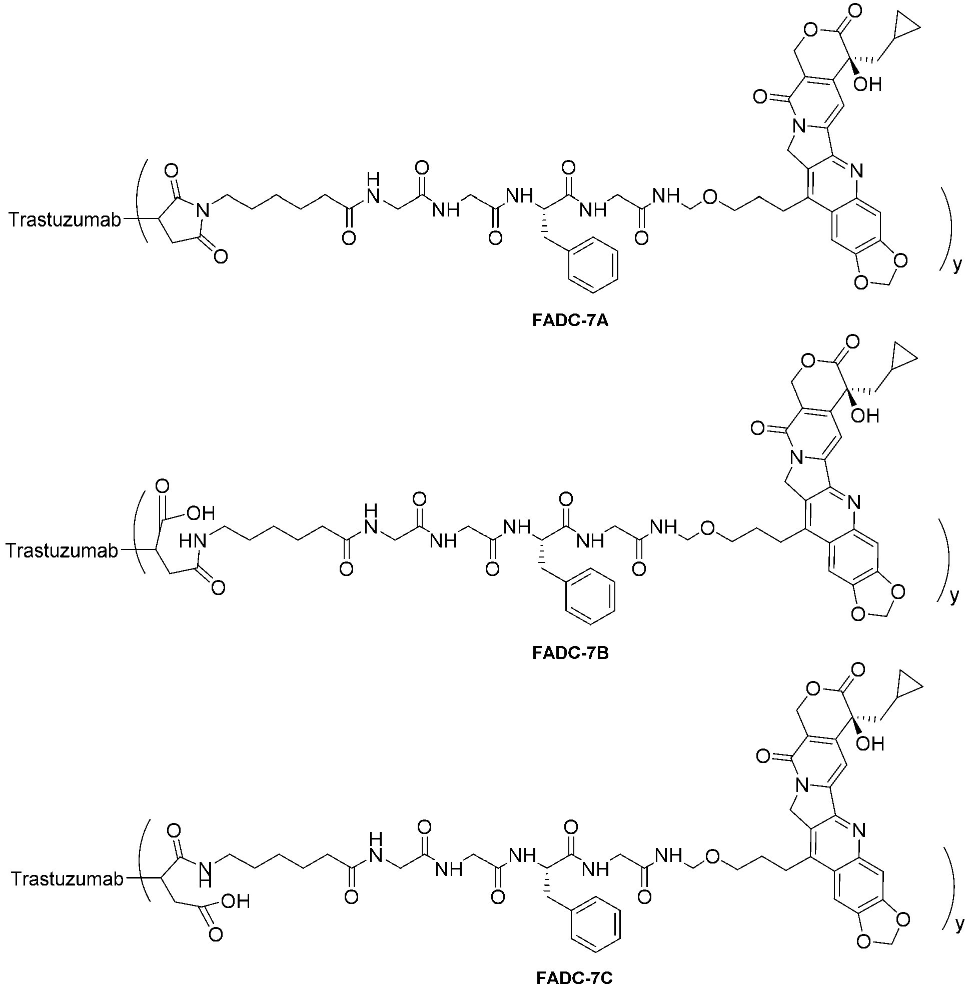

Using antibody Trastuzumab and **LD-7** as starting materials, and following Step B, an example product **ADC-7** of the conjugate mixture **(FADC-7A** and/or **FADC-7B** and/or **FADC-7C** mixture) was obtained in PBS buffer and stored at 4 °C.

### Example 4-8 ADC-8

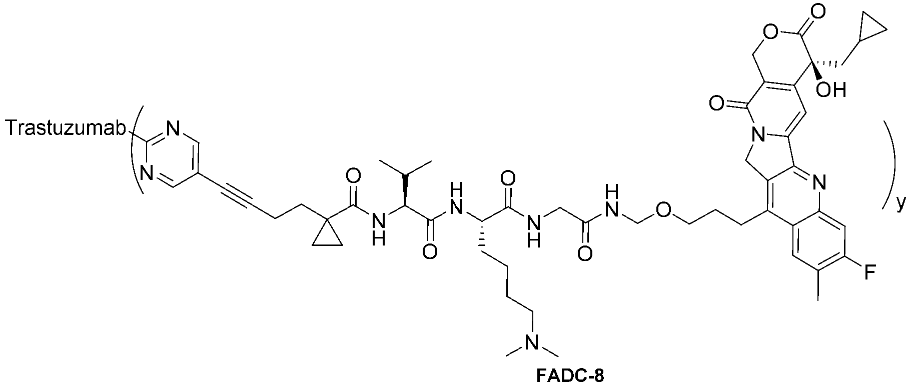

Using antibody Trastuzumab and **LD-8** as starting materials, and following Step A, an example product **ADC-8** of the conjugate mixture **FADC-8** was obtained in PBS buffer and stored at 4 °C. This is designated as Example 4-8.

### Example 4-9 ADC-9

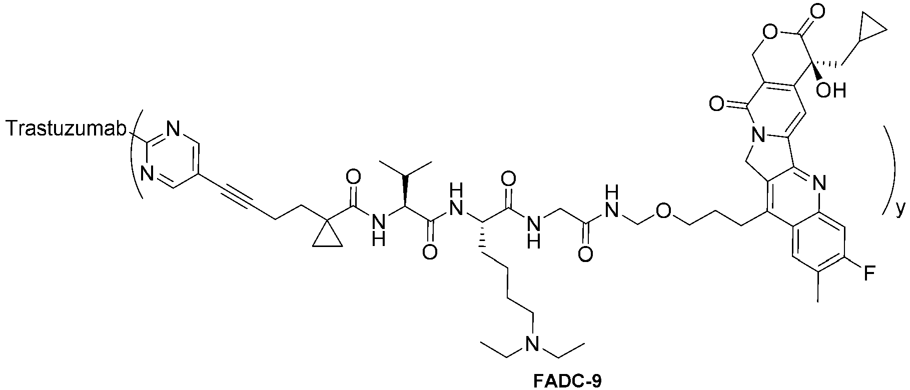

Using antibody Trastuzumab and **LD-9** as starting materials, and following Step A, an example product **ADC-9** of the conjugate mixture **FADC-9** was obtained in PBS buffer and stored at 4 °C. This is designated as Example 4-9.

### Example 4-10 ADC-10

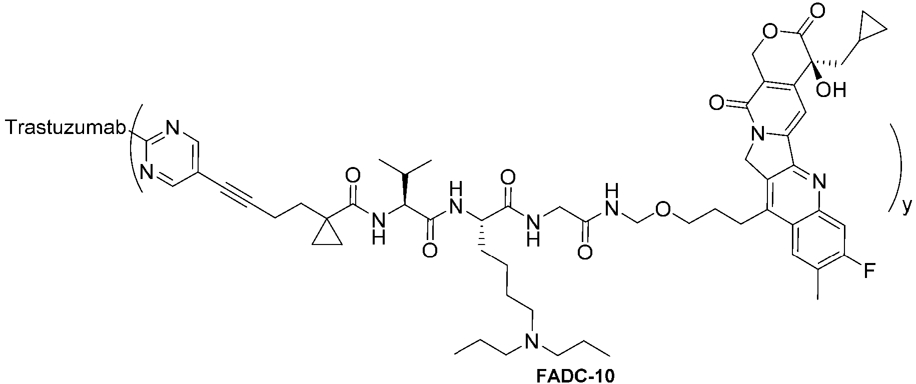

Using antibody Trastuzumab and **LD-10** as starting materials, and following Step A, an example product **ADC-10** of the conjugate mixture **FADC-10** was obtained in PBS buffer and stored at 4 °C. This is designated as Example 4-10.

The average drug loading was calculated by mass spectrometry: y = 8.00.

### Example 4-11 ADC-11

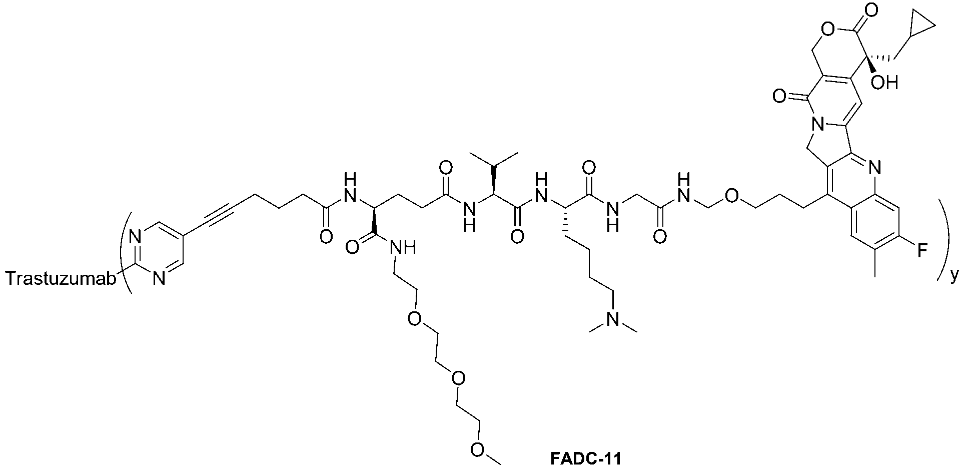

Using antibody Trastuzumab and **LD-11** as starting materials, and following Step A, an example product **ADC-11** of the conjugate mixture **FADC-11** was obtained in PBS buffer and stored at 4 °C. This is designated as Example 4-11.

### Example 4-12 ADC-12

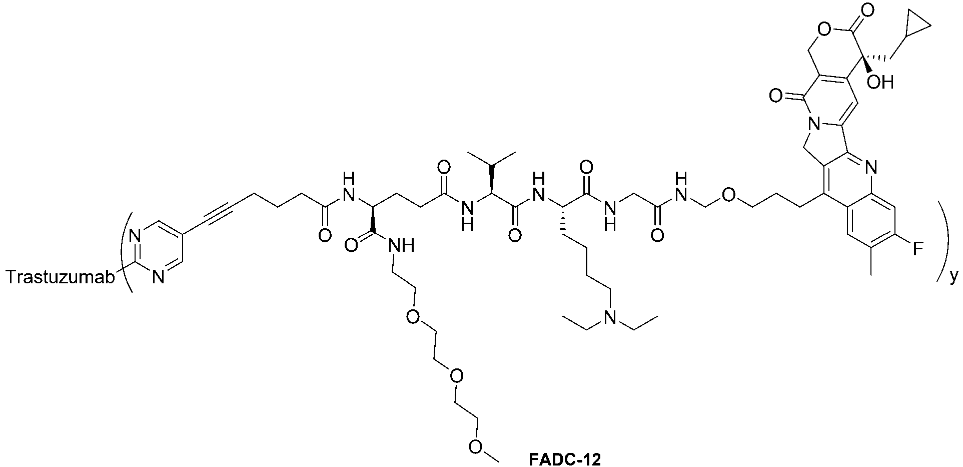

Using antibody Trastuzumab and **LD-12** as starting materials, and following Step A, an example product **ADC-12** of the conjugate mixture **FADC-12** was obtained in PBS buffer and stored at 4 °C. This is designated as Example 4-12.

### Example 4-13 ADC-13

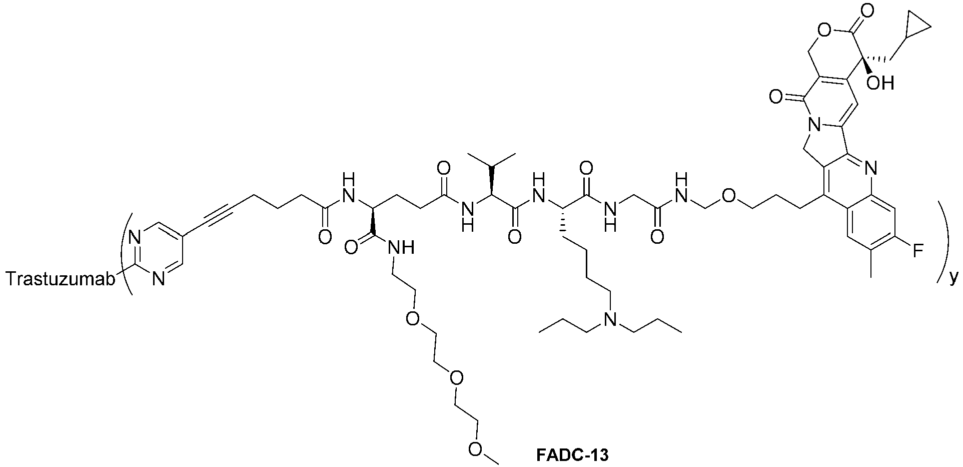

Using antibody Trastuzumab and **LD-13** as starting materials, and following Step A, an example product **ADC-13** of the conjugate mixture **FADC-13** was obtained in PBS buffer and stored at 4 °C. This is designated as Example 4-13.

### Example 4-14 ADC-14

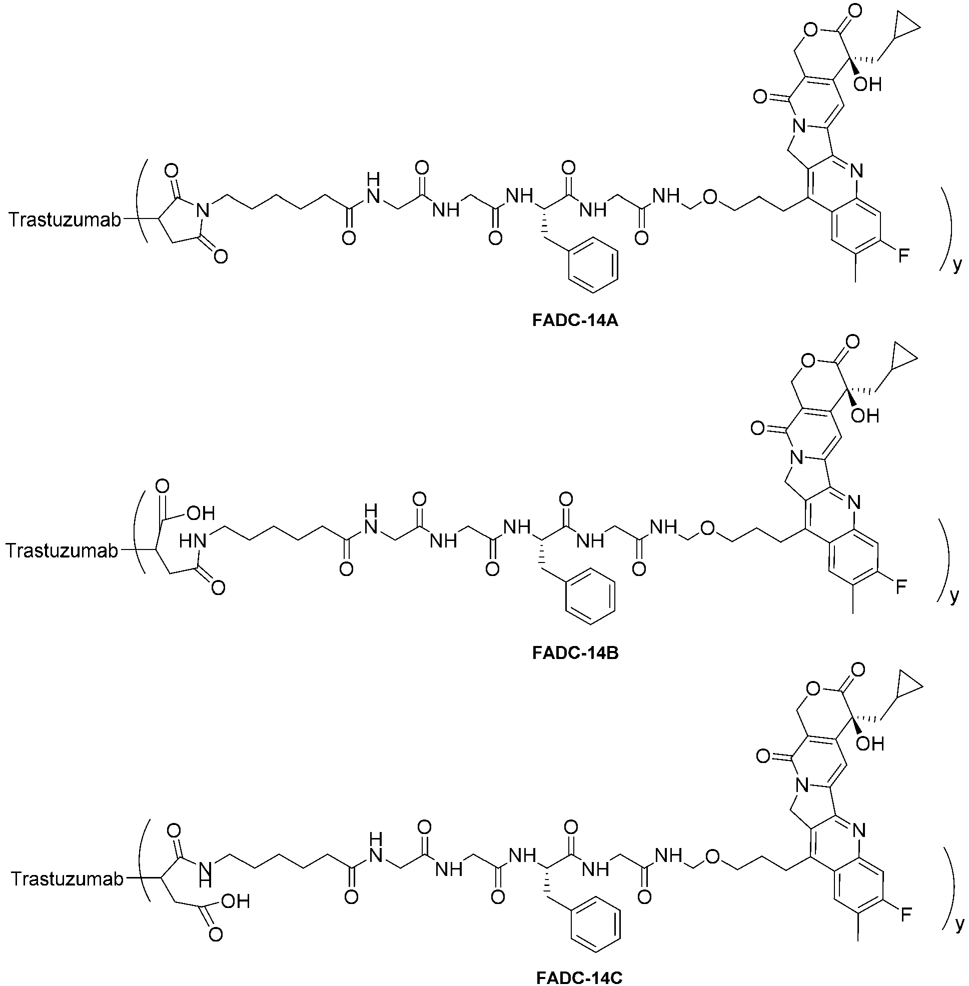

Using antibody Trastuzumab and **LD-14** as starting materials, and following Step B, an example product **ADC-14** of the conjugate mixture **(FADC-14A** and/or **FADC-14B** and/or **FADC-14C** mixture) was obtained in PBS buffer and stored at 4 °C.

### Example 4-15 ADC-15

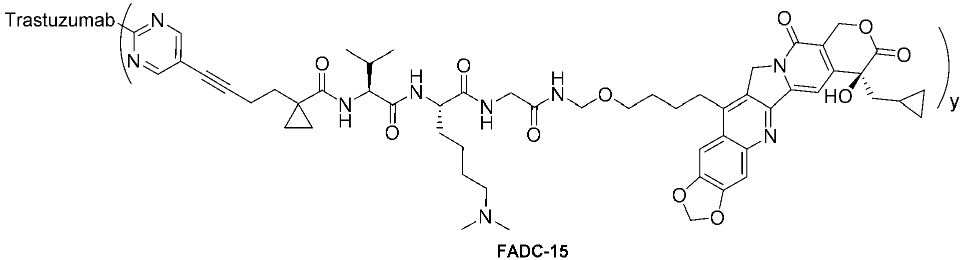

Using antibody Trastuzumab and **LD-15** as starting materials, and following Step A, an example product **ADC-15** of the conjugate mixture **FADC-15** was obtained in PBS buffer and stored at 4 °C. This is designated as Example 4-15.

### Example 4-16 ADC-16

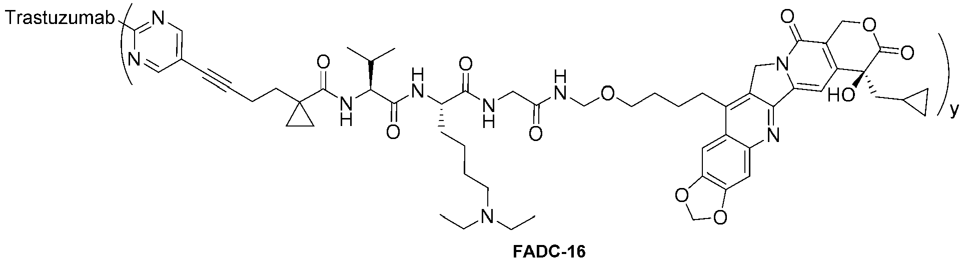

Using antibody Trastuzumab and **LD-16** as starting materials, and following Step A, an example product **ADC-16** of the conjugate mixture **FADC-16** was obtained in PBS buffer and stored at 4 °C. This is designated as Example 4-16.

### Example 4-17 ADC-17

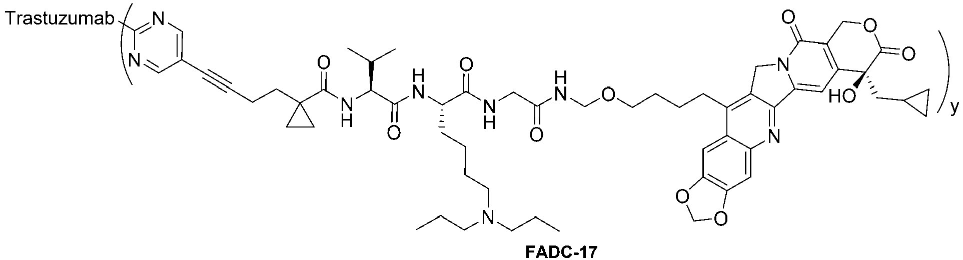

Using antibody Trastuzumab and **LD-17** as starting materials, and following Step A, an example product **ADC-17** of the conjugate mixture **FADC-17** was obtained in PBS buffer and stored at 4 °C. This is designated as Example 4-17.

### Example 4-18 ADC-18

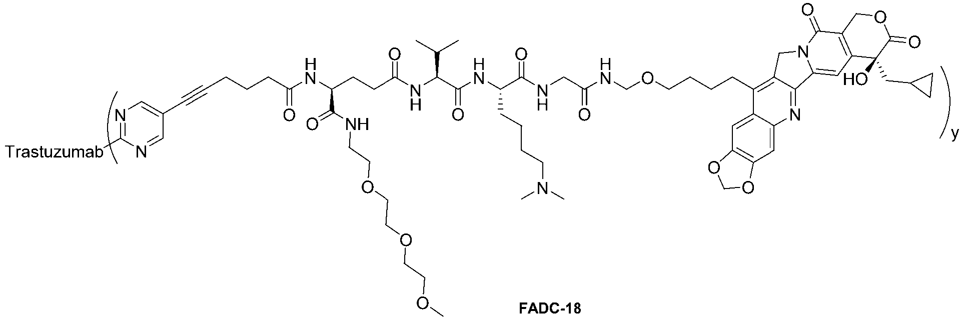

Using antibody Trastuzumab and **LD-18** as starting materials, and following Step A, an example product **ADC-18** of the conjugate mixture **FADC-18** was obtained in PBS buffer and stored at 4 °C. This is designated as Example 4-18.

### Example 4-19 ADC-19

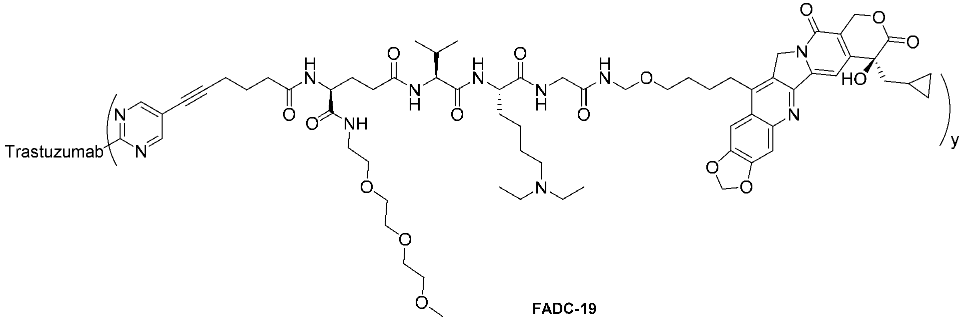

Using antibody Trastuzumab and **LD-19** as starting materials, and following Step A, an example product **ADC-19** of the conjugate mixture **FADC-19** was obtained in PBS buffer and stored at 4 °C. This is designated as Example 4-19.

### Example 4-20 ADC-20

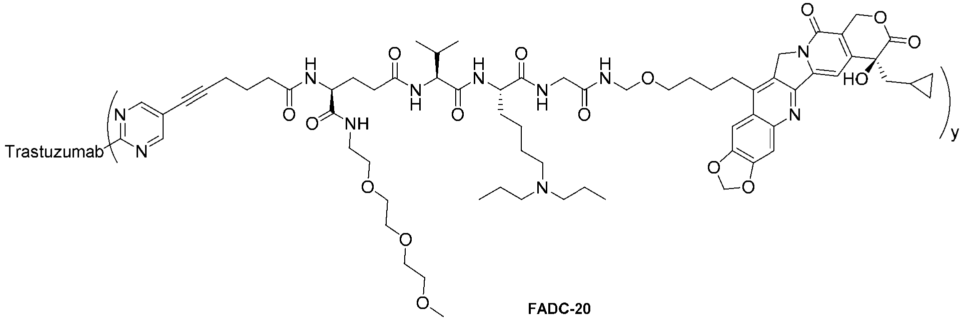

Using antibody Trastuzumab and **LD-20** as starting materials, and following Step A, an example product **ADC-20** of the conjugate mixture **FADC-20** was obtained in PBS buffer and stored at 4 °C. This is designated as Example 4-20.

### Example 4-21 ADC-21

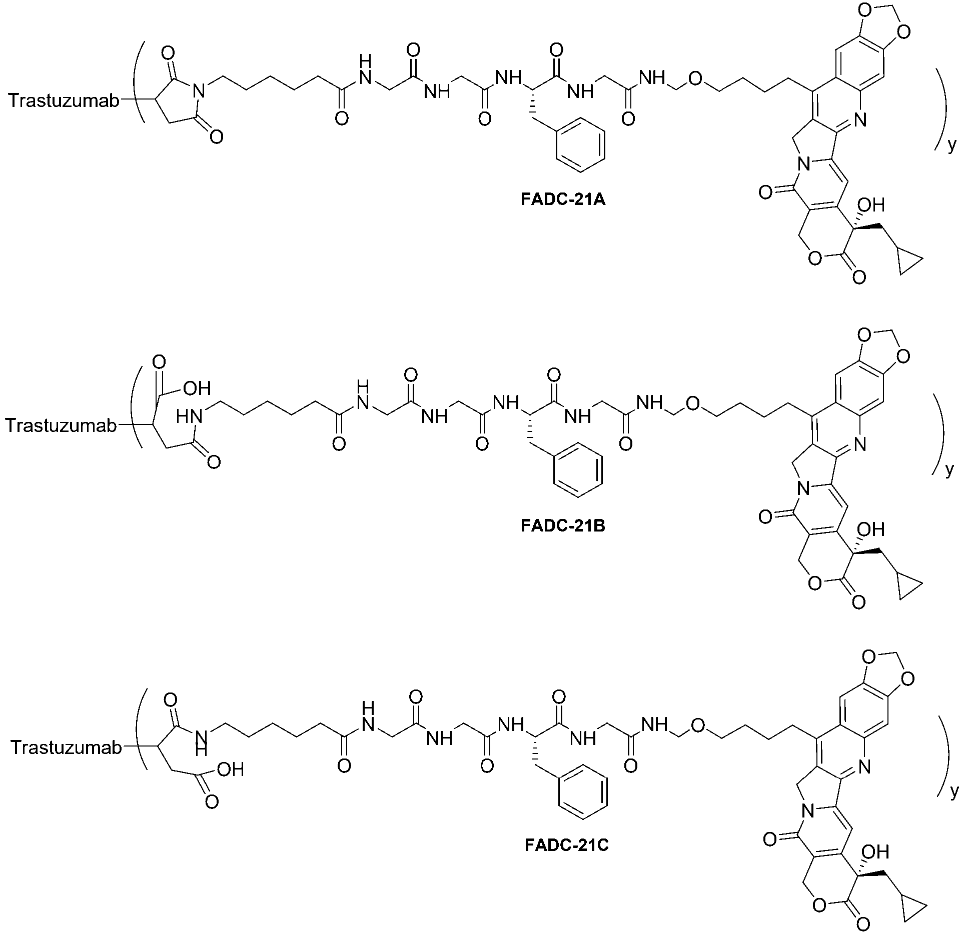

Using antibody Trastuzumab and **LD-21** as starting materials, and following Step B, an example product **ADC-21** of the conjugate mixture **(FADC-21A** and/or **FADC-21B** and/or **FADC-21C** mixture) was obtained in PBS buffer and stored at 4 °C.

### Example 4-22 ADC-22

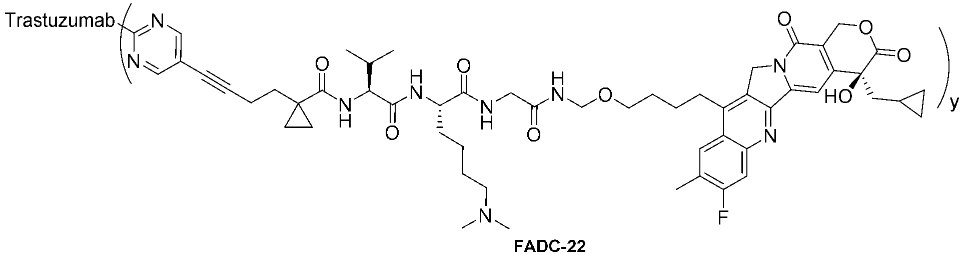

Using antibody Trastuzumab and **LD-22** as starting materials, and following Step A, an example product **ADC-22** of the conjugate mixture **FADC-22** was obtained in PBS buffer and stored at 4 °C. This is designated as Example 4-22.

### Example 4-23 ADC-23

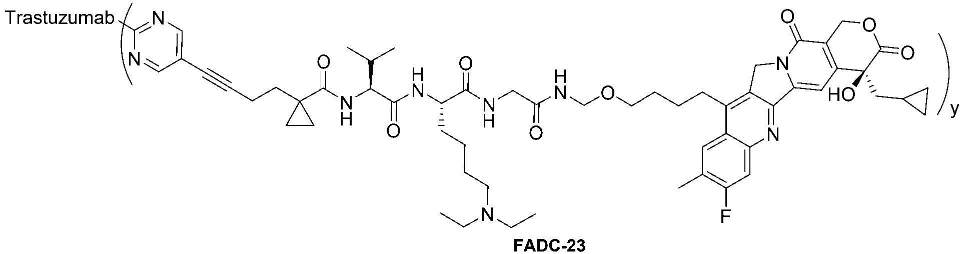

Using antibody Trastuzumab and **LD-23** as starting materials, and following Step A, an example product **ADC-23** of the conjugate mixture **FADC-23** was obtained in PBS buffer and stored at 4 °C. This is designated as Example 4-23.

### Example 4-24 ADC-24

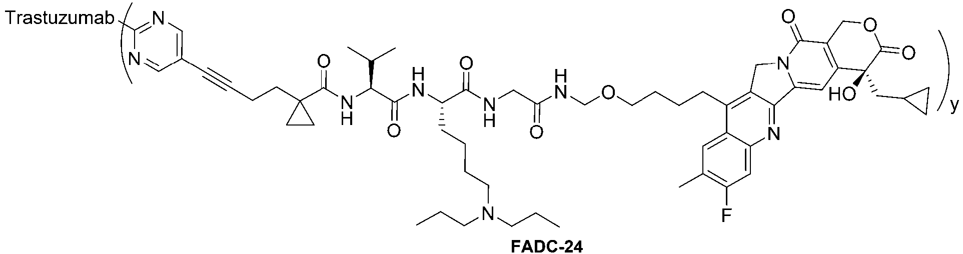

Using antibody Trastuzumab and **LD-24** as starting materials, and following Step A, an example product **ADC-24** of the conjugate mixture **FADC-24** was obtained in PBS buffer and stored at 4 °C. This is designated as Example 4-24.

### Example 4-25 ADC-25

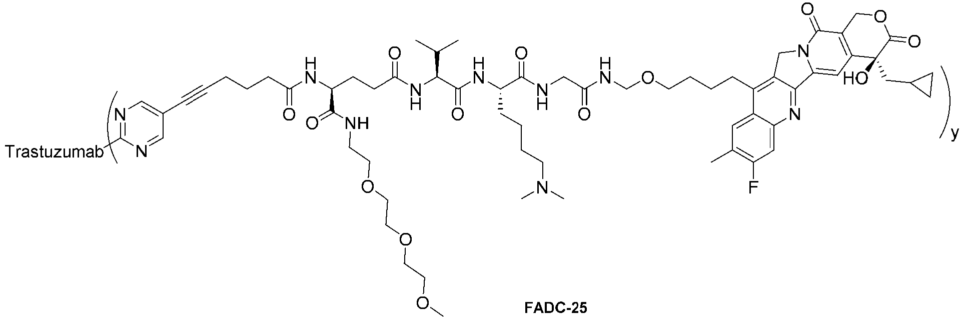

Using antibody Trastuzumab and **LD-25** as starting materials, and following Step A, an example product **ADC-25** of the conjugate mixture **FADC-25** was obtained in PBS buffer and stored at 4 °C. This is designated as Example 4-25.

### Example 4-26 ADC-26

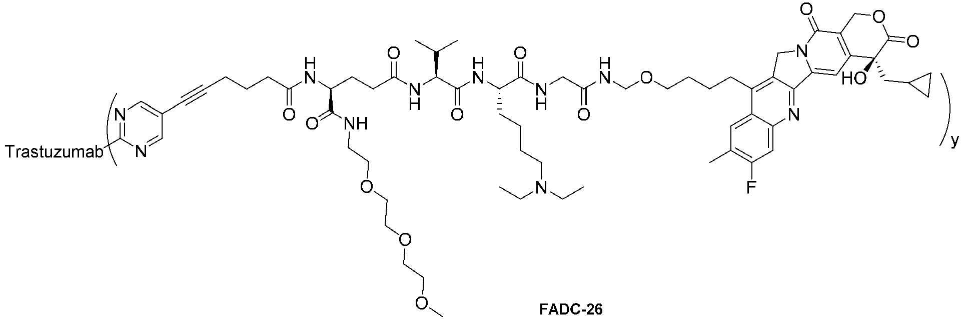

Using antibody Trastuzumab and **LD-26** as starting materials, and following Step A, an example product **ADC-26** of the conjugate mixture **FADC-26** was obtained in PBS buffer and stored at 4 °C. This is designated as Example 4-26.

### Example 4-27 ADC-27

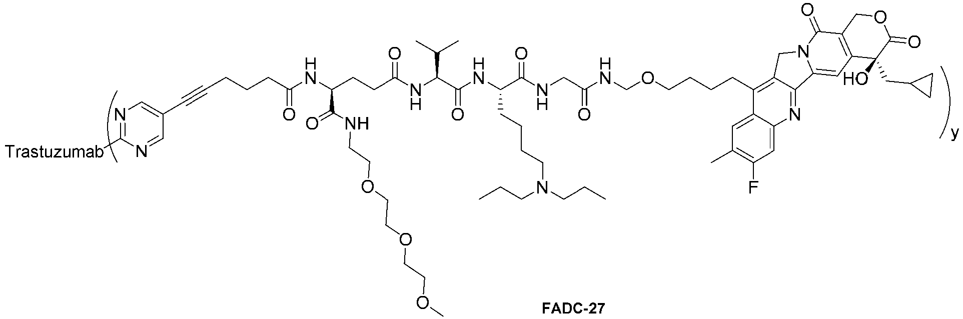

Using antibody Trastuzumab and **LD-27** as starting materials, and following Step A, an example product **ADC-27** of the conjugate mixture **FADC-27** was obtained in PBS buffer and stored at 4 °C. This is designated as Example 4-27.

### Example 4-28 ADC-28

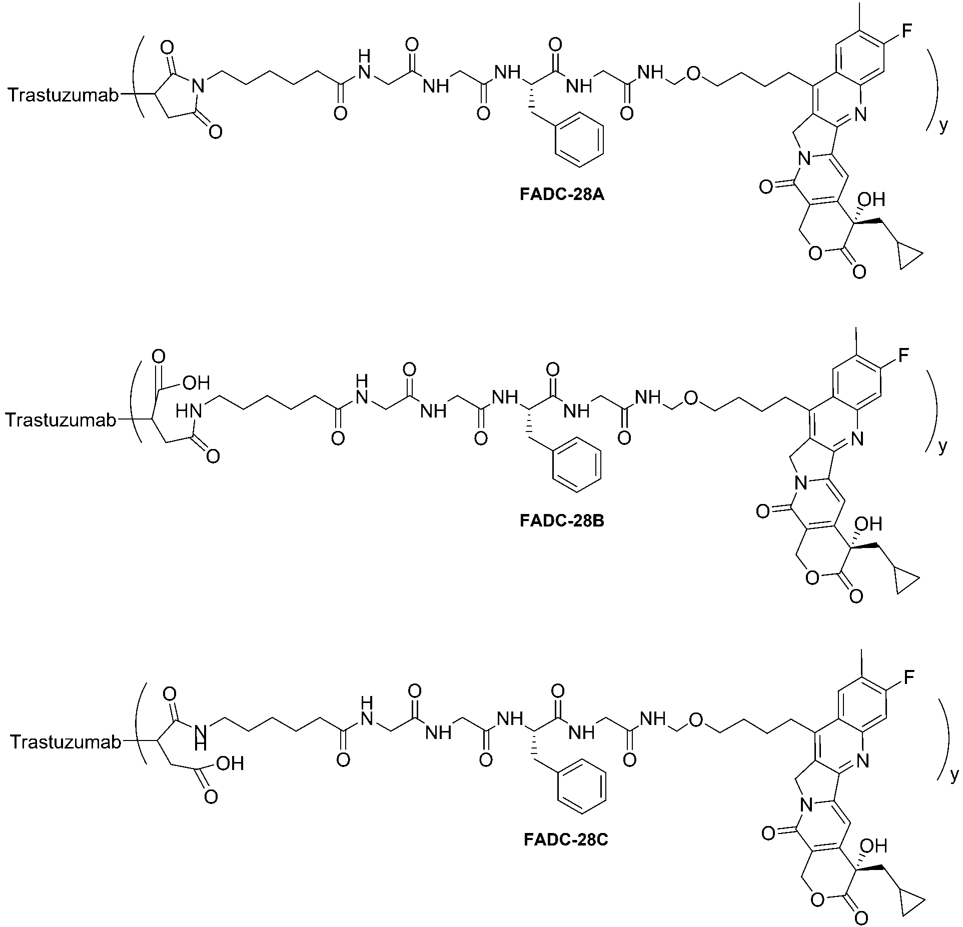

Using antibody Trastuzumab and **LD-28** as starting materials, and following Step B, an example product **ADC-28** of the conjugate mixture **(FADC-28A** and/or **FADC-28B** and/or **FADC-28C** mixture) was obtained in PBS buffer and stored at 4 °C.

### Example 4-29 ADC-29

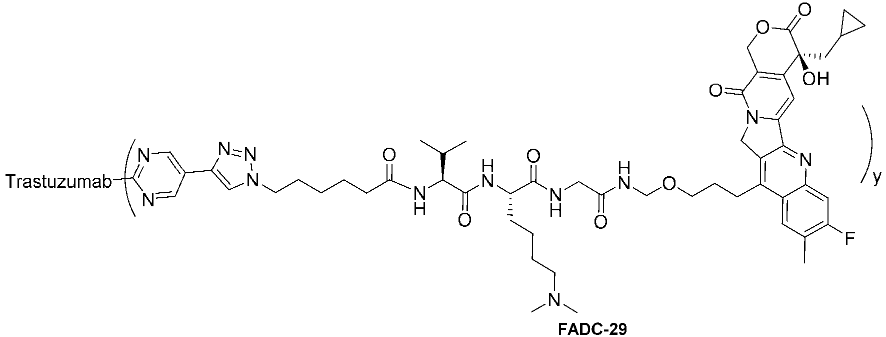

Using antibody Trastuzumab and **LD-29** as starting materials, and following Step A, an example product **ADC-29** of the conjugate mixture **FADC-29** was obtained in PBS buffer and stored at 4 °C. This is designated as Example 4-29.

### Example 4-30 ADC-30

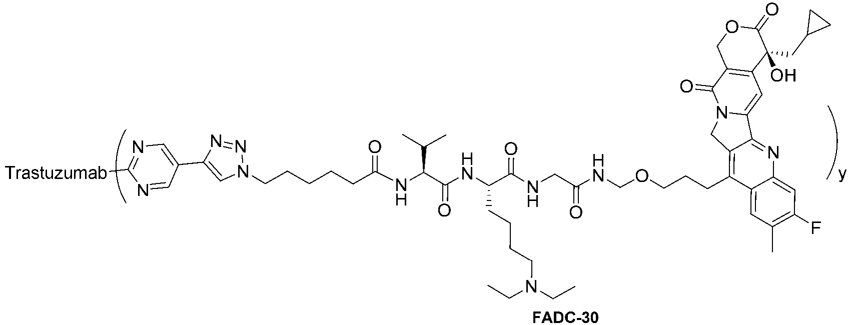

Using antibody Trastuzumab and **LD-30** as starting materials, and following Step A, an example product **ADC-30** of the conjugate mixture **FADC-30** was obtained in PBS buffer and stored at 4 °C. This is designated as Example 4-30.

### Example 4-31 ADC-31

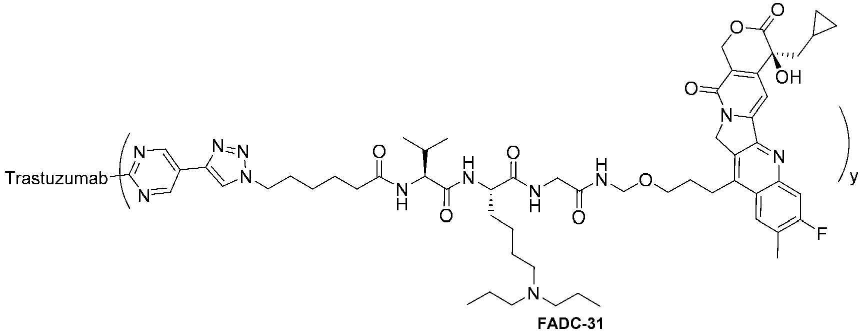

Using antibody Trastuzumab and **LD-31** as starting materials, and following Step A, an example product **ADC-31** of the conjugate mixture **FADC-31** was obtained in PBS buffer and stored at 4 °C. This is designated as Example 4-31.

### Example 4-32 ADC-32

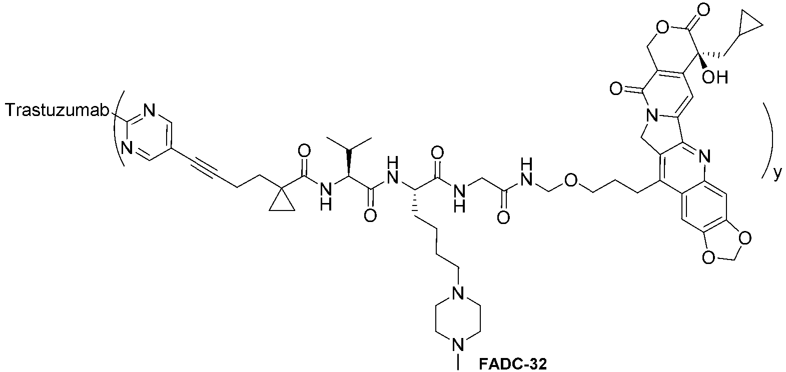

Using antibody Trastuzumab and **LD-32** as starting materials, and following Step A, an example product **ADC-32** of the conjugate mixture **FADC-32** was obtained in PBS buffer and stored at 4 °C. This is designated as Example 4-32.

### Example 4-33 ADC-33

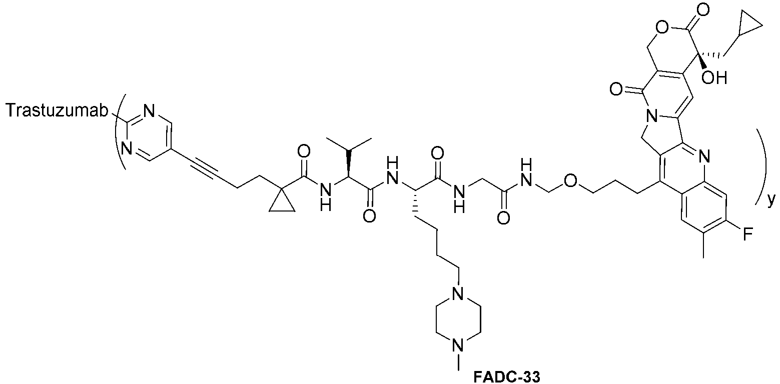

Using antibody Trastuzumab and **LD-33** as starting materials, and following Step A, an example product **ADC-33** of the conjugate mixture **FADC-33** was obtained in PBS buffer and stored at 4 °C. This is designated as Example 4-33.

### Example 4-34 ADC-34

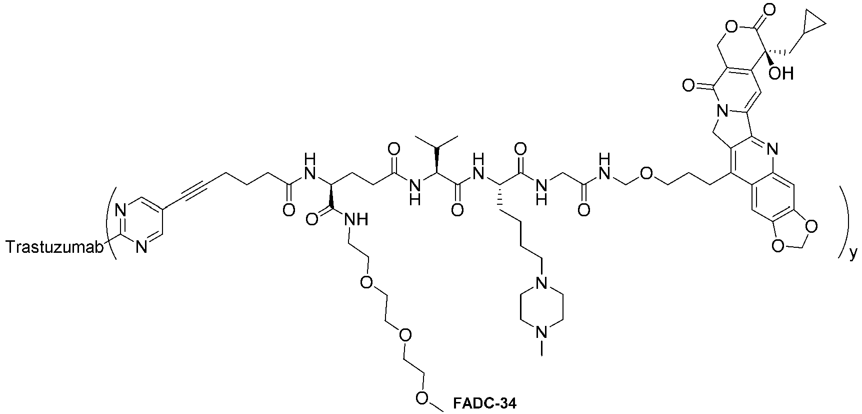

Using antibody Trastuzumab and **LD-34** as starting materials, and following Step A, an example product **ADC-34** of the conjugate mixture **FADC-34** was obtained in PBS buffer and stored at 4 °C. This is designated as Example 4-34.

### Example 4-35 ADC-35

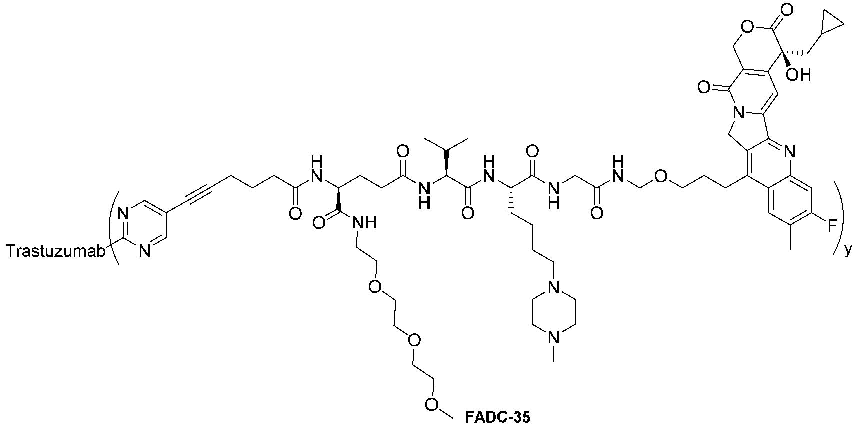

Using antibody Trastuzumab and **LD-35** as starting materials, and following Step A, an example product **ADC-35** of the conjugate mixture **FADC-35** was obtained in PBS buffer and stored at 4 °C. This is designated as Example 4-35.

### Example 4-36 ADC-36

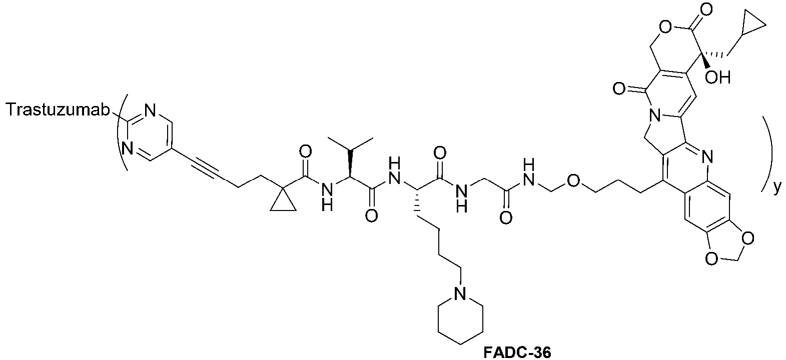

Using antibody Trastuzumab and **LD-36** as starting materials, and following Step A, an example product **ADC-36** of the conjugate mixture **FADC-36** was obtained in PBS buffer and stored at 4 °C. This is designated as Example 4-36.

### Example 4-37 ADC-37

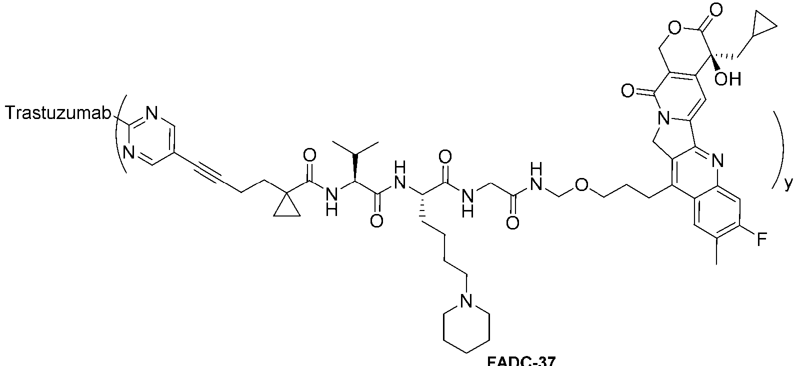

Using antibody Trastuzumab and **LD-37** as starting materials, and following Step A, an example product **ADC-37** of the conjugate mixture **FADC-37** was obtained in PBS buffer and stored at 4 °C. This is designated as Example 4-37.

### Example 4-38 ADC-38

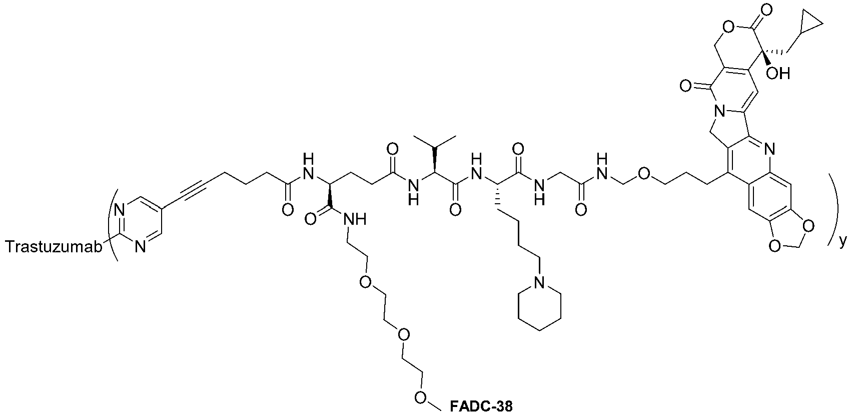

Using antibody Trastuzumab and **LD-38** as starting materials, and following Step A, an example product **ADC-38** of the conjugate mixture **FADC-38** was obtained in PBS buffer and stored at 4 °C. This is designated as Example 4-38.

### Example 4-39 ADC-39

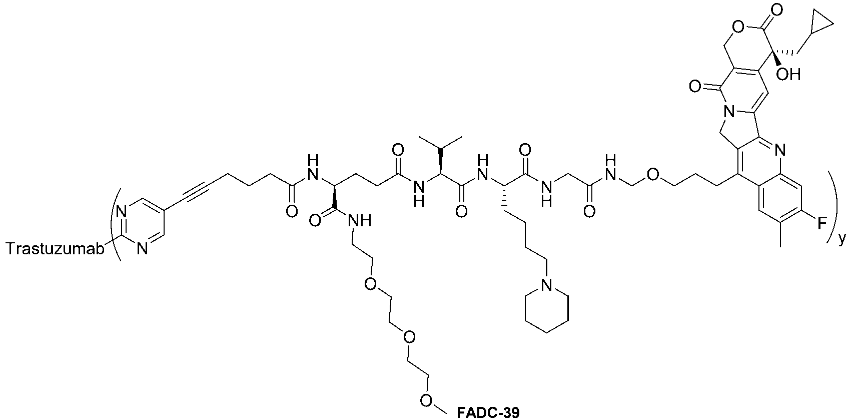

Using antibody Trastuzumab and **LD-39** as starting materials, and following Step A, an example product **ADC-39** of the conjugate mixture **FADC-39** was obtained in PBS buffer and stored at 4 °C. This is designated as Example 4-39.

### Example 4-40 ADC-40

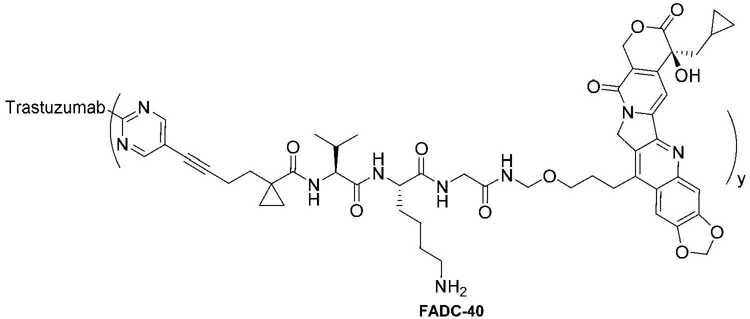

Using antibody Trastuzumab and **LD-40** as starting materials, and following Step A, an example product **ADC-40** of the conjugate mixture **FADC-40** was obtained in PBS buffer and stored at 4 °C. This is designated as Example 4-40.

### Example 4-41 ADC-41

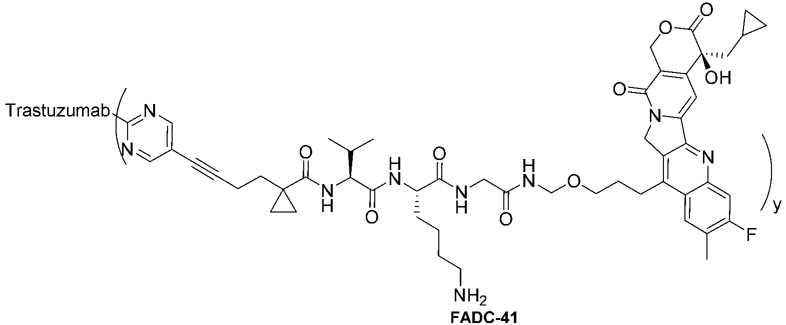

Using antibody Trastuzumab and **LD-41** as starting materials, and following Step A, an example product **ADC-41** of the conjugate mixture **FADC-41** was obtained in PBS buffer and stored at 4 °C. This is designated as Example 4-41.

### Example 4-42 ADC-42

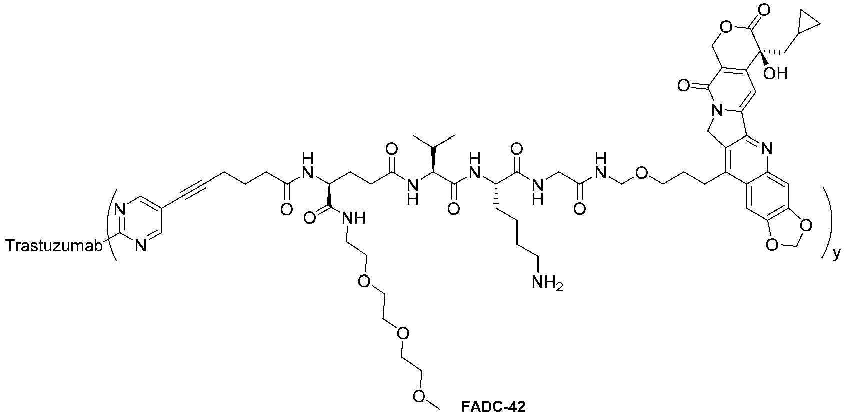

Using antibody Trastuzumab and **LD-42** as starting materials, and following Step A, an example product **ADC-42** of the conjugate mixture **FADC-42** was obtained in PBS buffer and stored at 4 °C. This is designated as Example 4-42.

### Example 4-43 ADC-43

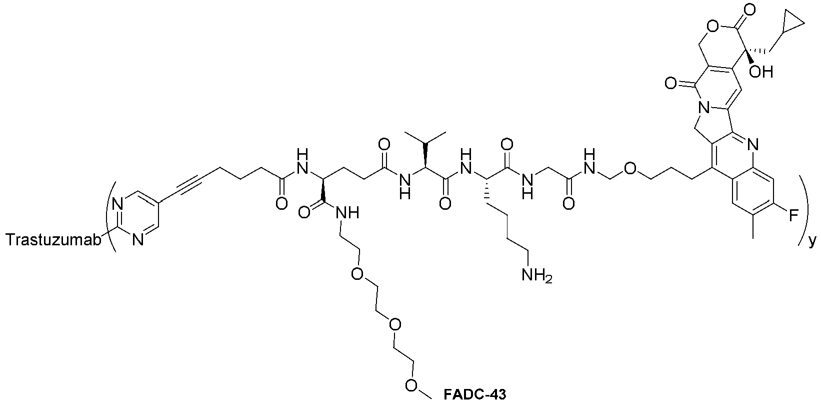

Using antibody Trastuzumab and **LD-43** as starting materials, and following Step A, an example product **ADC-43** of the conjugate mixture **FADC-43** was obtained in PBS buffer and stored at 4 °C. This is designated as Example 4-43.

### Example 4-44 ADC-44

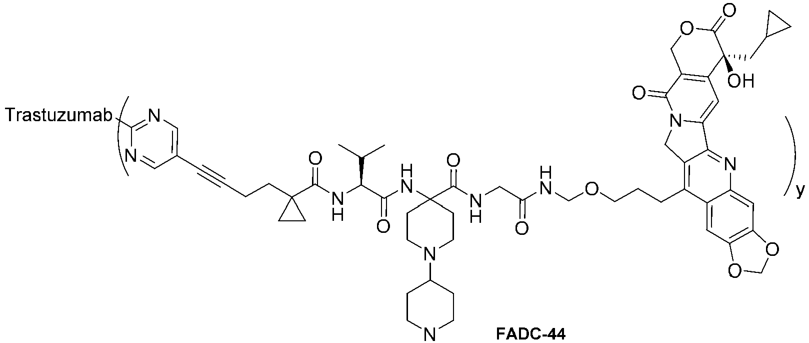

Using antibody Trastuzumab and **LD-44** as starting materials, and following Step A, an example product **ADC-44** of the conjugate mixture **FADC-44** was obtained in PBS buffer and stored at 4 °C. This is designated as Example 4-44.

### Example 4-45 ADC-45

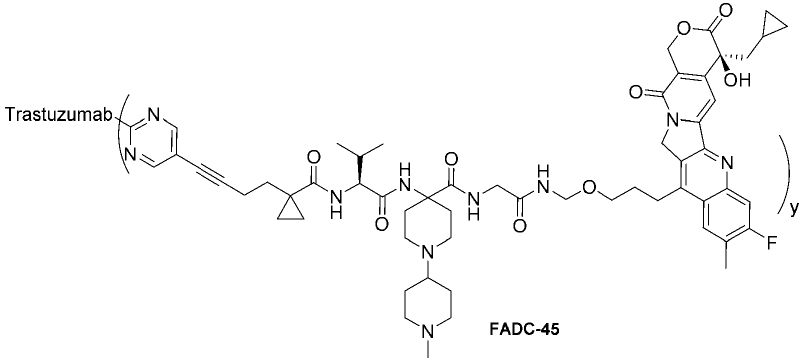

Using antibody Trastuzumab and **LD-45** as starting materials, and following Step A, an example product **ADC-45** of the conjugate mixture **FADC-45** was obtained in PBS buffer and stored at 4 °C. This is designated as Example 4-45.

### Example 4-46 ADC-46

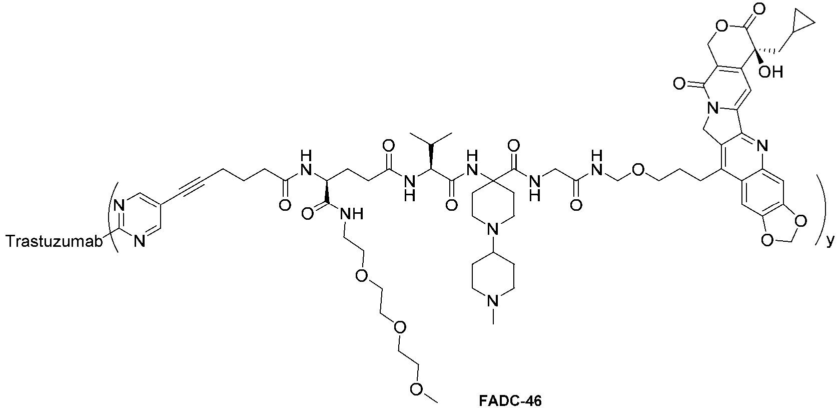

Using antibody Trastuzumab and **LD-46** as starting materials, and following Step A, an example product **ADC-46** of the conjugate mixture **FADC-46** was obtained in PBS buffer and stored at 4 °C. This is designated as Example 4-46.

### Example 4-47 ADC-47

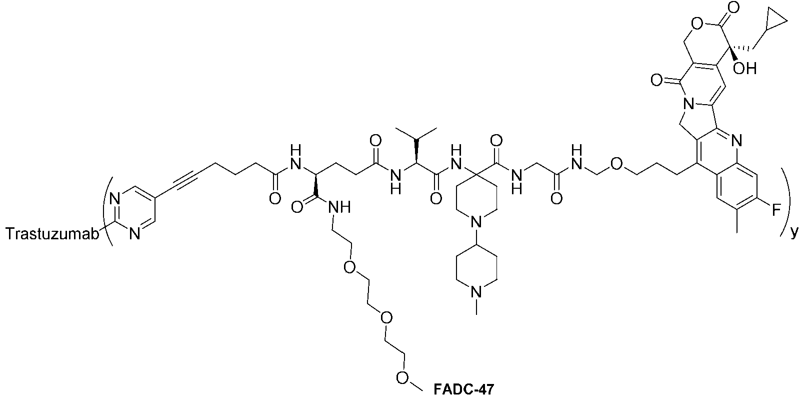

Using antibody Trastuzumab and **LD-47** as starting materials, and following Step A, an example product **ADC-47** of the conjugate mixture **FADC-47** was obtained in PBS buffer and stored at 4 °C. This is designated as Example 4-47.

### Example 4-48 ADC-48

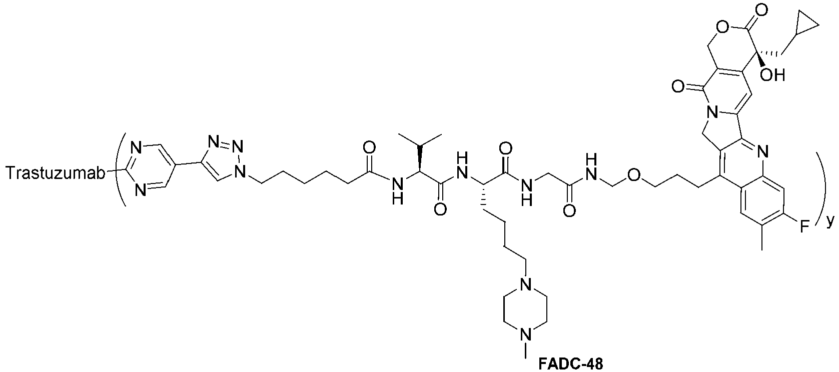

Using antibody Trastuzumab and **LD-48** as starting materials, and following Step A, an example product **ADC-48** of the conjugate mixture **FADC-48** was obtained in PBS buffer and stored at 4 °C. This is designated as Example 4-48.

### Example 4-49 ADC-49

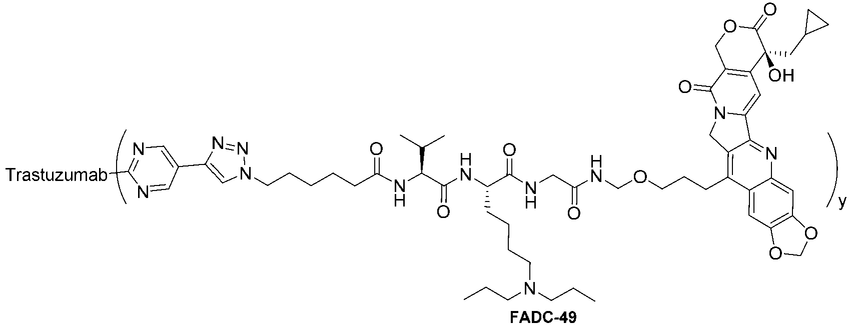

Using antibody Trastuzumab and **LD-49** as starting materials, and following Step A, an example product **ADC-49** of the conjugate mixture **FADC-49** was obtained in PBS buffer and stored at 4 °C. This is designated as Example 4-49.

### Example 4-50 ADC-50

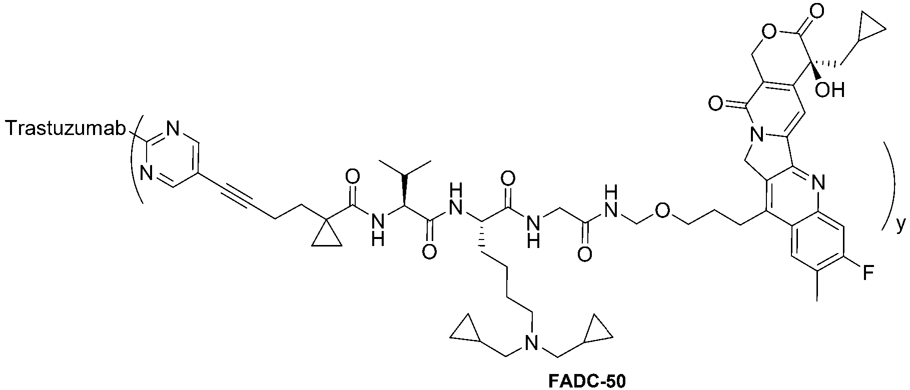

Using antibody Trastuzumab and **LD-50** as starting materials, and following Step A, an example product **ADC-50** of the conjugate mixture **FADC-50** was obtained in PBS buffer and stored at 4 °C. This is designated as Example 4-50.

### Example 4-51 ADC-51

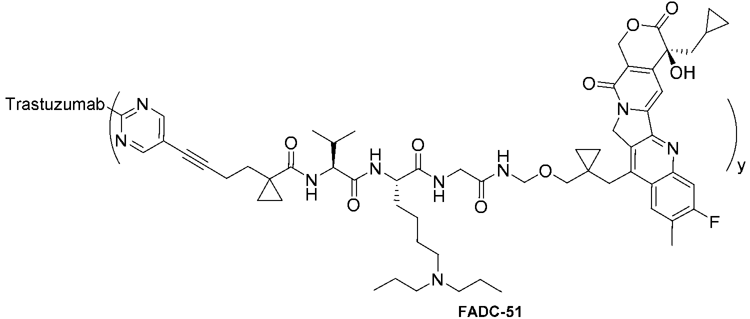

Using antibody Trastuzumab and **LD-51** as starting materials, and following Step A, an example product **ADC-51** of the conjugate mixture **FADC-51** was obtained in PBS buffer and stored at 4 °C. This is designated as Example 4-51.

### Example 4-52 ADC-52

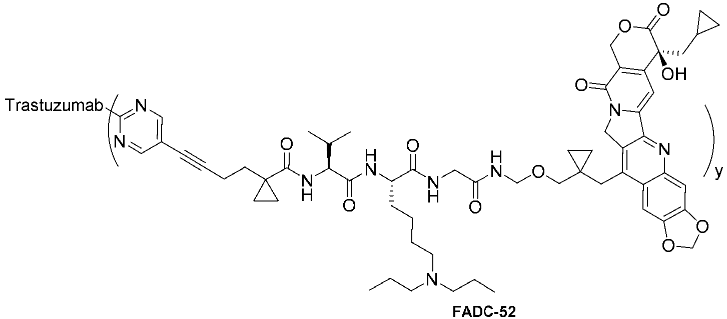

Using antibody Trastuzumab and **LD-52** as starting materials, and following Step A, an example product **ADC-52** of the conjugate mixture **FADC-52** was obtained in PBS buffer and stored at 4 °C. This is designated as Example 4-52.

### Example 4-53 ADC-53

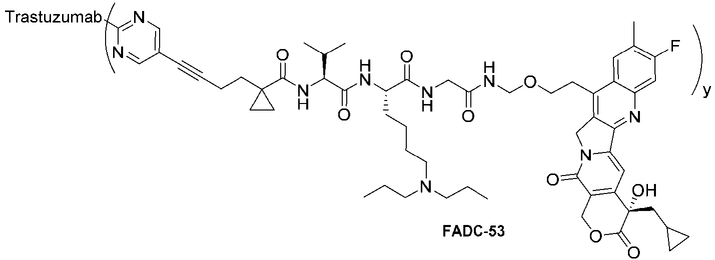

Using antibody Trastuzumab and **LD-53** as starting materials, and following Step A, an example product **ADC-53** of the conjugate mixture **FADC-53** was obtained in PBS buffer and stored at 4 °C. This is designated as Example 4-53.

### Example 4-54 ADC-54

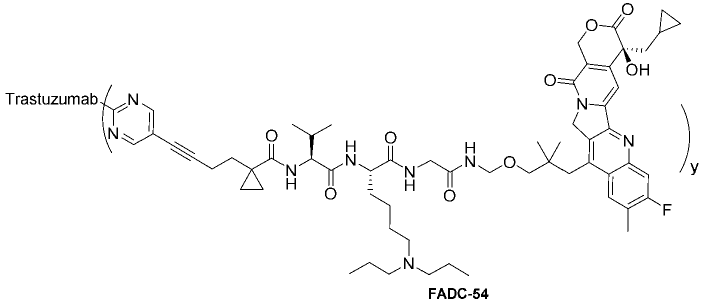

Using antibody Trastuzumab and **LD-54** as starting materials, and following Step A, an example product **ADC-54** of the conjugate mixture **FADC-54** was obtained in PBS buffer and stored at 4 °C. This is designated as Example 4-54.

### Example 4-55 ADC-55

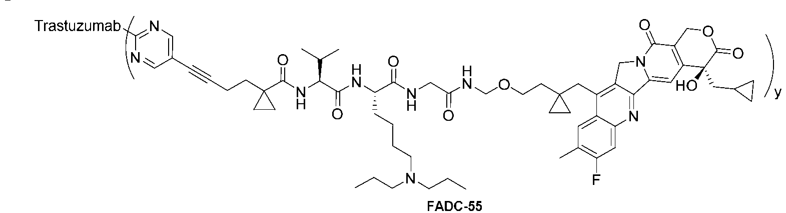

Using antibody Trastuzumab and **LD-55** as starting materials, and following Step A, an example product **ADC-55** of the conjugate mixture **FADC-55** was obtained in PBS buffer and stored at 4 °C. This is designated as Example 4-55.

### Example 4-56 ADC-56

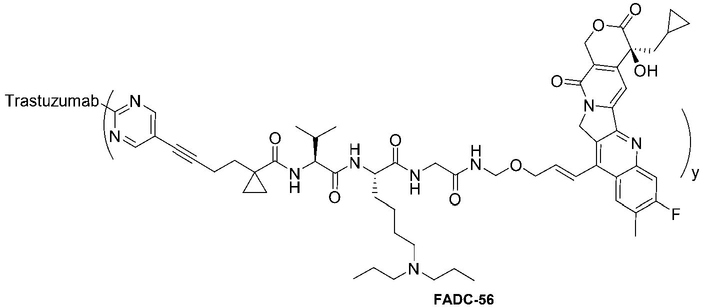

Using antibody Trastuzumab and **LD-56** as starting materials, and following Step A, an example product **ADC-56** of the conjugate mixture **FADC-56** was obtained in PBS buffer and stored at 4 °C. This is designated as Example 4-56.

### Example 4-57 ADC-57

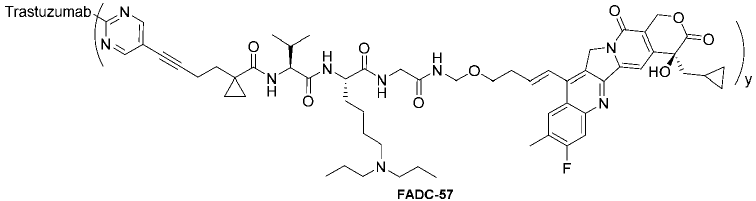

Using antibody Trastuzumab and **LD-57** as starting materials, and following Step A, an example product **ADC-57** of the conjugate mixture **FADC-57** was obtained in PBS buffer and stored at 4 °C. This is designated as Example 4-57.

### Example 4-58 ADC-58

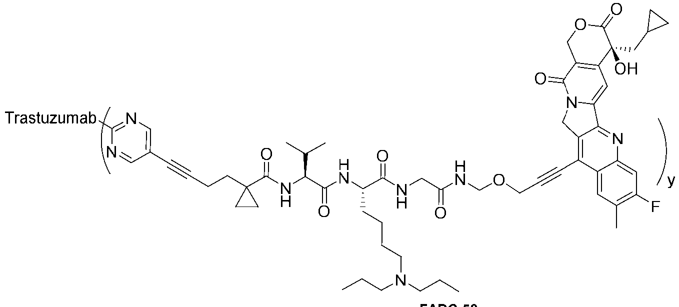

Using antibody Trastuzumab and **LD-58** as starting materials, and following Step A, an example product **ADC-58** of the conjugate mixture **FADC-58** was obtained in PBS buffer and stored at 4 °C. This is designated as Example 4-58.

### Example 4-59 ADC-59

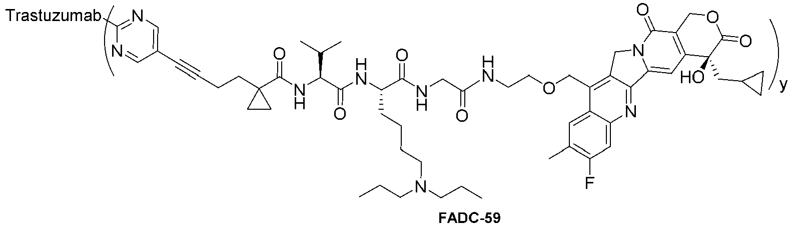

Using antibody Trastuzumab and **LD-59** as starting materials, and following Step A, an example product **ADC-59** of the conjugate mixture **FADC-59** was obtained in PBS buffer and stored at 4 °C. This is designated as Example 4-59.

### Example 4-60 ADC-60

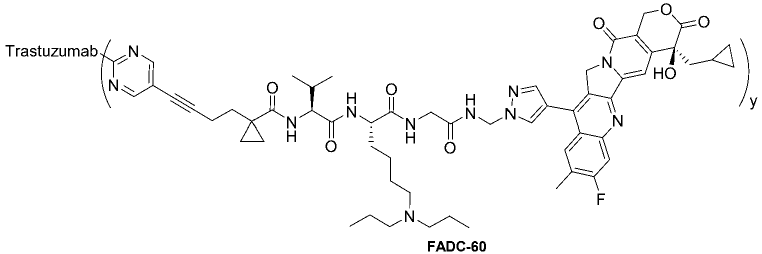

Using antibody Trastuzumab and **LD-60** as starting materials, and following Step A, an example product **ADC-60** of the conjugate mixture **FADC-60** was obtained in PBS buffer and stored at 4 °C. This is designated as Example 4-60.

### Example 4-61 ADC-61

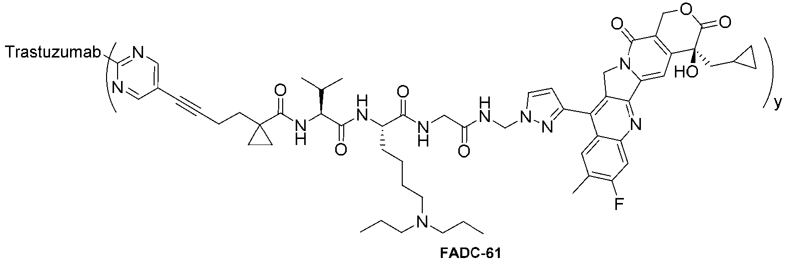

Using antibody Trastuzumab and **LD-61** as starting materials, and following Step A, an example product **ADC-61** of the conjugate mixture **FADC-61** was obtained in PBS buffer and stored at 4 °C. This is designated as Example 4-61.

### Example 4-62 ADC-62

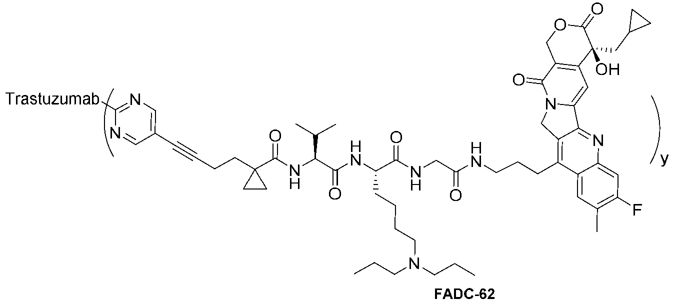

Using antibody Trastuzumab and **LD-62** as starting materials, and following Step A, an example product **ADC-62** of the conjugate mixture **FADC-62** was obtained in PBS buffer and stored at 4 °C. This is designated as Example 4-62.

### Example 4-63 ADC-63

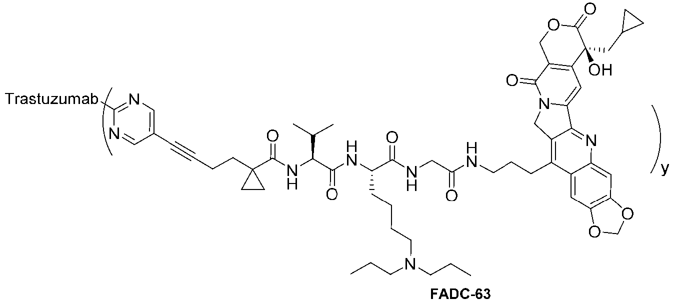

Using antibody Trastuzumab and **LD-63** as starting materials, and following Step A, an example product **ADC-63** of the conjugate mixture **FADC-63** was obtained in PBS buffer and stored at 4 °C. This is designated as Example 4-63.

### Example 4-64 ADC-64

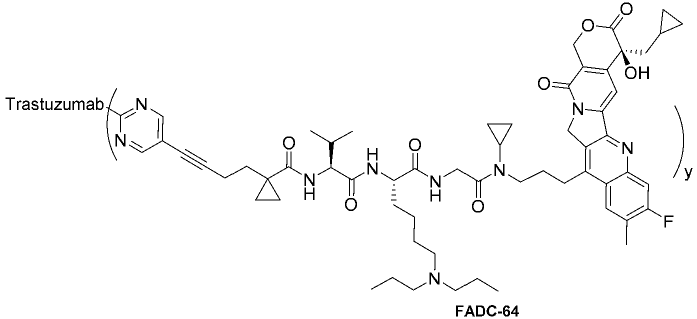

Using antibody Trastuzumab and **LD-64** as starting materials, and following Step A, an example product **ADC-64** of the conjugate mixture **FADC-64** was obtained in PBS buffer and stored at 4 °C. This is designated as Example 4-64.

### Example 4-65 ADC-65

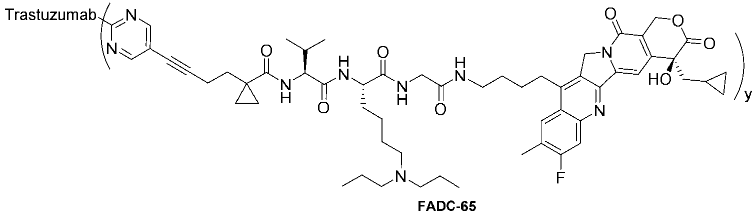

Using antibody Trastuzumab and **LD-65** as starting materials, and following Step A, an example product **ADC-65** of the conjugate mixture **FADC-65** was obtained in PBS buffer and stored at 4 °C. This is designated as Example 4-65.

### Example 4-66 ADC-66

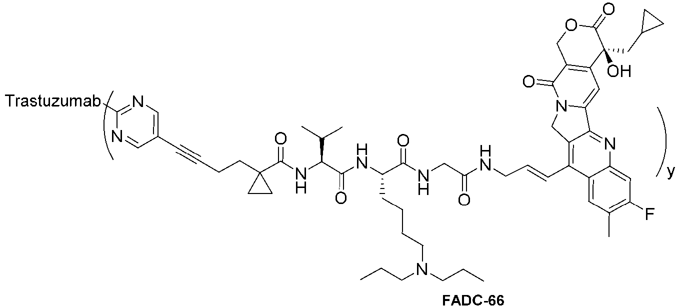

Using antibody Trastuzumab and **LD-66** as starting materials, and following Step A, an example product **ADC-66** of the conjugate mixture FADC-66 was obtained in PBS buffer and stored at 4 °C. This is designated as Example 4-66.

### Example 4-67 ADC-67

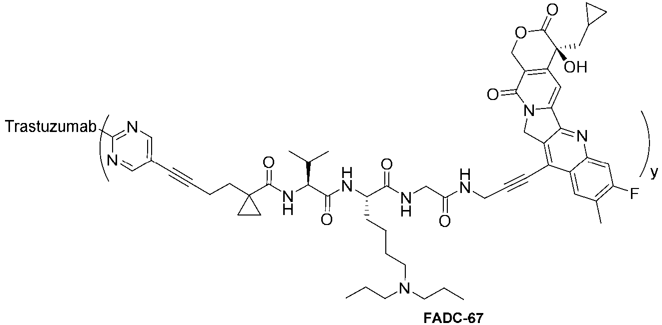

Using antibody Trastuzumab and **LD-67** as starting materials, and following Step A, an example product **ADC-67** of the conjugate mixture **FADC-67** was obtained in PBS buffer and stored at 4 °C. This is designated as Example 4-67.

### Example 4-68 ADC-68

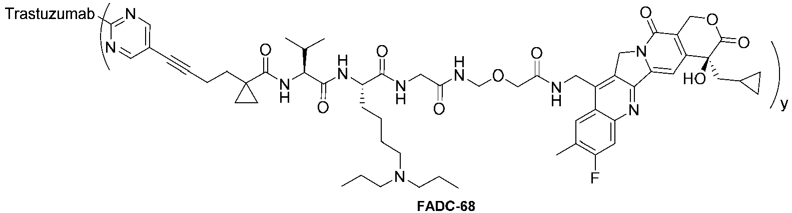

Using antibody Trastuzumab and **LD-68** as starting materials, and following Step A, an example product **ADC-68** of the conjugate mixture **FADC-68** was obtained in PBS buffer and stored at 4 °C. This is designated as Example 4-68.

### Example 4-69 ADC-69

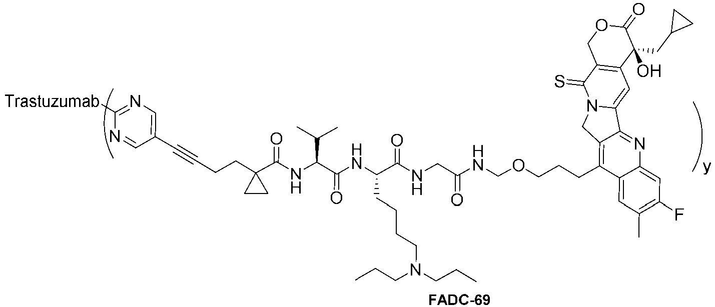

Using antibody Trastuzumab and **LD-69** as starting materials, and following Step A, an example product **ADC-69** of the conjugate mixture **FADC-69** was obtained in PBS buffer and stored at 4 °C. This is designated as Example 4-69.

### Example 4-70 ADC-70

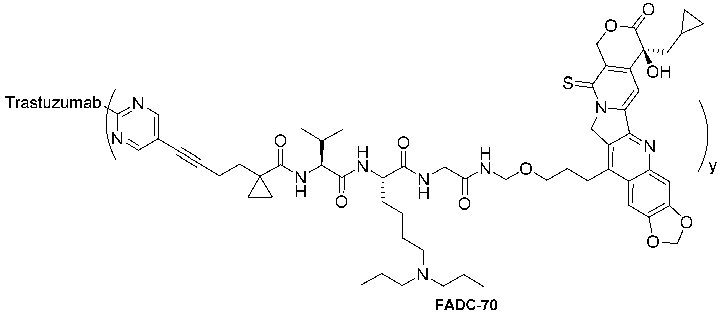

Using antibody Trastuzumab and **LD-70** as starting materials, and following Step A, an example product **ADC-70** of the conjugate mixture **FADC-70** was obtained in PBS buffer and stored at 4 °C. This is designated as Example 4-70.

### Example 4-71 ADC-71

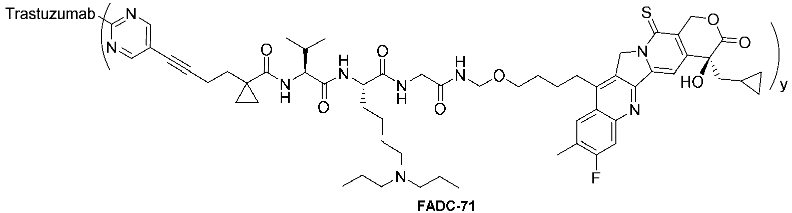

Using antibody Trastuzumab and **LD-71** as starting materials, and following Step A, an example product **ADC-71** of the conjugate mixture **FADC-71** was obtained in PBS buffer and stored at 4 °C. This is designated as Example 4-71.

### Example 4-72 ADC-72

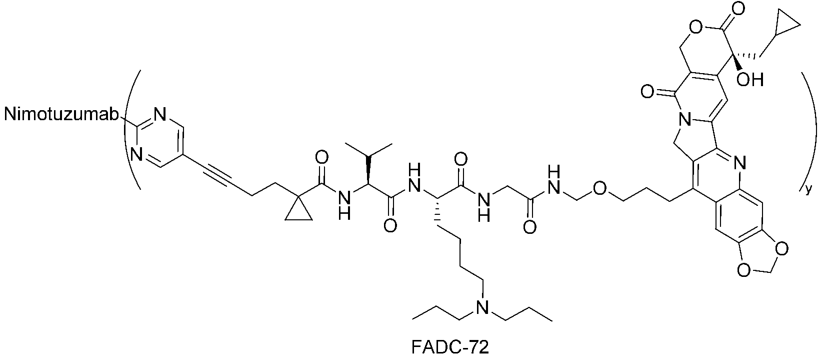

Using antibody nimotuzumab and **LD-1** as starting materials, and following Step A, an example product **ADC-72** of the conjugate mixture **FADC-72** was obtained in PBS buffer and stored at 4 °C. This is designated as Example 4-72.

The average drug loading was calculated by mass spectrometry: y = 8.00.

### Example 4-73 ADC-73

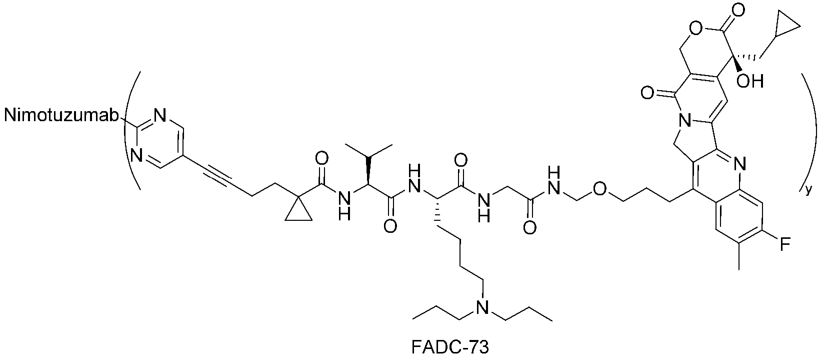

Using antibody nimotuzumab and **LD-10** as starting materials, and following Step A, an example product **ADC-73** of the conjugate mixture **FADC-73** was obtained in PBS buffer and stored at 4 ° C. This is designated as Example 4-73.

The average drug loading was calculated by mass spectrometry: y = 8.00.

### Example 4-74 ADC-74

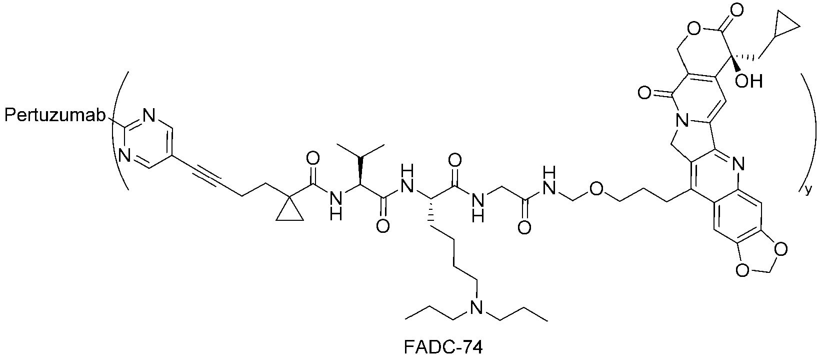

Using antibody Pertuzumab and **LD-1** as starting materials, and following Step A, an example product **ADC-74** of the conjugate mixture **FADC-74** was obtained in PBS buffer and stored at 4 °C. This is designated as Example 4-74.

The average drug loading was calculated by mass spectrometry: y = 8.00.

### Example 4-75 ADC-75

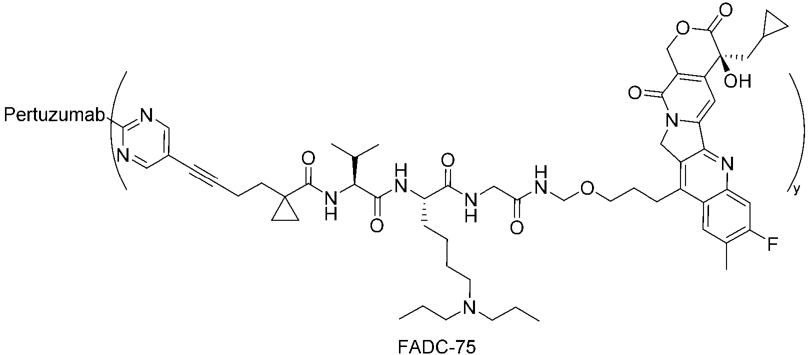

Using antibody Pertuzumab and **LD-10** as starting materials, and following Step A, an example product **ADC-75** of the conjugate mixture **FADC-75** was obtained in PBS buffer and stored at 4 °C. This is designated as Example 4-75.

The average drug loading was calculated by mass spectrometry: y = 8.00.

### Example 4-76 Reference 2

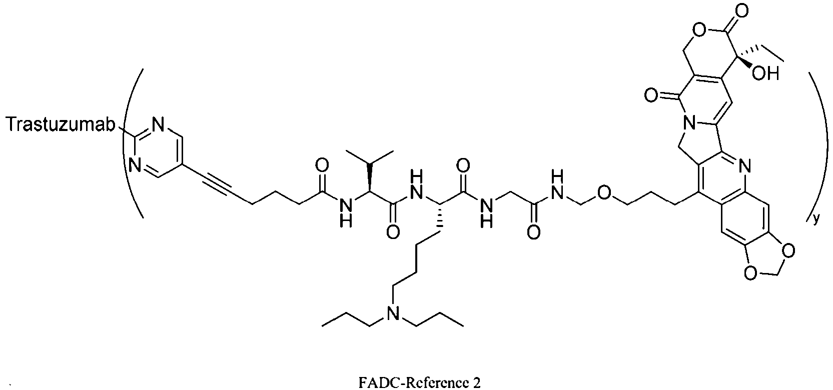

Using antibody Trastuzumab and N-((11S,14S)-11-(4-di-n-propylamino) butyl)-1-((S)-7-ethyl-7-hydroxy-8,11-dioxo-7,8,11,13-tetrahydro-10H-[1,3] dioxolo [4,5-g] pyrano [3', 4':6,7] indolazino [1,2-b] quinolin-14-yl)-15-methyl-7,10,13-trioxo-4-oxa-6,9,12-triazahexadecan-14-yl)-6-(2-(methylsulfonyl) pyrimidin-5-yl) hexan-5-ynamide (prepared according to the method disclosed in Example 2.37 on page 273 of the Patent Application "WO2022170971 Al") as starting materials, and following Step A, an example product **Reference 2** of the conjugate mixture **FADC-Reference** 2 was obtained in PBS buffer and stored at 4 ° C. This is designated as Example 4-76.

The average drug loading was calculated by mass spectrometry: y = 8.00.

### Example 4-77 Reference 3

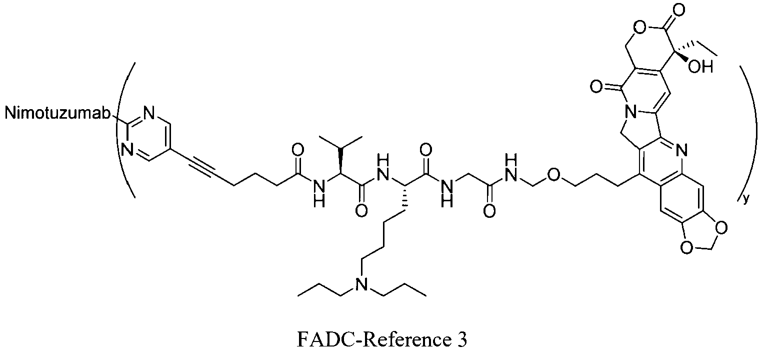

Using antibody nimotuzumab and N-((11S,14S)-11-(4-di-n-propylamino) butyl)-1-((S)-7-ethyl-7-hydroxy-8,11-dioxo-7,8,11,13-tetrahydro-10H-[1,3] dioxolo [4,5-g] pyrano [3',4': 6,7] indolazino [1,2-b] quinolin-14-yl)-15-methyl-7,10,13-trioxo-4-oxa-6,9,12-triazahexadecan-14-yl)-6-(2-(methylsulfonyl) pyrimidin-5-yl) hexan-5-ynamide (prepared according to the method disclosed in Example 2.37 on page 273 of the Patent Application "WO2022170971 Al") as starting materials, and following Step A, an example product **Reference 3** of the conjugate mixture **FADC-Reference 3** was obtained in PBS buffer and stored at 4 °C. This is designated as Example 4-77.

The average drug loading was calculated by mass spectrometry: y = 8.00.

### Example 4-78 Reference 4

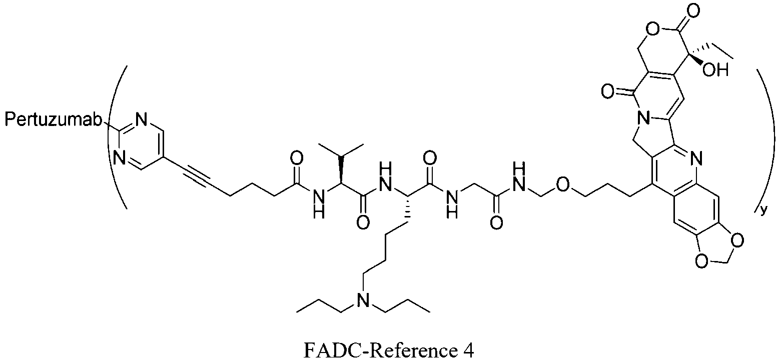

Using antibody Pertuzumab and N-((11S,14S)-11-(4-di-n-propylamino) butyl)-1-((S)-7-ethyl-7-hydroxy-8,11-dioxo-7,8,11,13-tetrahydro-10H-[1,3] dioxolo [4,5-g] pyrano [3',4': 6,7] indolazino [1,2-b] quinolin-14-yl)-15-methyl-7,10,13-trioxo-4-oxa-6,9,12-triazahexadecan-14-yl)-6-(2-(methylsulfonyl) pyrimidin-5-yl) hexan-5-ynamide (prepared according to the method disclosed in Example 2.37 on page 273 of the Patent Application "WO2022170971 Al") as starting materials, and following Step A, an example product Reference 4 of the conjugate mixture **FADC-Reference 4** was obtained in PBS buffer and stored at 4 °C. This is designated as Example 4-78.

The average drug loading was calculated by mass spectrometry: y = 7.60.

### Drug Loading Analysis of ADC Stock Solution

### Experimental object and principle

ADC stock solution is an antibody conjugate drug, and its mechanism of treating diseases is to deliver toxin molecules into cells depending on the targeting property of antibodies, and then kill cells. Drug loading plays a decisive role in drug efficacy. Drug loading of ADC stock solutions was determined by mass spectrometry and reversed-phase high performance liquid chromatography.

Method 1: Reversed-Phase High-Performance Liquid Chromatography:
(1) Experimental method: The test sample was diluted to 0.5 mg/mL with ultrapure water, reduced with 25 mM dithiothreitol at 37 °C for 30 min, and injected directly, with an injection volume of 5 µL. The chromatographic column was a reverse-phase chromatographic column (Agilent PLRP-S 1000 Å (5 µm) 50 × 2.1 mm) with gradient elution using mobile phases A and B (A: 0.1% formic acid-0.025% trifluoroacetic acid-water, B: 0.1% formic acid-0.025% trifluoroacetic acid-acetonitrile). The flow rate was 0.25 mL/min, the detection wavelength was 280 nm, and the column temperature was 70 °C. (2) Data analysis: The positions of light and heavy chains were distinguished by comparing the spectra of the sample with that of the naked antibody, and then the DAR value was calculated by integrating the spectra of the detected sample. The calculation formula is as follows: LC: 0 (number of drugs linked), LC+1: 2 (number of drugs linked), HC: 0 (number of drugs linked), HC+1: 2 (number of drugs linked), HC+2: 4 (number of drugs linked), HC+3: 6 (number of drugs linked). Sum of LC peak areas = LC peak area + LC+1 peak area; Sum of HC peak areas = HC peak area + HC+1 peak area + HC+2 peak area + HC+3 peak area; LC DAR = Σ (number of linked drugs ×peak area percentage)/sum of LC peak areas; HC DAR = Σ (number of linked drugs ×peak area percentage)/sum of HC peak areas; DAR = LC DAR + HC DAR

Method 2: Mass spectrometry: (1) Experimental method: 10 µL of the test sample with a concentration of 1 mg/mL was transferred into a 1.5 mL centrifuge tube, 0.5 µL of Rapid PNGase F (Adamas) was added, vortexed well, and incubated at 37 °C for 60 min. After the N-cut sugar was completed, 6 µL of ultrapure water and 4 µL of 0.5 M dithiothreitol in water were added to the centrifuge tube, vortexed and incubated at 37 °C for 30 min. After completion of the reaction, the supernatant was centrifuged and transferred into an injection vial and injected 2 µL. The chromatographic column was a reverse-phase chromatographic column (Agilent PLRP-S 1000 Å (5 µm) 50 × 2.1 mm), gradient elution was performed with mobile phases A and B (A: 0.1% formic acid-water, B: 0.1% formic acid-acetonitrile), flow rate was 0.5 mL/min, column temperature was 60 °C, detection wavelength was 280 nm, ESI-Tof (LC-MS) was used to collect m/z, mass spectrometric acquisition mode was positive ion mode, m/z range was 200 ~ 3200, and the original mass spectrum was deconvoluted by software. (2) Data analysis: The DAR values were calculated using the peak height values in a similar manner as described above for reversed-phase high-performance liquid chromatography.

### III. Test Examples

### Biological evaluation

### Test Example 1: Determination of in vitro proliferation Inhibitory Activity of Compounds of Formula (GH) on A549, SK-BR-3, and NCI-N87 Tumor Cells

### 1. Test Object:

The object of this experiment is to test the in vitro killing activity of the compounds of the formula (GH) of the present disclosure on A549 tumor cells (human lung cancer cells, Beijing Tongren Hospital), SK-BR-3 tumor cells (human breast cancer cells, Wuhan Punosai, Cat. No. CL-0211) and NCI-N87 tumor cells (human gastric cancer cells, Cell Resource Center, Institute of Basic Medical Sciences, Chinese Academy of Medical Sciences, Cat. No. 3111C0001CCC000481). The cells were treated with different concentrations of the compounds in vitro and cultured for 6 days, then the in vitro activity of the test compounds was evaluated based on IC₅₀ values by measuring the number of viable cells using CCK8 (Cell Counting Kit-8, Cat. No. TS547) reagent.

### 2. Experimental method:

Taking the test method of in vitro killing activity against A549 cells, SK-BR-3 cells and NCI-N87 cells as an example, the test was carried out as follows. It should be understood that this method is also applicable to, but not limited to, testing of in vitro proliferation inhibitory activity on other tumor cells (HCC1569, JIMT-1, DIFI, etc.).
(1) Cell culture: SK-BR-3 cells and NCI-N87 cells were cultured in RPMI 1640 medium containing 10% FBS (Shanghai Yuanpei Biotechnology Co., Ltd., Cat. No.: G211018); A549 cells were cultured in DMEM/F12 medium containing 10% FBS (Shanghai Yuanpei Biotechnology Co., Ltd., Cat. No. L310KJ).
(2) Cell preparation: A549 cells, SK-BR-3 cells and NCI-N87 cells in logarithmic growth phase were washed once with PBS (phosphate buffer, Shanghai Yuanpei Biotechnology Co., Ltd., Cat. No. E211004), then 2-3 mL trypsin (0.25% Trypsin-EDTA (1x), Shanghai Yuanpei Biotechnology Co., Ltd., Cat. No. A121002) was added for digestion for 2-3 min. After the cells were completely digested, 10 mL cell culture medium containing 10% FBS was added, the digested cells were eluted, centrifuged at 300 g for 5 min, and the supernatant was discarded. Then 10-20 mL cell culture medium containing 2% FBS was added to re-suspend the cells to prepare a single cell suspension.
(3) Cell plating: The single-cell suspension was mixed well, and the cell density of viable cells was adjusted with a cell culture medium containing 2% FBS (the plating density of SK-BR-3 was 3 × 10⁴ cells/mL, the plating density of A549 was 2 × 10⁴ cells/mL, and the plating density of NCI-N87 was 4 × 10⁴ cells/mL). The cell suspension after density adjustment was mixed well and added into a 96-well cell culture plate at 100 µL/well. Only 200 µL PBS was added to the peripheral wells of the 96-well plate. The plate was incubated in an incubator (37 °C, 5% CO₂) for 24 hours.
(4) Compound Preparation: The compound was dissolved in DMSO (dimethyl sulfoxide, SIGMA) to make a 2 mM stock solution.
   2 mM stock solution of a small molecule compound sample was diluted with a cell culture medium containing 2% FBS to 600 nM, and filtered with a 0.22 µM filter head for sterilization, then 300 µL of different samples to be tested was added in the first column of 96-well U-bottom dispensing plate, with sample concentration of 600 nM; 210 µL of cell culture medium with 2% FBS was added to each well in columns 2 through 10. 70 µL of sample in the first column was transferred to 210 µL of cell culture medium in the second column, mixed well, transferred 70 µL to the third column, and so on to column 9.
(5) Sample addition operation: 100 µL of the prepared samples to be tested at different concentrations were added to the culture plate, with duplicate wells for each sample. The plate was incubated in an incubator (37 °C, 5% CO₂) for 6 days.
(6) Chromogenic operation: The 96-well cell culture plate was taken out, the cell culture supernatant was patted off, 100 µL of basal medium containing 10% CCK8 (without FBS) was added, and then the plate was placed in an incubator for incubation for about 2 hours (37 °C, 5% CO₂).
(7) Plate reading operation: the 96-well cell culture plate was taken out, placed in a microplate reader (MD SpectraMax i3X), and A₄₅₀ was determined using the microplate reader.
(8) Data analysis: Microsoft Excel, Graphpad Prism 5 was used to process and analyze the data.

The results of the above test are summarized in Table 1:

**Table 1: IC₅₀ values of the tested compounds for inhibition proliferation of A549, SK-BR-3, NCI-N87 cells in vitro.**

| Compound No. | IC₅₀ (nM) | | |
|---|---|---|---|
| | A549 | SK-BR-3 | NCI-N87 |
| Compound 1 of Example 2-1 | 1.53 | 0.25 | 0.45 |
| Compound 2 of Example 2-2 | 1.30 | 0.29 | 0.68 |
| Compound 3 of Examples 2-3 | 1.68 | 0.24 | 0.44 |
| Reference 1 of Examples 2-26 | 13.22 | 1.29 | 3.21 |

Conclusion: The small molecular compounds in the present disclosure had significant proliferation inhibitory activity on A549, SK-BR-3, and NCI-N87 cells. Compound 1, Compound 2, and Compound 3 showed significantly higher inhibitory effect on the proliferation of A549, SK-BR-3 and NCI-N87 cells in vitro than Reference 1 at the same dose.

### Test Example 2: In Vitro Proliferation Inhibition Test of the Antibody-Drug Conjugates of the present disclosure on HER2-Targeted Tumor Cells

### 1. Test Object:

The object of this experiment is to test the inhibitory activity of the HER2-targeted antibody-drug conjugates of the present disclosure on the in vitro proliferation of SK-BR-3 cells (human breast cancer cells, Wuhan Punosai, Cat. No. CL-0211) and NCI-N87 cells (human gastric cancer cells, Cell Resource Center, Institute of Basic Medical Sciences, Chinese Academy of Medical Sciences, Cat. No. 3111C0001CCC000481). The cells were treated in vitro with various concentrations of the conjugate and cultured for 6 days, then the proliferation of the cells was measured using CCK8 (Cell Counting Kit-8, Cat. No. TS547) reagent to evaluate the in vitro activity based on IC₅₀ values.

### 2. Experimental method:

Taking the in vitro proliferation activity test method for SK-BR-3 cells and NCI-N87 cells as an example, the test was carried out as follows. It should be understood that this method is also applicable to, but not limited to, testing of in vitro proliferation inhibitory activity on other tumor cells (HCC1569, JIMT-1, DIFI, etc.).
(1) Cell culture: SK-BR-3 cells and NCI-N87 cells were cultured in RPMI 1640 medium containing 10% FBS (Shanghai Yuanpei Biotechnology Co., Ltd., Cat. No.: L210KJ), respectively.
(2) Cell preparation: Cells in logarithmic growth phase were washed once with PBS (phosphate buffer, Shanghai Youpei Biotechnology Co., Ltd., Cat. No.: E211004), then 1 mL trypsin (0.25% Trypsin-EDTA (1x), Shanghai Youpei Biotechnology Co., Ltd., Cat. No.: A121002) was added for digestion for 2-3 min. After the cells were completely digested, 10 mL cell culture medium was added to elute the digested cells, centrifuged at 300g for 5 min, and the supernatant was discarded. Then 10 mL cell culture medium was added to re-suspend the cells to prepare a single cell suspension.
(3) Cell plating: The single-cell suspension was mixed well, and the density of viable cells was adjusted to 3 × 10⁴ cells/mL with a cell culture medium. The cell suspension after density adjustment was mixed well and added to a 96-well cell culture plate at 100 µL/well. Only 200 µL PBS was added to the peripheral wells of the 96-well plate, and the plate was incubated in an incubator (37 °C, 5% CO₂) for 24 hours.
(4) Preparation of sample to be tested: The sample to be tested was diluted to 400 nM with a cell culture medium containing 2% FBS, and 300 µL of sample was added to the first well of a 96-well U-bottom dispensing plate at a sample concentration of 400 nM; 210 µL of a cell culture medium with 2% FBS was added to each well in columns 2 through 10. 70 µL of sample in the first column was transferred to 210 µL of cell culture medium in the second column, mixed well, transferred 70 µ L to the third column, and so on to column 9.
(5) Sample addition operation: 100 µL of the prepared samples to be tested at different concentrations were added to the culture plate, with two replicate wells for each sample. The plate was incubated in an incubator (37 °C, 5% CO₂) for 6 days.
(6) Chromogenic operation: the 96-well cell culture plate was taken out, the culture medium supernatant was patted off, 100 µL of 10% CCK8 basal medium (without FBS) was added to each well, and incubated at room temperature for 2 h.
(7) Plate reading operation: the 96-well cell culture plate was taken out, placed in a microplate reader (SpectraMax i3X), and A₄₅₀ was determined using the microplate reader.
(8) Data analysis: Microsoft Excel, Graphpad Prism 5 was used to process and analyze the data.

The results of the above test are summarized in Table 2:

**Table 2: IC₅₀ values of the antibody-drug conjugates of the present disclosure for in vitro proliferation inhibition of HER2-targeted tumor cells.**

| Compound No. | IC₅₀ (nM) | |
|---|---|---|
| | SK-BR-3 | NCI-N87 |
| Example 4-1 ADC-1 | 0.04 | 0.08 |

Conclusion: The HER2-targeting antibody-drug conjugates of the present disclosure had significant proliferation inhibitory activity against HER2 positive cells SK-BR-3 and NCI-N87.

### Test Example 3 in Vitro Proliferation Inhibition Test of the Antibody-Drug Conjugates of the present disclosure on EGFR-Targeted Tumor Cells

### 1. Test object:

The object of this experiment is to test the inhibitory activity of the antibody-drug conjugates of the present disclosure against EGFR targets on the in vitro proliferation of MDA-MB-468 cells (human breast cancer cells, Cell Center, Shanghai Institute of Biological Sciences, Chinese Academy of Sciences, Cat. No. 3131C0001000700120), SK-BR-3 cells (human breast cancer cells, Wuhan Punosai, Cat. No. CL-0211) and SW620 cells (human colon cancer cells, Biofeng, bc067). The cells were treated in vitro with various concentrations of the conjugate and cultured for 6 days, then the proliferation of the cells was measured using CCK8 (Cell Counting Kit-8, Cat. No. TS547) reagent to evaluate the in vitro activity based on IC₅₀ values.

### 2. Experimental method:

Taking the in vitro proliferation activity test method for MDA-MB-468 cells, SK-BR-3 cells and SW620 cells as an example, the test was carried out as follows. It should be understood that this method is also applicable to, but not limited to, testing of in vitro proliferation inhibitory activity on other tumor cells (HCC1569, JIMT-1, DIFI, etc.).
(1) Cell culture: MDA-MB-468 cells and SW620 cells were cultured in L-15 medium containing 10% FBS (Gibco, Cat. No. 11415-064), respectively; SK-BR-3 cells were cultured in RPMI 1640 medium containing 10% FBS (Shanghai Yuanpei Biotechnology Co., Ltd., article No.: L210KJ).
(2) Cell preparation: Cells in logarithmic growth phase were washed once with PBS (phosphate buffer, Shanghai Youpei Biotechnology Co., Ltd., Cat. No.: E211004), then 1 mL trypsin (0.25% Trypsin-EDTA (1x), Shanghai Youpei Biotechnology Co., Ltd., Cat. No.: A121002) was added for digestion for 2-3 min. After the cells were completely digested, 10 mL corresponding cell culture medium containing 10% FBS was added, the digested cells were eluted, centrifuged at 300g for 5 min, and the supernatant was discarded. Then 10 mL cell culture medium was added to re-suspend the cells to prepare a single cell suspension.
(3) Cell plating: The single-cell suspension was mixed well, and the density of viable cells was adjusted to 3 × 10⁴ cells/mL with corresponding cell culture medium containing 2% FBS. The cell suspension after density adjustment was mixed well and added to a 96-well cell culture plate at 100 µL/well. Only 200 µL PBS was added to the peripheral wells of the 96-well plate. Plates of MDA-MB-468 and SW620 cells were incubated in a CO₂-free incubator for 24 hours (37 °C), and plates of SK-BR-3 cells were incubated in a 5% CO₂ incubator for 24 hours (37 °C).
(4) Preparation of sample to be tested:
   The samples to be tested were diluted to 400 nM with the cell culture medium containing 2% FBS corresponding to the added cells, respectively, and then 300 µL samples were added to the first well of a 96-well U-bottom dispensing plate at a sample concentration of 400 nM; 210 µL of the corresponding FBS-containing cell culture medium was added to each well in columns 2 through 10. 70 µL of sample in the first column was transferred to 210 µL of cell culture medium in the second column, mixed well, transferred 70 µL to the third column, and so on to column 9.
(5) Sample addition operation: 100 µL of the prepared samples to be tested at different concentrations were added to the culture plate, with two replicate wells for each sample. The cell culture plates of MDA-MB-468 cells and SW620 cells were incubated in a CO₂-free incubator for 6 days (37 °C), respectively, and the cell culture plate of SK-BR-3 cells was incubated in a 5% CO₂ incubator for 6 days (37 °C).
(6) Chromogenic operation: the 96-well cell culture plate was taken out, the culture medium supernatant was patted off, 100 µL of 10% CCK8 (prepared with RPMI 1640 basal medium, without FBS) solution was added to each well, and incubate at room temperature for 2h.
(7) Plate reading operation: the 96-well cell culture plate was taken out, placed in a microplate reader (SpectraMax i3X), and A₄₅₀ was determined using the microplate reader.
(8) Data analysis: Microsoft Excel, Graphpad Prism 5 was used to process and analyze the data.

The results of the above test are summarized in Table 3:

**Table 3: IC₅₀ values of the antibody-drug conjugates of the present disclosure for in vitro proliferation inhibition of EGFR-targeted tumor cells.**

| Compound No. | IC50 (nM) | | |
|---|---|---|---|
| | MDA-MB-468 | SK-BR-3 | SW620 |
| Example 4-72 ADC-72 | 0.7 | 10.23 | > 50 |
| Example 4-73 ADC-73 | 2.34 | > 50 | > 50 |

Conclusion: The EGFR-targeted antibody-drug conjugates in the present disclosure had significant proliferation inhibitory activity on EGFR positive cells MDA-MB-468; At the same time, they had weak inhibitory activity on proliferation of EGFR negative SW620 cells. It has good selectivity.

### Test Example 4: In Vitro Proliferation Inhibition Test 2of the Antibody-Drug Conjugates of the present disclosure on HER2-Targeted Tumor Cells

### 1. Test object:

The object of this experiment is to test the inhibitory activity of the HER2-targeted antibody drug conjugates of the present disclosure on the in vitro proliferation of SK-BR-3 cells (human breast cancer cells, Wuhan Punosai, Cat. No. CL-0211) and MDA-MB-468 cells (human breast cancer cells, Cell Center, Shanghai Institute for Biological Sciences, Chinese Academy of Sciences, Cat. No. 3131C0001000700120). The cells were treated in vitro with various concentrations of the conjugate and cultured for 6 days, then the proliferation of the cells was measured using CCK8 (Cell Counting Kit-8, Cat. No. TS547) reagent to evaluate the in vitro activity based on IC₅₀ values.

### 2. Experimental method:

Taking the in vitro proliferation activity test method for SK-BR-3 cells and MDA-MB-468 cells as an example, the test was carried out as follows. It should be understood that this method is also applicable to, but not limited to, testing of in vitro proliferation inhibitory activity on other tumor cells (HCC1569, JIMT-1, DIFI, etc.).
(1) Cell culture: SK-BR-3 cells and MDA-MB-468 cells were cultured in RPMI 1640 medium containing 2% FBS (Shanghai Yuanpei Biotechnology Co., Ltd., article No.: L210KJ), respectively.
(2) Cell preparation: Cells in logarithmic growth phase were washed once with PBS (phosphate buffer, Shanghai Youpei Biotechnology Co., Ltd., Cat. No.: E211004), then 1 mL trypsin (0.25% Trypsin-EDTA (1x), Shanghai Youpei Biotechnology Co., Ltd., Cat. No.: A121002) was added for digestion for 2-3 min. After the cells were completely digested, 10 mL cell culture medium was added to elute the digested cells, centrifuged at 300g for 5 min, and the supernatant was discarded. Then 10 mL cell culture medium was added to re-suspend the cells to prepare a single cell suspension.
(3) Cell plating: The single-cell suspension was mixed well, and the density of viable cells was adjusted to 3 × 10⁴ cells/mL with cell culture medium. The cell suspension after density adjustment was mixed well and added to a 96-well cell culture plate at 100 µL/well. Only 200 µL PBS was added to the peripheral wells of the 96-well plate. The plates were incubated in an incubator (37 °C, 5% CO₂) for 24 hours.
(4) Preparation of sample to be tested:
   The samples to be tested were diluted to 400 nM with cell culture medium containing 2% FBS, and 300 µL of samples were added to the first well of a 96-well U-bottom dispensing plate at a sample concentration of 400 nM; 210 µL of cell culture medium containing 2% FBS was added to each well in columns 2 through 10. 70 µL of sample in the first column was transferred to 210 µL of cell culture medium in the second column, mixed well, transferred 70 µL to the third column, and so on to column 9.
(5) Sample addition operation: 100 µL of the prepared samples to be tested at different concentrations were added to the culture plate, with two replicate wells for each sample. The plates were incubated in an incubator (37 °C, 5% CO₂) for 6 days.
(6) Chromogenic operation: the 96-well cell culture plate was taken out, the culture medium supernatant was patted off, 100 µL of 10% CCK8 (prepared with RPMI 1640 basal medium, without FBS) solution was added to each well, and incubated at room temperature for 2 h.
(7) Plate reading operation: the 96-well cell culture plate was taken out, placed in a microplate reader (SpectraMax i3X), and A₄₅₀ was determined using the microplate reader.
(8) Data analysis: Microsoft Excel, Graphpad Prism 5 was used to process and analyze the data.

The results of the above test are summarized in Table 4:

**Table 4: IC₅₀ values of antibody-drug conjugates of the present disclosure for in vitro proliferation inhibition of HER2-targeted tumor cells.**

| Compound No. | IC50 (nM) | |
|---|---|---|
| | SK-BR-3 | MDA-MB-468 |
| Example 4-1 ADC-1 | 0.21 | > 50 |
| Example 4-74 ADC-74 | 0.17 | > 50 |
| Example 4-76 Reference 2 | 0.51 | > 50 |
| Example 4-78 Reference 4 | 0.60 | > 50 |

Conclusion: The HER2-targeted antibody-drug conjugates in the present disclosure had significant proliferation inhibitory activity on HER2 positive cell SK-BR-3, and ADC-1 and ADC-74 had better inhibitory effect on SK-BR-3 cell proliferation in vitro than Reference 2 and Reference 4 at the same dose. At the same time, they had weak inhibitory activity on the proliferation of HER2-negative MDA-MB-468 cells. It has good selectivity.

### Test Example 5: Test for Bystander Killing Effect of the Antibody-Drug Conjugates of the present disclosure on HER2-Targeted Tumor Cells

HER2-positive SK-BR-3 cells (human breast cancer cells, Wuhan Punosai, Cat. No. CL-0211) and HER2-negative MDA-MB-468 cells (human breast cancer cells, Cell Center, Shanghai Institute for Biological Sciences, Chinese Academy of Sciences, Cat. No. 3131C0001000700120) were adjusted to 1.2 × 10⁵ cells/ml and 4 × 10⁴ cells/ml using RPMI-1640 +10% FBS, respectively. 1 mL of SK-BR-3 cells and MDA-MB-468 cells were added to a 6-well plate, and mixed and cultured overnight at 37 °C in 5% CO₂. Sample ADC-113 was prepared to a concentration of 4.5 µg/mL (30 nM), and 1 mL was added to the cells to make a total volume of 3mL and a final concentration of 10 nM. A vehicle control group was set and incubated at 37 °C in 5% CO₂ for 5 days. Trypsin (0.25% Trypsin-EDTA (1x), Shanghai Yangpei Biotechnology Co., Ltd., Cat. No.: A121002) was added to a 6-well plate to digest cells, 1mL FACS buffer (PBS + 4% FBS) was added to re-suspend the cells, and trypan blue staining was performed on the cells and counted. The remaining cells were centrifuged, Trastuzumab was diluted to 10 µg/mL with FACS buffer, re-suspended with 200 µL of the antibody solution, incubated on ice for 30 min, centrifuged to discard the supernatant, washed once with 1 mL FACS buffer, 200 µL of which was added to AF647 labeled goat anti-human Fc antibody (Jackson, Cat. No. 109-606-170) solution, incubated on ice for 15 min, PI (Sigma, Cat. No. 31845) solution at the concentration of 5 µg/mL was added, incubated on ice for 5 min, centrifuged to discard the supernatant, washed once with 1 mL FACS buffer, and re-suspended with 400 µL PBS, analyzed by BD Celesta flow cytometer. The ratio of the two types of cells and the number of cells were determined according to the results of flow cytometry and counting.

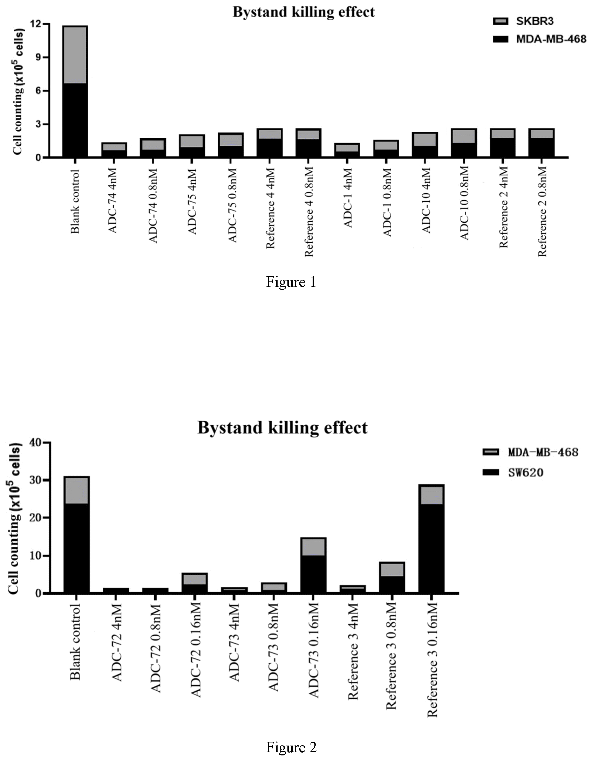

The results of the above tests, summarized in FIG. 1, showed that the antibody-drug conjugates of the present disclosure had excellent bystander killing effects. The bystander killing effect of ADC-1, ADC-10, ADC-74 and ADC-75 on HER2-targeted tumor cells is superior to that of Reference 2 and Reference 4 at the same dose.

### Test Example 6: Test for Bystander Killing Effect of the Antibody-Drug Conjugates of the Present Disclosure on EGFR-Targeted Tumor Cells

EGFR-positive MDA-MB-468 cells (human breast cancer cells, Cell Center, Shanghai Institute for Biological Sciences, Chinese Academy of Sciences, Cat. No.: 3131C0001000700120) and EGFR-negative SW620 cells (human colon cancer cells, Biofeng, bc067) were adjusted to a cell density of 1.2 × 10⁵ cells/ml and 4 × 10⁴ cells/ml, respectively, using RPMI-1640 +10% FBS. 1 mL of MDA-MB-468 and SW620 cells were added into 6-well plates, and mixed and cultured overnight at 37 °C in 5% CO₂. ADC samples were prepared to a concentration of 4.5 µg/mL (30 nM) and 1 mL was added to the cells to make a total volume of 3mL and a final concentration of 10 nM. A vehicle control group was set and incubated at 37 °C in 5% CO₂ for 5 days. Trypsin (0.25% Trypsin-EDTA (1x), Shanghai Yangpei Biotechnology Co., Ltd., Cat. No.: A121002) was added to a 6-well plate to digest cells, 1mL FACS buffer (PBS + 4% FBS) was added to re-suspend the cells, and trypan blue staining was performed on the cells and counted. The remaining cells were centrifuged, Nimotuzumab was diluted to 10 µg/mL with FACS buffer, the cells were re-suspended with 200 µL of the antibody solution, incubated on ice for 30 min, centrifuged to discard the supernatant, washed once with 1 mL FACS buffer, 200 µL of which was added to AF647-labeled goat anti-human Fc antibody (Jackson, Cat. No. 109-606-170) solution, incubated on ice for 15 min, PI (Sigma, Cat. No. 31845) solution at a concentration of 5 µg/mL was added, incubated on ice for 5 min, centrifuged to discard the supernatant, washed once with 1 mL FACS buffer, and re-suspended with 400 µL PBS, analyzed by BD Celesta flow cytometer. The ratio and number of the two types of cells were determined according to the results of flow cytometry and counting.

The results of the above tests, summarized in Figure 2, indicated that the nimotuzumab antibody-drug conjugates of the present disclosure had excellent bystander killing effects. The bystander killing effect of ADC-72 and ADC-73 on EGFR-targeted tumor cells is significantly better than that of reference 3 at the same dose.

Embodiments of the technical solutions of the present disclosure are exemplary described above. It should be understood that the scope of the disclosure is not limited to the aforementioned embodiments. Any modification, equivalent replacement, improvement, etc. made by those skilled in the art within the spirit and principles of the disclosure shall be covered by the scope of the claims of the present application.

## Claims

1. A ligand-drug conjugate represented by the following formula (C), or a stereoisomer, racemate, tautomer, isotopologue, isotope-labeled compound, N-oxide, prodrug, solvate, or pharmaceutically acceptable salt thereof:
Tp-L-G (C)
wherein, Tp represents a targeting moiety;
L is selected from a chemical bond or a linker;
G is a group represented by the following formula (G): 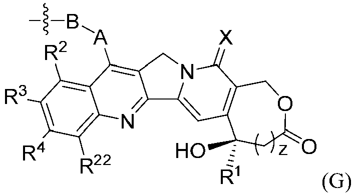
wherein, A is absent or selected from the group consisting of alkylene, alkenylene, alkynylene, cycloalkyl, aryl, heteroaryl, heterocyclyl, and a combination of two or more of them, which are unsubstituted or optionally substituted with one, two or more R^{A}; wherein the alkylene, alkenylene, alkynylene, cycloalkyl, aryl, heteroaryl, heterocyclyl or a combination of two or more of them may be uninterrupted or optionally interrupted by one, two or more groups selected from-O-, -S-, -NR⁵⁻, -NR⁶C(=O)-, - C(=O)NR⁶-, -C(=O)-, -NR⁷C(=O)NR⁸- or -OC(=O)-;
R¹ is selected from the following groups unsubstituted or optionally substituted with one, two or more R^{B}: cycloalkyl, cycloalkylalkyl, cycloalkyloxy, heterocyclyl, heterocyclylalkyl, or heterocyclyloxy;
X is selected from O or S;
Z is selected from 0 or 1;
R² is selected from the group consisting of hydrogen, deuterium, halogen, hydroxy, mercapto, amino, cyano, and the following groups which are unsubstituted or optionally substituted with one, two or more R^{C}: alkyl, alkyloxy, cycloalkyl, cycloalkylalkyl, or cycloalkyloxy;
R²² is selected from the group consisting of hydrogen, deuterium, halogen, hydroxy, mercapto, amino, cyano, and the following groups which are unsubstituted or optionally substituted with one, two or more R^{C}: alkyl, alkyloxy, cycloalkyl, cycloalkylalkyl, or cycloalkyloxy;
R³ is selected from the group consisting of hydrogen, deuterium, halogen, hydroxy, mercapto, amino, cyano, and the following groups which are unsubstituted or optionally substituted with one, two or more R^{C}: alkyl, alkyloxy, cycloalkyl, cycloalkylalkyl, or cycloalkyloxy;
R⁴ is selected from the group consisting of hydrogen, deuterium, halogen, hydroxy, mercapto, amino, cyano, and the following groups which are unsubstituted or optionally substituted with one, two or more R^{C}: alkyl, alkyloxy, cycloalkyl, cycloalkylalkyl, or cycloalkyloxy;
alternatively, R² and R³, together with the atoms to which they are attached form a ring structure which is unsubstituted or optionally substituted with one, two or more R^{D};
alternatively, R³ and R⁴, together with the atoms to which they are attached form a ring structure which is unsubstituted or optionally substituted with one, two or more R^{D};
alternatively, R²² and R⁴, together with the atoms to which they are attached form a ring structure which is unsubstituted or optionally substituted with one, two or more R^{D};
B is absent or selected from-O-, -S-, -S(=O)-, -C(=O)-, -NR⁵-, -C=N-NR⁹-, -C=N-O- or -NR¹⁰-NR¹¹-;
R⁵, R⁶, R⁷, R⁸, R⁹, R¹⁰ and R¹¹ are identical or different and are independently selected from the group consisting of hydrogen, deuterium, and the following groups which are unsubstituted or optionally substituted with one, two or more R^{E}: alkyl, cycloalkyl, cycloalkylalkyl, aryl, arylalkyl, heteroaryl, heteroarylalkyl, heterocyclyl, heterocyclylalkyl, HC(=O)-, alkyl-C(=O)-, cycloalkyl-C(=O)NH-, heterocyclyl-C(=O)NH-, aryl-C(=O)NH-, or heteroaryl-C(=O)NH-;
each R^{A}, R^{B}, R^{C}, R^{D} and R^{E} is identical or different and is independently selected from the group consisting of deuterium, halogen, hydroxy, cyano, nitro, oxo (=O), and the following groups which are unsubstituted or optionally substituted with one, two or more R^{F}: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, NH₂, HC(=O)NH-, alkyl-C(=O)NH-, cycloalkyl-C(=O)NH-, heterocyclyl-C(=O)NH-, aryl-C(=O)NH-, or heteroaryl-C(=O)NH-;
each R^{F} is identical or different and is independently selected from the group consisting of deuterium, halogen, hydroxy, cyano, nitro, oxo (=O), alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, NH₂, HC(=O)NH-, alkyl-C(=O)NH-, cycloalkyl-C(=O)NH-, heterocyclyl-C(=O)NH-, aryl-C(=O)NH-, and heteroaryl-C(=O)NH-;
the wavy line represents the site linked to L.

2. The ligand-drug conjugate, or a stereoisomer, racemate, tautomer, isotopologue, isotope-labeled compound, N-oxide, prodrug, solvate, or pharmaceutically acceptable salt thereof according to claim 1, wherein the definitions of groups in formula (G) are independently selected from:
wherein, A is absent or selected from the group consisting of C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl, and a combination of two or more of them, which are unsubstituted or optionally substituted with one, two or more R^{A}; wherein the C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl, or a combination of two or more of them may be uninterrupted or optionally interrupted by one, two or more groups selected from -O-, -S-, -NR⁵-, -NR⁶C(=O)-, -C(=O)NR⁶-, -C(=O)-, -NR⁷C(=O)NR⁸-, or -OC(=O)-; preferably, A is absent or selected from the group consisting of C₁₋₆ alkylene, C₂₋₆ alkenylene, C₂₋₆ alkynylene, C₃₋₆ cycloalkyl, C₆₋₁₀ aryl, 5-10 membered heteroaryl, 5-10 membered heterocyclyl, and a combination of two or more of them, which are unsubstituted or optionally substituted with one, two or more R^{A}; wherein the C₁₋₆ alkylene, C₂₋₆ alkenylene, C₂₋₆ alkynylene, C₃₋₆ cycloalkyl, C₆₋₁₀ aryl, 5-10-membered heteroaryl, 5-10-membered heterocyclyl, or a combination of two or more of them may be uninterrupted or optionally interrupted by one, two or more groups selected from-O-, -S-, -NR⁵-, -NR⁶C(=O)-, -C(=O)NR⁶-, -C(=O)-, -NR⁷C(=O)NR⁸-, or -OC(=O)-;
R¹ is selected from the group consisting of C₃₋₁₀ cycloalkyl, C₃₋₁₀ cycloalkyl-C₁₋₁₀ alkyl, C₃₋₁₀ cycloalkyloxy, 3-10 membered heterocyclyl, 3-10 membered heterocyclyl-C₁₋₁₀ alkyl, and 3-10 membered heterocyclyloxy, which are unsubstituted or optionally substituted with one, two or more R^{B}; preferably, R¹ is selected from the group consisting of C₃₋₆ cycloalkyl, C₃₋₆ cycloalkyl-C₁₋₆ alkyl, C₃₋₆ cycloalkyloxy, 3-6 membered heterocyclyl, 3-6 membered heterocyclyl-C₁₋₆ alkyl, and 3-6 membered heterocyclyloxy, which are unsubstituted or optionally substituted with one, two or more R^{B};
X is selected from O or S;
Z is selected from 0 or 1;
R² is selected from the group consisting of hydrogen, deuterium, halogen, hydroxy, mercapto, amino, cyano, and the following groups which are unsubstituted or optionally substituted with one, two or more R^{C}: C₃₋₁₀ alkyl, C₃₋₁₀ alkyloxy, C₃₋₁₀ cycloalkyl, C₃₋₁₀ cycloalkyl-C₁₋₁₀ alkyl, or C₃₋₁₀ cycloalkyloxy; preferably, R² is selected from the group consisting of hydrogen, deuterium, halogen, hydroxy, mercapto, amino, cyano, and the following groups which are unsubstituted or optionally substituted with one, two or more R^{C}: C₁₋₆ alkyl, C₁₋₆ alkyloxy, C₃₋₆ cycloalkyl, C₃₋₆ cycloalkyl-C₁₋₆ alkyl, or C₃₋₆ cycloalkyloxy;
R²² is selected from the group consisting of hydrogen, deuterium, halogen, hydroxy, mercapto, amino, cyano, and the following groups which are unsubstituted or optionally substituted with one, two or more R^{C}: C₃₋₁₀ alkyl, C₃₋₁₀ alkyloxy, C₃₋₁₀ cycloalkyl, C₃₋₁₀ cycloalkyl-C₁₋₁₀ alkyl, C₃₋₁₀ cycloalkyloxy; preferably, R²² is selected from the group consisting of hydrogen, deuterium, halogen, hydroxy, mercapto, amino, cyano, and the following groups which are unsubstituted or optionally substituted with one, two or more R^{C}: C₁₋₆ alkyl, C₁₋₆ alkyloxy, C₃₋₆ cycloalkyl, C₃₋₆ cycloalkyl-C₁₋₆ alkyl, or C₃₋₆ cycloalkyloxy;
R³ is selected from the group consisting of hydrogen, deuterium, halogen, hydroxy, mercapto, amino, cyano, and the following groups which are unsubstituted or optionally substituted with one, two or more R^{C}: C₁₋₁₀ alkyl, C₁₋₁₀ alkyloxy, C₃₋₁₀ cycloalkyl, C₃₋₁₀ cycloalkyl-C₁₋₁₀ alkyl, or C₃₋₁₀ cycloalkyloxy; preferably, R³ is selected from the group consisting of hydrogen, deuterium, halogen, hydroxy, mercapto, amino, cyano, and the following groups which are unsubstituted or optionally substituted with one, two or more R^{C}: C₁₋₆ alkyl, C₁₋₆ alkyloxy, C₃₋₆ cycloalkyl, C₃₋₆ cycloalkyl-C₁₋₆ alkyl, or C₃₋₆ cycloalkyloxy;
R⁴ is selected from the group consisting of hydrogen, deuterium, halogen, hydroxy, mercapto, amino, cyano, and the following groups which are unsubstituted or optionally substituted with one, two or more R^{C}: C₁₋₁₀ alkyl, C₁₋₁₀ alkyloxy, C₃₋₁₀ cycloalkyl, C₃₋₁₀ cycloalkyl-C₁₋₁₀ alkyl, or C₃₋₁₀ cycloalkyloxy; preferably, R⁴ is selected from the group consisting of hydrogen, deuterium, halogen, hydroxy, mercapto, amino, cyano, and the following groups which are unsubstituted or optionally substituted with one, two or more R^{C}: C₁₋₆ alkyl, C₁₋₆ alkyloxy, C₃₋₆ cycloalkyl, C₃₋₆ cycloalkyl-C₁₋₆ alkyl, or C₃₋₆ cycloalkyloxy;
alternatively, R² and R³, together with the atoms to which they are attached form a 4-10 membered ring structure which is unsubstituted or optionally substituted with one, two or more R^{D}; wherein the 4-10 membered ring structure may be selected from, for example, a 4-, 5-, 6-, 7-, 8-, 9-, or 10-membered hetero-monocyclic hydrocarbon, hetero-bicyclic hydrocarbon, monocyclic hydrocarbon, or bicyclic hydrocarbon; wherein the hetero-monocyclic hydrocarbon and hetero-bicyclic hydrocarbon contain one, two or more O, S, N or carbonyl or any combination thereof;
alternatively, R³ and R⁴, together with the atoms to which they are attached form a 4-10 membered ring structure which is unsubstituted or optionally substituted with one, two or more R^{D}; wherein the 4-10 membered ring structure may be selected from, for example, a 4-, 5-, 6-, 7-, 8-, 9-, or 10-membered monocyclic hydrocarbon, bicyclic hydrocarbon, hetero-monocyclic hydrocarbon, or hetero-bicyclic hydrocarbon; wherein the hetero-monocyclic hydrocarbon and hetero-bicyclic hydrocarbon contain one, two or more O, S, N or carbonyl or any combination thereof;
alternatively, R²² and R⁴, together with the atoms to which they are attached form a 4-10 membered ring structure which is unsubstituted or optionally substituted with one, two or more R^{D}; wherein the 4-10 membered ring structure may be selected from, for example, a 4-, 5-, 6-, 7-, 8-, 9-, or 10-membered monocyclic hydrocarbon, bicyclic hydrocarbon, hetero-monocyclic hydrocarbon, or hetero-bicyclic hydrocarbon; wherein the hetero-monocyclic hydrocarbon and hetero-bicyclic hydrocarbon contain one, two or more O, S, N or carbonyl or any combination thereof;
B is absent or selected from-O-, -S-, -S(=O)-, -C(=O)-, -NR⁵-, -C=N-NR⁹-, -C=N-O- or -NR¹⁰-NR¹¹-;
R³, R⁶, R⁷, R⁸, R⁹, R¹⁰ and R¹¹ are identical or different and are independently selected from the group consisting of hydrogen, deuterium, and the following groups which are unsubstituted or optionally substituted with one, two or more R^{E}: C₁₋₁₀ alkyl, C₃₋₁₀ cycloalkyl, C₃₋₁₀ cycloalkyl-C₁₋₁₀ alkyl, C₆₋₁₂ aryl, C₆₋₁₂ aryl-C₁₋₁₀ alkyl, 5-12 membered heteroaryl, 5-12 membered heteroaryl-C₁₋₁₀ alkyl, 5-12 membered heterocyclyl, 5-12 membered heterocyclyl-C₁₋₁₀ alkyl, HC(=O)-, C₁₋₁₀ alkyl-C(=O)-, C₃₋₁₀ cycloalkyl-C(=O)NH-, 5-12 membered heterocyclyl-C(=O)NH-, C₆₋₁₂ aryl-C(=O)NH-, or 5-12 membered heteroaryl-C(=O)NH-; preferably, R⁵, R⁶, R⁷, R⁸, R⁹, R¹⁰ and R¹¹ are identical or different and are independently selected from the following group consisting of hydrogen, deuterium, and the following groups which are unsubstituted or optionally substituted with one, two or more R^{E}: C₁₋₆ alkyl, C₃₋₆ cycloalkyl, C₃₋₆ cycloalkyl-C₁₋₆ alkyl, C₆₋₁₀ aryl, C₆₋₁₀ aryl-C₁₋₆ alkyl, 5-6 membered heteroaryl, 5-6 membered heteroaryl-C₁₋₆ alkyl, 5-6 membered heterocyclyl, 5-6 membered heterocyclyl-C₁₋₆ alkyl, HC(=O)-, C₁₋₆ alkyl-C(=O)-, C₃₋₆ cycloalkyl-C(=O)NH-, 5-6 membered heterocyclyl-C(=O)NH-, C₆₋₁₀ aryl-C(=O)NH-, or 5-6 membered heteroaryl-C(=O)NH-;
each of R^{A}, R^{B}, R^{C}, R^{D} and R^{E} is identical or different and is independently selected from the group consisting of deuterium, halogen, hydroxy, cyano, nitro, and the following groups which are unsubstituted or optionally substituted with one, two or more R^{F}: C₁₋₁₀ alkyl, C₁₋₁₀ alkyloxy, C₃₋₁₀ cycloalkyl, C₃₋₁₀ cycloalkyl-C₁₋₁₀ alkyl, C₃₋₁₀ cycloalkyloxy, C₆₋₁₂ aryl, C₆₋₁₂ aryl-C₁₋₁₀ alkyl, C₆₋₁₂ aryloxy, 5-12-membered heteroaryl, 5-12-membered heteroaryl-C₁₋₁₀ alkyl, 5-12-membered heteroaryloxy, 5-12-membered heterocyclyl, 5-12-membered heterocyclyl-C₁₋₁₀ alkyl, 5-12-membered heterocyclyloxy, NH₂, HC(=O)NH-, C₁₋₁₀ alkyl-C(=O)NH-, C₃₋₁₀ cycloalkyl-C(=O)NH-, 5-12 membered heterocyclyl-C(=O)NH-, C₆₋₁₂ aryl-C(=O)NH-, or 5-12 membered heteroaryl-C(=O)NH-; preferably, each of R^{A}, R^{B}, R^{C}, R^{D} and R^{E} is identical or different and is independently selected from the group consisting of deuterium, halogen, hydroxy, cyano, nitro, and the following groups which are unsubstituted or optionally substituted with one, two or more R^{F}: C₁₋₆ alkyl, C₁₋₆ alkyloxy, C₃₋₆ cycloalkyl, C₃₋₆ cycloalkyl-C₁₋₆ alkyl, C₃₋₆ cycloalkyloxy, C₆₋₁₀ aryl, C₆₋₁₀ aryl-C₁₋₆ alkyl, C₆₋₁₀ aryloxy, 5-6-membered heteroaryl, 5-6-membered heteroaryl-C₁₋₆ alkyl, 5-6-membered heteroaryloxy, 5-6-membered heterocyclyl, 5-6-membered heterocyclyl-C₁₋₆ alkyl, 5-6-membered heterocyclyloxy, NH₂, HC(=O)NH-, C₁₋₆ alkyl-C(=O)NH-, C₃₋₆ cycloalkyl-C(=O)NH-, 5-6 membered heterocyclyl-C(=O)NH-, C₆₋₁₀ aryl-C(=O)NH-, or 5-6 membered heteroaryl-C(=O)NH-;
each R^{F} is identical or different and is independently selected from the group consisting of deuterium, halogen, hydroxy, cyano, nitro, alkyl, C₁₋₁₀ alkyloxy, C₃₋₁₀ cycloalkyl, C₃₋₁₀ cycloalkyl-C₁₋₁₀ alkyl, C₃₋₁₀ cycloalkyloxy, C₆₋₁₂ aryl, C₆₋₁₂ aryl-C₁₋₁₀ alkyl, C₆₋₁₂ aryloxy, 5-12 membered heteroaryl, 5-12 membered heteroaryl-C₁₋₁₀ alkyl, 5-12 membered heteroaryloxy, 5-12 membered heterocyclyl, 5-12 membered heterocyclyl-C₁₋₁₀ alkyl, 5-12 membered heterocyclyloxy, NH₂, HC(=O)NH-, C₁₋₁₀ alkyl-C(=O)NH-, C₃₋₁₀ cycloalkyl-C(=O)NH-, 5-12-membered heterocyclyl-C(=O)NH-, C₆₋₁₂ aryl-C(=O)NH-, and 5-12-membered heteroaryl-C(=O)NH-; preferably, each R^{F} is identical or different and is independently selected from the group consisting of deuterium, halogen, hydroxy, cyano, nitro, alkyl, C₁₋₆ alkyloxy, C₃₋₆ cycloalkyl, C₃₋₆ cycloalkyl-C₁₋₆ alkyl, C₃₋₆ cycloalkyloxy, C₆₋₁₀ aryl, C₆₋₁₀ aryl-C₁₋₆ alkyl, C₆₋₁₀ aryloxy, 5-6 membered heteroaryl, 5-6 membered heteroaryl-C₁₋₆ alkyl, 5-6 membered heteroaryloxy, 5-6 membered heterocyclyl, 5-6 membered heterocyclyl-C₁₋₆ alkyl, 5-6 membered heterocyclyloxy, NH₂, HC(=O)NH-, C₁₋₆ alkyl C(=O)NH-, C3-6 cycloalkyl-C(=O)NH-, 5-6-membered heterocyclyl-C(=O)NH-, C₆₋₁₀ aryl-C(=O)NH-, and 5-6-membered heteroaryl-C(=O)NH-.

3. The ligand-drug conjugate, or a stereoisomer, racemate, tautomer, isotopologue, isotope-labeled compound, N-oxide, prodrug, solvate, or pharmaceutically acceptable salt thereof according to any one of claims 1-2, wherein:
A is absent or selected from one of the following substructures, wherein the substructure may be unsubstituted or optionally substituted with one, two or more R^{A}:
-(CH₂)ₙ₁-, -O(CH₂)ₙ₂-, -S(CH₂)ₙ₃-, -NR⁵(CH₂)ₙ₄-, -NR⁶C(=O)(CH₂)ₙ₅-, -C(=O)(CH₂)ₙ₆-, - NR⁷C(=O)NR⁸(CH₂)ₙ₇-, -OC(=O)(CH₂)ₙ₈-, -C=C(CH₂)ₙ₉-, -C≡C(CH₂)ₙ₁₀-, 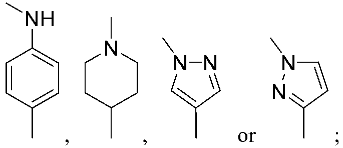
n1, n2, n3, n4, n5, n6, n7, n8, n9 and n10 are each independently selected from any integer between 0 and 6;
R¹ is selected from the following groups which are unsubstituted or optionally substituted with one, two or more R^{B}: C₃₋₆ cycloalkyl, or C₃₋₆ cycloalkyl-C₁₋₆ alkyl; for example, R¹ is selected from the following group unsubstituted or optionally substituted with one, two or more R^{B}: 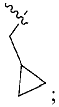
R² is selected from hydrogen;
R²² is selected from hydrogen;
R³ is selected from C₁₋₆ alkyl which is unsubstituted or optionally substituted with one, two or more R^{C}; for example, R³ is selected from methyl, ethyl or propyl;
R⁴ is selected from halogen;
B is absent or selected from-O-, -S- or NR⁵; R5 is selected from the group consisting of H, deuterium, C₁₋₆ alkyl, and C₃₋₆ cycloalkyl;
alternatively, R³ and R⁴, together with the phenyl ring to which they are attached form one of the following substructures, wherein the substructure may be unsubstituted or optionally substituted with one, two or more R^{D}: 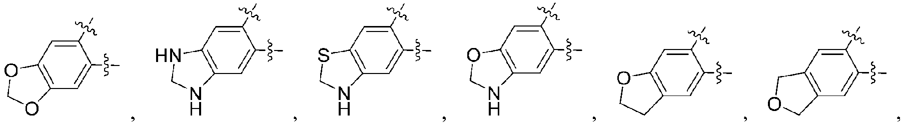 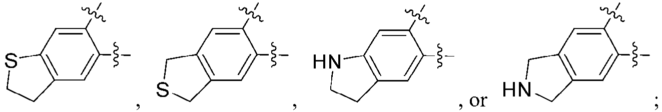
wherein R^{D} independently has the definition as described in any one of claims 1-2.

4. The ligand-drug conjugate, or a stereoisomer, racemate, tautomer, isotopologue, isotope-labeled compound, N-oxide, prodrug, solvate, or pharmaceutically acceptable salt thereof according to any one of claims 1-3, wherein:
A is absent or selected from one of the following substructures, wherein the substructure may be unsubstituted or optionally substituted with one, two or more R^{A}: 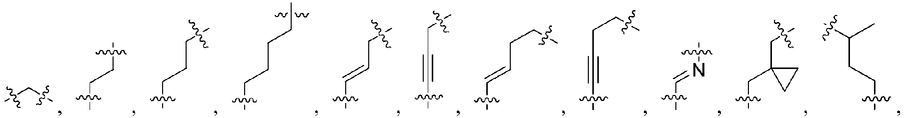 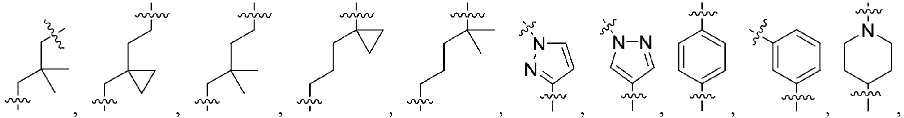 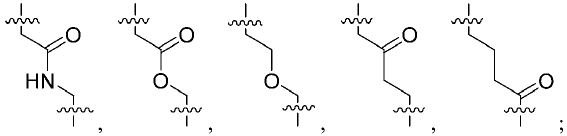
B is absent or selected from-O-, -S- or NR⁵; R⁵ is selected from the group consisting of H, deuterium, C₁₋₆ alkyl, and C₃₋₆ cycloalkyl.

5. The ligand-drug conjugate, or a stereoisomer, racemate, tautomer, isotopologue, isotope-labeled compound, N-oxide, prodrug, solvate, or pharmaceutically acceptable salt thereof according to any one of claims 1-4, wherein G is selected from a group represented by the following Formula (G-1) or (G-2): 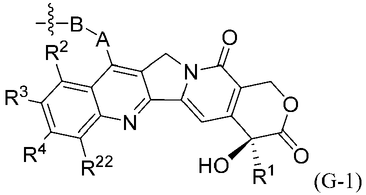 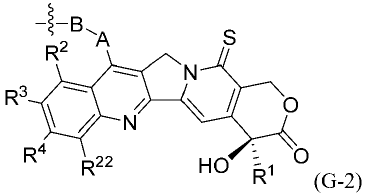 wherein A, B, R¹, R², R²², R³ and R⁴ independently have a definition as described in any one of claims 1-4.

6. The ligand-drug conjugate, or a stereoisomer, racemate, tautomer, isotopologue, isotope-labeled compound, N-oxide, prodrug, solvate, or pharmaceutically acceptable salt thereof according to any one of claims 1-4, wherein G is selected from a group represented by Formula (G-3), (G-4), (G-5), or (G-6): 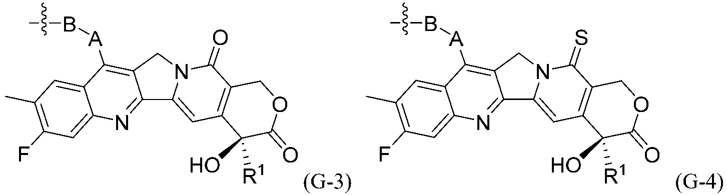 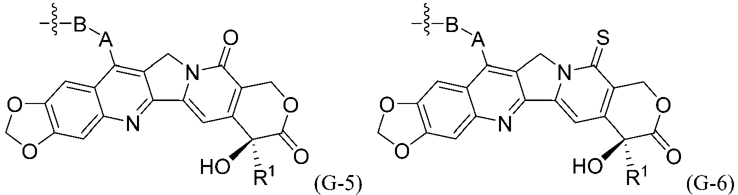 wherein, A, B and R¹ have definitions as described in any one of claims 1-4.

7. The ligand-drug conjugate, or a stereoisomer, racemate, tautomer, isotopologue, isotope-labeled compound, N-oxide, prodrug, solvate, or pharmaceutically acceptable salt thereof according to any one of claims 1-4, wherein, G is selected from the following group: 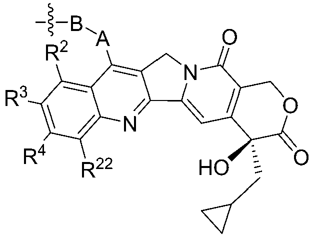 wherein, A, B, R², R²², R³ and R⁴ independently have a definition as described in any one of claims 1-4.

8. The ligand-drug conjugate, or a stereoisomer, racemate, tautomer, isotopologue, isotope-labeled compound, N-oxide, prodrug, solvate, or pharmaceutically acceptable salt thereof according to any one of claims 1-4, wherein, G is selected from the following group: 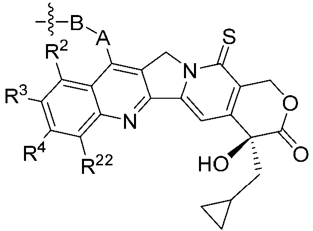 wherein, A, B, R², R²², R³ and R⁴ independently have a definition as described in any one of claims 1-4.

9. The ligand-drug conjugate, or a stereoisomer, racemate, tautomer, isotopologue, isotope-labeled compound, N-oxide, prodrug, solvate, or pharmaceutically acceptable salt thereof according to any one of claims 1-4, wherein, G is selected from the groups consisting of:

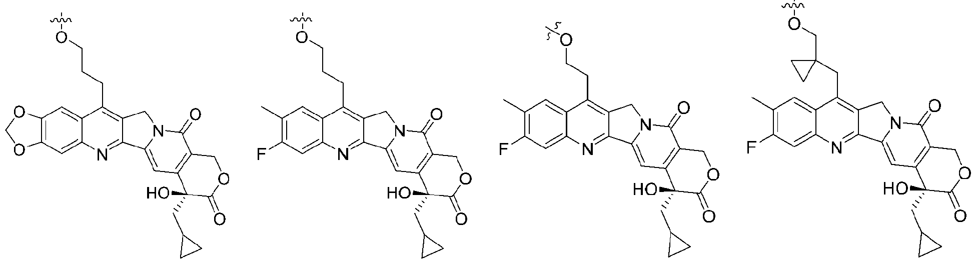

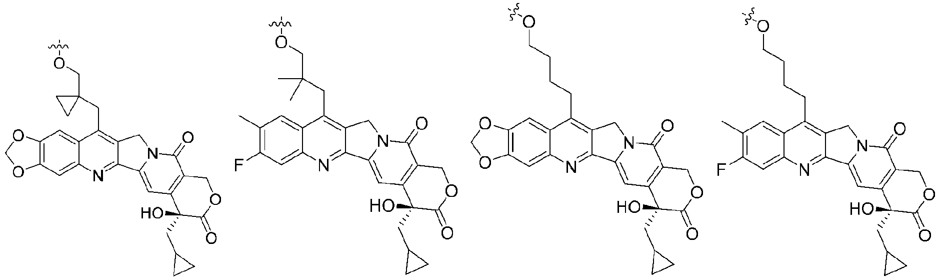

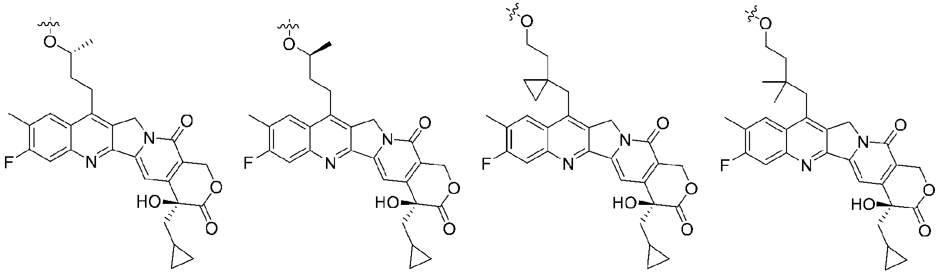

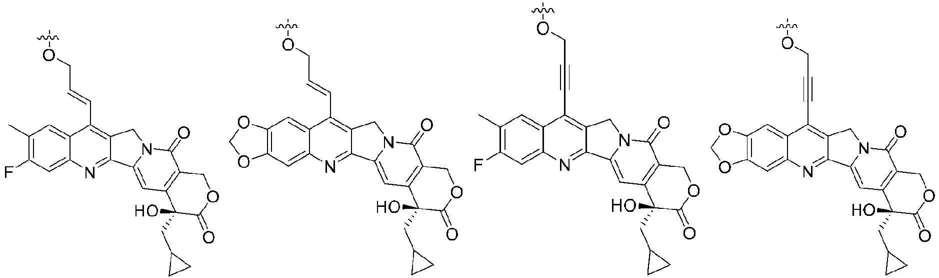

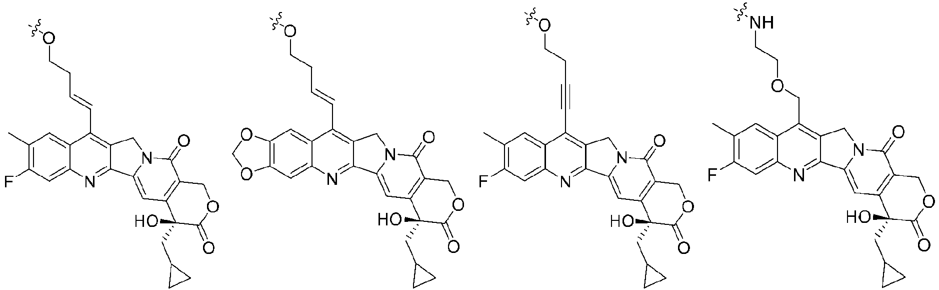

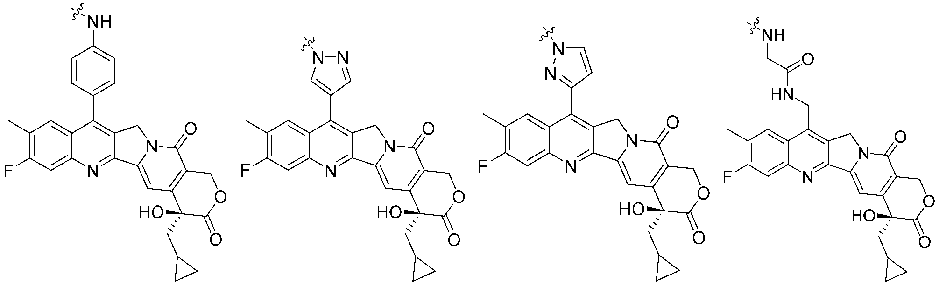

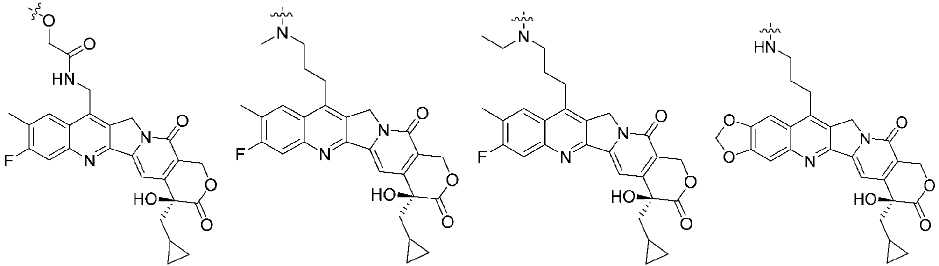

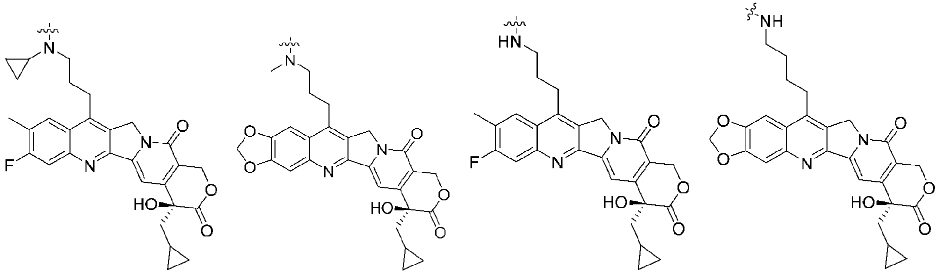

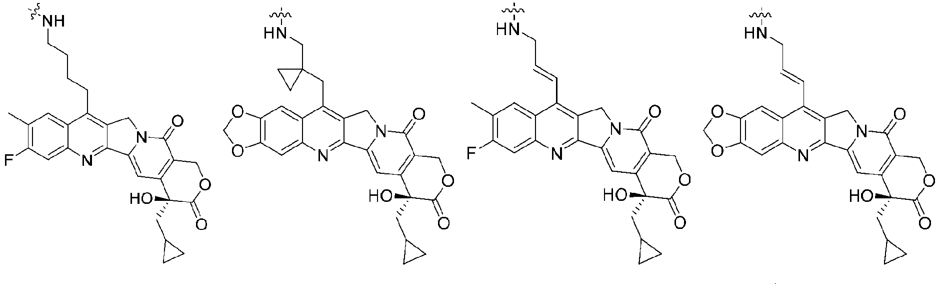

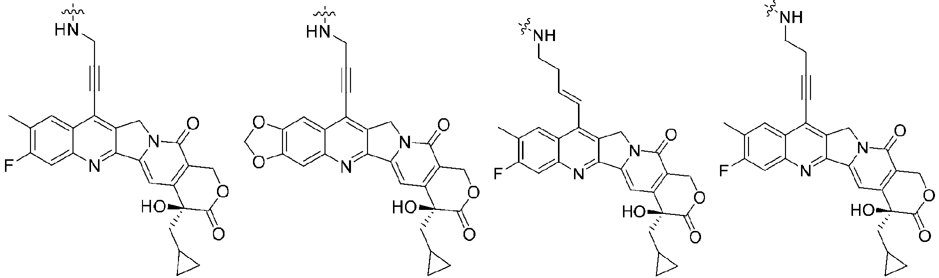

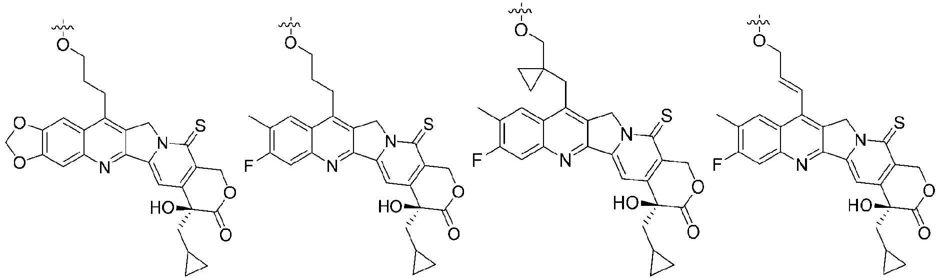

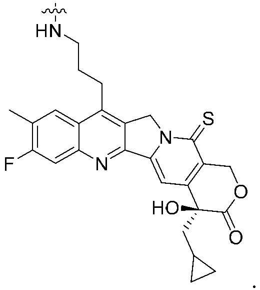

10. The ligand-drug conjugate, or a stereoisomer, racemate, tautomer, isotopologue, isotope-labeled compound, N-oxide, prodrug, solvate, or pharmaceutically acceptable salt thereof according to any one of claims 1-4,
wherein L is selected from a chemical bond or a linker represented by formula (L) as defined below:
#L¹-L²-L³-L⁴ * (L)
wherein L¹ is a linking moiety to the targeting moiety Tp, which is formed by reacting a reactive group L^{1'} with the targeting moiety Tp, and # represents a linking site to the targeting moiety Tp;
for example, L^{1'} is a pyrimidine group or a maleimide group, then L¹ has the following structure: 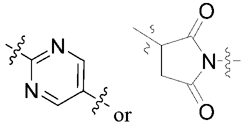
or its open-loop form: 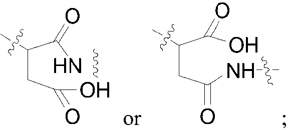
L² is absent or is a spacer between L¹ and L³;
L³ is a peptide moiety;
L⁴ is absent, or is a linking moiety between the peptide moiety to a biologically active molecule G, which is formed by reacting a reactive group L^{4'} with the biologically active molecule G or an intermediate thereof, and * represents a linking site attached to the biologically active molecule G;
optionally:
a following hydrophilic moiety is contained between the aforementioned moieties of L, preferably located between L¹ and L², or between L² and L³, or in a form of replacement of L², or in a form of insertion into the L² unit:
wherein the hydrophilic moiety is a divalent unit substituted with one, two or more hydrophilic groups, such as a phenylene substituted with one, two or more hydrophilic groups or an amino acid residue substituted with one, two or more hydrophilic groups, preferably the hydrophilic moiety is: 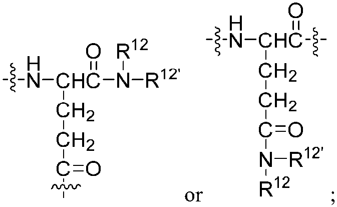 the hydrophilic moiety may also be a divalent unit formed by hydrophilic group itself, such as 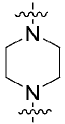 or-(CH₂CH₂O)ₚ-;
R¹², R^{12'} are identical or different, at least one is selected from a hydrophilic group and the other is selected from the groups consisting of: hydrogen, halogen, cyano, amino, nitro, and the following substituents which are unsubstituted or optionally substituted with one, two or more R^{zg}: C₁₋₁₀ alkyl, C₁₋₁₀ alkyloxy, C₃₋₁₀ cycloalkyl, C₃₋₁₀ cycloalkyl-C₁₋₁₀ alkyl, C₃₋₁₀ cycloalkyloxy;
the hydrophilic group is selected from a polyethylene glycol group or a polyethylene glycol divalent unit, a C₁₋₁₀ alkyl substituted with 1 to 10 hydroxyl, a group containing a sugar ring or a heterocyclic divalent unit containing a nitrogen atom such as a piperidinyl or piperazinyl, preferably a polyethylene glycol group, further preferably - (CH₂CH₂O)ₚ-C₁₋₁₀ alkyl, -C(=O)-NH-(CH₂CH₂O)ₚ-C₁₋₁₀ alkyl or -NH-(CH₂CH₂O)ₚ-C₁₋₁₀ alkyl, or preferably a C₁₋₁₀ alkyl substituted with 1 to 10 hydroxyl, further preferably 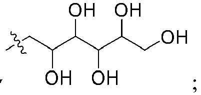
each p is identical or different and is independently selected from an integer from 0 to 10, preferably 1, 2, 3, 4, 5, 6, 7 or 8;
each R^{zg} is identical or different, and is independently selected from the following groups: halogen, hydroxy, amino, cyano, nitro, C₁₋₁₀ alkyl, C₁₋₁₀ alkyloxy, C₃₋₁₀ cycloalkyl, C₃₋₁₀ cycloalkyl-C₁₋₁₀ alkyl, C₃₋₁₀ cycloalkyloxy;
preferably, each R^{zg} is identical or different and is independently selected from the following groups: halogen, hydroxy, amino, cyano, nitro, C₁₋₆ alkyl, C₁₋₆ alkyloxy, C₃₋₆ cycloalkyl, C₃₋₆ cycloalkyl-C₁₋₆ alkyl, C₃₋₆ cycloalkyloxy;
the hydrophilic moiety as defined above is present or absent;
L¹-L⁴ or the hydrophilic moiety may be linked by any chemical bond, such as a direct bond, an ester bond (-CO-O-), an amide bond (-CO-NH-), an ether bond (-O-), a thioether bond (-S-), a carbamate (-N-CO-O-), or a ureido group (-O-CO-O-);
preferably, the hydrogen in each peptide bond or amide bond in L may optionally be substituted with methyl.

11. The ligand-drug conjugate, or a stereoisomer, racemate, tautomer, isotopologue, isotope-labeled compound, N-oxide, prodrug, solvate, or pharmaceutically acceptable salt thereof according to claim 10,
L¹ is formed from any group L^{1'} which reacts with the targeting moiety Tp, and L^{1'} is preferably a thiol reactive group, an amino reactive group, a carboxyl reactive group, a dithiol bridging group, or the like; for an antibody directed to introduce an unnatural amino acid, it may also be selected from a click chemical group such as a ketone, a hydrazine or a hydrazide, an azide, an alkyne, cyclopropene or a diene; when Tp is an antibody, the linking site comprises any applicable amino acid residue or N297 sugar chain coupling of a CH₂ domain, such as fucose, galactose, N-acetylgalactosamine (GalNAc), N-acetylglucosamine (GlcNAc) and sialic acid (SA) introduced by sugar engineering; wherein the linking reaction comprises a chemical reaction or an enzymatic reaction, such as the transfer of an amine-containing linker-drug or reactive spacer into a deglycosylated antibody using glutamine transaminase (MTGase);
L^{1'} is preferably a thiol reactive group;
L^{1'} is preferably a thiol reactive group of the following structure:
Hal-Het-
Hal is selected from the group consisting of halogen, OMs, OTs, OTf, nitro, and the following groups optionally substituted with one, two or more R^{z8}: C₁₋₁₀ alkyl thioether, C₆₋₁₂ aryl thioether, 5-12 membered heteroaryl thioether, C₁₋₁₀ alkyl sulfoxide, C₆₋₁₂ aryl sulfoxide, 5-12 membered heteroaryl sulfoxide, C₁₋₁₀ alkylsulfonyl, C₆₋₁₂ arylsulfonyl, or 5-12 membered heteroarylsulfonyl; wherein, R^{z8} is independently selected from the group consisting of H (hydrogen), D (deuterium), halogen, CN, nitro, C₁₋₆ alkyl, halo-C₁₋₆ alkyl, C₁₋₆ alkoxy, C₆₋₁₂ aryl, and 5-12 membered heteroaryl;
Het is selected from the group consisting of 5-12 membered heteroaryl optionally substituted with one, two or more R^{z9}; wherein, R^{z9} is independently selected from the group consisting of H (hydrogen), D (deuterium), halogen, CN, nitro, C₁₋₁₀ alkyl, and halogenated C₁₋₁₀ alkyl; preferably, Het is selected from the group consisting of 5-10 membered heteroaryl optionally substituted with one, two or more R^{z9}; wherein, R^{z9} is independently selected from the group consisting of H (hydrogen), D (deuterium), halogen, CN, nitro, C₁₋₄ alkyl, and halogenated C₁₋₄ alkyl;
Hal is preferably methanesulfonyl and Het is preferably pyrimidine;
Hal-Het- is preferably: 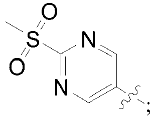
the corresponding L¹ structure is: 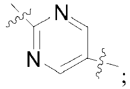
and L^{1'}-L² is preferably the following structure: 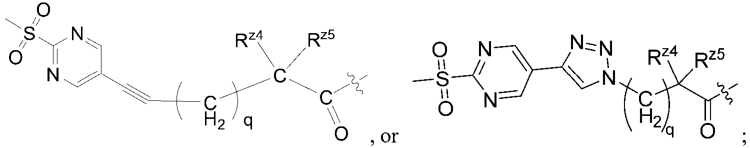
q is an integer selected from 0 to 10, preferably 1, 2, 3, 4, 5, 6, 7 or 8;
R^{z4} and R^{z5} are identical or different and independently selected from H, C₁₋₄ alkyl, C₃₋₆ cycloalkyl, C₃₋₆ cycloalkyl-C₁₋₄ alkyl or R^{z4} and R^{z5} together form C₃₋₆ cycloalkyl;
L^{1'}-L² is further preferably the following structure: 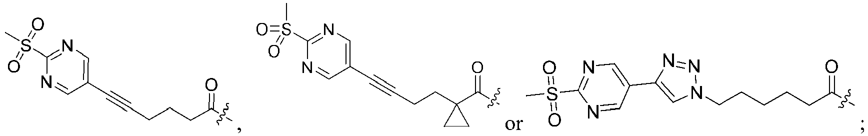
or L^{1'} is further preferably a maleimide group or a substituted maleimide group, and L¹-L² is preferably of the following structure:
a fragment prepared from (N-maleimidomethyl)-carboxylic acid-N-hydroxysuccinimide ester having the structure of: 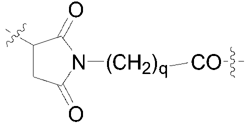 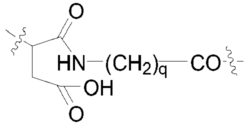 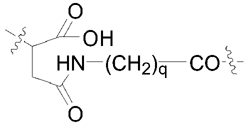
(q is an integer from 0 to 10, preferably 1, 2, 3, 4, 5, 6, 7 or 8);
or a fragment prepared from m-maleimidobenzoyl-N-hydroxysuccinimide ester (MBS) having the structure: 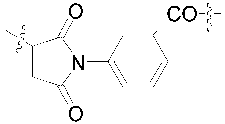 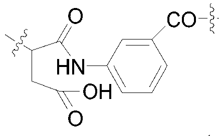 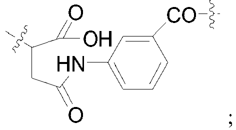
or a fragment prepared from 4-(N-maleimidomethyl)-cyclohexane-1-carboxylic acid succinimidyl ester (SMCC) having the structure:

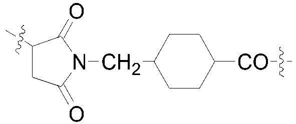

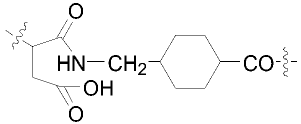

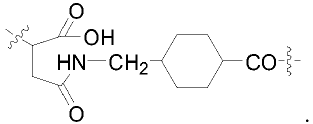

12. A ligand-drug conjugate, or a stereoisomer, racemate, tautomer, isotopologue, isotope-labeled compound, N-oxide, prodrug, solvate, or pharmaceutically acceptable salt thereof according to claim 10,
L² is absent or selected from the group consisting of C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl, and a combination of two or more of them; wherein the C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl or a combination of two or more of them may be uninterrupted or optionally interrupted by carbonyl, O, S, or N; wherein the C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl or a combination of two or more of them may optionally be substituted with C₁₋₆ alkyl, C₃₋₆ cycloalkyl, halogen, or halo-C₁₋₆ alkyl; optionally, the C₁₋₆ alkyl or halo-C₁₋₆ alkyl may form a C₃₋₆ cycloalkyl with the C atom to which it is attached, and L² is attached to the L¹ or L³ fragment via a functional group or a covalent bond;
preferably, L² is selected from -(CH₂)_{q}-, -C(=O)-NH-(CH₂)_{q}-C(=O)-, -(CH₂)_{q}-C(=O)-, -(CH₂)_{q}-NH-C(=O)-, - (CH₂)_{q}-NCH₃-C(=O)-, -(C≡C)-(CH₂)_{q}-C(R^{z4}R^{z5})-C(-O)- or -Cy-(CH₂)_{q}-C(R^{z4}R^{z5})-C(=O)-, wherein q is an integer selected from 0 to 10, preferably 1, 2, 3, 4, 5, 6, 7 or 8;
wherein Cy is a 5-12 membered heteroaryl or heterocyclyl optionally containing heteroatom of S, O or N, and is optionally substituted with H (hydrogen), D (deuterium), halogen, CN, nitro, C₁₋₄ alkyl and halogenated C₁₋₄ alkyl, preferably at least three atoms in Cy are N, further preferably three consecutive atoms in Cy are N, further preferably Cy is 1, 2, 3-triazolyl;
preferably, R^{z4} and R^{z5} are identical or different and independently selected from H, C₁₋₄ alkyl, C₃₋₆ cycloalkyl, C₃₋₆ cycloalkyl-C₁₋₄ alkyl, or R^{z4} and R^{z5} together form a C₃₋₆ cycloalkyl;
preferably, L² is linked to the N-terminus of peptide fragment L³ via-NHC(=O)-, -NCH₃C(=O)- or -C(=O)-.

13. The ligand-drug conjugate, or a stereoisomer, racemate, tautomer, isotopologue, isotope-labeled compound, N-oxide, prodrug, solvate, or pharmaceutically acceptable salt thereof according to claim 10,
wherein L³ is selected from a bivalent peptide radicals comprising 2 to 8 amino acid residues, which are natural or non-natural, of L- or D-configuration, and are optionally substituted; each of the amino acid residues is identical or different, and is independently selected from the residues of the following amino acids: Alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), selenocysteine (Sec), pyrrolysine (Pyl), homoserine, homocysteine, demethylpyrrolysine, an analog of the above amino acid, or an amino acid residue represented by AA¹ or a stereoisomer thereof, 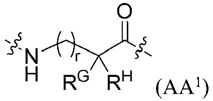
wherein R^{G} and R^{H} are not simultaneously H and are each independently selected from H, 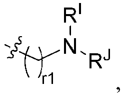 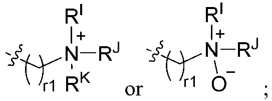
alternatively, R^{G} and R^{H} together with the carbon atoms to which they are jointly attached form a C₃₋₁₀ cycloalkyl or 3-10 membered heterocyclyl, which is unsubstituted or optionally substituted with one, two or more R^{L};
r and r1 are each independently selected from any integer from 0 to 10;
R^{I}, R^{J} and R^{K} are each independently selected from H, and the groups consisting of C₁₋₆ alkyl, C₃₋₁₀ cycloalkyl, C₃₋₁₀ cycloalkyl-C₁₋₁₀ alkyl, or an ester group, which are unsubstituted or optionally substituted with one, two or more R^{M};
alternatively, R^{I} and R^{J}, together with the nitrogen atom to which they are jointly attached form a 4-10 membered heterocyclyl which is unsubstituted or optionally substituted with one, two or more R^{L};
R^{M} and R^{L} are identical or different and are independently selected from the group consisting of deuterium, halogen, hydroxy, cyano, nitro, alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, NH₂, alkylamino, dialkylamino, HC(=O)NH-, alkyl-C(=O)NH-, cycloalkyl-C(=O)NH-, heterocyclyl-C(=O)NH-, aryl-C(=O)NH-, and heteroaryl-C(=O)NH-; optionally, two R^{M} or R^{L} attached to the same carbon atom together form a C₃₋₆ cycloalkyl;
further preferably, L³ is selected from a bivalent peptide group comprising a combination of 2, 3, 4, 5 or 6 amino acid residues, which are optionally substituted, natural or non-natural, and of L- or D-configuration, or amino acid residues of AA¹; each of the amino acid residues is identical or different, independently selected from the following amino acid residues: Alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), selenocysteine (Sec), pyrrolysine (Pyl), homoserine, homocysteine, demethylpyrrolysine, an analog of the above amino acid, or AA¹; for example, -ValCit-, -ValAA¹-, -CitVal-, -AlaAla-, -AlaCit-, -CitAla-, -AsnCit-, -CitAsn-, -CitCit-, -ValGlu-, - GluVal-, -SerCit-, -CitSer-, -LysCit-, -CitLys-, -AspCit-, -CitAsp-, -AlaVal-, -ValAla-, -PheAla-, -AlaPhe-, - PheLys-, -LysPhe-, -ValLys-, -GlyAA¹-, -LysVal-, -AlaLys-, -LysAla-, -PheCit-, -CitPhe-, -LeuCit-, -CitLeu-, - IleCit-, -CitIle-, -PheArg-, -ArgPhe-, -CitTrp-, -TrpCit-, -AlaAlaAla-, -PhePheLys-, -ValAA¹Gly-, -AlaAA¹Gly-, - GlyAA¹Gly-, -LysPhePhe-, -DPhePheLys-, -DLysPhePhe-, -GlyPheLys-, -LysPheGly-, -GlyPheLeuGly-, - GlyLeuPheGly-, -AlaLeuAlaLeu-, -GlyGlyGly-, -GlyGlyGlyGly-, -GlyPheValGly-, -GlyValPheGly-, - GlyGlyPheGly-, or -GlyGlyValGly-;
in the amino acid residues of AA¹, preferably,
r and r1 are each independently selected from any integer from 0 to 5;
either of R^{G} and R^{H} is H and the other is selected from: 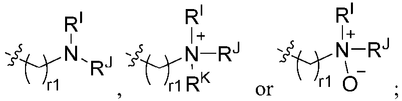
alternatively, R^{G} and R^{H} together with the carbon atoms to which they are jointly attached form a 5-6 membered heterocyclyl which is unsubstituted or optionally substituted with one, two or more R^{L};
R^{I}, R^{J} and R^{K} are each independently selected from the group consisting of H, methyl, ethyl, n-propyl, n-butyl, C₃₋₆ cycloalkyl, C₃₋₆ cycloalkyl-C₁₋₆ alkyl,-COOCH₃, COOCH₂CH₃, -COOCH₂CH₂CH₃, -COOCH(CH₃)₂, - COOC(CH₃)₃, and -COOCH₂CH₂CH₂CH₃, which is unsubstituted or optionally substituted with one, two or more R^{M};
alternatively, R^{I} and R^{J}, together with the nitrogen atom to which they are jointly attached form a 5-6 membered heterocyclyl that is unsubstituted or optionally substituted with one, two or more R^{L};
most preferably,
L³ is selected from -ValAA¹Gly-;
in the amino acid residues of AA¹, r is 0 and r1 is 4;
R^{G} and R^{H}, either is H and the other is selected from: 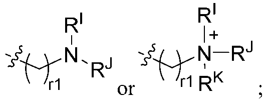
alternatively, R^{G} and R^{H}, together with the carbon atoms to which they are jointly attached form 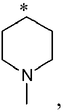 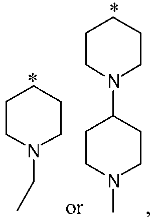 wherein * indicates the carbon atoms to which R^{G} and R^{H} are commonly attached;
R^{I}, R^{J} and R^{K} are each independently selected from H, and the following groups which are unsubstituted or optionally substituted with one, two or more R^{M}: methyl, ethyl, n-propyl, n-butyl, C₃₋₆ cycloalkyl, or C₃₋₆ cycloalkyl-C₁₋₄ alkyl;
alternatively, R^{I} and R^{J}, together with the nitrogen atoms to which they are jointly attached form

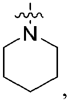

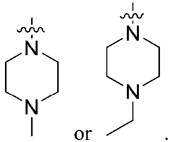

14. The ligand-drug conjugate, or a stereoisomer, racemate, tautomer, isotopologue, isotope-labeled compound, N-oxide, prodrug, solvate, or pharmaceutically acceptable salt thereof according to any one of claims 10-13, wherein:
AA¹ amino acid residues are selected from one of the following substructures:

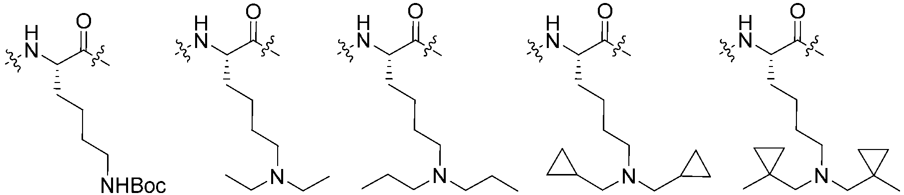

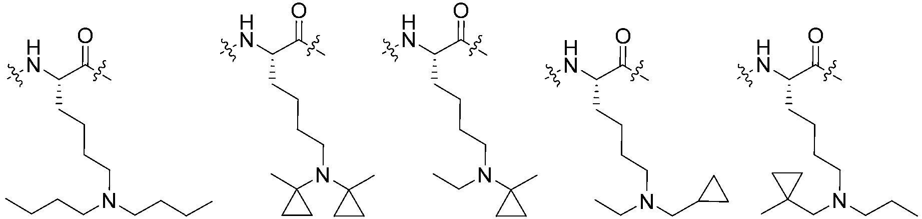

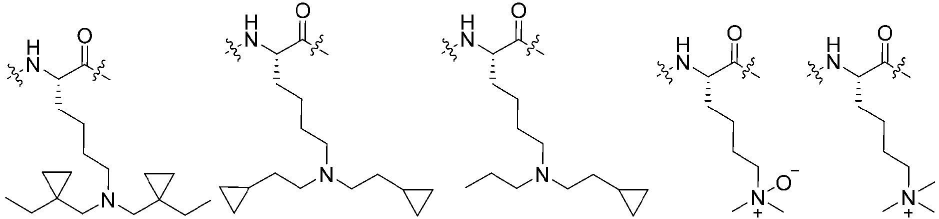

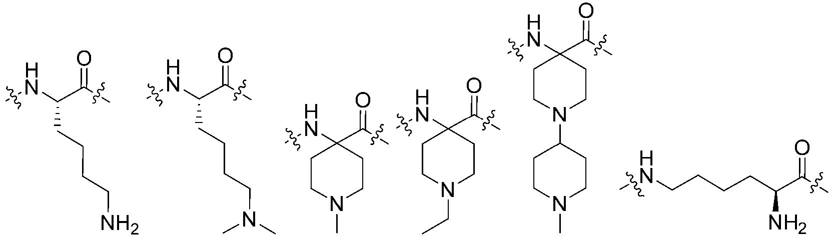

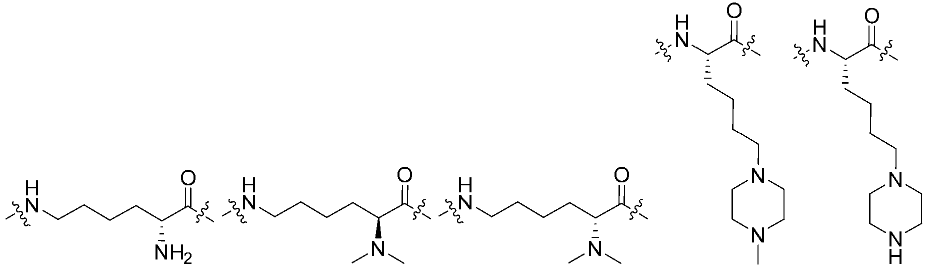

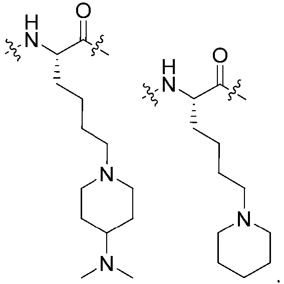

15. The ligand-drug conjugate, or a stereoisomer, racemate, tautomer, isotopologue, isotope-labeled compound, N-oxide, prodrug, solvate, or pharmaceutically acceptable salt thereof according to claim 10,
L⁴ is absent or selected from: 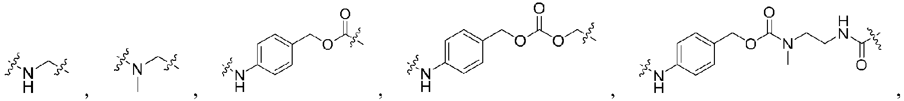 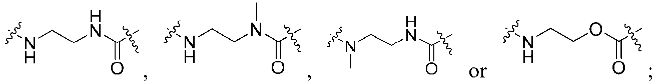
further preferably, L⁴ is: 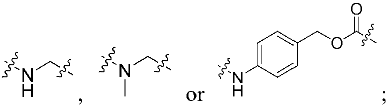
most preferably, L⁴ is:

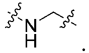

16. The ligand-drug conjugate, or a stereoisomer, racemate, tautomer, isotopologue, isotope-labeled compound, N-oxide, prodrug, solvate, or pharmaceutically acceptable salt thereof according to any one of claims 10-15, wherein:
L¹ is generated by coupling Tp with a thiol-reactive group selected from a maleimide group, a substituted maleimide group or Hal-Het-;
the hydrophilic moiety is contained between L¹ and L², or between L² and L³, or in a form of replacement of L², or in a form of insertion into the L².

17. The ligand-drug conjugate, or a stereoisomer, racemate, tautomer, isotopologue, isotope-labeled compound, N-oxide, prodrug, solvate, or pharmaceutically acceptable salt thereof according to any one of claims 10-15,
L¹ is generated by coupling Tp with a thiol-reactive group selected from a maleimide group or a substituted maleimide group.

18. The ligand-drug conjugate, or a stereoisomer, racemate, tautomer, isotopologue, isotope-labeled compound, N-oxide, prodrug, solvate, or pharmaceutically acceptable salt thereof according to any one of claims 10-15,
L¹ is generated by coupling Tp with a thiol-reactive group selected from Hal-Het-;
L⁴ is:

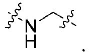

19. The ligand-drug conjugate, or a stereoisomer, racemate, tautomer, isotopologue, isotope-labeled compound, N-oxide, prodrug, solvate, or pharmaceutically acceptable salt thereof according to any one of claims 10-15,
L¹-L² is formed by coupling Tp with L^{1'}-L² of the following structure: 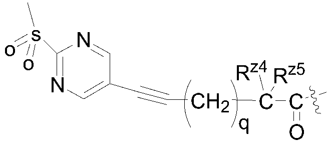
q is an integer selected from 0 to 10, preferably 1, 2, 3, 4, 5, 6, 7 or 8;
R^{z4} and R^{z5} are identical or different and independently selected from H, C₁₋₄ alkyl, C₃₋₆ cycloalkyl, C₃₋₆ cycloalkyl-C₁₋₄ alkyl, or R^{z4} and R^{z5} together form a C₃₋₆ cycloalkyl, wherein at least one of R^{z4} or R^{z5} is not H;
further preferably, R^{z4} and R^{z5} together form a C₃₋₆ cycloalkyl;
most preferably, L¹-L² is generated by coupling 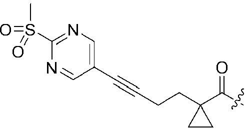 with Tp.

20. The ligand-drug conjugate, or a stereoisomer, racemate, tautomer, isotopologue, isotope-labeled compound, N-oxide, prodrug, solvate, or pharmaceutically acceptable salt thereof according to any one of claims 1-19,
Tp is a targeting moiety selected from the group consisting of a small molecule ligand, a protein, a polypeptide and a non-protein agent (e.g., a sugar, RNA or DNA);
preferably, the target of Tp is selected from the group consisting of epidermal growth factor, Trop-2, CD37, HER2, CD70, EGFRvIII, Mesothelin, Folate receptor1, Mucin 1, CD138, CD20, CD19, CD30, SLTRK6, Nectin 4, Tissue factor, Mucin16, Endothelin receptor, STEAP1, SLC39A6, Guanylylcyclase C, PSMA, CCD79b, CD22, Sodium phosphate cotransporter 2B, GPNMB, Trophoblast glycoprotein, AGS-16, EGFR, CD33, CD66e, CD74, CD56, PD-L1, TACSTD2, DR5, E16, 0772P, MPF, Napi3b, Sema 5b, PSCA hlg, ETBR, MSG783, STEAP2, TrpM4, CRIPTO, CD21, CD79b, FcRH2, NCA, MDP, IL20Rα, Brevican, EphB2R, ASLG659, PSCA, GEDA, BAFF-R, CD79a, CXCR5, HLA-DOB, P2X5, CD72, LY64, FcRH1, IRTA2, TENB2, Integrin α5β6, α4β7, FGF2, FGFR2, Her3, CA6, DLL3, DLL4, P-cadherin, EpCAM, pCAD, CD223, LYPD3, LY6E, EFNA4, ROR1, SLITRK6, 5T4, ENPP3, Claudin18.2, BMPR1B, Tyro7, c-Met, ApoE, CD11c, CD40, CD45(PTPRC), CD49D(ITGA4), CD80, CSF1R, CTSD, GZMB, Ly86, MS4A7, PIK3AP1, PIK3CD, CCR5, IFNG, IL10RA1, IL-6, ACTA2, COL7A1, LOX, LRRC15, MCPT8, MMP10, NOG, SERPINE1, STAT1, TGFBR1, CTSS, PGF, VEGFA, C1QA, C1QB, ANGPTL4, EGLN, EGLN3, BNIP3, AIF1, CCL5, CXCL10, CXCL11, IFI6, PLOD2, KISS1R, STC2, DDIT4, PFKFB3, PGK1, PDK1, AKR1C1, AKR1C2, CADM1, CDH11, COL6A3, CTGF, HMOX1, KRT33A, LUM, WNT5A, IGFBP3, MMP14, CDCP1, PDGFRA, TCF4, TGF, TGFB1, TGFB2, CD11b, ADGRE1, EMR2, TNFRSF21, UPK1B, TNFSF9, MMP16, MFI2, IGF-1R, RNF43, NaPi2b and BCMA;
preferably, Tp is a small molecule ligand, such as a folic acid derivative, a glutamic acid urea derivative, a somatostatin derivative, an arylsulfonamide derivative (e.g., a carbonic anhydrase IX inhibitor), an ICG dye, a cyanine dye or a derivative thereof;
preferably, Tp is selected from an antibody or antigen-binding fragment thereof, and the antibody is selected from the group consisting of a chimeric antibody, a humanized antibody, and a fully human antibody; preferably a monoclonal antibody;
preferably, the antibody or antigen-binding fragment thereof is at least one selected from the following antibodies or antigen-binding fragments thereof: anti-CD20 antibody, anti-CD22 antibody, anti-CD30 antibody, anti-CD33 antibody, anti-CD44 antibody, anti-CD56 antibody, anti-CD70 antibody, anti-CD73 antibody, anti-CD105 antibody, anti-CEA antibody, anti-A33 antibody, anti-Cripto antibody, anti-EphA2 antibody, anti-G250 antibody, anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-MUCl antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody or anti-Mesothelin antibody, and the antibody may be a bispecific antibody or a multispecific antibody;
further preferably, the antibody or antigen-binding fragment thereof is at least one selected from the following antibodies or antigen-binding fragments thereof: Trastuzumab, Pertuzumab, Nimotuzumab, Enoblituzumab, Emibetuzumab, Inotuzumab, Pinatuzumab, Brentuximab, Gemtuzumab, Bivatuzumab, Lorvotuzumab, cBR96, and Glembatumumab.

21. The ligand-drug conjugate, or a stereoisomer, racemate, tautomer, isotopologue, isotope-labeled compound, N-oxide, prodrug, solvate, or pharmaceutically acceptable salt thereof according to any one of claims 1-20, wherein the conjugate, linker, or linker-drug thereof is selected from one of the following:
wherein, u is selected from an integer of 0 to 10, G has the definition as described in any one of claims 1-20, and LG has the definition of Tp as decribed in any one of claims 1-20;
| No. | Conjugate, linker or linker-drug thereof |
|---|---|
| 1 | 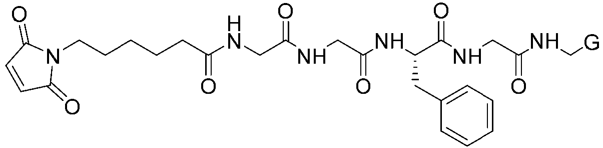 |
| 2 | 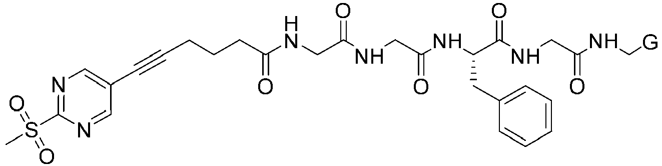 |
| 3 | 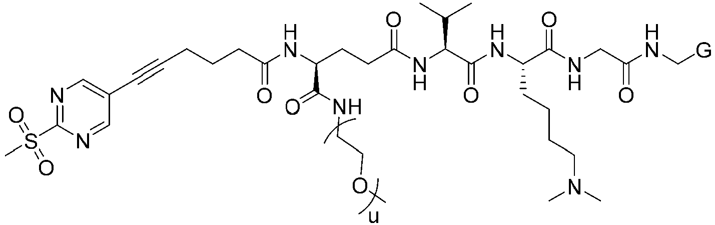 |
| 4 | 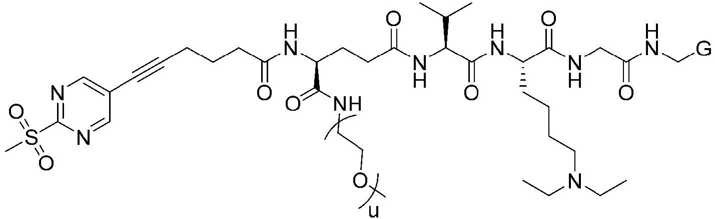 |
| 5 | 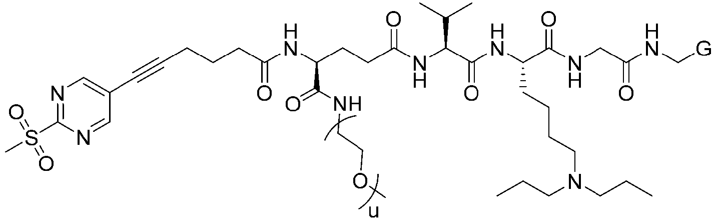 |
| 6 | 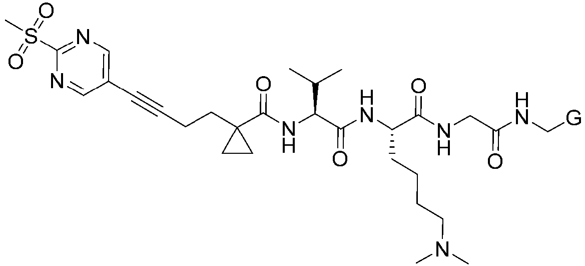 |
| 7 | 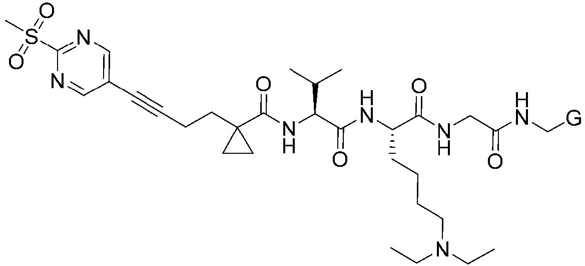 |
| 8 | 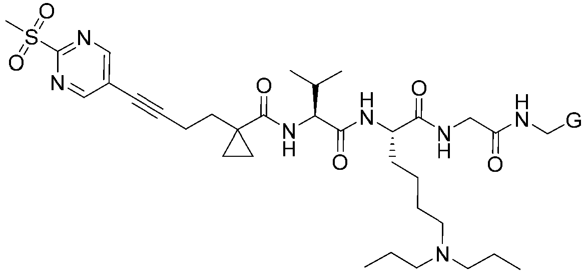 |
| 9 | 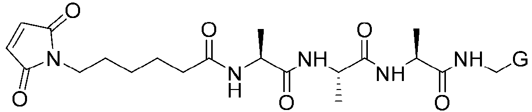 |
| 10 | 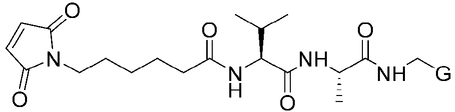 |
| 11 | 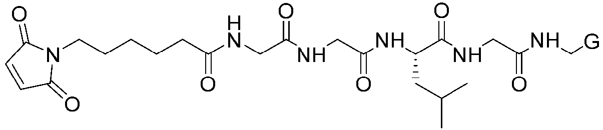 |
| 12 | 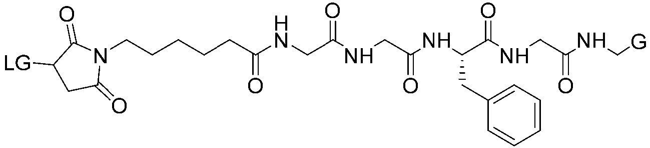 |
| 13 | 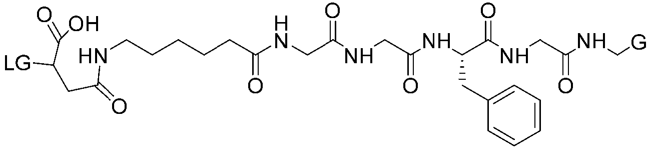 |
| 14 | 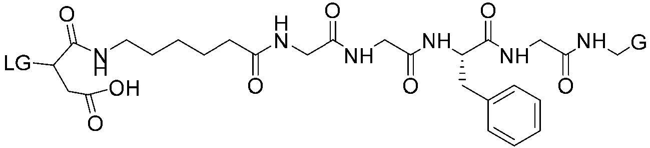 |
| 15 | 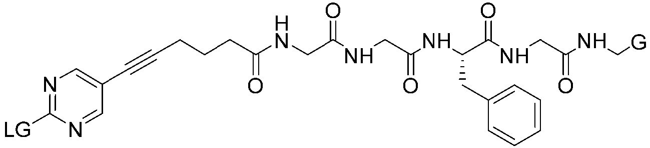 |
| 16 | 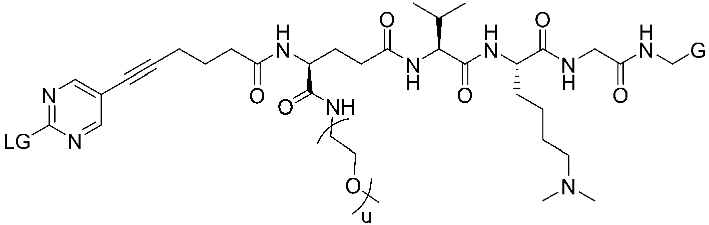 |
| 17 | 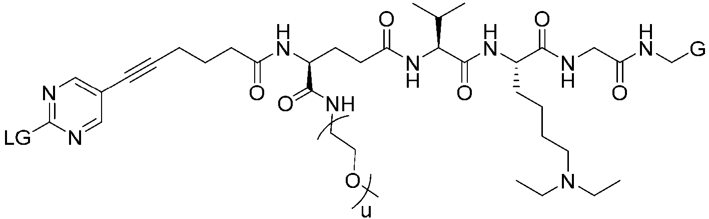 |
| 18 | 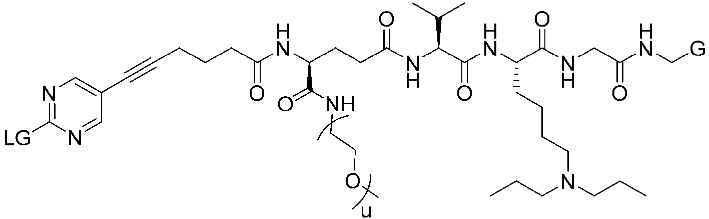 |
| 19 | 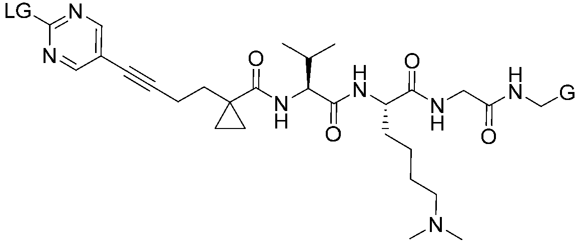 |
| 20 | 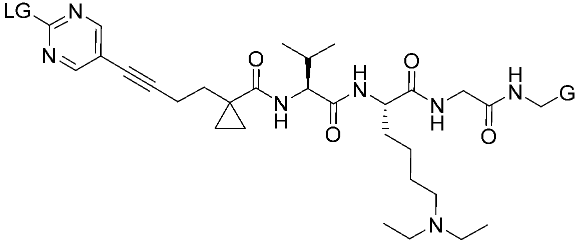 |
| 21 | 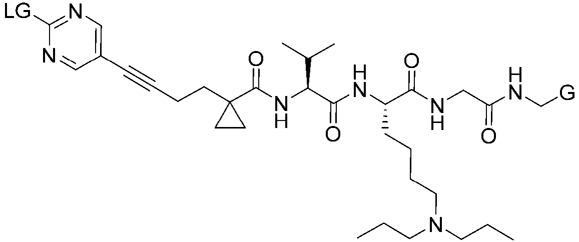 |
| 22 | 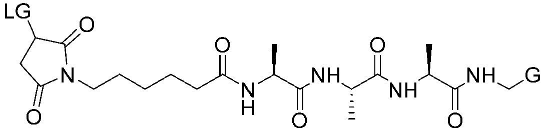 |
| 23 | 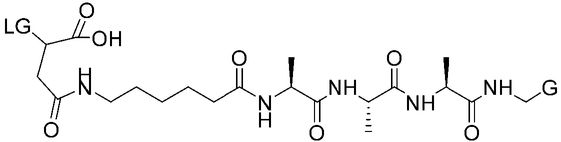 |
| 24 | 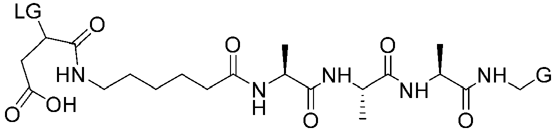 |
| 25 | 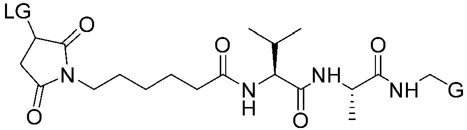 |
| 26 | 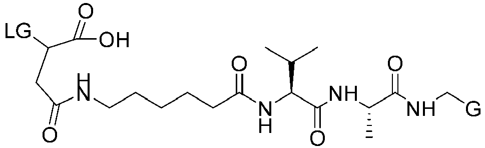 |
| 27 | 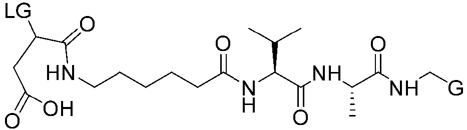 |
| 28 | 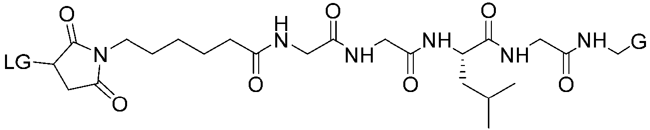 |
| 29 | 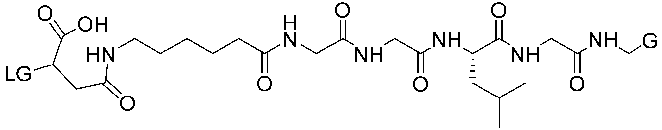 |
| 30 | 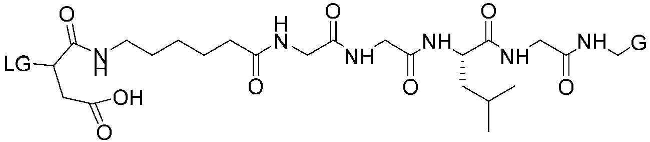 |
| 31 | 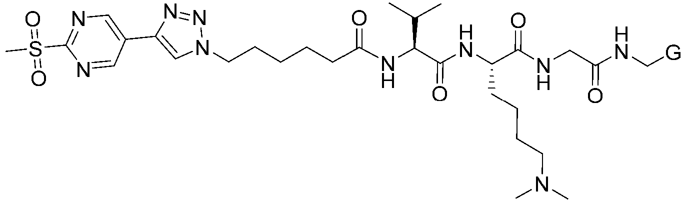 |
| 32 | 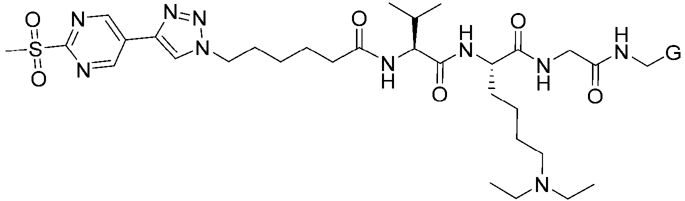 |
| 33 | 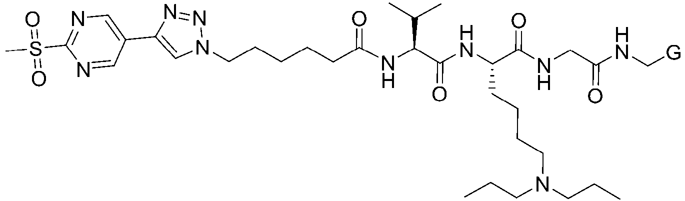 |
| 34 | 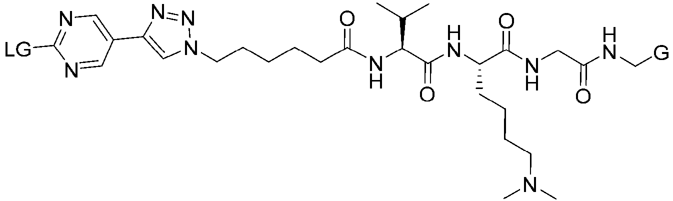 |
| 35 | 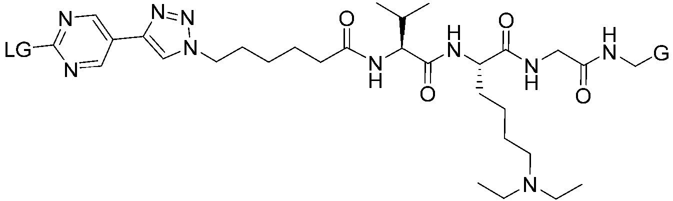 |
| 36 | 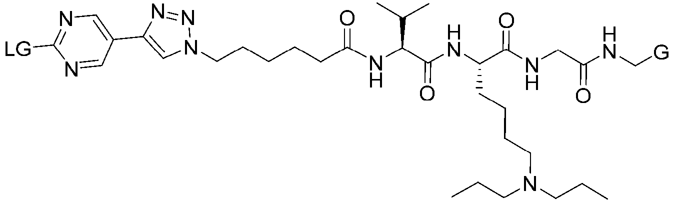 |
| 37 | 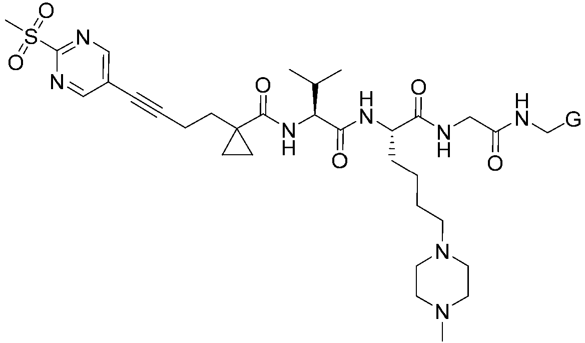 |
| 38 | 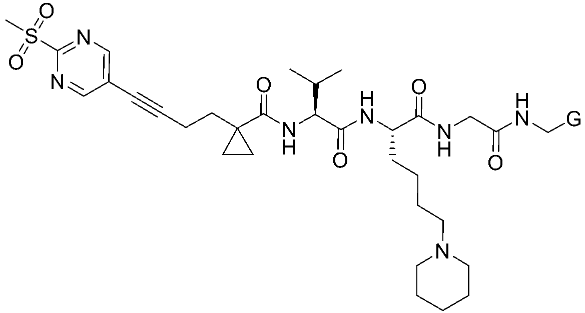 |
| 39 | 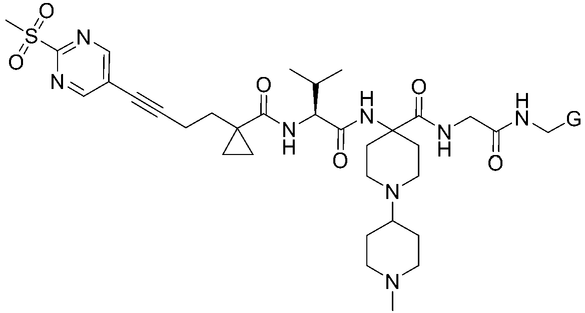 |
| 40 | 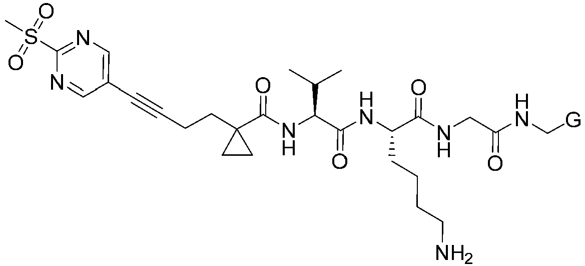 |
| 41 | 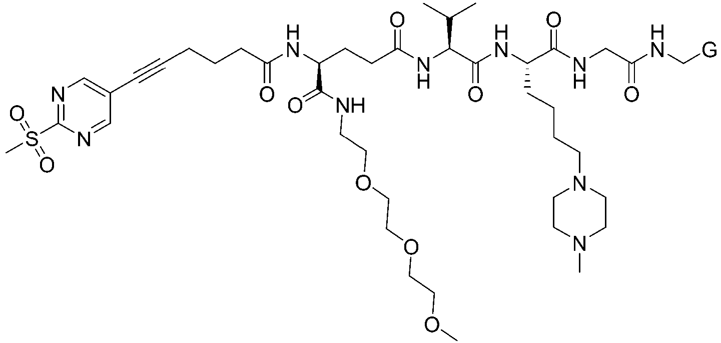 |
| 42 | 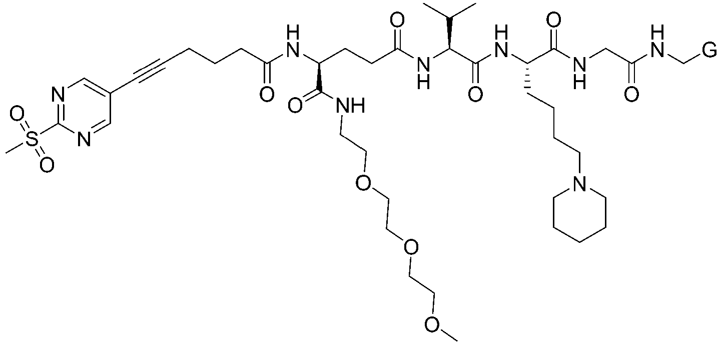 |
| 43 | 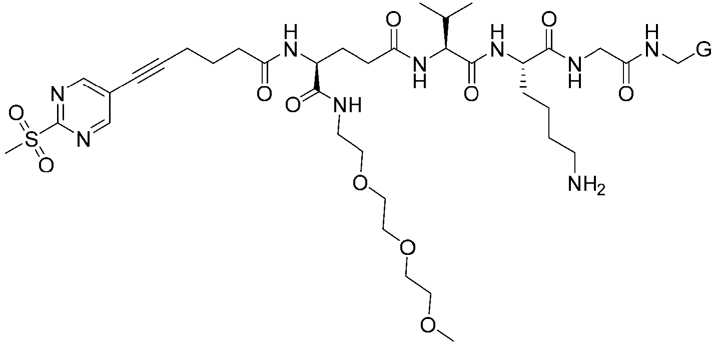 |
| 44 | 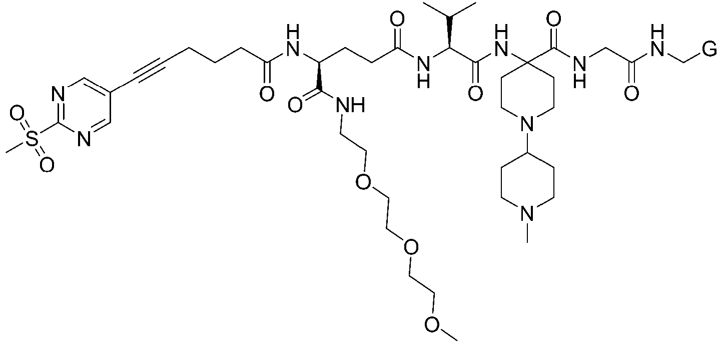 |
| 45 | 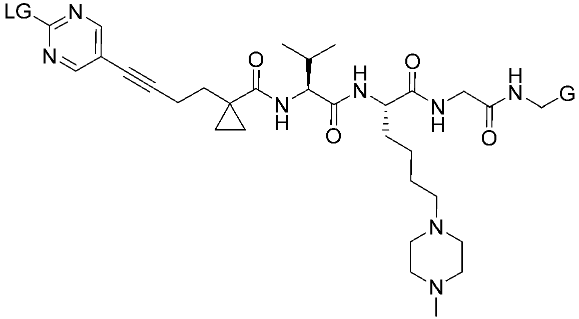 |
| 46 | 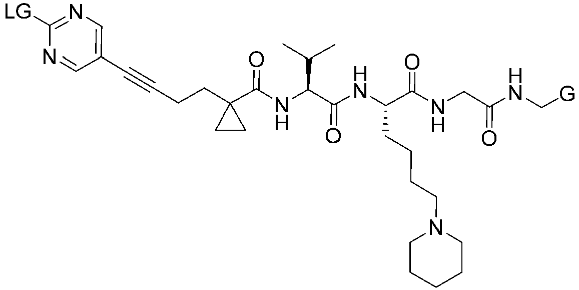 |
| 47 | 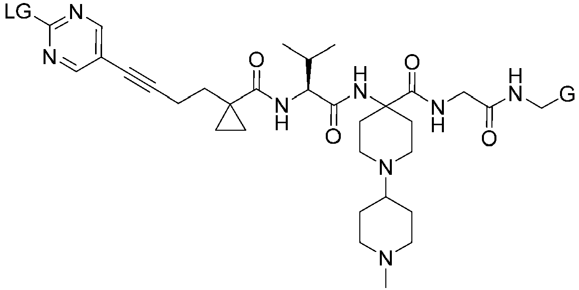 |
| 48 | 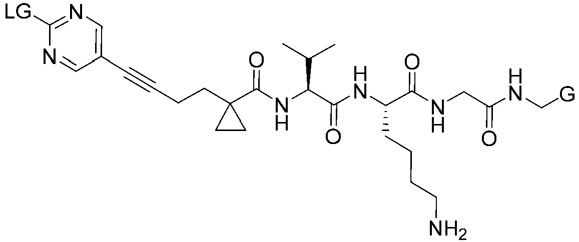 |
| 49 | 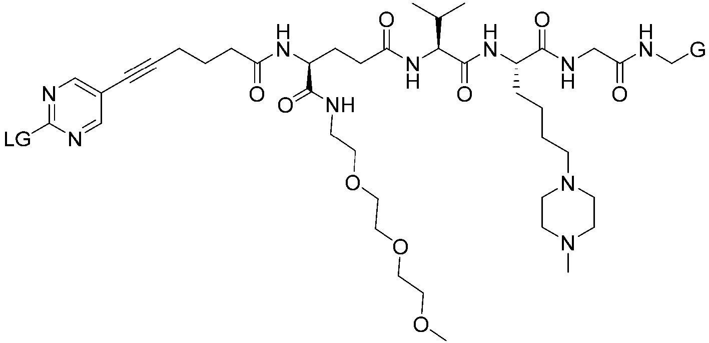 |
| 50 | 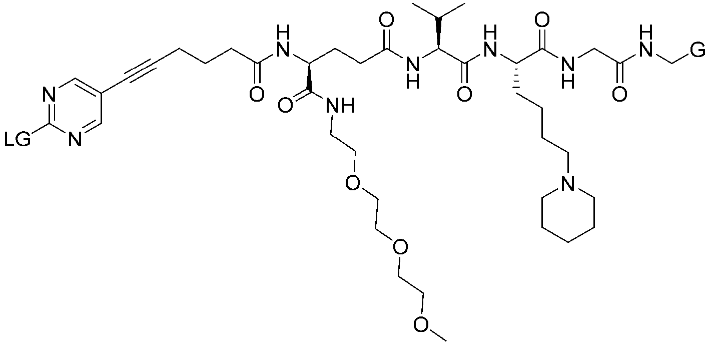 |
| 51 | 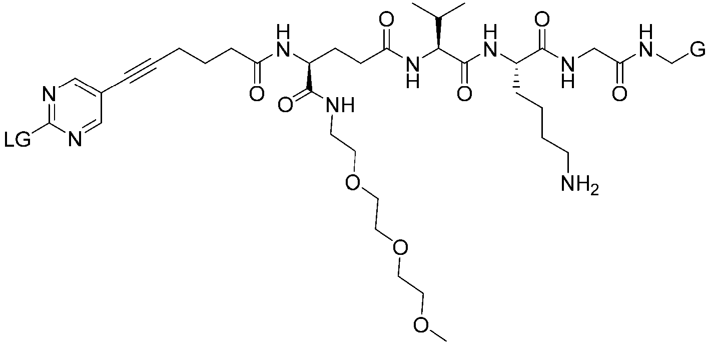 |
| 52 | 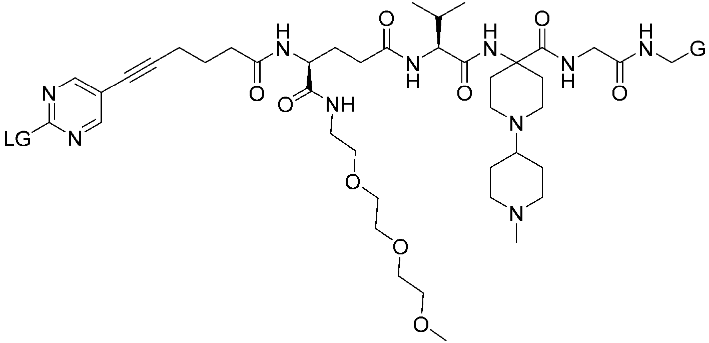 |
| 53 | 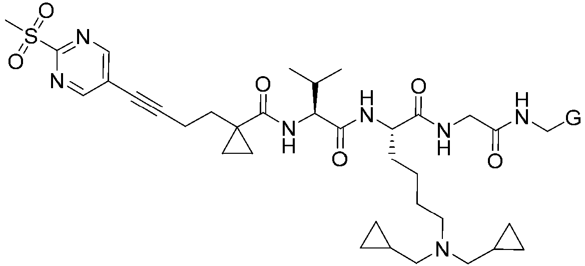 |
| 54 | 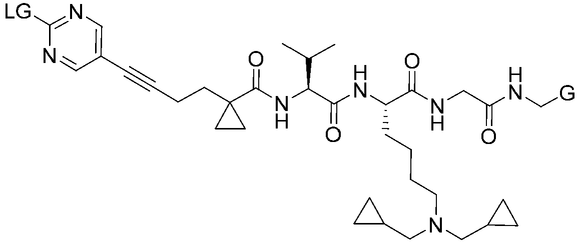 |
| 55 | 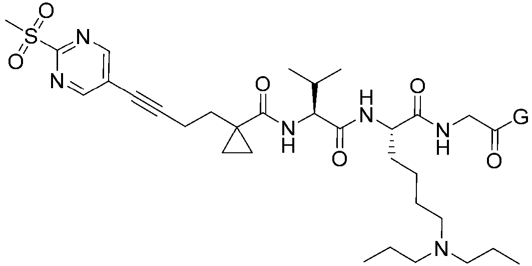 |
| 56 | 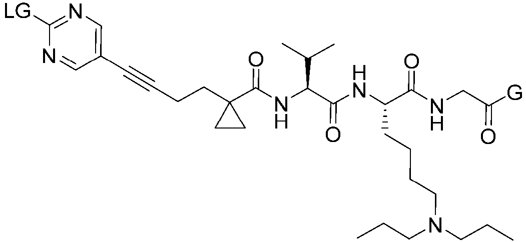 |

22. The ligand-drug conjugate, or a stereoisomer, racemate, tautomer, isotopologue, isotope-labeled compound, N-oxide, prodrug, solvate, or pharmaceutically acceptable salt thereof according to any one of claims 1-20, wherein the conjugate has a structure as shown in the following formula: 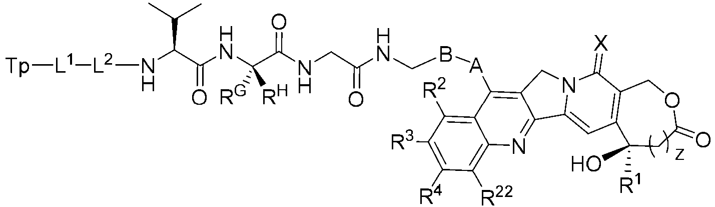 wherein, R¹, R², R²², R³, R⁴, R^{G}, R^{H}, A, B, X, Z, L¹, L² and Tp have definitions as described in any one of claims 1-20.

23. The ligand-drug conjugate, or a stereoisomer, racemate, tautomer, isotopologue, isotope-labeled compound, N-oxide, prodrug, solvate, or pharmaceutically acceptable salt thereof according to any one of claims 1-20, wherein the conjugate has a structure as shown in the following formula: 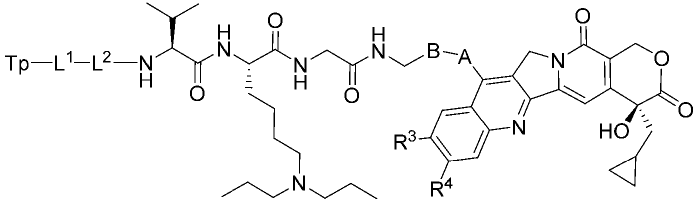 wherein, A, B, R³, R⁴, L¹, L² and Tp have definitions as described in any one of claims 1-20.

24. The ligand-drug conjugate, or a stereoisomer, racemate, tautomer, isotopologue, isotope-labeled compound, N-oxide, prodrug, solvate, or pharmaceutically acceptable salt thereof according to any one of claims 1-20, wherein the conjugate has a structure as shown in the following formula: 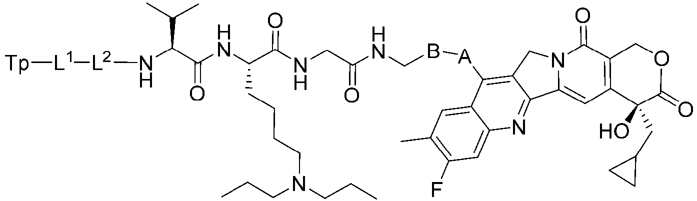 wherein, A, B, L¹, L² and Tp have definitions as described in any one of claims 1-20.

25. The ligand-drug conjugate, or a stereoisomer, racemate, tautomer, isotopologue, isotope-labeled compound, N-oxide, prodrug, solvate, or pharmaceutically acceptable salt thereof according to any one of claims 1-20, wherein the conjugate is selected from one of the followings: 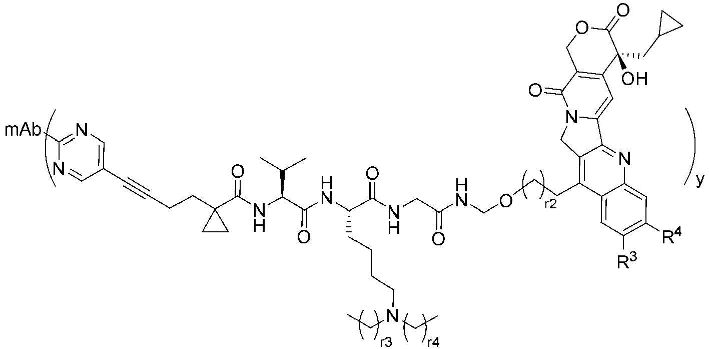 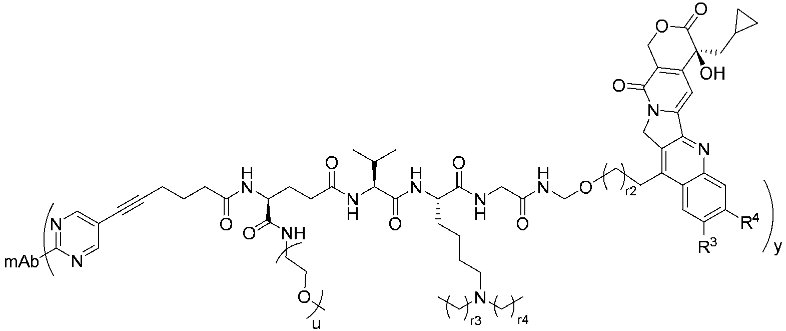 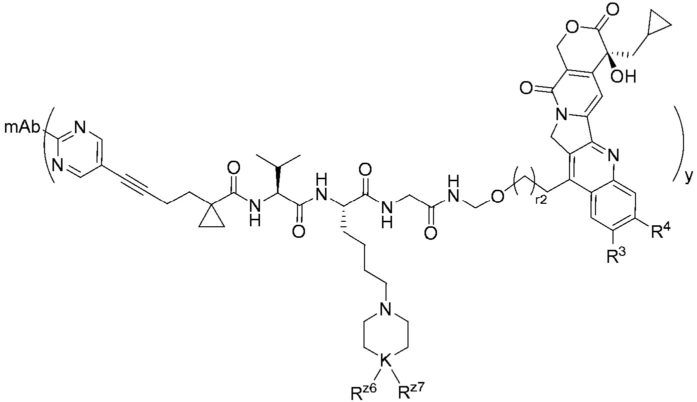 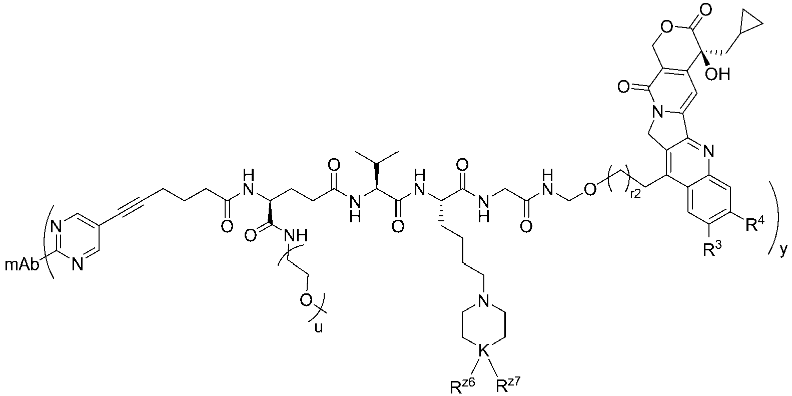 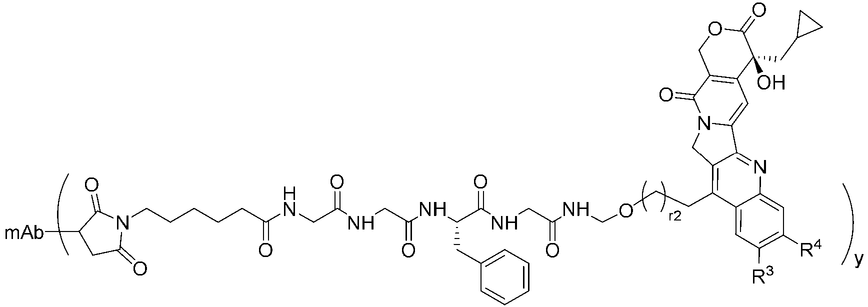 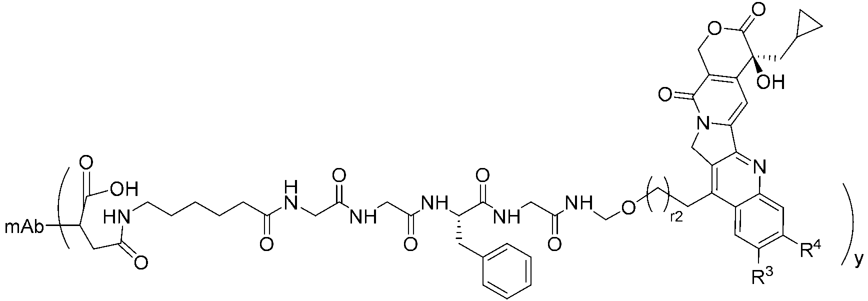 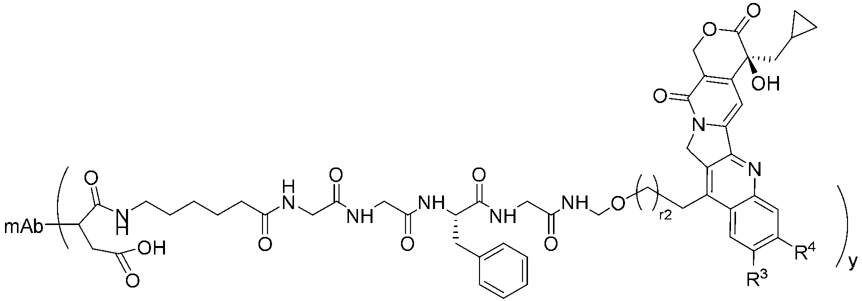 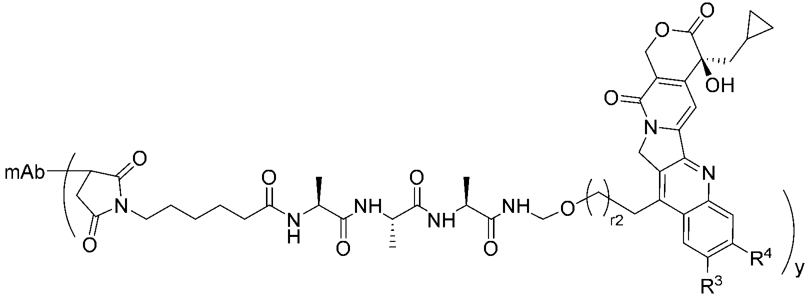 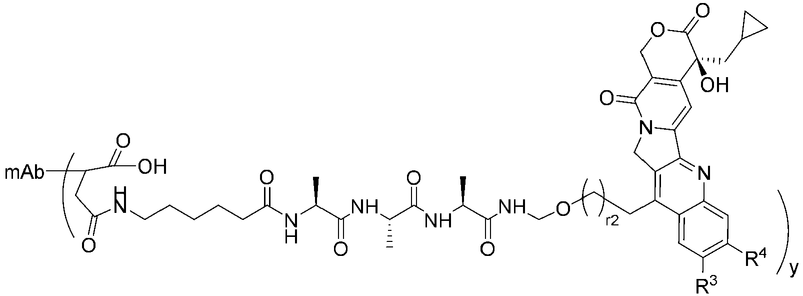 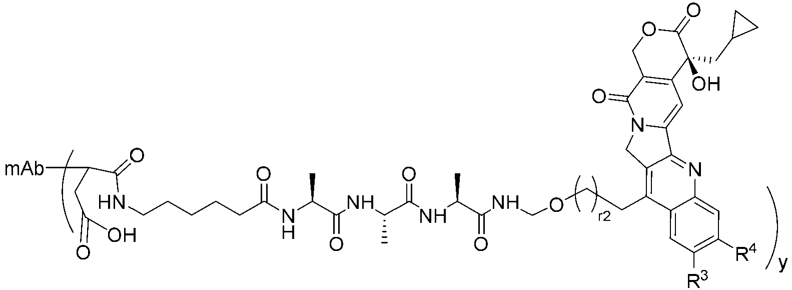 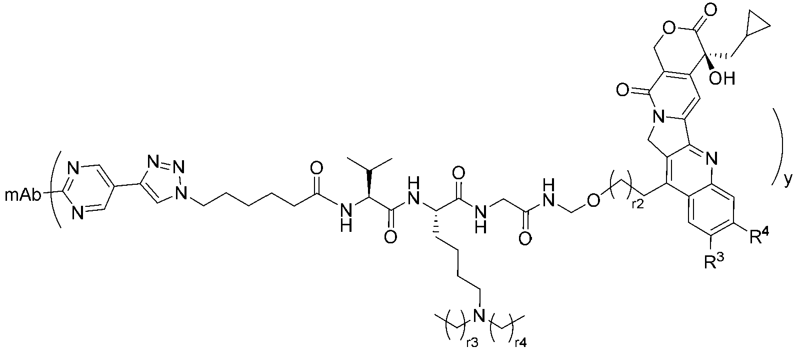 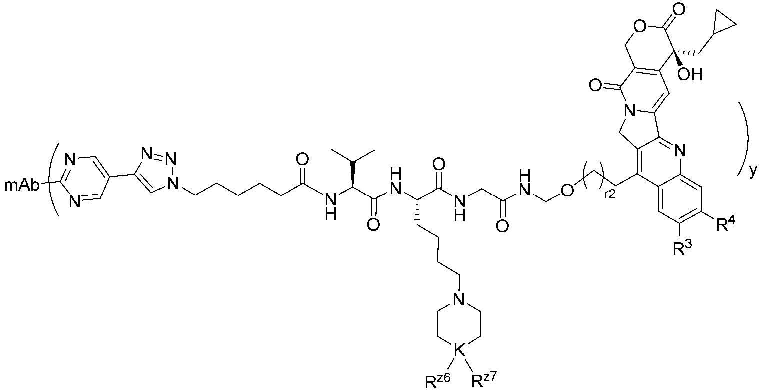 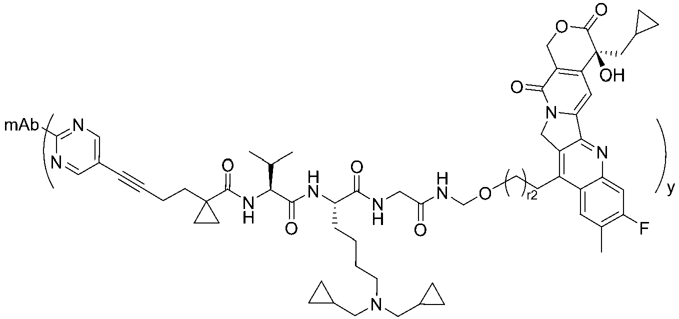
wherein, R³ and R⁴, independently of one another, have definitions as described in any one of claims 1-20;
u is an integer selected from 0 to 10;
r2 is an integer selected from 0 to 4; r3 and r4 are independently an integer selected from 0 to 3;
K is C or N;
R^{z6} and R^{z7} are identical or different, independently selected from hydrogen, amino, alkylamino or dialkylamino, alkyl, alkyloxy, cycloalkyl, cycloalkylalkyl, or cycloalkyloxy;
alternatively, R^{z6} and R^{z7}, together with the atoms to which they are attached form a ring structure; preferably, form a 5-6 membered heterocyclyl optionally substituted with a C₁₋₄ alkyl, preferably the 5-6 membered heterocyclyl is piperidinyl or piperazinyl;
mAb represents a monoclonal antibodies;
y represents the average number of small molecule drugs attached to each mAb (DAR), which may be selected from an integer or decimal, e.g., an integer or decimal from 1 to 50, an integer or decimal from 1 to 20, or an integer or decimal from 1 to 10.

26. The ligand-drug conjugate, or a stereoisomer, racemate, tautomer, isotopologue, isotope-labeled compound, N-oxide, prodrug, solvate, or pharmaceutically acceptable salt thereof according to any one of claims 1-25, wherein the conjugate is selected from one of the followings:
y represents the average number of small molecule drugs attached to each monoclonal antibody (DAR), which may be selected from an integer or decimal, e.g. an integer or decimal of 1 to 50, an integer or decimal of 1 to 20, or an integer or decimal of 1 to 10;

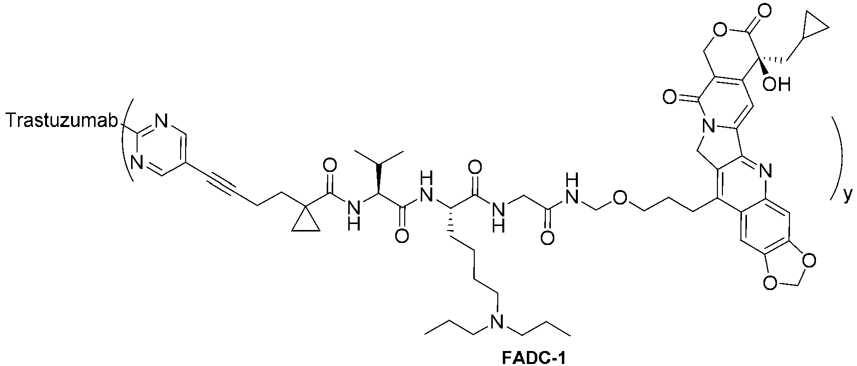

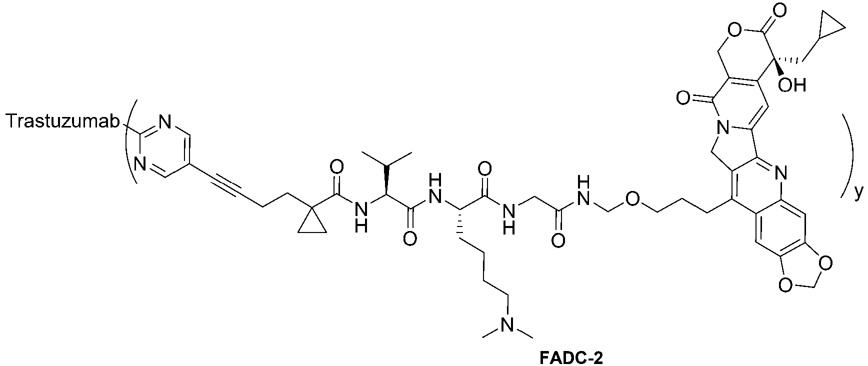

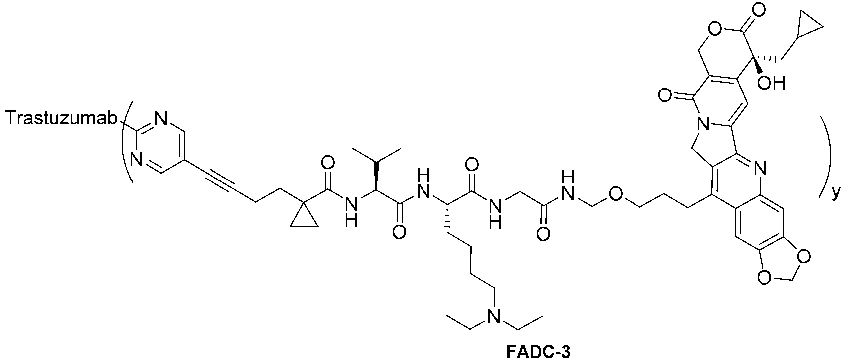

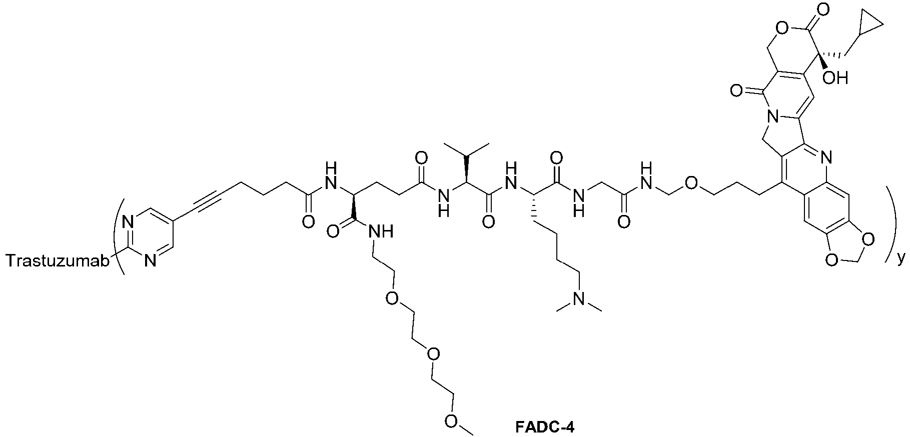

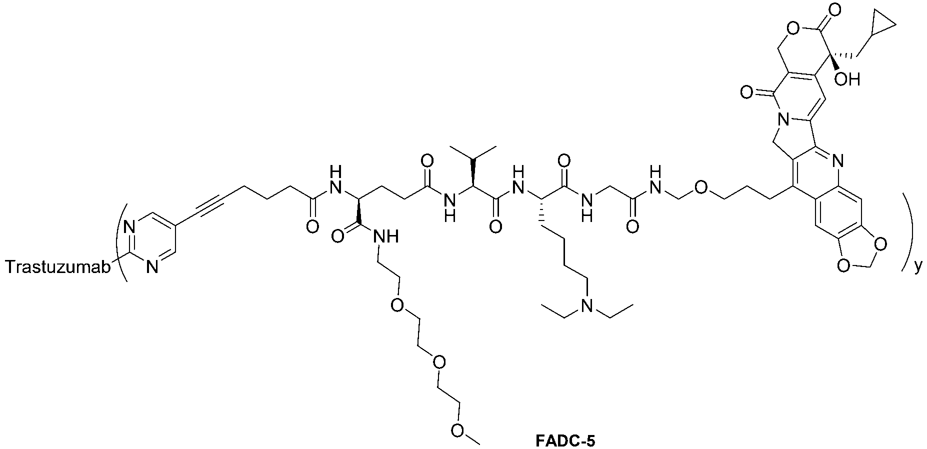

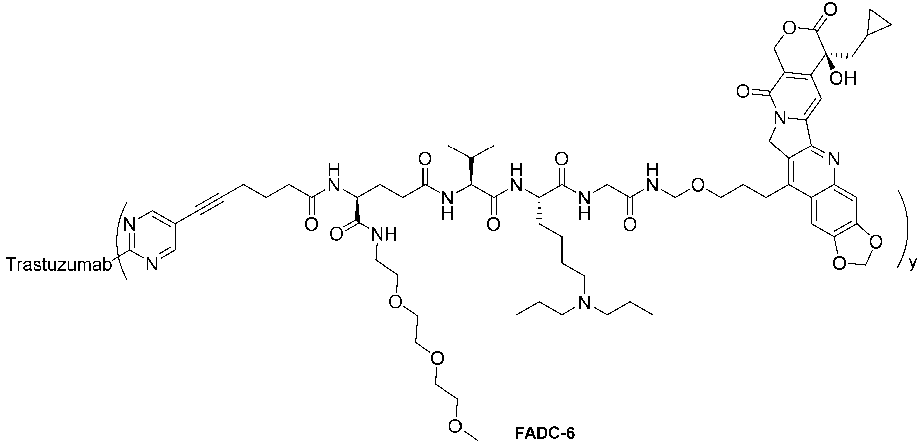

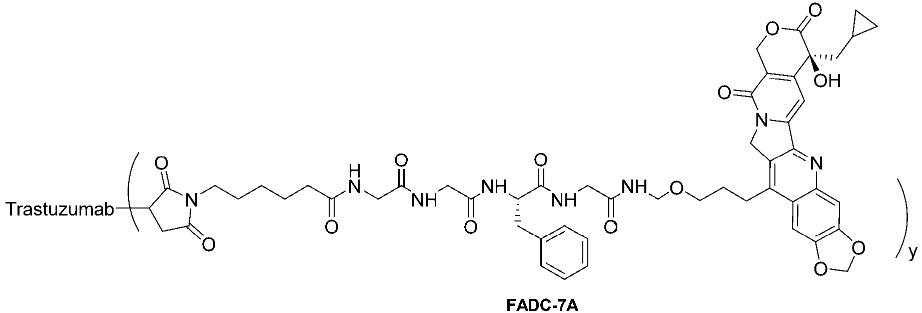

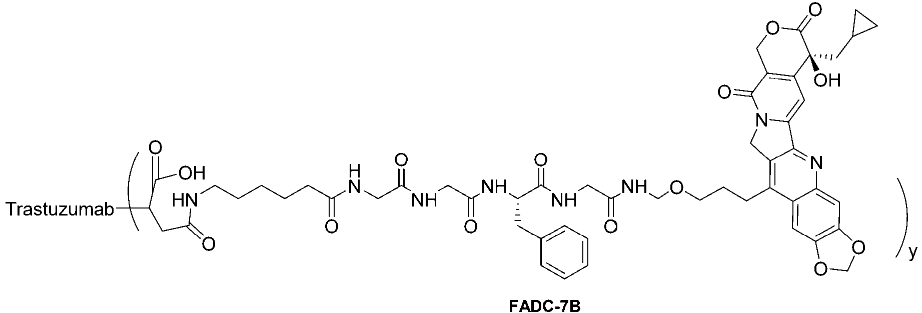

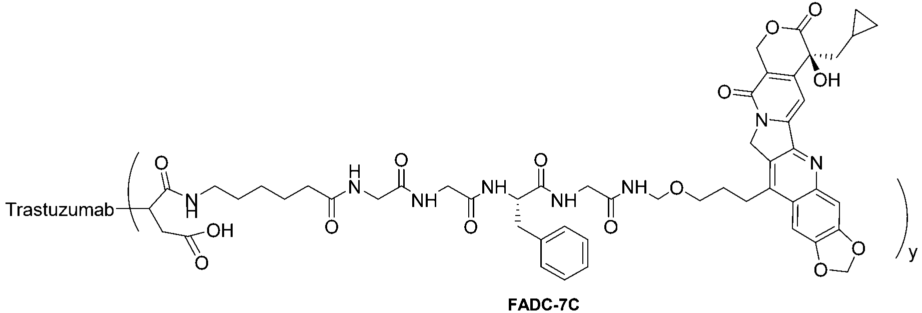

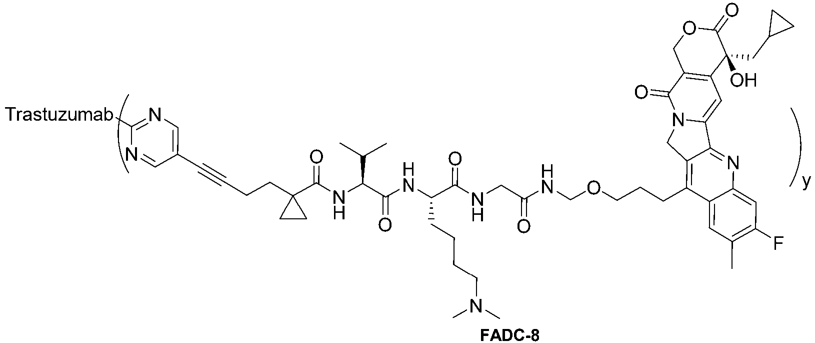

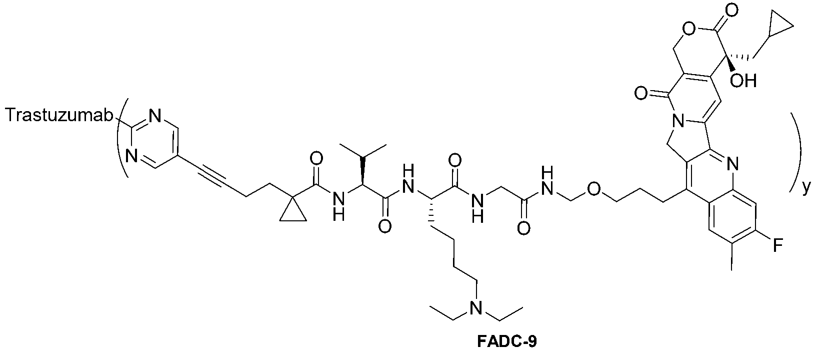

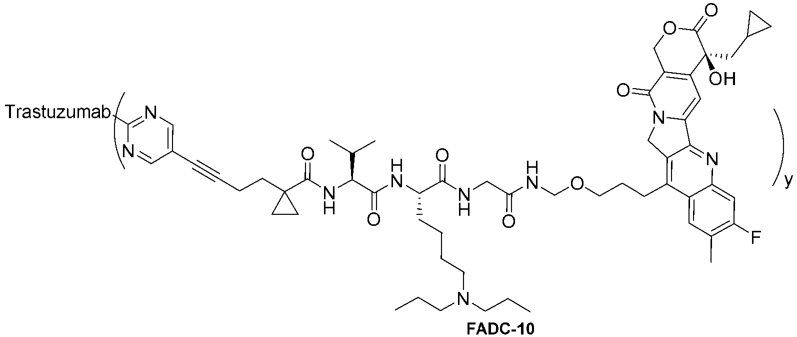

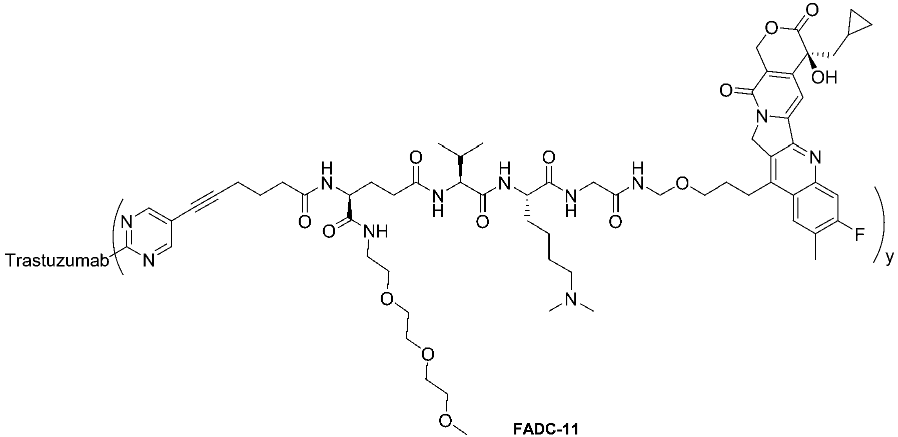

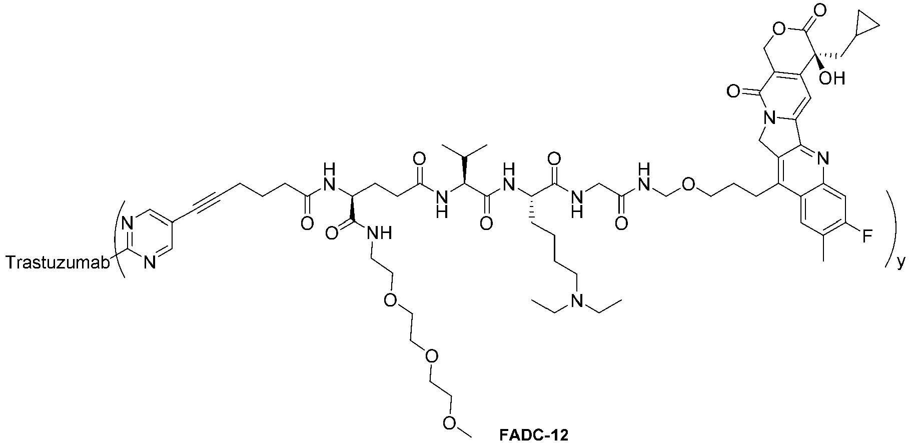

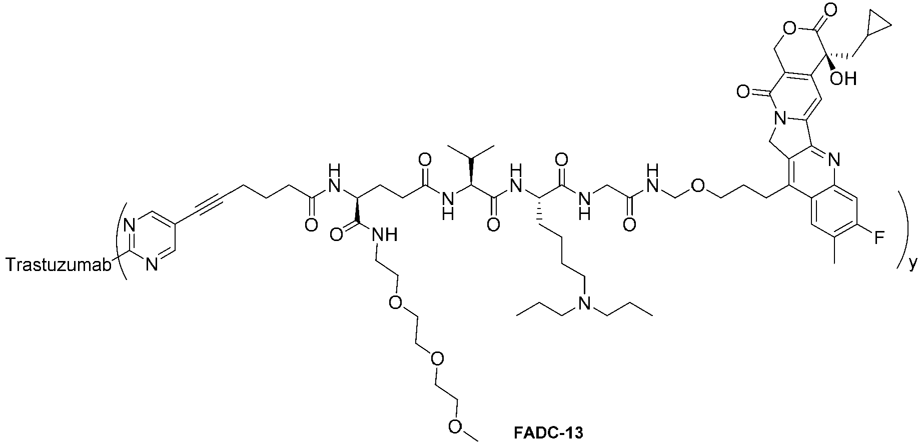

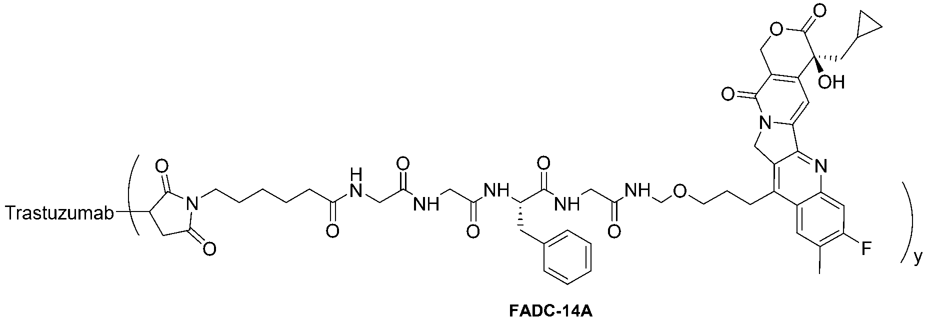

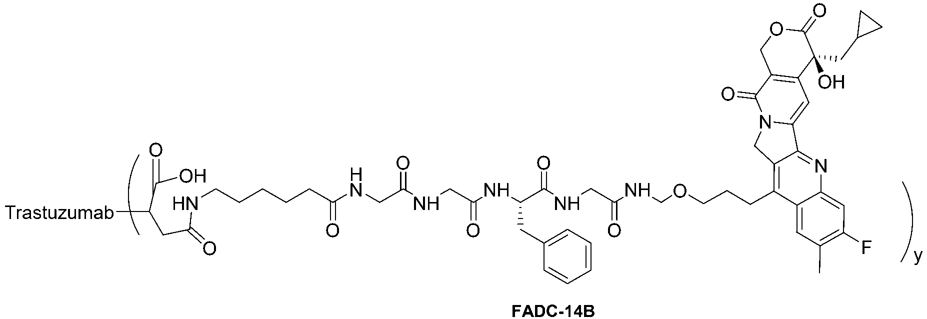

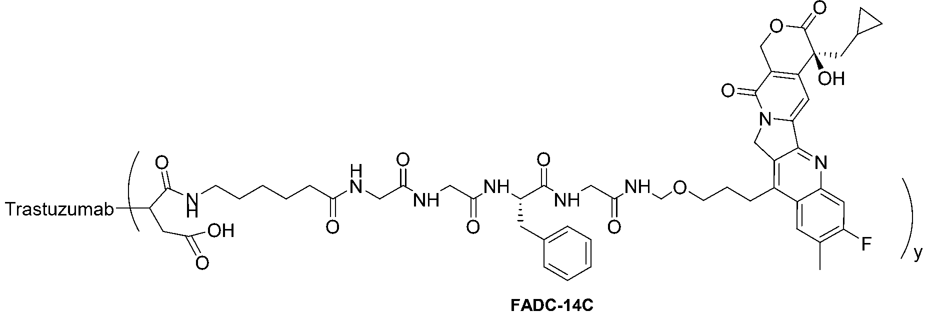

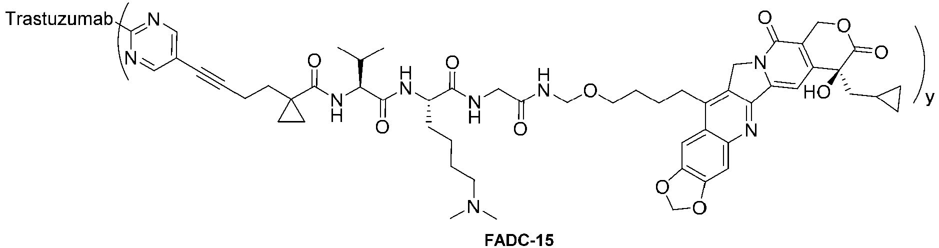

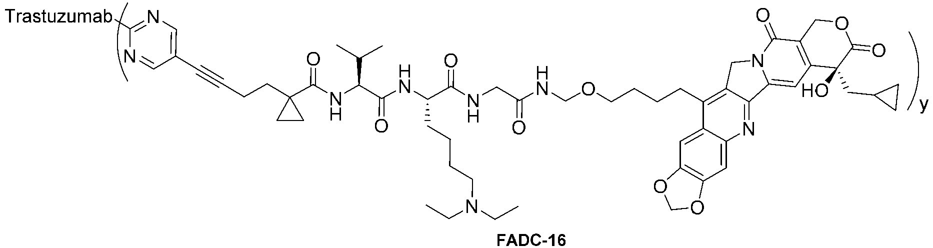

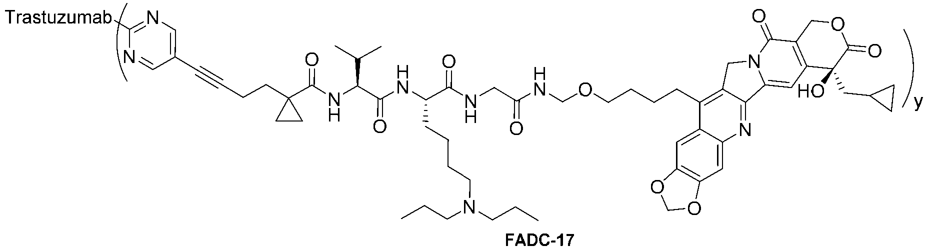

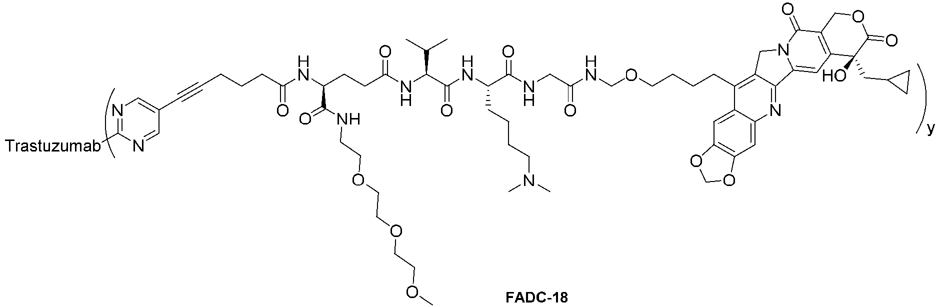

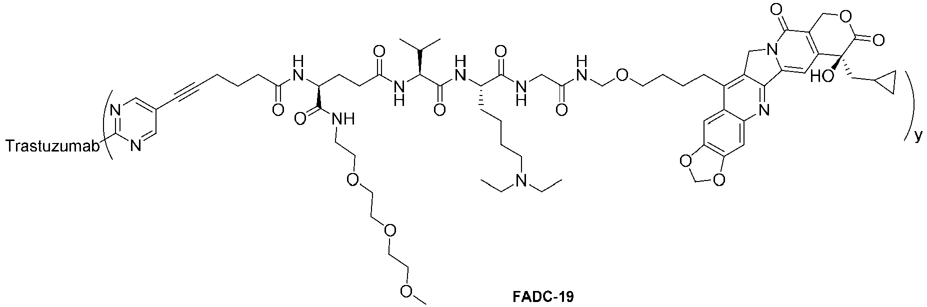

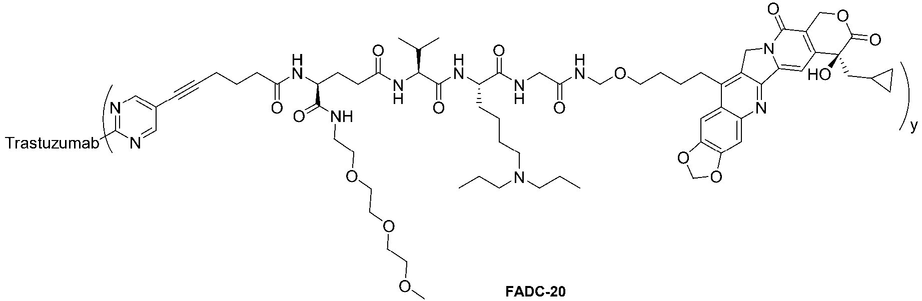

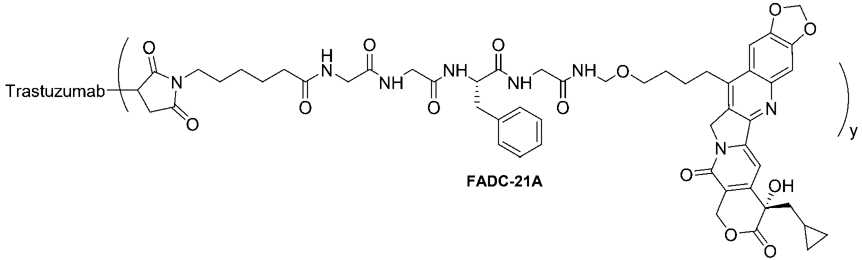

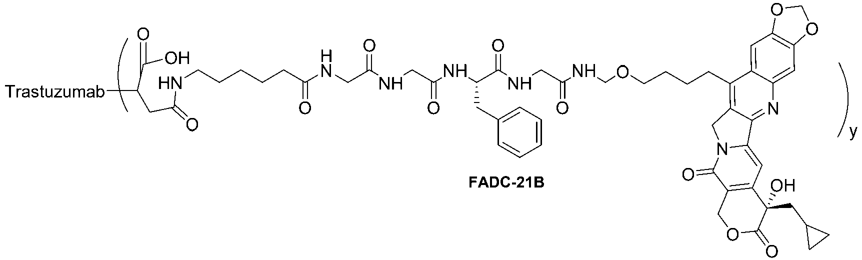

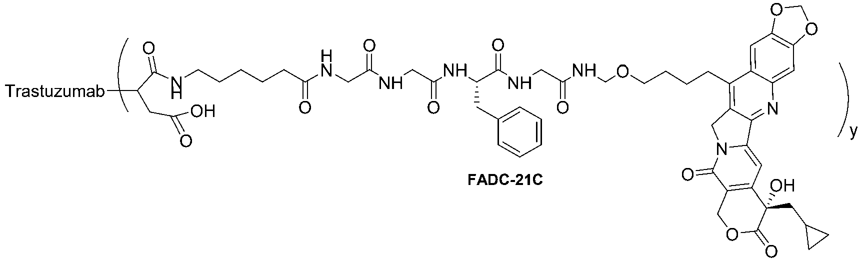

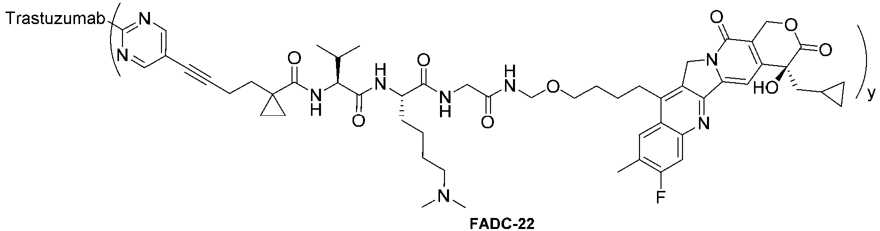

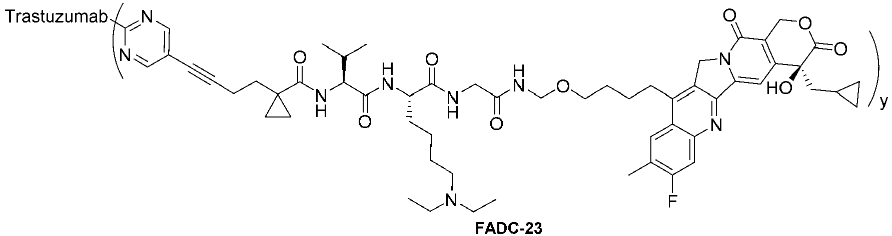

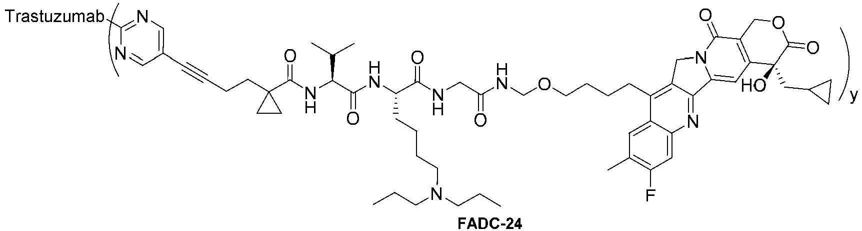

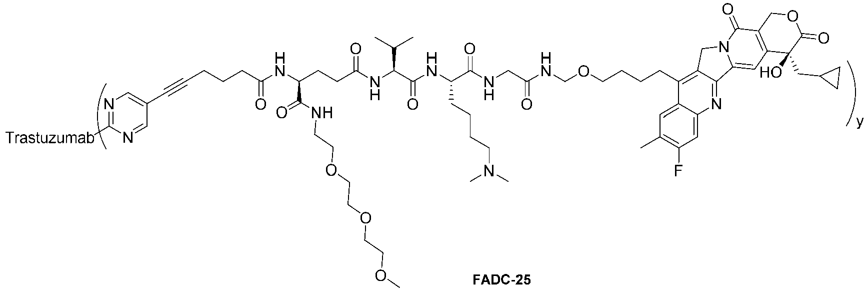

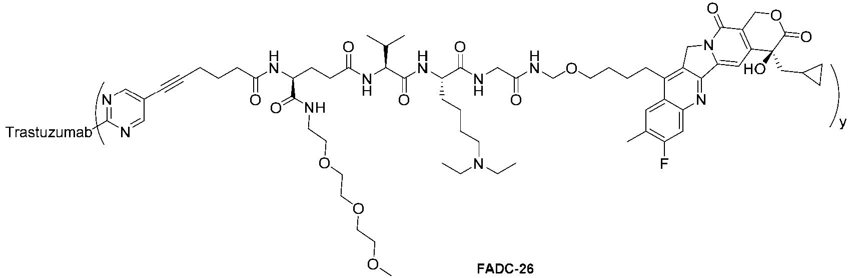

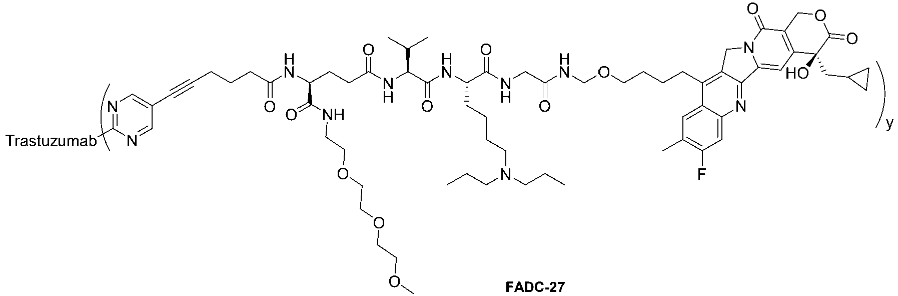

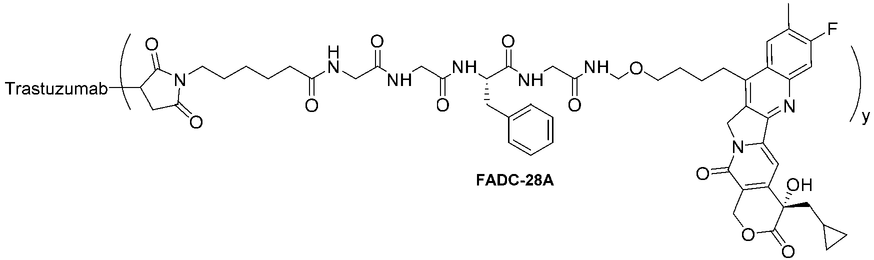

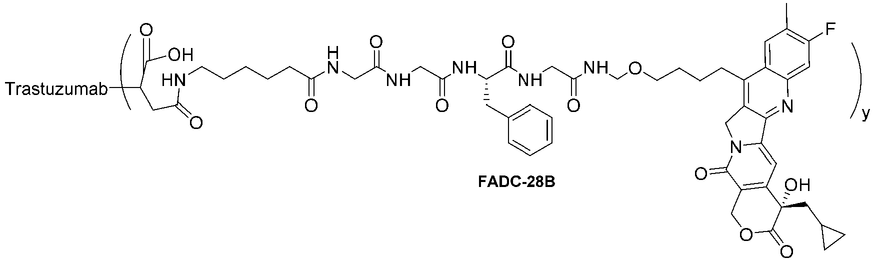

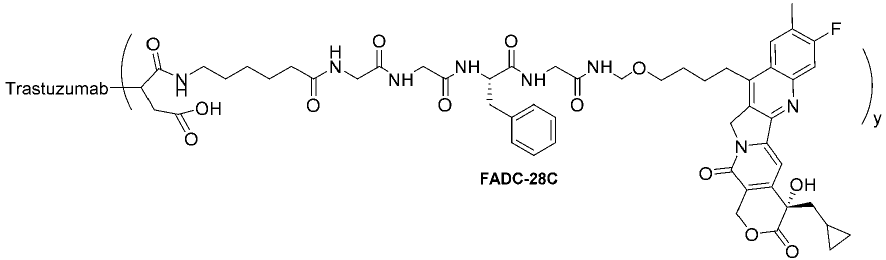

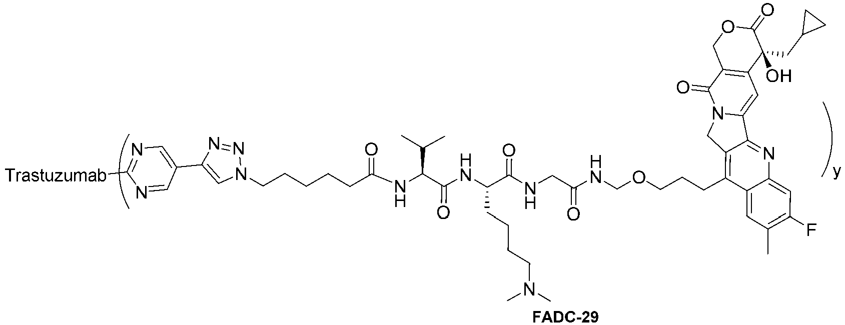

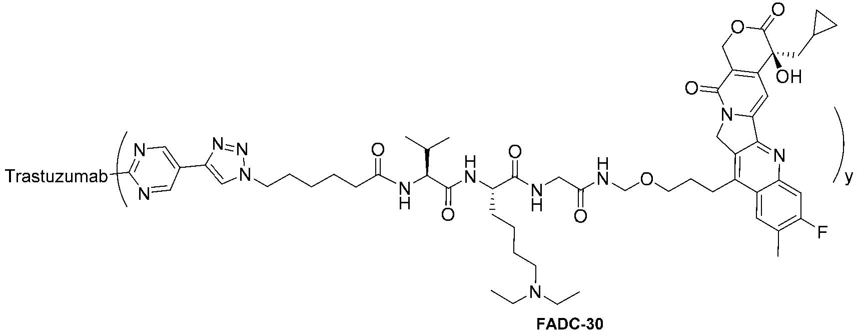

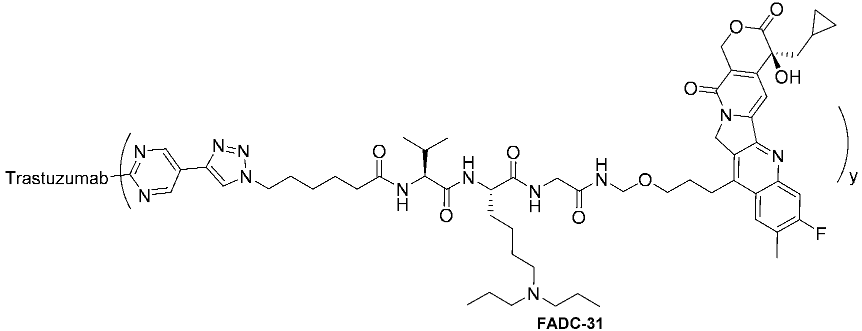

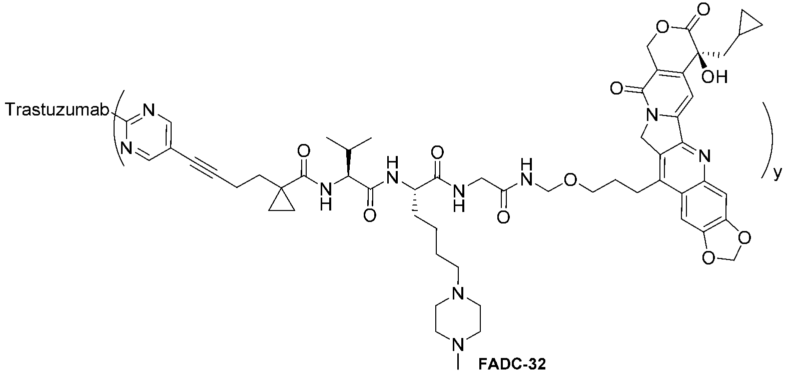

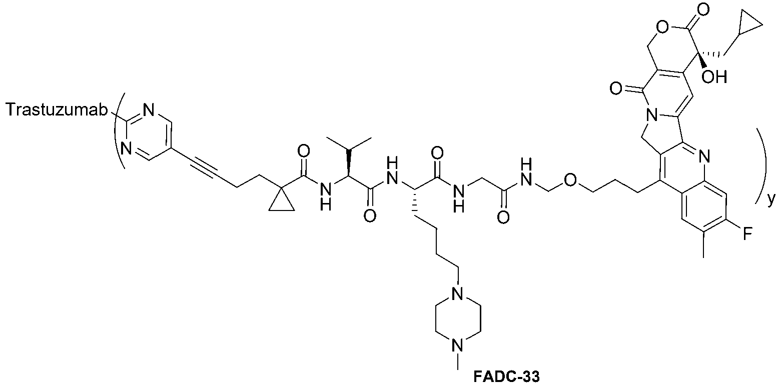

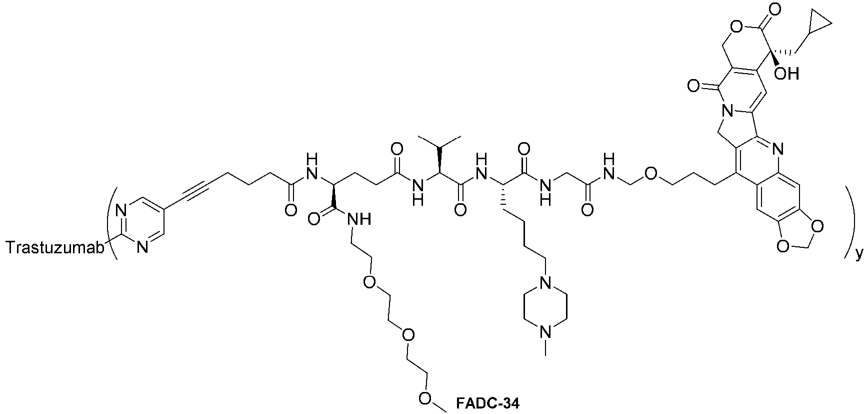

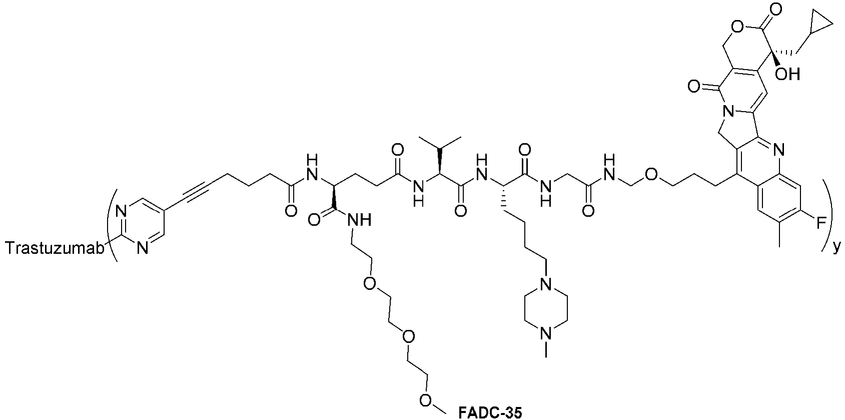

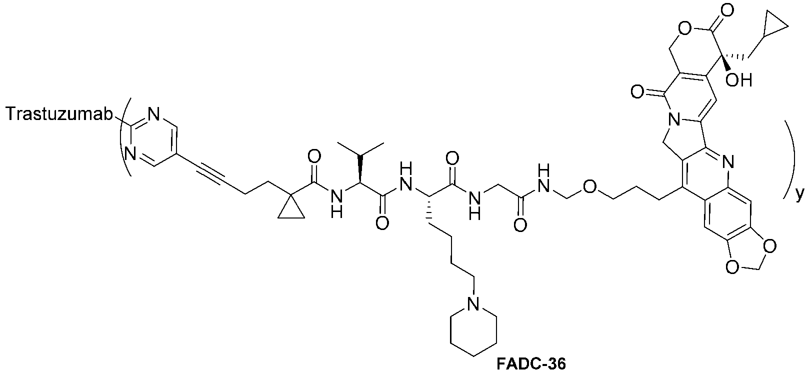

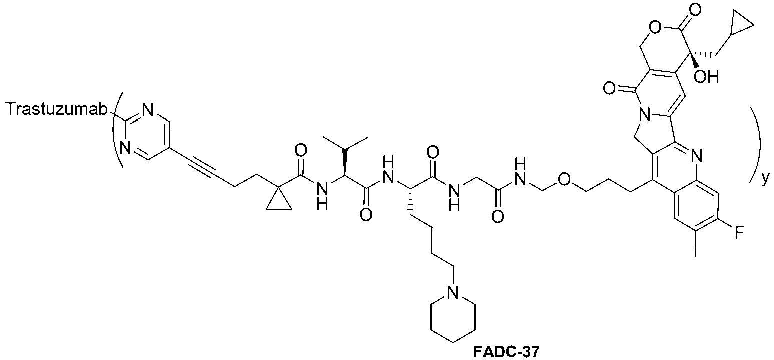

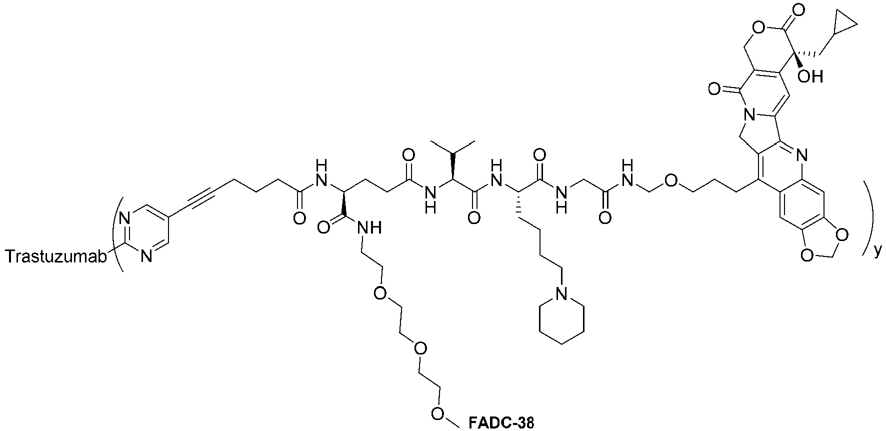

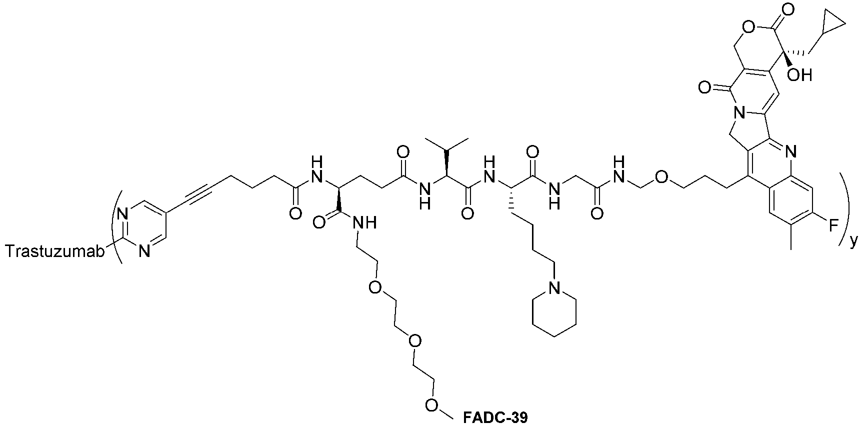

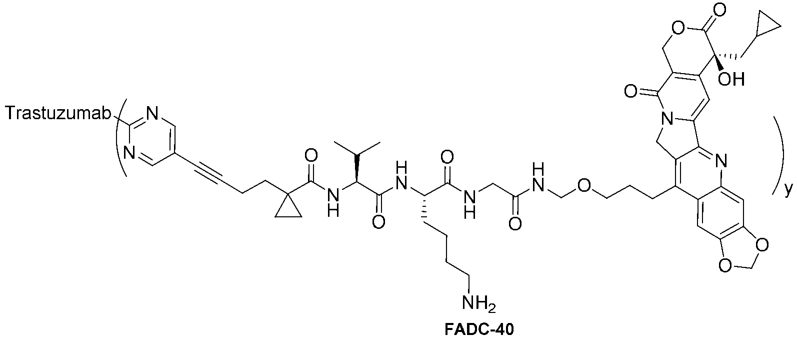

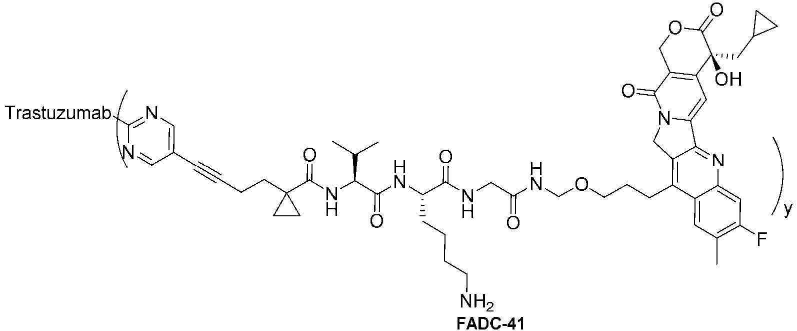

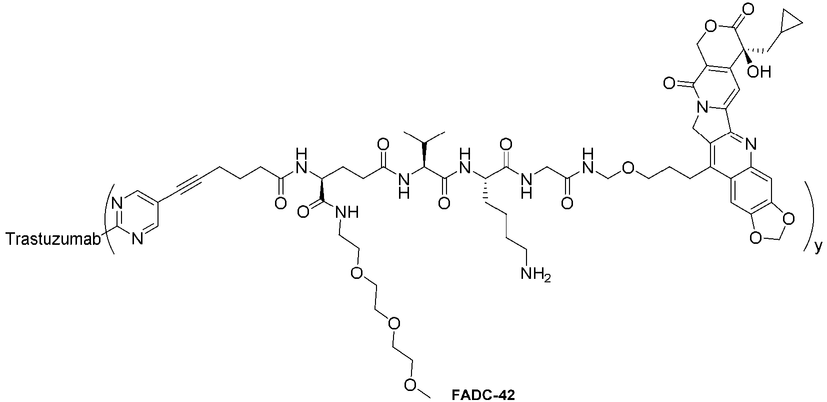

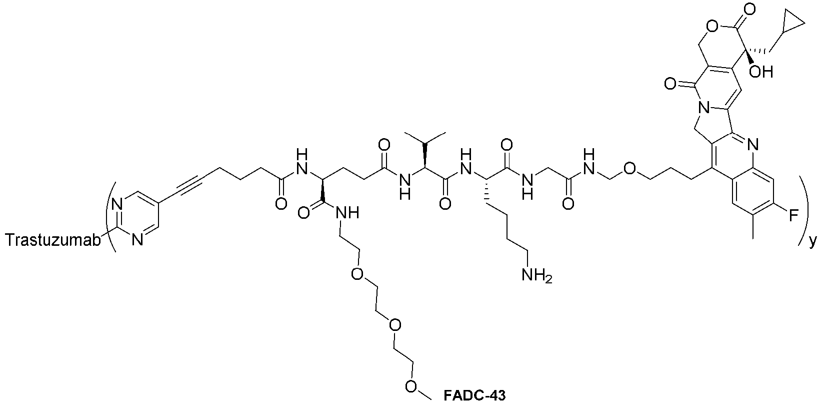

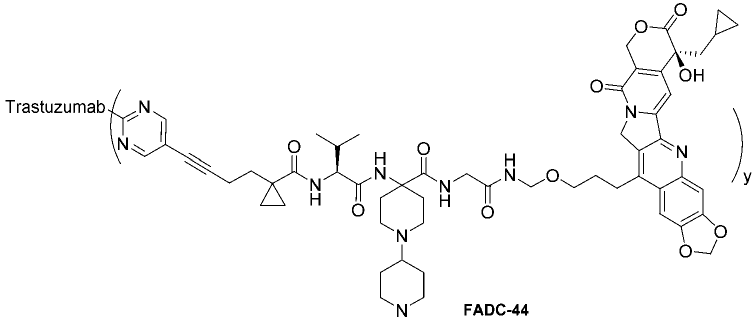

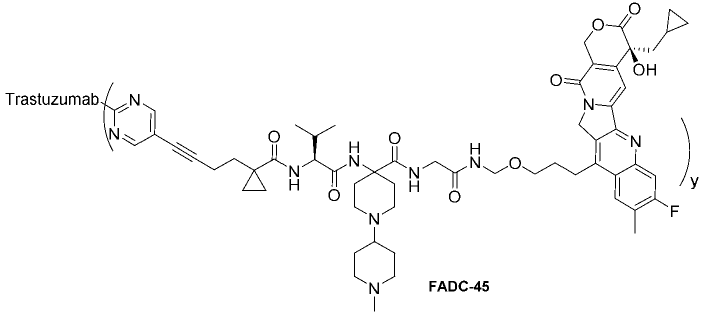

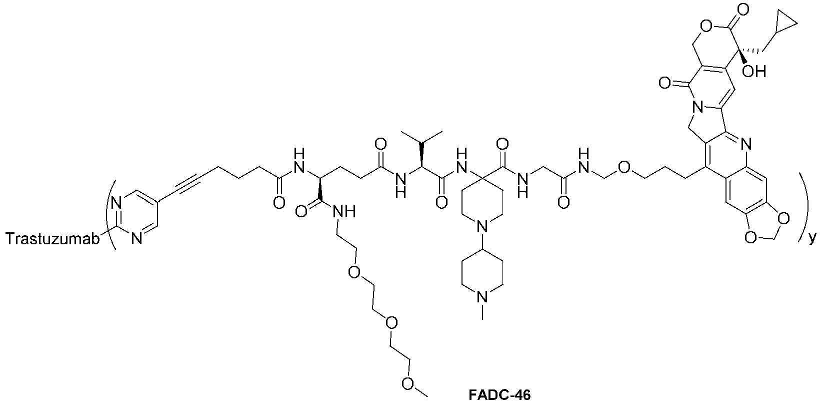

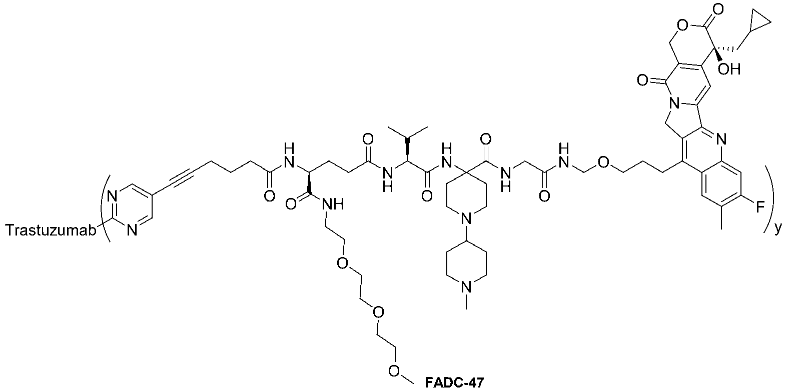

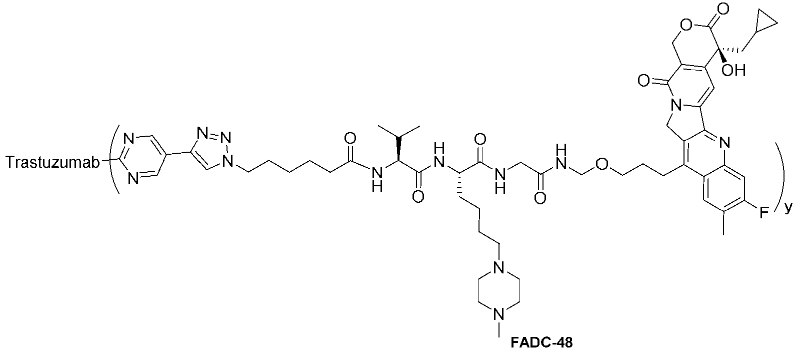

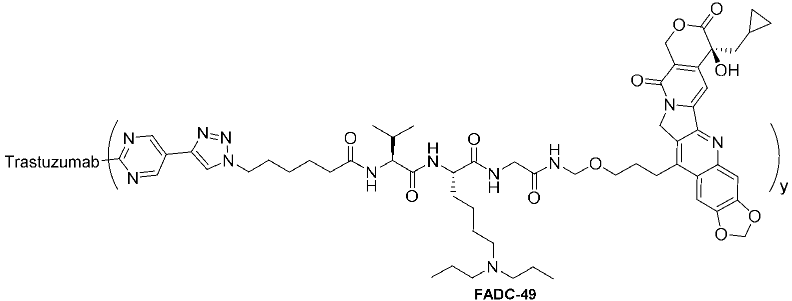

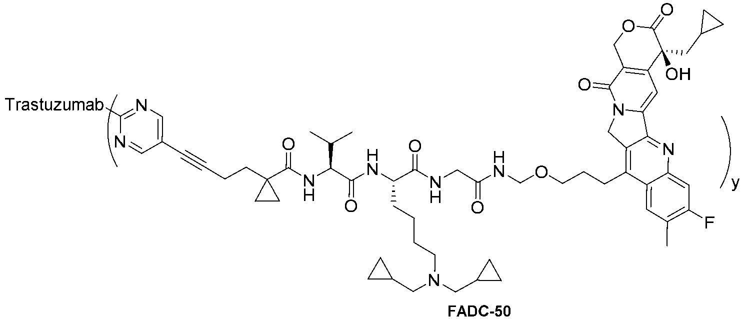

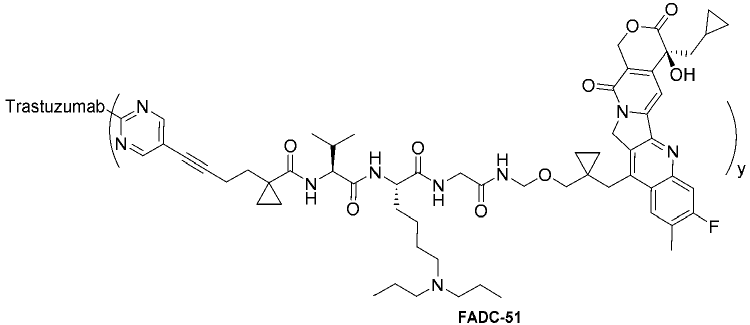

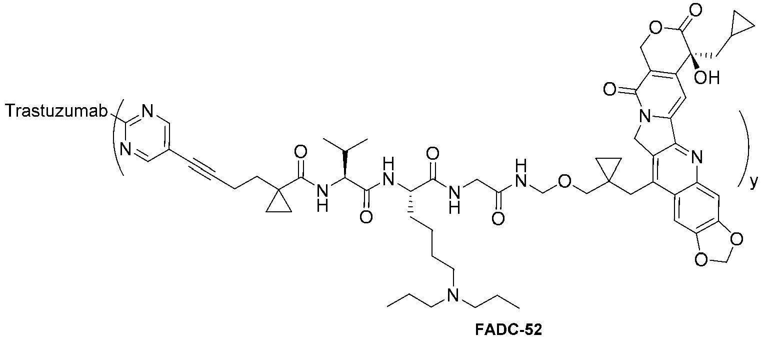

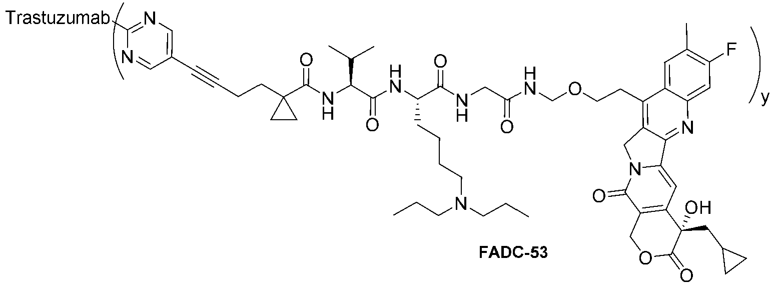

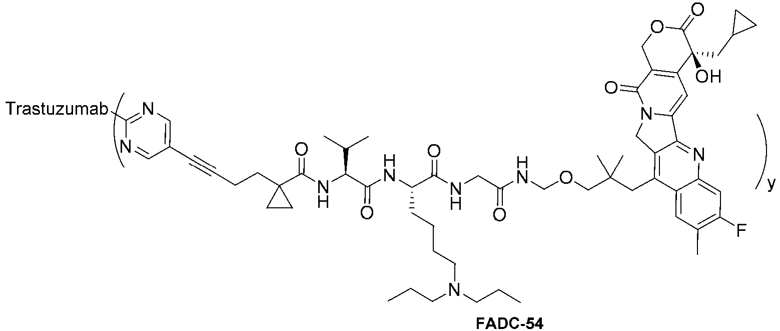

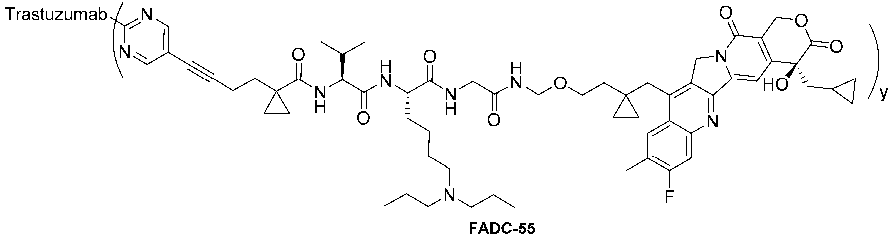

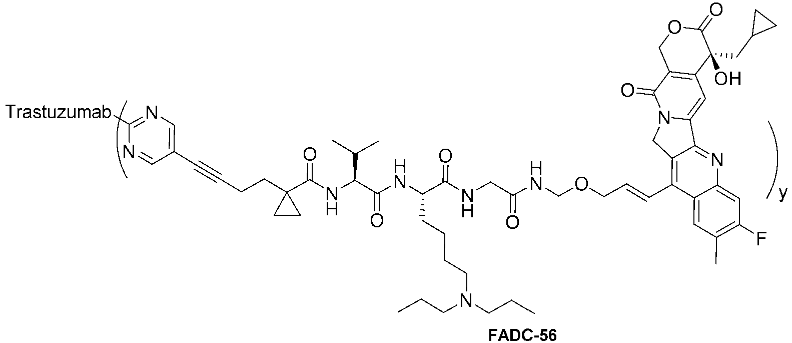

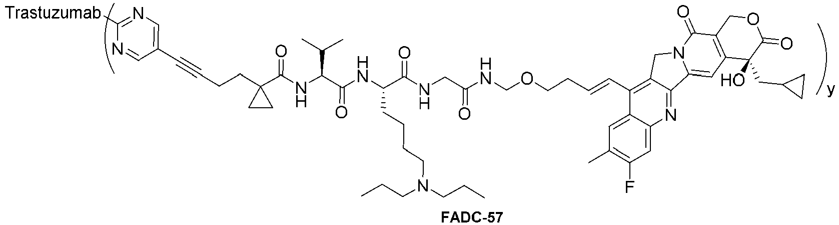

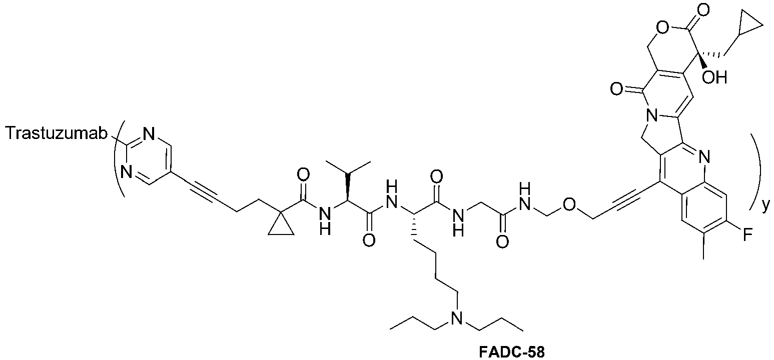

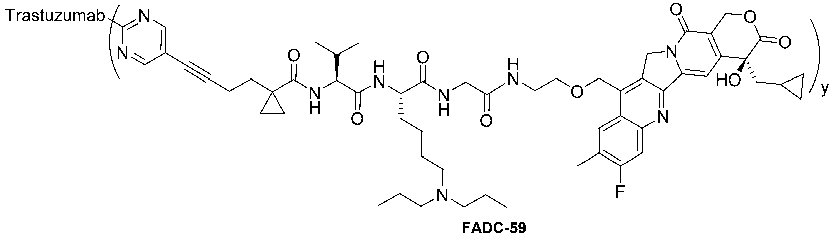

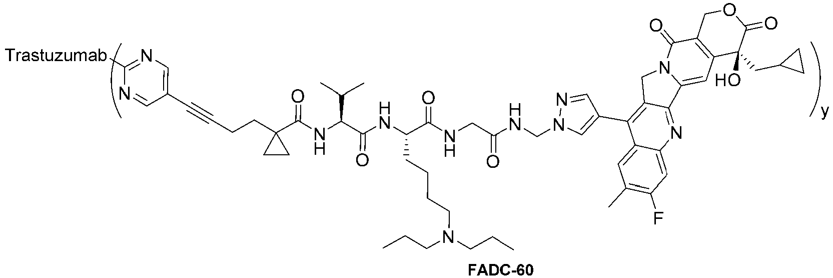

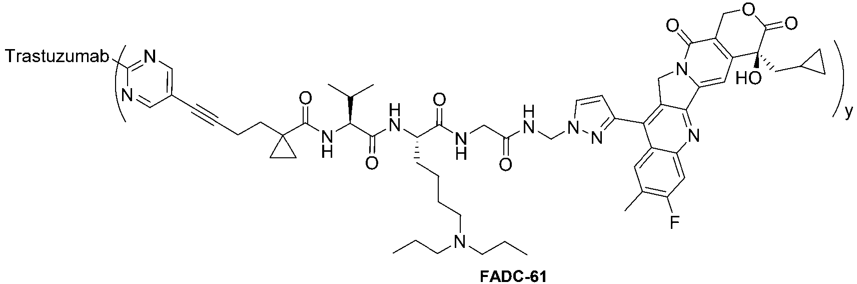

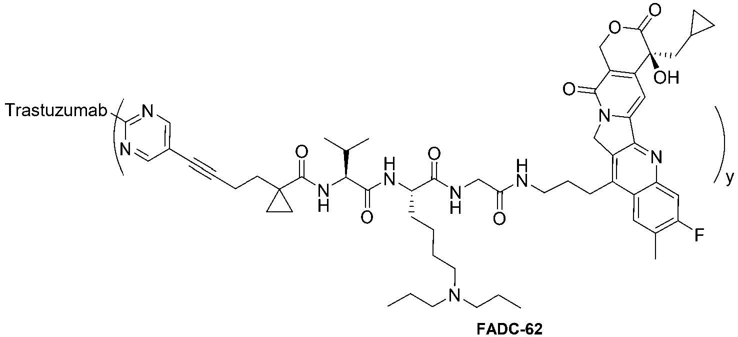

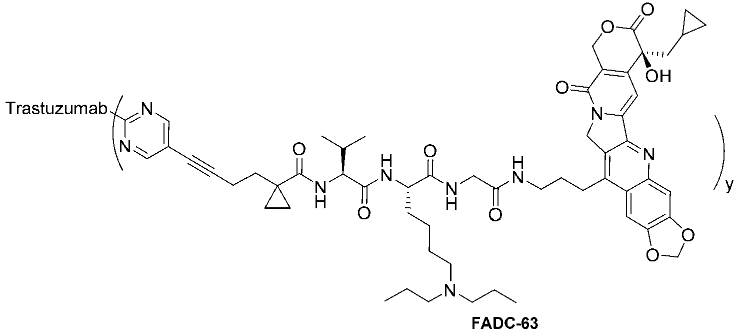

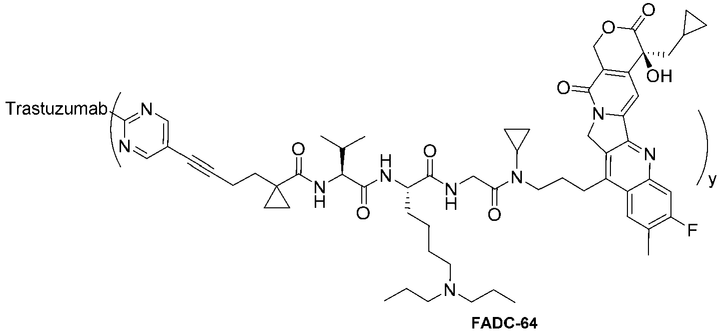

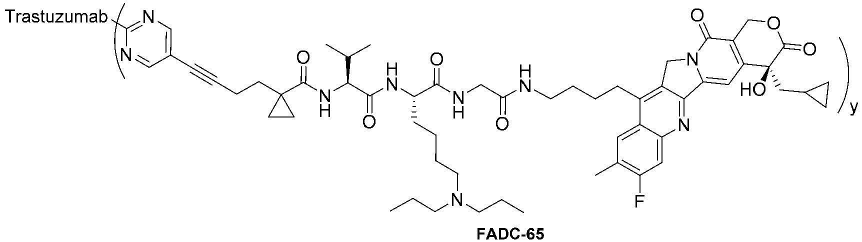

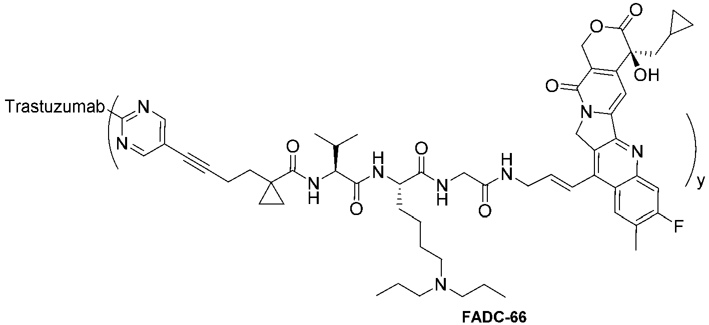

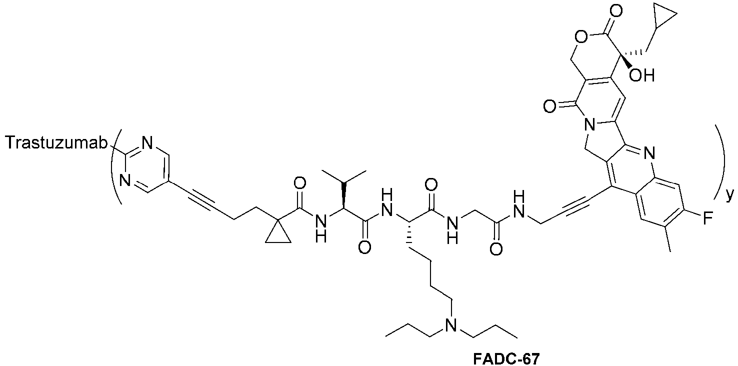

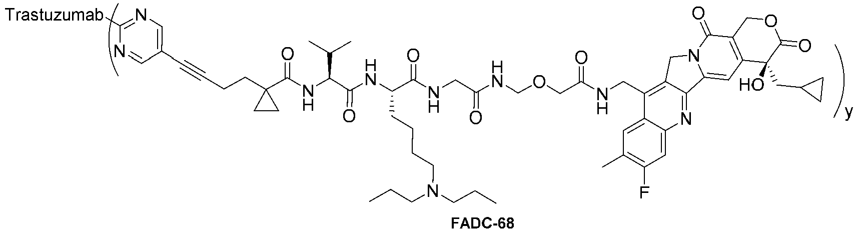

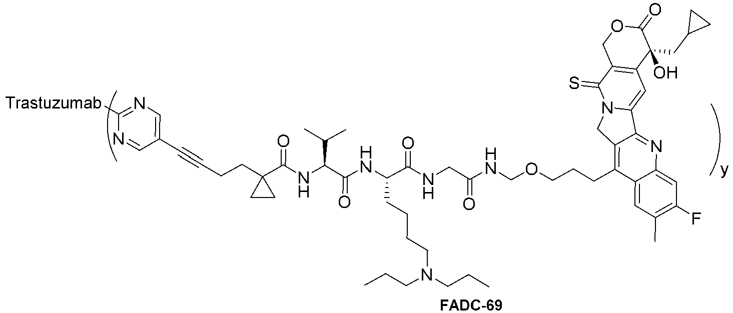

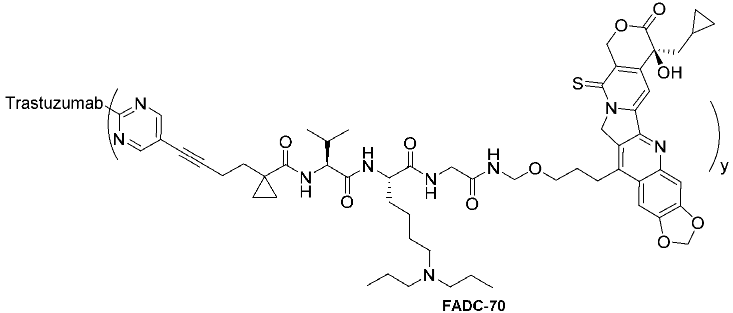

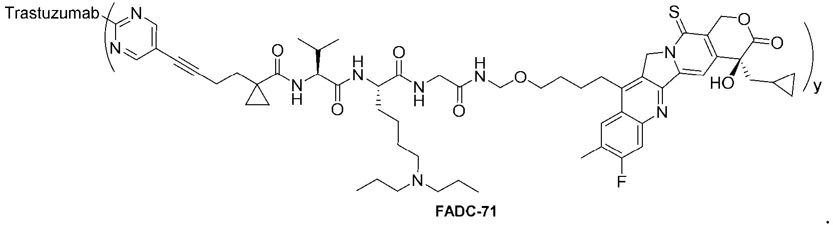

27. A method for preparing the ligand-drug conjugate, or a stereoisomer, racemate, tautomer, isotopologue, isotope-labeled compound, N-oxide, prodrug, solvate, or pharmaceutically acceptable salt thereof according to any one of claims 1-26, wherein:
the method is Method A, comprising the following steps:
step 1: providing a linker as shown in L1'-L²-L³-L^{4'} (L');
preferably, in the linker,
L^{4'} is acetate of 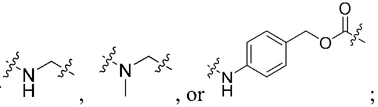
further preferably, L^{4'} is an acetate of 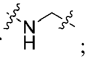
step 2: coupling the linker with a compound of formula (GH) to obtain a coupled intermediate of L¹'-L²-L³-L⁴-G(C');
wherein the structure of formula (GH) is: 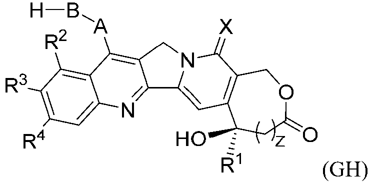
wherein, A, B, Z, X, R¹, R², R³, R⁴, L^{1'}, L¹, L², L³, L⁴ and L^{4'} independently have the definitions as described in any one of claims 1-26;
preferably, the preparation method further comprises step 3: coupling the coupled intermediate of formula (C') with the targeting moiety Tp;
or Method B, comprising the following steps:
step 1: providing a linker fragment comprising L^{4'},the L^{4'} has the definitions as described above;
step 2: coupling the linker fragment containing L^{4'} with the compound of formula (GH) to obtain a L^{4'}-containing linker fragment-G intermediate;
step 3: providing another linker fragment to react with the above intermediate to form a conjugated intermediate of the structure L^{1'}-L²-L³-L⁴-G (C') containing the complete linker;
preferably, the linker fragment comprising L^{4'} is: 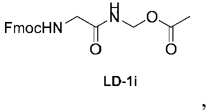 which reacts with GH in which B is -O-to form an ether linkage. 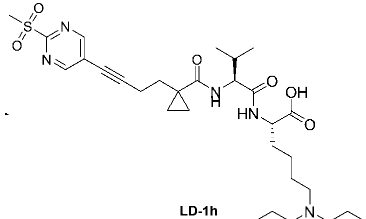
preferably, the another linker fragment is: ;
preferably, the preparation method further comprises step 4:coupling the conjugated intermediate of formula (C ') with the targeting moiety Tp;
optionally, if necessary, functional groups of the reaction substrates may also be protectedwith a protecting group known in the art, to allow the reaction to proceed smoothly, and the protecting group is removed after the reaction is completed.

28. A compound represented by the following formula (GH), or a stereoisomer, racemate, tautomer, isotopologue, isotope-labeled compound, N-oxide, prodrug, solvate, or pharmaceutically acceptable salt thereof: 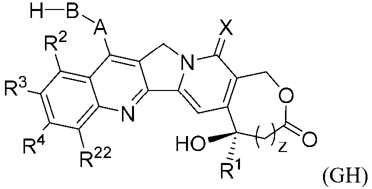 wherein, A, B, X, Z, R¹, R², R²², R³ and R⁴, independently of one another, have the definitions as described in any one of claims 1-26.

29. The compound, or a stereoisomer, racemate, tautomer, isotopologue, isotope-labeled compound, N-oxide, prodrug, solvate, or pharmaceutically acceptable salt thereof according to claim 28, wherein the compound represented by Formula (GH) can be selected from the compound represented by the following Formula (GH-1): 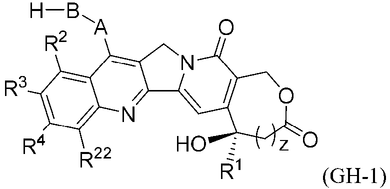 wherein A, B, Z, R¹, R², R²², R³ and R⁴ independently of one another have the definitions as described in claim 28.

30. The compound, or a stereoisomer, racemate, tautomer, isotopologue, isotope-labeled compound, N-oxide, prodrug, solvate, or pharmaceutically acceptable salt thereof according to claim 28, wherein the compound represented by Formula (GH) can be selected from the compound represented by the following Formula (GH-2): 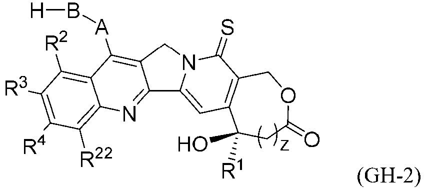 wherein A, B, Z, R¹, R², R²², R³, R⁴, independently of one another, have the definitions as described in claim 28.

31. The compound, or a stereoisomer, racemate, tautomer, isotopologue, isotope-labeled compound, N-oxide, prodrug, solvate, or pharmaceutically acceptable salt thereof according to claim 28, wherein the compound is selected from the following compounds:

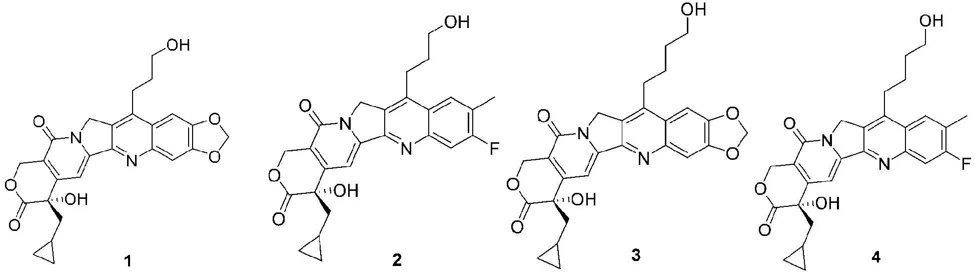

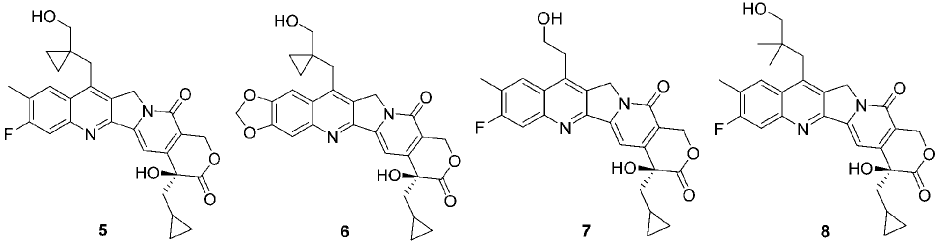

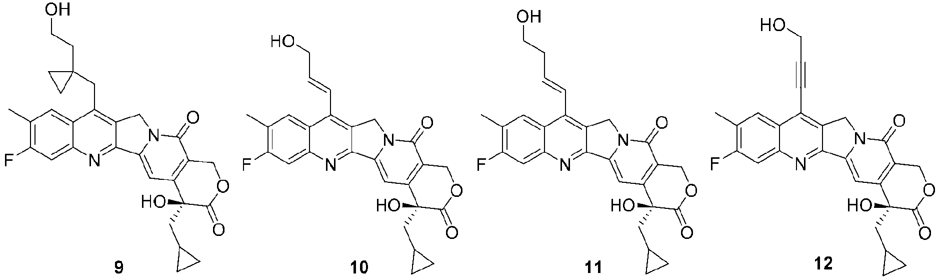

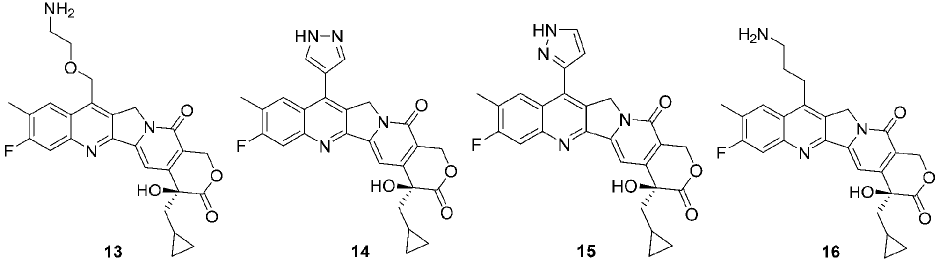

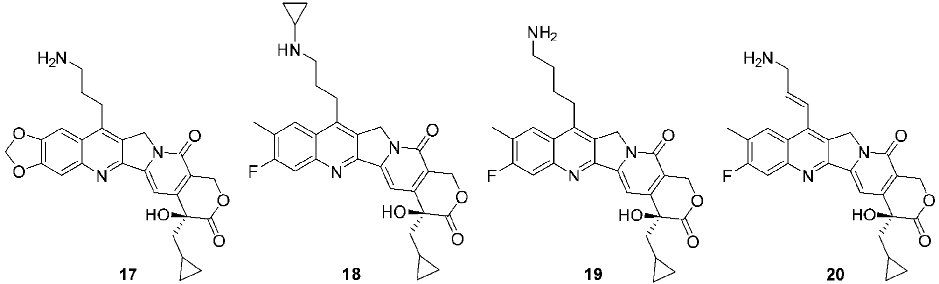

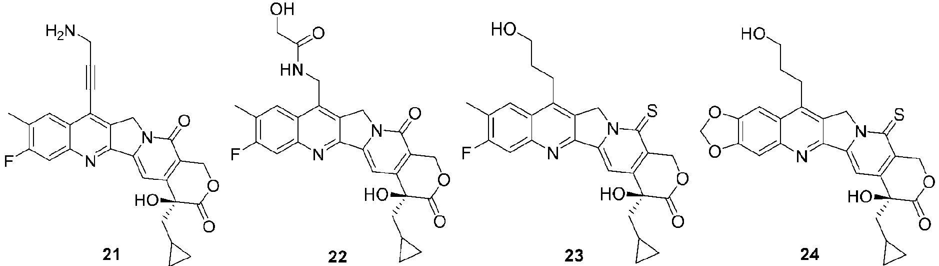

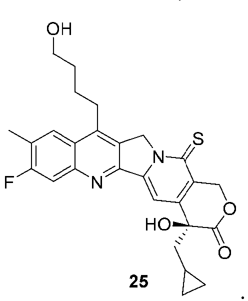

32. A method for preparing the compound of formula (GH), or a stereoisomer, racemate, tautomer, isotopologue, isotope-labeled compound, N-oxide, prodrug, solvate, or pharmaceutically acceptable salt thereof according to claim 28, wherein the method comprises a step of reacting a compound of formula (i) with a compound of formula (ii) to obtain a compound of formula (G'): 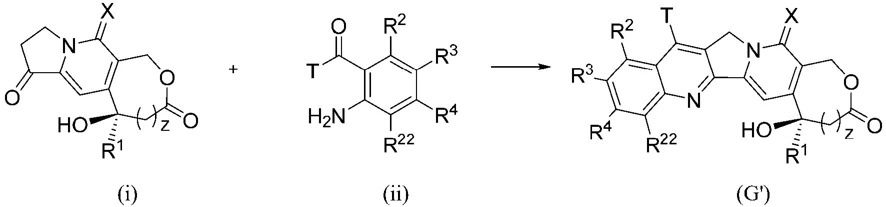
wherein, T is 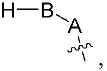 or is a group which can be converted into 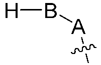 by a reaction method such as oxidation or reduction;
preferably, a group containing an active functional group such as alkenyl, aldehyde, carbonyl, nitro or the like is selected as T;
preferably, when the active functional group is a carbon-containing structure such as alkenyl, aldehyde, carbonyl or the like, the residue obtained by removing the active functional group from T is a structure with one fewer carbon than A; when the active functional group is nitro, the residue obtained by removing the nitro group from T is A.

33. The method for preparing the compound of formula (GH), or a stereoisomer, racemate, tautomer, isotopologue, isotope-labeled compound, N-oxide, prodrug, solvate, or pharmaceutically acceptable salt thereof according to claim 28, wherein the method comprises the step of reacting a compound of formula (G') to obtain a compound of formula (GH): 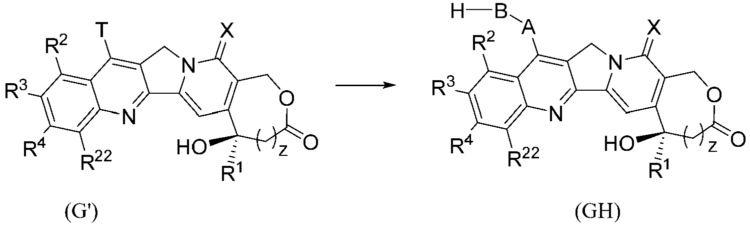
wherein, A, B, R¹, R², R²², R³, R⁴, X and Z independently have a definition as described in any one of claims 28-31;
wherein, T is a group which can be converted into 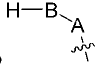 by a reaction method such as oxidation and reduction;
preferably, a group containing an active functional group such as alkenyl, aldehyde, carbonyl, nitro or the like can be selected as T;
preferably, when the active functional group is a carbon-containing structure such as alkenyl, aldehyde, carbonyl or the like, the residue obtained by removing the active functional group from T is a structure with one fewer carbon than A; when the active functional group is nitro, the residue obtained by removing the nitro group from T is A.

34. A compound represented by formula (i): 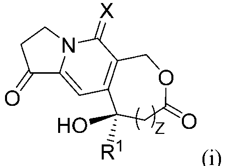 wherein, R¹, X and Z independently have a definition as described in any one of claims 28-31.

35. A compound represented by formula (G'): 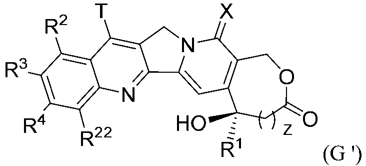 wherein, R¹, R², R²², R³, R⁴, X, Z and T independently have a definition as described in any one of claims 28-32.

36. A conjugate of the following structure:
Tp-L-D (D)
wherein, Tp is a targeting moiety;
D is a biologically active molecular fragment, preferably a molecular fragment with anti-tumor biological activity;
provided that:
L is selected from the linker represented by formula (L):
# L¹-L²-L³-L⁴* (L)
wherein L¹ is a linking moiety to the targeting moiety Tp, formed from a reactive group L1' and a targeting moiety Tp, # represents the linking site to the Tp, preferably L¹ is 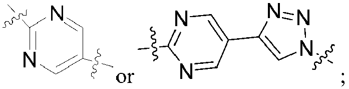
L² is selected from -(C≡C)-(CH₂)_{q}-C(R^{z4}R^{z5})-C(=O)- or -(CH₂)_{q}-C(R^{z4}R^{z5})-C(=O)-;
wherein L^{1'}-L² has the following structure: 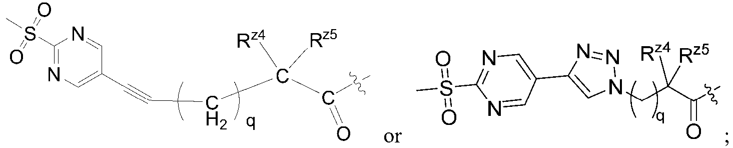
q is an integer selected from 0 to 10, preferably 1, 2, 3, 4, 5, 6, 7 or 8;
R^{z4} and R^{z5} are identical or different and independently selected from H, C₁₋₄ alkyl, C₃₋₆ cycloalkyl, C₃₋₆ cycloalkyl-C₁₋₄ alkyl, or R^{z4} and R^{z5} together form C₃₋₆ cycloalkyl, wherein at least one of R^{z4} or R^{z5} is not H;
or
L² is selected from -(C≡C)-(CH₂)_{q}-C(=O)-, and between L² and L³, a hydrophilic moiety as defined in claim 10 is contained: 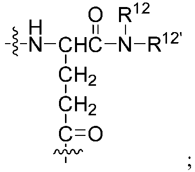
L³ is-ValAA¹Gly-, wherein AA¹ is as defined in any one of claims 13-14;
L⁴ is absent or is a linking moiety of a peptide moiety to a biologically active molecule D, formed by reacting a reactive group L^{4'} with the biologically active molecule, and * represents a linking site to the biologically active molecule D.

37. The conjugate according to claim 36, wherein the biologically active molecule D is a compound with biological activity or potential biological activity as disclosed in the Chinese Pharmacopoeia, The United States Pharmacopoeia or European Pharmacopoeia or as disclosed in other publications;
the drug may be selected from the group consisting of a cytotoxic drug, a cytostatic drug, or an immunosuppressive drug, preferably an anti-tubulin agent, a tubulin inhibitor, a DNA minor groove binder, a DNA replication inhibitor, an alkylating agent, an antibiotic, an antifolate, an antimetabolite, a chemosensitizer, a topoisomerase inhibitor, a vinca alkaloid, and the like;
the drug is further preferably selected from the group consisting of auristatin, camptothecin, duocarmycin, etoposide, maytansinoid and a maytansinoid alkaloid, a taxane, a benzodiazepine or a benzodiazepine-containing drug, and a vinca alkaloid.

38. A pharmaceutical composition comprising at least one selected from the group consisting of:
the ligand-drug conjugate represented by formula (C), or a stereoisomer, racemate, tautomer, isotopologue, isotope-labeled compound, N-oxide, prodrug, solvate or pharmaceutically acceptable salt thereof according to any one of claims 1-26; and/or
the compound represented by formula (GH), or a stereoisomer, racemate, tautomer, isotopologue, isotope-labeled compound, N-oxide, prodrug, solvate or pharmaceutically acceptable salt thereof according to any one of claims 28-31; and/or
the ligand-drug conjugate represented by formula (D), or a stereoisomer, racemate, tautomer, isotopologue, isotope-labeled compound, N-oxide, prodrug, solvate, or pharmaceutically acceptable salt thereof according to any one of claims 36-37;
preferably, the compound represented by formula (GH), or a stereoisomer, racemate, tautomer, isotopologue, isotope-labeled compound, N-oxide, prodrug, solvate, or pharmaceutically acceptable salt thereof, or the ligand-drug conjugate represented by formula (C) or (D), or a stereoisomer, racemate, tautomer, isotopologue, isotope-labeled compound, N-oxide, prodrug, solvate, or pharmaceutically acceptable salt thereof in the pharmaceutical composition is present in a therapeutically effective amount.

39. Use of the compound represented by formula (GH), or a stereoisomer, racemate, tautomer, isotopologue, isotope-labeled compound, N-oxide, prodrug, solvate or pharmaceutically acceptable salt thereof according to any one of claims 28-31, or the ligand-drug conjugate represented by formula (C) according to any one of claims 1-26, or the ligand-drug conjugate represented by formula (D), or a stereoisomer, racemate, tautomer, isotopologue, isotope-labeled compound, N-oxide, prodrug, solvate or pharmaceutically acceptable salt thereof according to any one of claims 36-37, or the pharmaceutical composition according to claim 38, in the prevention and/or treatment of a disease or disorder, and/or in the preparation of a medicament;
preferably, the disease or disorder can be selected from a tumor, such as a solid tumor or a hematological cancer.

40. The use according to claim 39, wherein the medicament is used for the prevention and/or treatment of a disease or disorder;
preferably, the disease or disorder may be selected from a tumor, such as a solid tumor or a hematological cancer;
the solid tumor is selected from the group consisting of malignancies of various organ systems, e.g., sarcomas, adenocarcinomas, blastomas, and carcinomas, such as those affecting liver, lung, breast, lymphoid, biliary bowel (e.g., colon), genitourinary tract (e.g., kidney, urothelial cells), prostate, and pharynx; adenocarcinomas include malignancies such as most of colon cancers, rectum cancers, renal cell cancers, liver cancers, small cell lung cancers, non-small cell lung cancers, small intestine cancers and esophagus cancers;
the hematological cancer is selected from the group consisting of leukemias, lymphomas, and malignant lymphoproliferative disorders affecting blood, bone marrow, and lymphatic systems.

41. A linker-drug conjugate, having the following structure:
L^{1'}-L ²-L³-L⁴-G (C')
Wherein: G has the definition as described in any one of claims 1-9;
L^{1'}, L², L³ and L⁴ have the definition as described in any one of claims 10-19.

42. The linker-drug conjugate according to claim 41, wherein the conjugate is selected from one of the following:

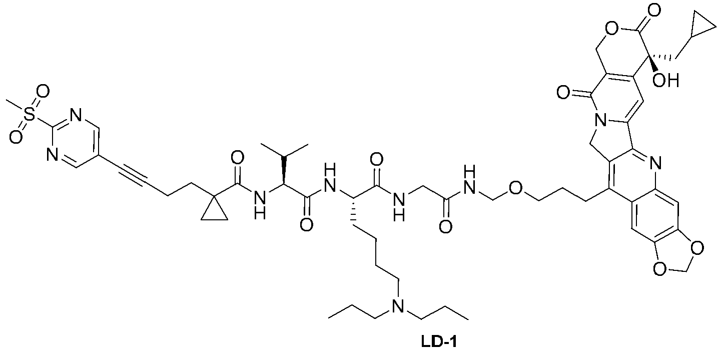

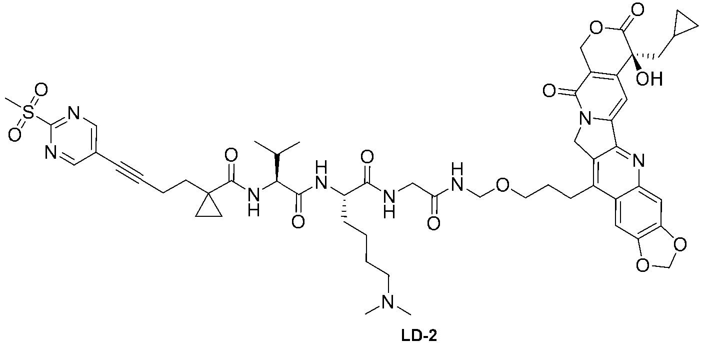

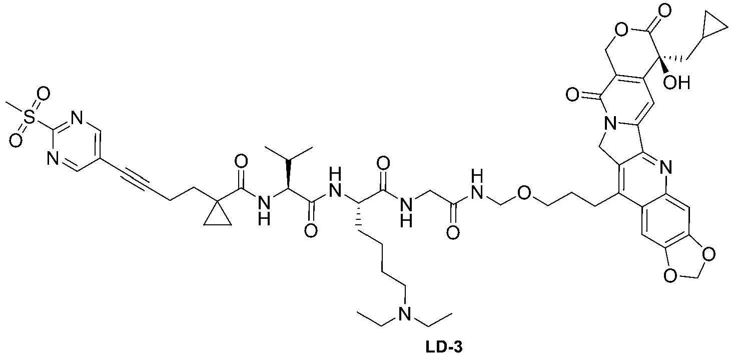

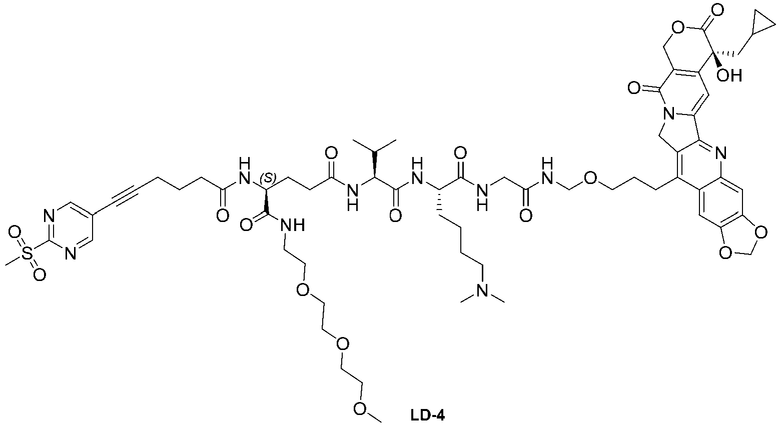

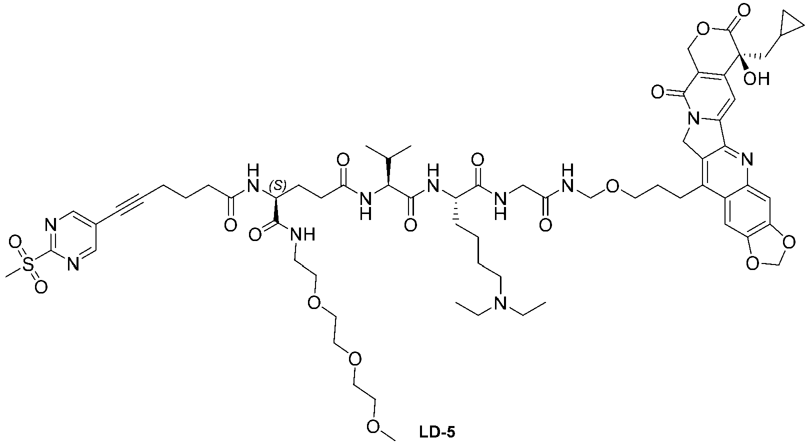

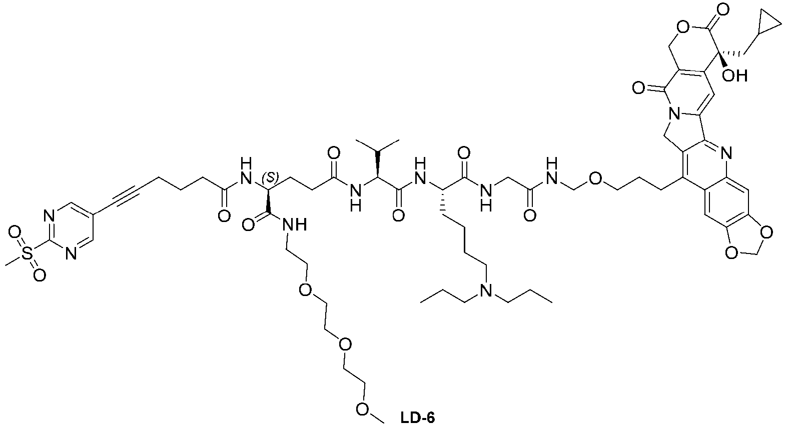

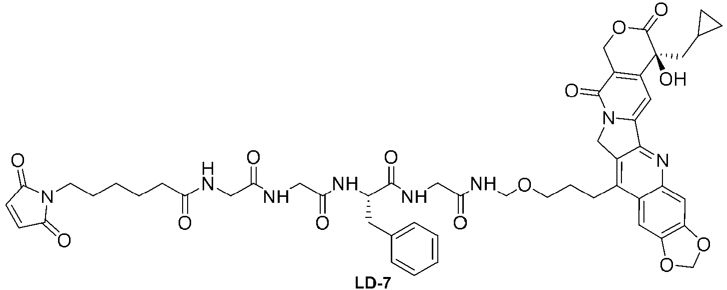

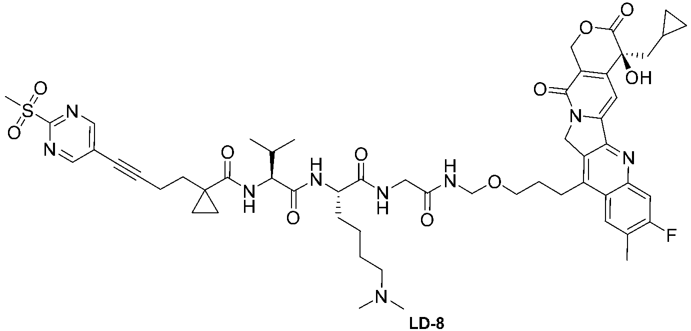

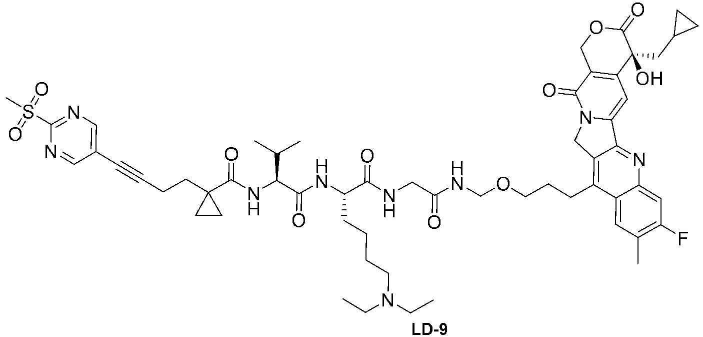

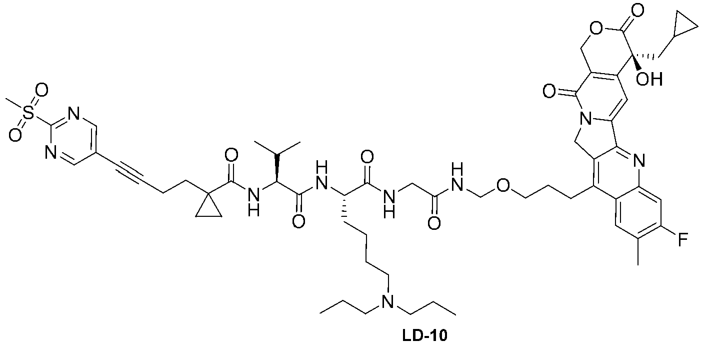

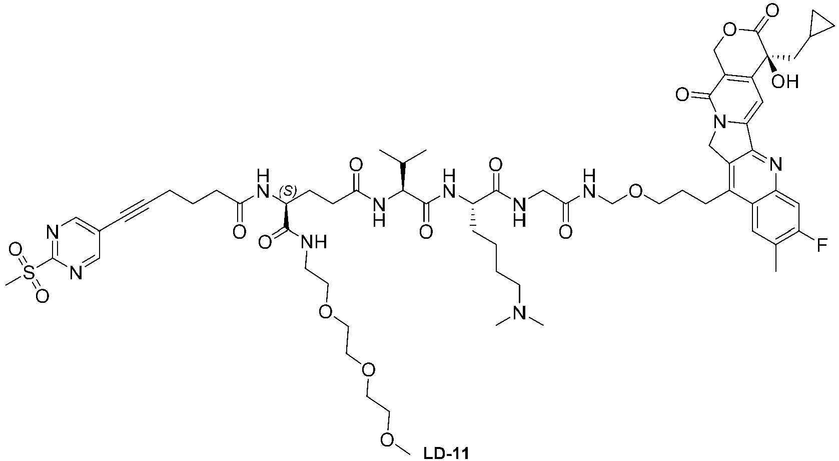

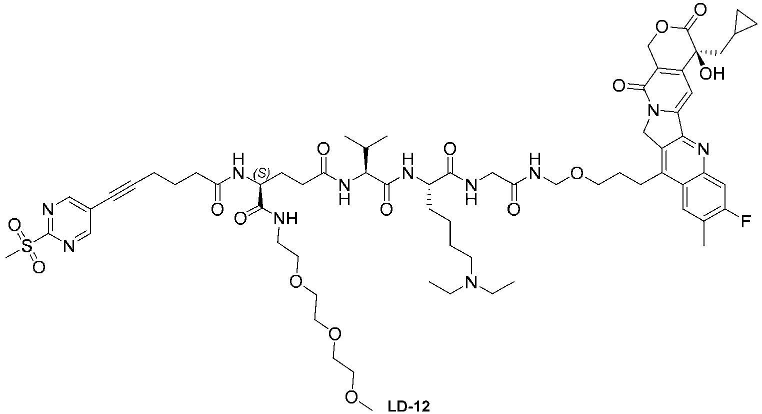

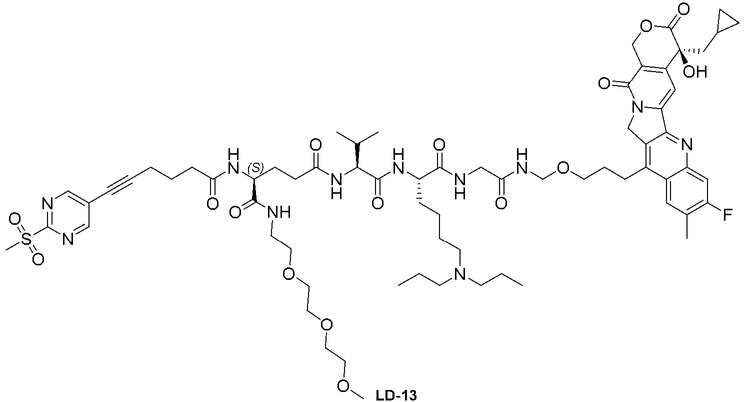

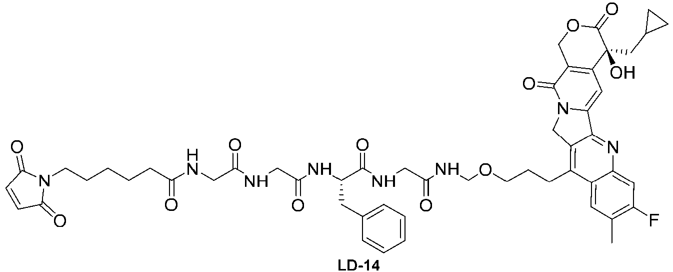

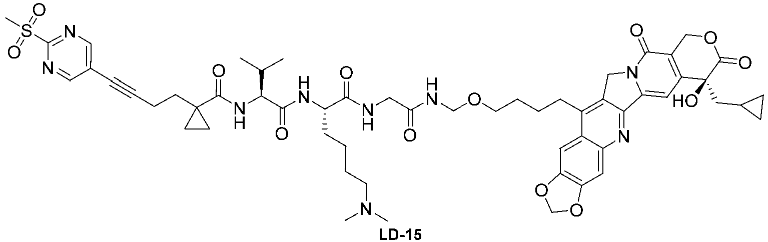

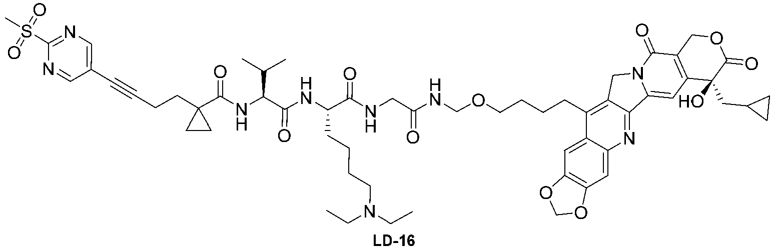

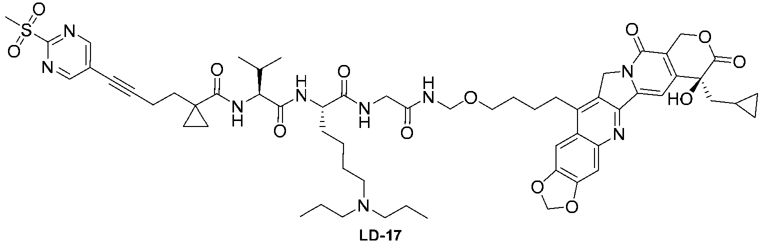

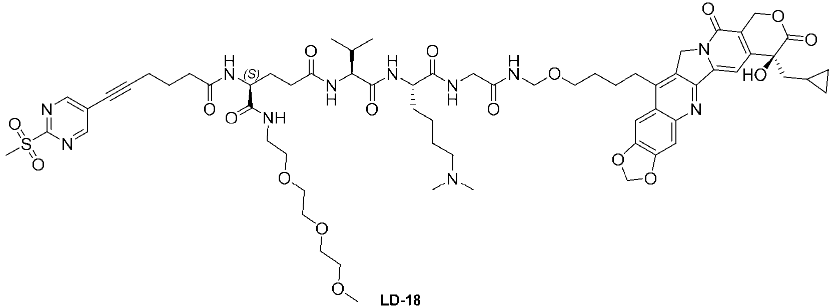

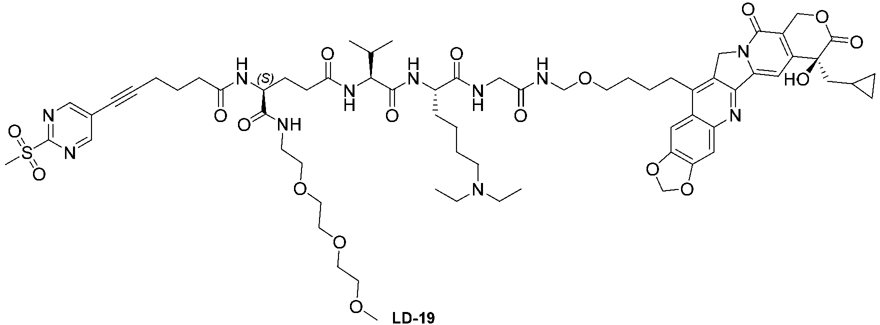

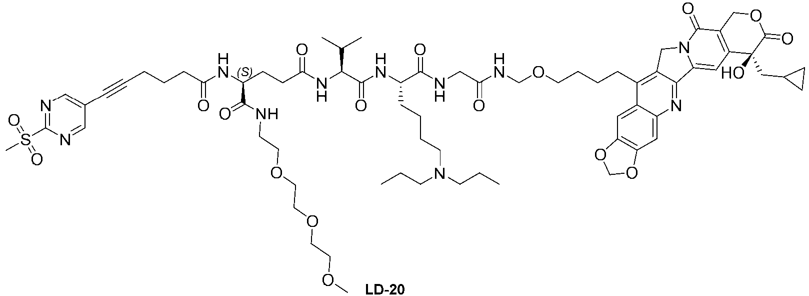

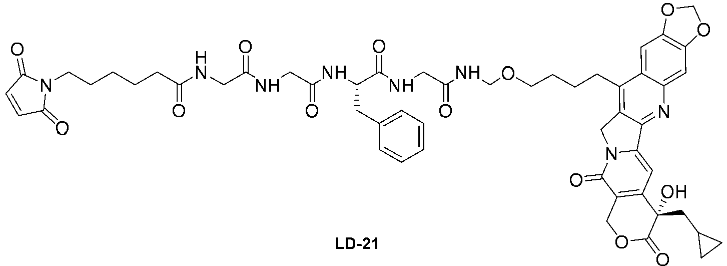

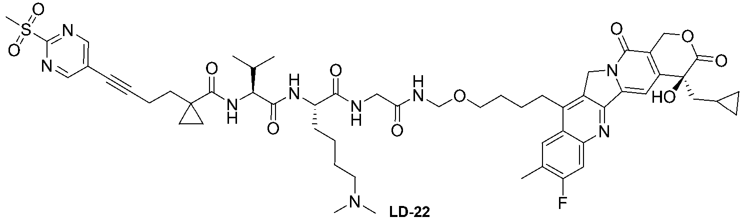

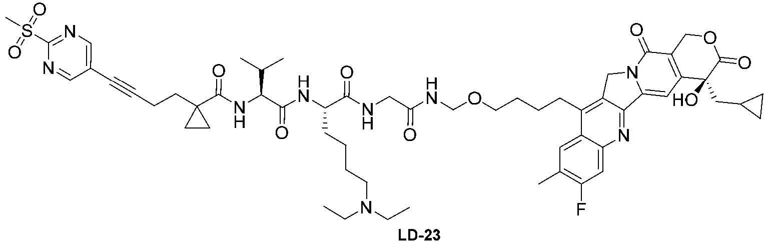

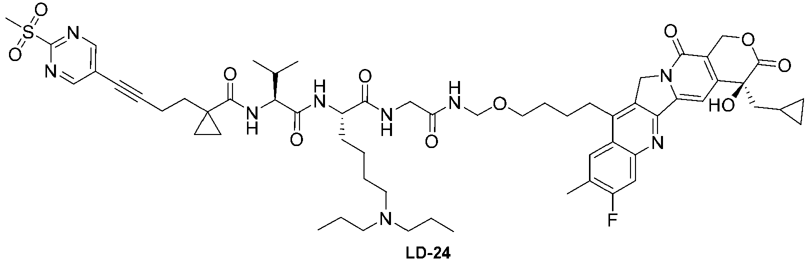

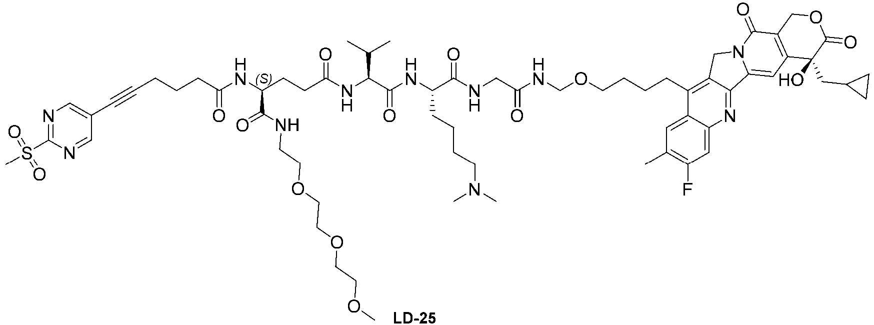

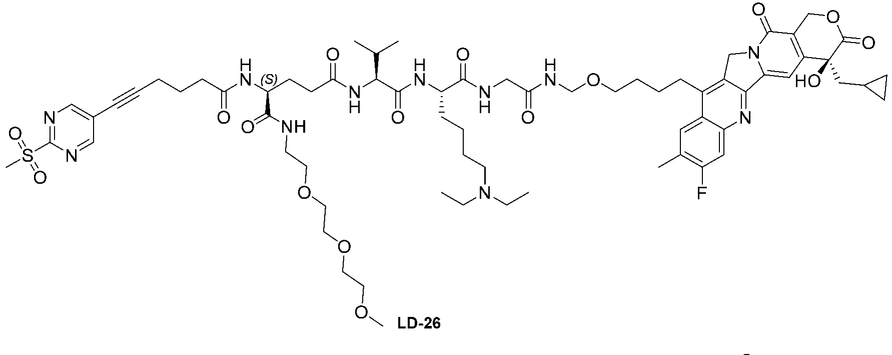

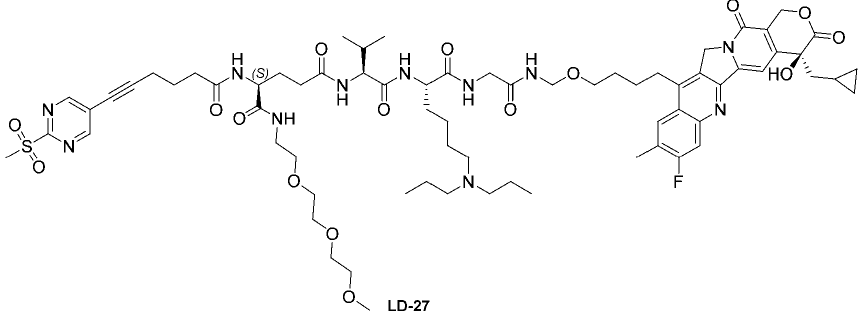

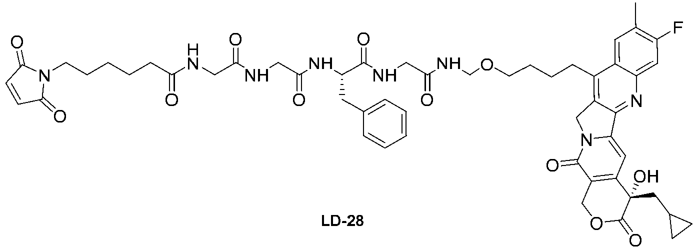

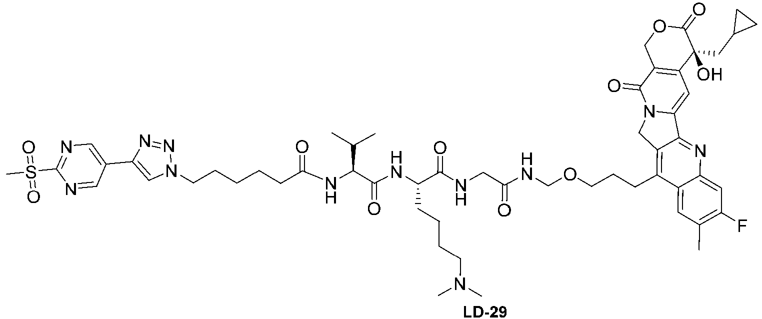

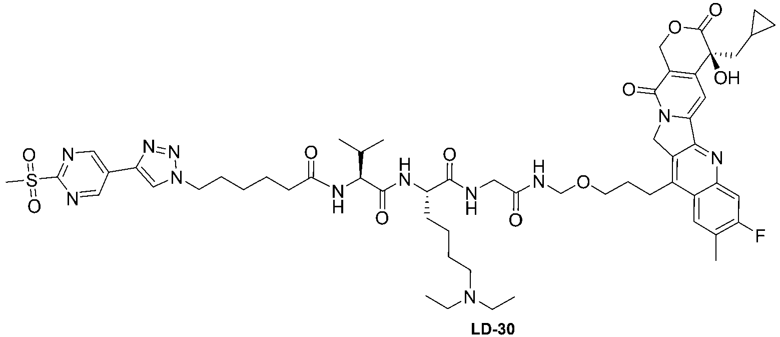

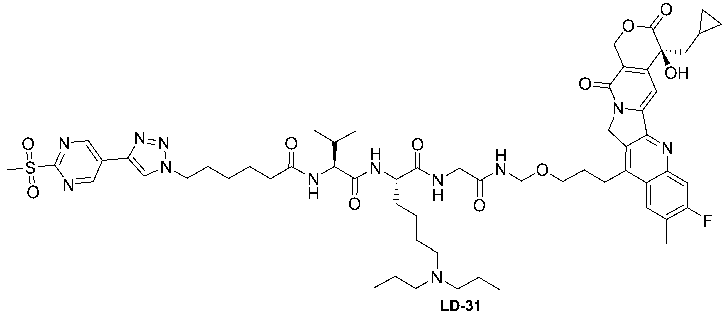

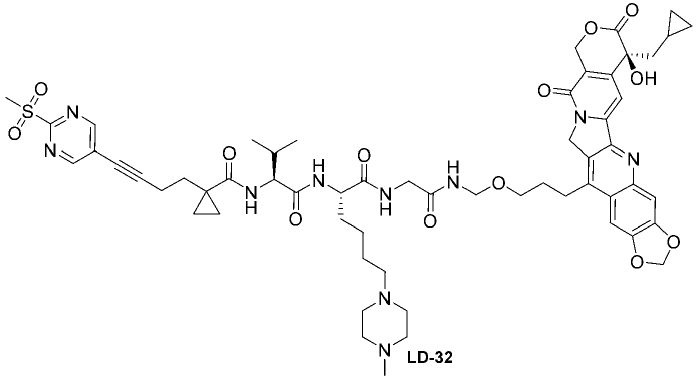

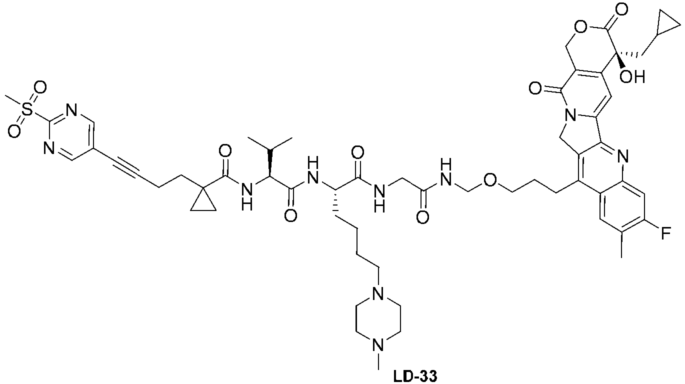

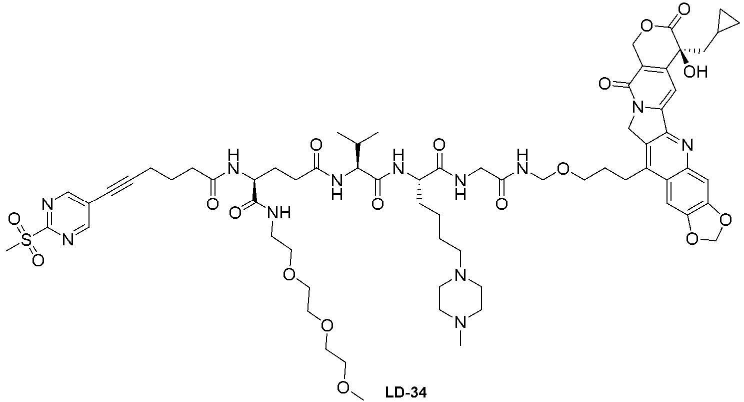

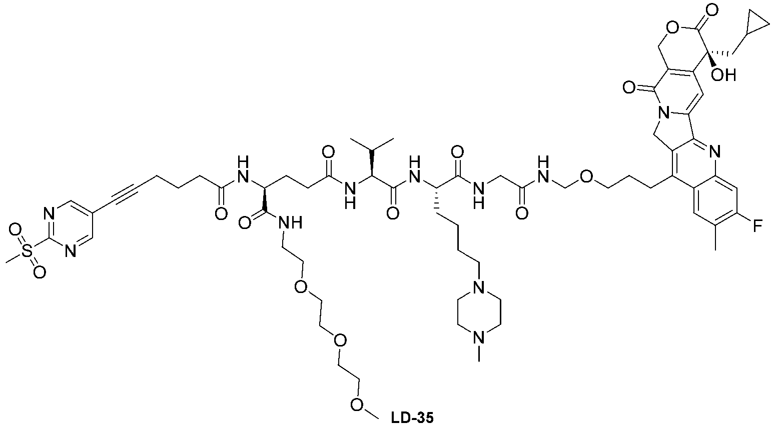

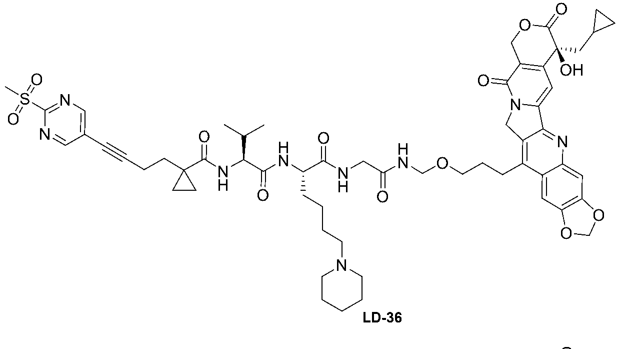

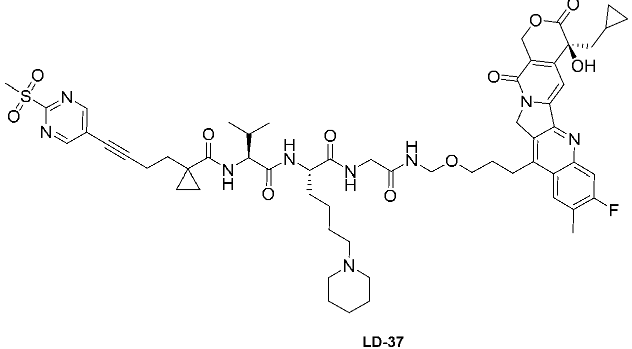

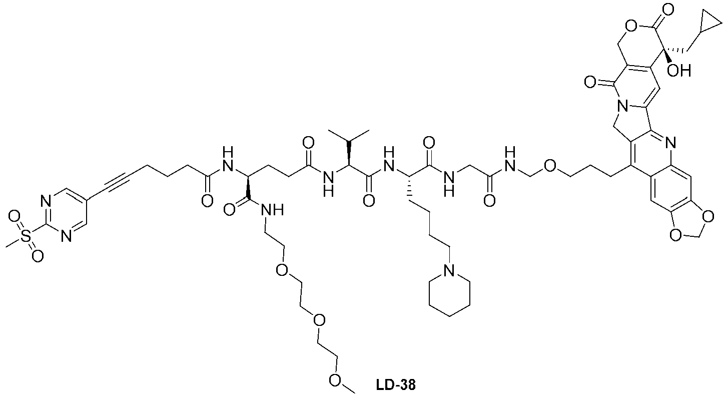

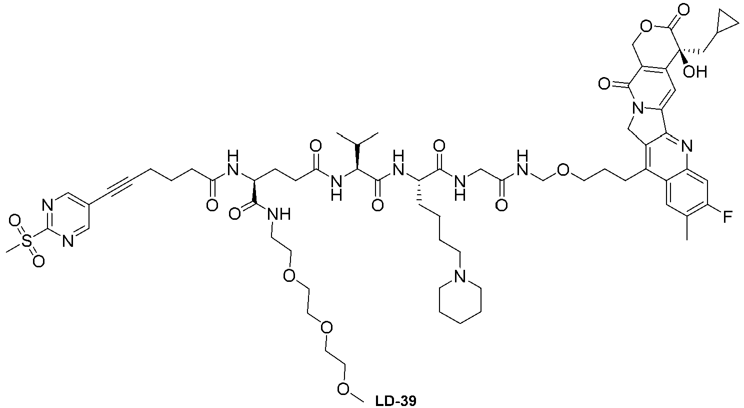

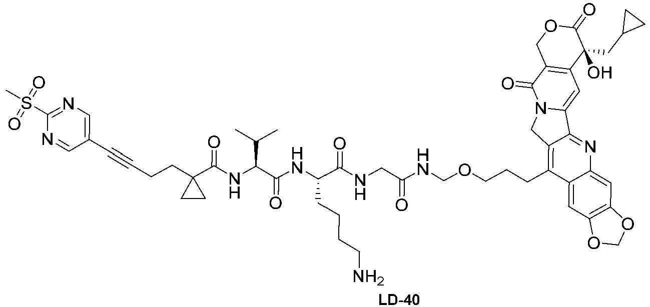

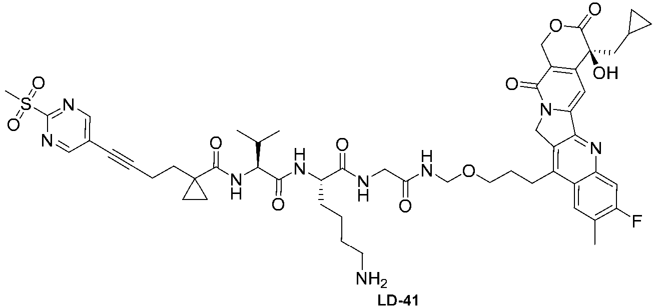

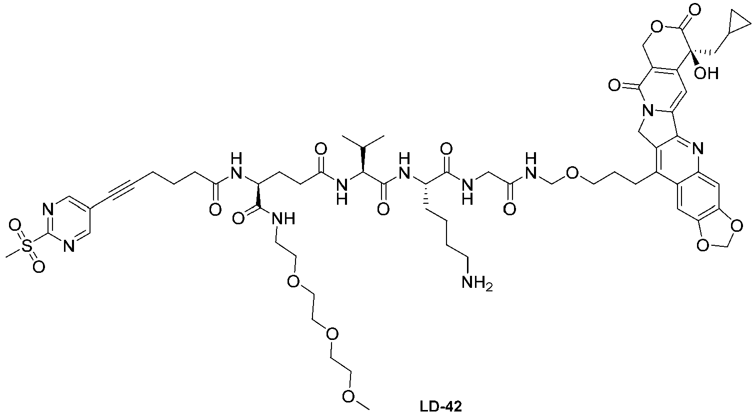

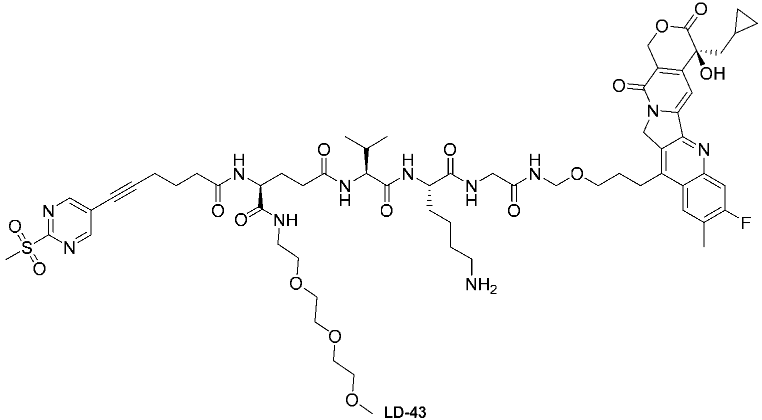

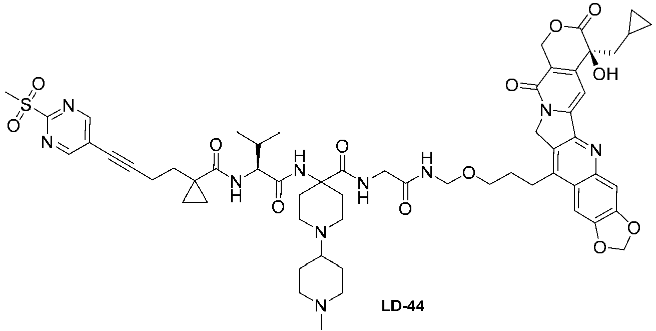

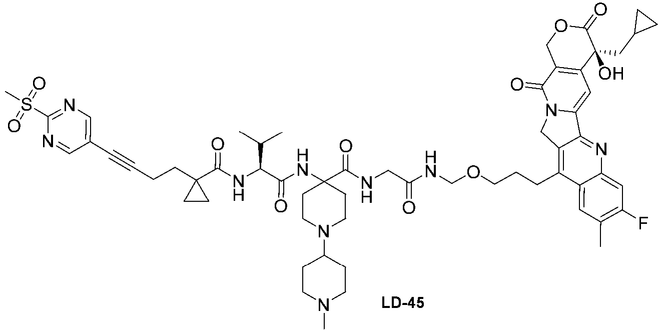

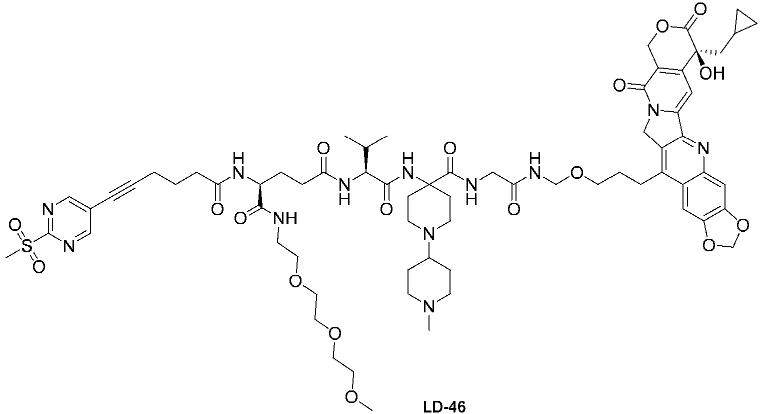

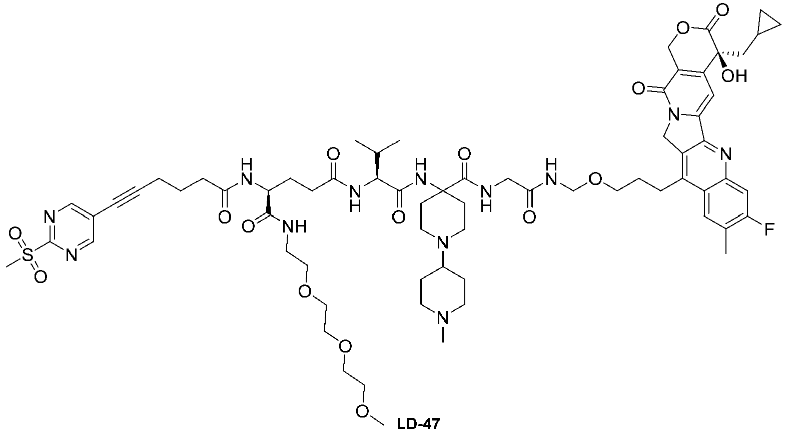

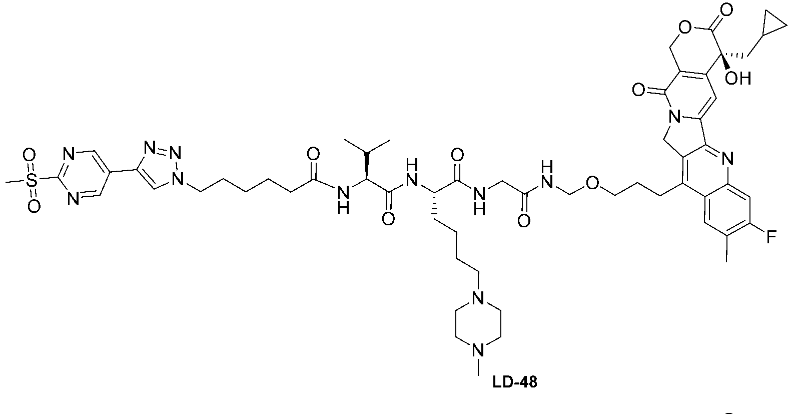

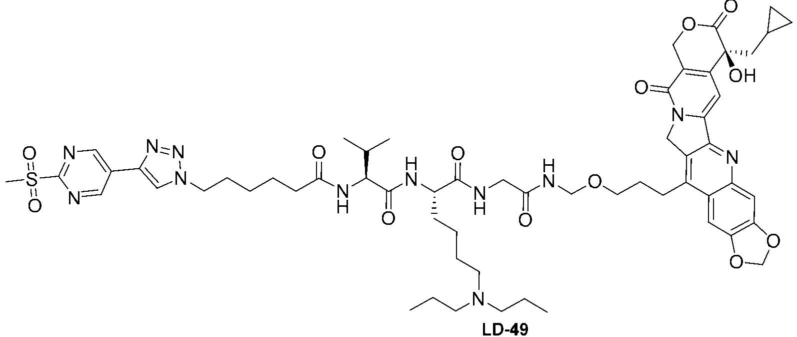

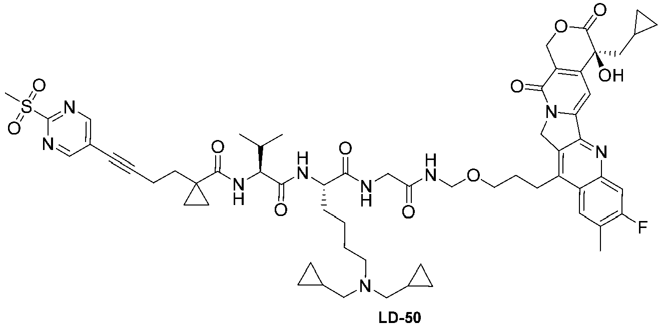

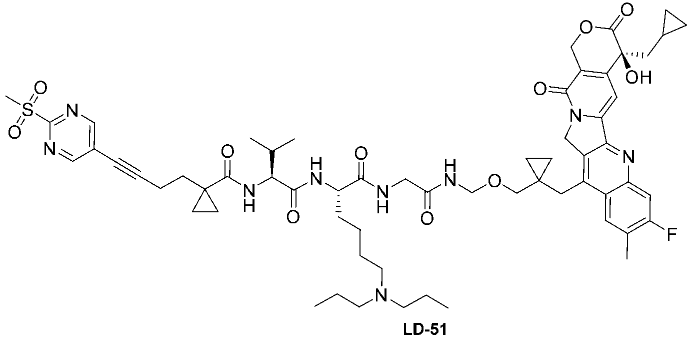

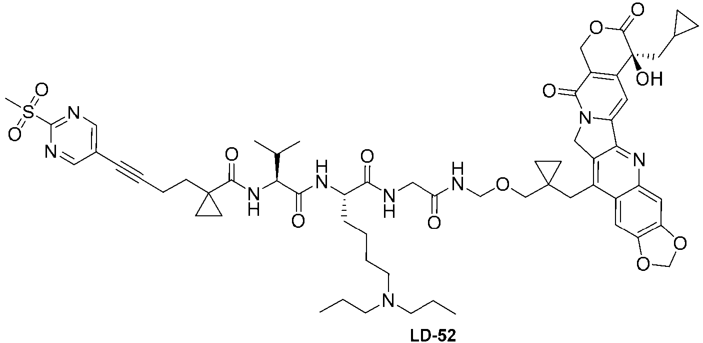

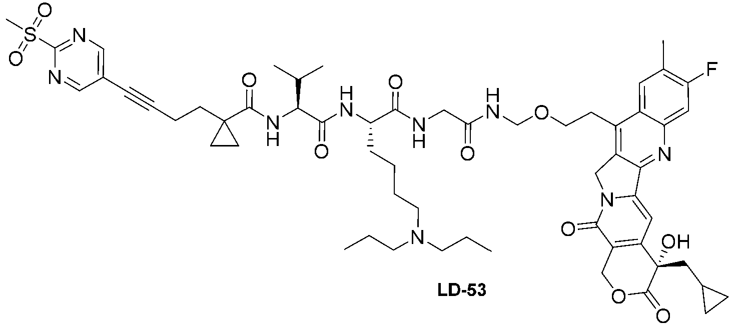

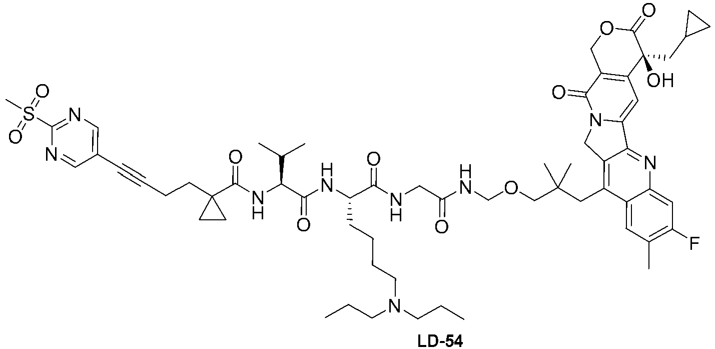

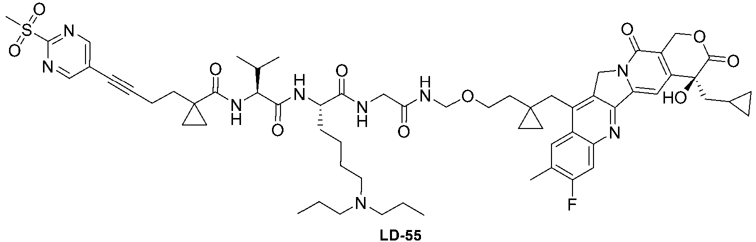

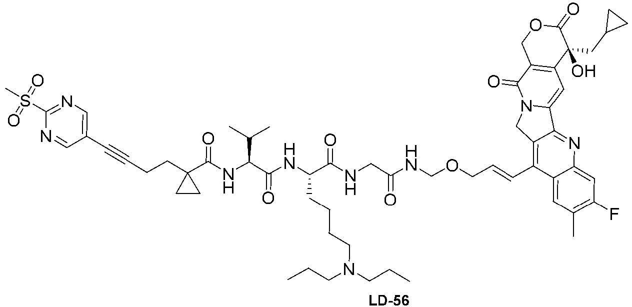

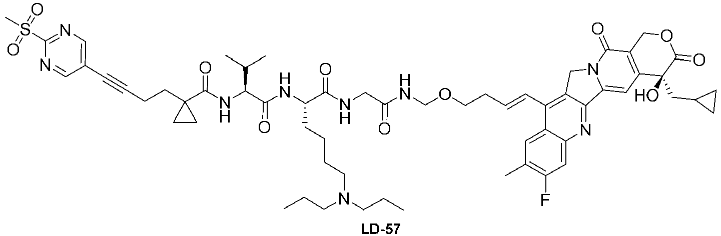

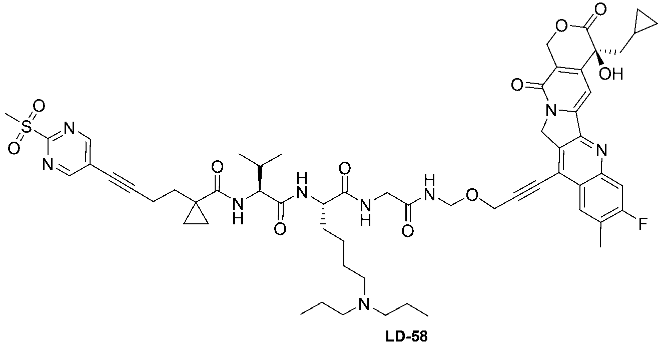

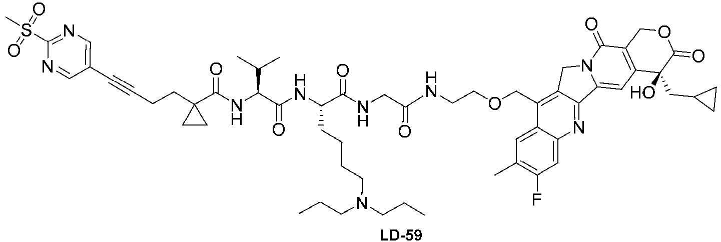

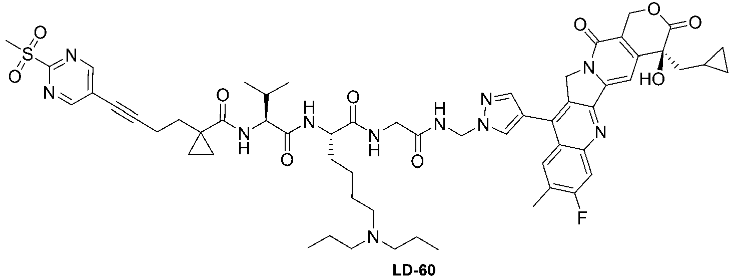

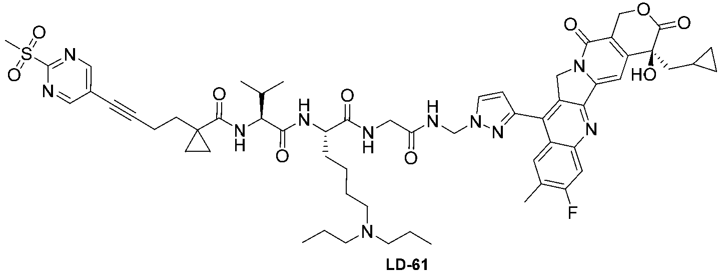

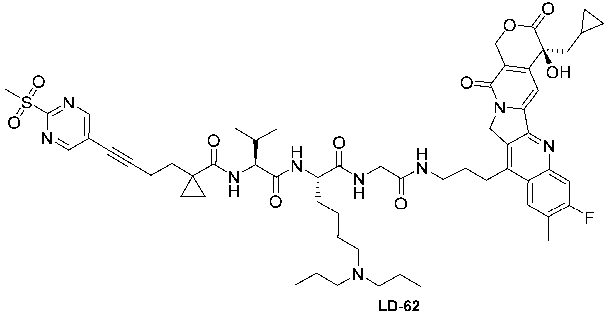

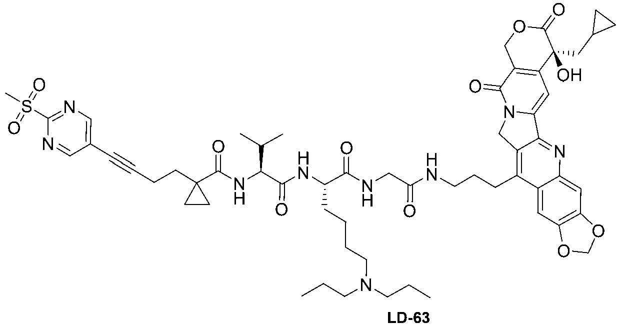

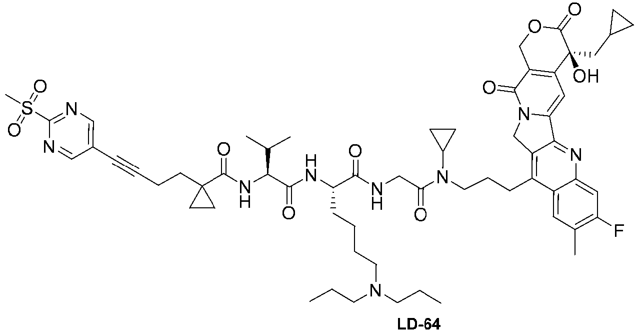

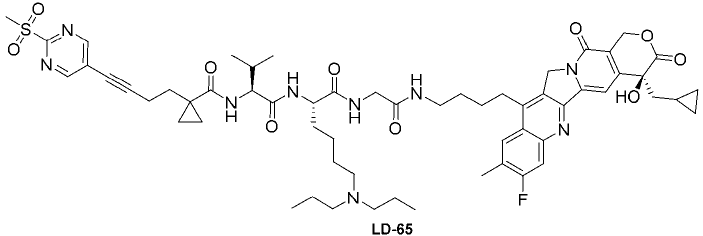

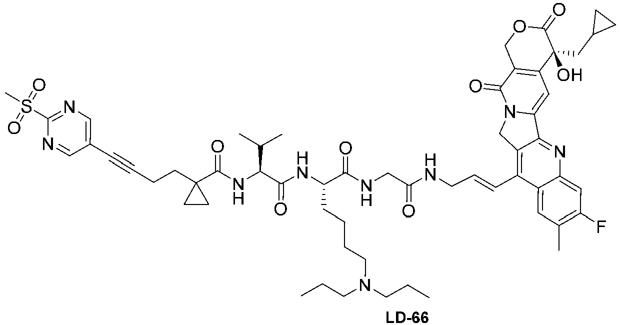

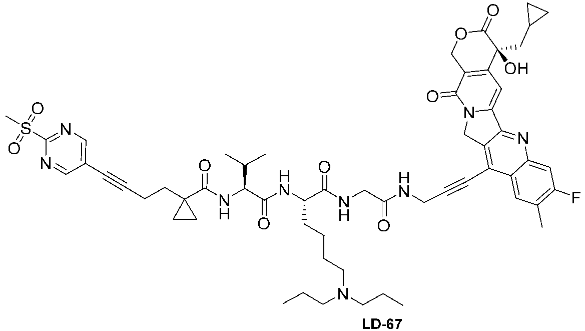

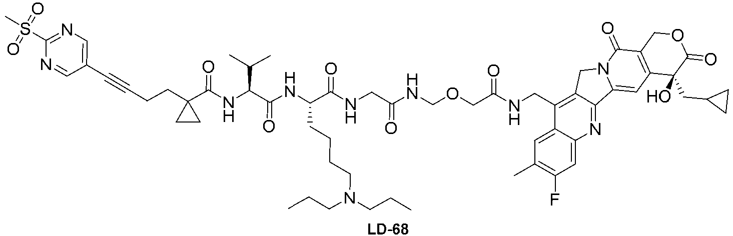

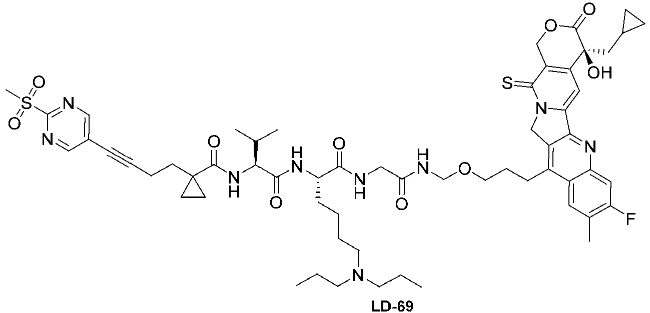

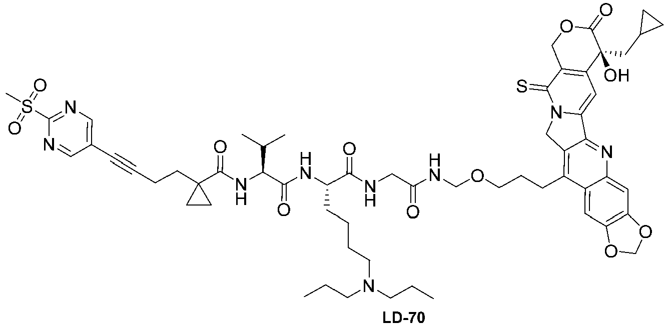

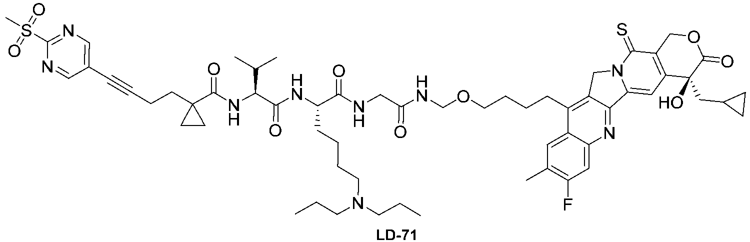

43. A linker-drug conjugate, having the following structure:
L^{1'}-L²-L³-L⁴-D
wherein, D has the definition as described in any one of claims 36-37,
L^{1'} is a reactive group, preferably 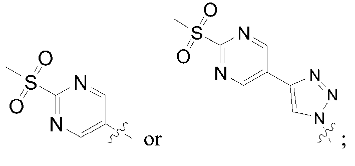
L² is selected from -(C≡C)-(CH₂)_{q}-C(R^{z4}R^{z5})-C(=O)- or -(CH₂)_{q}-C(R^{z4}R^{z5})-C(=O);
wherein L^{1'}-L² has the following structure: 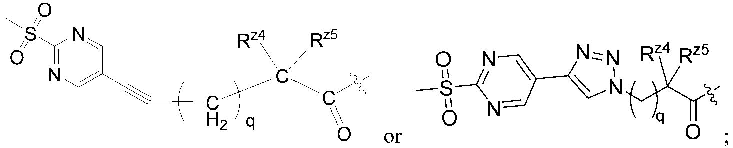
q is an integer selected from 0 to 10, preferably 1, 2, 3, 4, 5, 6, 7 or 8;
R^{z4} and R^{z5} are identical or different and independently selected from H, C₁₋₄ alkyl, C₃₋₆ cycloalkyl, C₃₋₆ cycloalkyl-C₁₋₄ alkyl, or R^{z4} and R^{z5} together form a C₃₋₆ cycloalkyl, wherein at least one of R^{z4} or R^{z5} is not H,
or
L² is selected from -(C≡C)-(CH₂)_{q}-C(=O)-, and between L² and L³, a hydrophilic moiety as defined in claim 10 is contained: 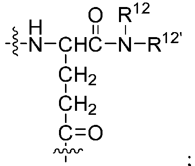
L³ is -ValAA¹Gly-, wherein AA¹ is as defined above;
L⁴ is absent or is a linking moiety of a peptide moiety to a biologically active molecule D, formed by reacting a reactive group L^{4'} with the biologically active molecule, and * represents a linking site to the biologically active molecule D;
optionally, a hydrophilic moiety as defined in claim 10 is contained between L¹ and L², or between L² and L3, or in a form of replacement of L2, or in a form of insertion into the L2.

44. A linker, having the following structure:
L^{1'}-L2-L3-L^{4'} (L')
wherein, L^{1'} is a reactive group, preferably 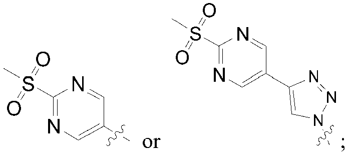
L² is selected from -(C≡C)-(CH₂)_{q}-C(R^{z4}R^{z5})-C(=O)- or -(CH₂)_{q}-C(R^{z4}R^{z5})-C(=O);
wherein, L^{1'}-L² has the following structure: 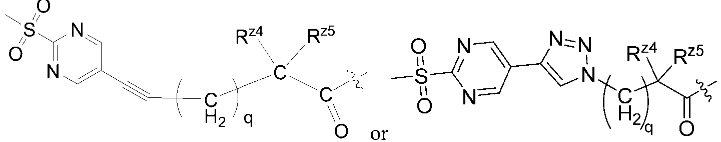
R^{z4} and R^{z5} are identical or different and independently selected from H, C₁₋₄ alkyl, C₃₋₆ cycloalkyl, C₃₋₆ cycloalkyl-C₁₋₄ alkyl, or R^{z4} and R^{z5} together form a C₃₋₆ cycloalkyl, wherein at least one of R^{z4} or R^{z5} is not H and q is an integer selected from 0 to 10, preferably 1, 2, 3, 4, 5, 6, 7 or 8;
or
L² is selected from -(C≡C)-(CH₂)_{q}-C(=O)-, and between L² and L³, a hydrophilic moiety as defined in claim 10 is contained: 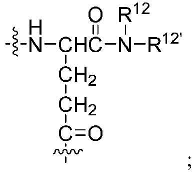 and
L³ is-ValAA¹Gly-, wherein AA¹ has the definition as described in any one of claims 13-14.
L' is preferably: 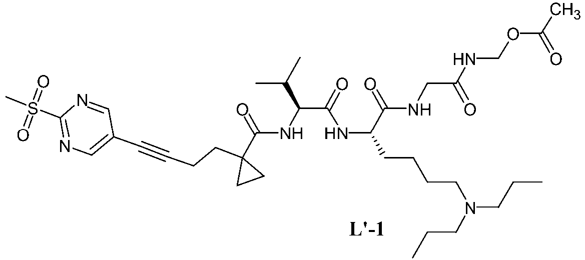 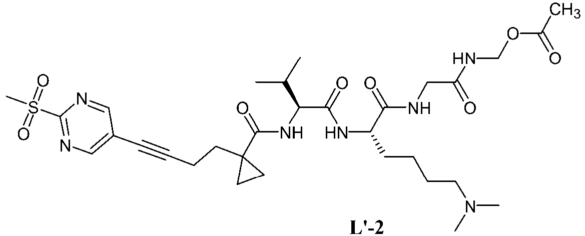 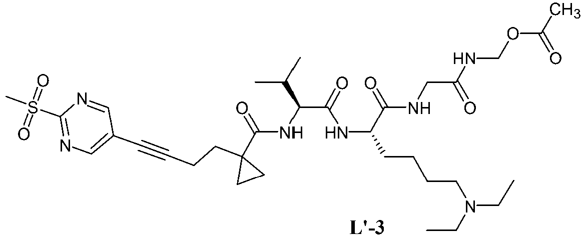 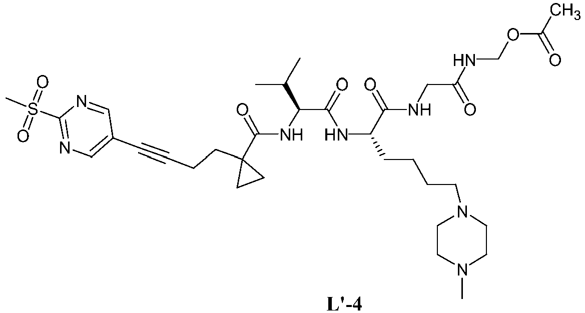 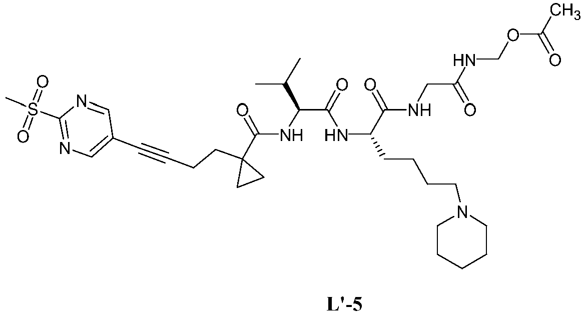 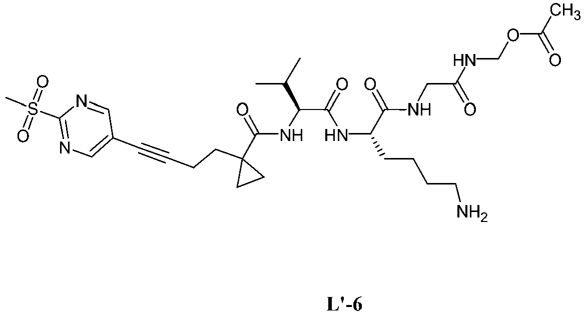 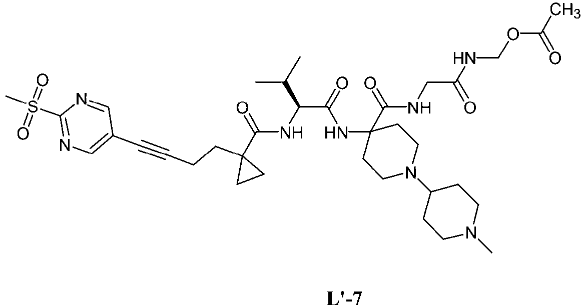 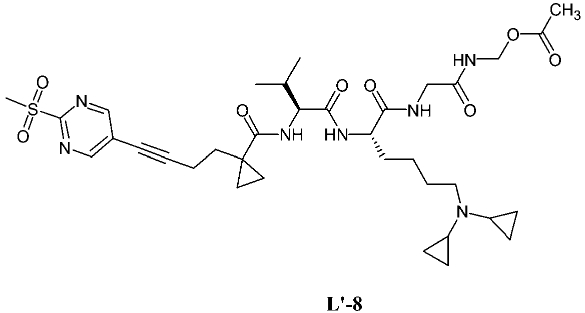 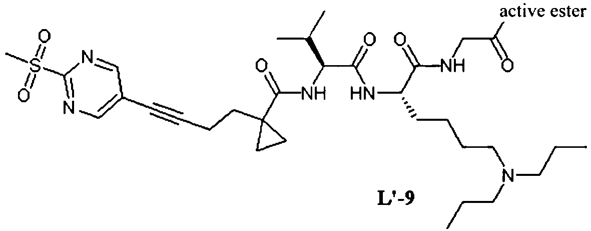 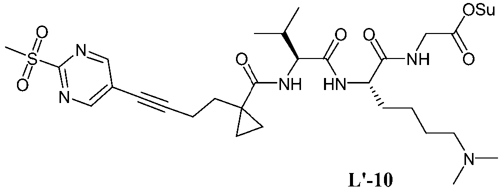 Wherein OSu represents succinimide active ester

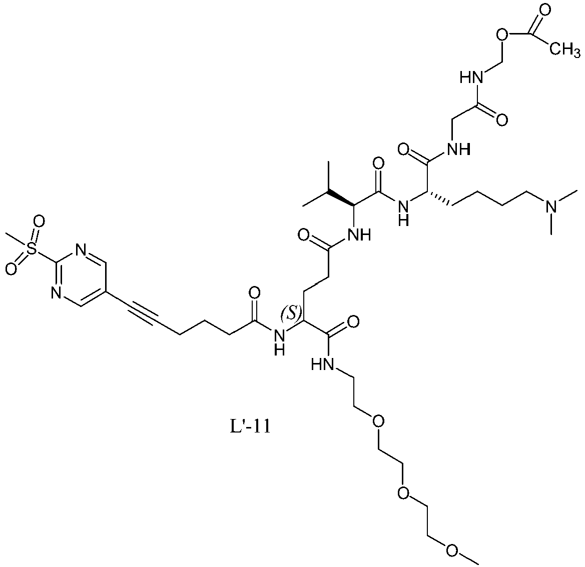

45. A conjugated intermediate, having the following structure: 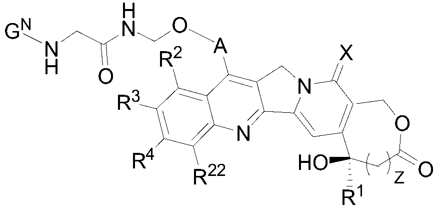 wherein G^{N} is H or an optional amino protecting group and A, R¹, R², R²², R³, R⁴, X and Z independently have the definitions as described in any one of claims 28-31.

46. A linker fragment compound
L^{1'}-L²-ValAA¹' (L")
Wherein L^{1'} is a reactive group, preferably 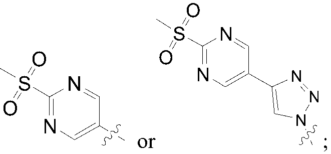
L² is selected from -(C≡C)-(CH₂)_{q}-C(R^{z4}R^{z5})-C(=O)- or -(CH₂)_{q}-C(R^{z4}R^{z5})-C(=O);
wherein L^{1'}-L² has the following structure: 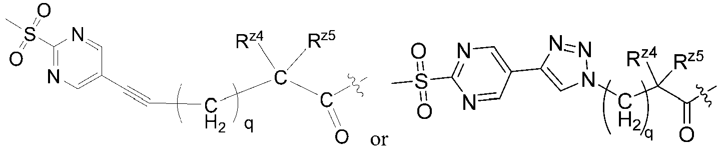
R^{z4} and R^{z5} are identical or different and independently selected from H, C₁₋₄ alkyl, C₃₋₆ cycloalkyl, C₃₋₆ cycloalkyl-C₁₋₄ alkyl, or R^{z4} and R^{z5} together form a C₃₋₆ cycloalkyl, wherein at least one of R^{z4} or R^{z5} is not H;
q is an integer selected from 0 to 10, preferably 1, 2, 3, 4, 5, 6, 7, or 8;
or
L₂ is selected from -(C≡C)-(CH₂)_{q}-C(=O)-, and between L² and L³, a hydrophilic moiety as defined in claim 10 is contained: 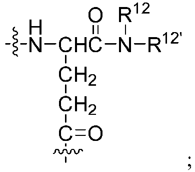
AA¹' is AA¹ or a reactive form thereof such as an active ester, wherein AA¹ has the definition as described in any one of claims 13 to 14.
